(19)

**Europäisches Patentamt**
**European Patent Office**
**Office européen des brevets**

(11) **EP 4 141 127 B1**

(12) **EUROPEAN PATENT SPECIFICATION**

(45) Date of publication and mention
of the grant of the patent:
**09.10.2024  Bulletin 2024/41**

(21) Application number: **21193861.8**

(22) Date of filing: **30.08.2021**

(51) International Patent Classification (IPC):
**C12Q 1/6827** (2018.01)     **C12Q 1/6886** (2018.01)

(52) Cooperative Patent Classification (CPC):
(C-Sets available)
**C12Q 1/6886; C12Q 1/6827;** C12Q 2600/106;
C12Q 2600/156                                    (Cont.)

(54) **METHOD FOR ASSESSING HOMOLOGOUS RECOMBINATION DEFICIENCY IN OVARIAN CANCER CELLS**

VERFAHREN ZUR BEWERTUNG DES MANGELS AN HOMOLOGER REKOMBINATION IN EIERSTOCKKREBSZELLEN

PROCÉDÉ D'ÉVALUATION DE DÉFICIENCE DE RECOMBINAISON HOMOLOGUE DANS DES CELLULES DU CANCER DE L'OVAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.03.2023  Bulletin 2023/09**

(73) Proprietors:
• **Zentrum Familiärer Brust- und Eierstockkrebs Universitätsklinik Köln**
  **50937 Köln (DE)**
• **Stichting Het Netherlands Kanker Insituut - Antoni van Leeuwenhoek ziekenhuis**
  **1066 CX Amsterdam (NL)**

(72) Inventors:
• **SCHMUTZLER, Rita**
  **50858 Köln (DE)**
• **SCHOUTEN, Philip**
  **CB29AB Cambridge (GB)**
• **HAHNEN, Eric**
  **53113 Bonn (DE)**
• **LINN, Sabine**
  **1052 HH Amsterdam (NL)**

(74) Representative: **Patentanwälte Bauer Vorberg Kayser**
  **Partnerschaft mbB**
  **Goltsteinstraße 87**
  **50968 Köln (DE)**

(56) References cited:
WO-A1-2011/048495     WO-A1-2015/080585
WO-A1-2015/086473     WO-A1-2017/191074
WO-A1-2021/122620

• **HELEN DAVIES ET AL: "HRDetect is a predictor of BRCA1 and BRCA2 deficiency based on mutational signatures", NATURE MEDICINE, vol. 23, no. 4, 13 March 2017 (2017-03-13), New York, pages 517 - 525, XP055386173, ISSN: 1078-8956, DOI: 10.1038/nm.4292**
• **LADAN MARJOLIJN M. ET AL: "Homologous Recombination Deficiency Testing for BRCA-Like Tumors: The Road to Clinical Validation", CANCERS, vol. 13, no. 5, 28 February 2021 (2021-02-28), pages 1004, XP055886909, DOI: 10.3390/cancers13051004**
• **SEVERSON TESA M. ET AL: "The BRCA1ness signature is associated significantly with response to PARP inhibitor treatment versus control in the I-SPY 2 randomized neoadjuvant setting", BREAST CANCER RESEARCH, vol. 19, no. 1, 25 August 2017 (2017-08-25), XP055886950, Retrieved from the Internet <URL:http://link.springer.com/content/pdf/10.1186/s13058-017-0861-2.pdf> DOI: 10.1186/s13058-017-0861-2**

- P. A. KONSTANTINOPOULOS ET AL: "Gene Expression Profile of BRCAness That Correlates With Responsiveness to Chemotherapy and With Outcome in Patients With Epithelial Ovarian Cancer", JOURNAL OF CLINICAL ONCOLOGY, vol. 28, no. 22, 14 June 2010 (2010-06-14), pages 3555 - 3561, XP055070579, ISSN: 0732-183X, DOI: 10.1200/JCO.2009.27.5719
- SCHOUTEN PHILIP C ET AL: "RobustBRCA1-like classification of copy number profiles of samples repeated across different datasets and platforms", MOLECULAR ONCOLOGY, ELSEVIER, vol. 9, no. 7, 1 August 2015 (2015-08-01), pages 1274 - 1286, XP029252523, ISSN: 1574-7891, DOI: 10.1016/J.MOLONC.2015.03.002
- SIMON A JOOSSE ET AL: "Prediction of BRCA1-association in hereditary non-BRCA1/2 breast carcinomas with array-CGH", BREAST CANCER RESEARCH AND TREATMENT, KLUWER ACADEMIC PUBLISHERS, BO, vol. 116, no. 3, 14 August 2008 (2008-08-14), pages 479 - 489, XP019727887, ISSN: 1573-7217
- TIBSHIRANI R ET AL: "Diagnosis of multiple cancer types by shrunken centroids of gene expression", PROCEEDINGS OF THE NATIONAL ACADEMY OF SCIENCES, NATIONAL ACADEMY OF SCIENCES, vol. 99, no. 10, 14 May 2002 (2002-05-14), pages 6567 - 6572, XP002988576, ISSN: 0027-8424, DOI: 10.1073/PNAS.082099299
- ZHANG MIN ET AL: "Copy number deletion of RAD50 as predictive marker of BRCAness and PARP inhibitor response in BRCA wild type ovarian cancer", GYNECOLOGIC ONCOLOGY, ACADEMIC PRESS, LONDON, GB, vol. 141, no. 1, 23 March 2016 (2016-03-23), pages 57 - 64, XP029470964, ISSN: 0090-8258, DOI: 10.1016/J.YGYNO.2016.01.004

(52) Cooperative Patent Classification (CPC): (Cont.)

C-Sets
C12Q 1/6827, C12Q 2537/143, C12Q 2537/16, C12Q 2537/165

**Description**

FIELD OF THE INVENTION

The present invention relates to methods involved in assessing homologous recombination deficiency (HRD) status in a patient having ovarian cancer based on the determination of copy number variants (CNVs) in a genomic locus. The invention also provides methods for predicting a patient's response to an anti-cancer therapy and thus to the identification of ovarian cancer patients likely to respond to a particular cancer treatment regime based on their HRD status.

BACKGROUND OF THE INVENTION

Epithelial ovarian cancer (OC) is the second leading cause of death among gynecologic cancers worldwide as there were 286,000 incident cases and mortality of 176,000 reported in 2017 (1). Since screening tools are ineffective and early clinical warning signs are rare, the majority of OC cases present in late clinical stages. Even though therapeutic strategies improved within the past years, the prognosis is still poor, with an average five-year survival-rate of 48.6% (2).
Cytoreductive surgery aiming for complete resection, followed by platinum-based chemotherapy, has been the backbone of OC treatment for decades (3,4). Carboplatin, combined with paclitaxel +/- bevacizumab, as an initial systemic regimen leads to a response rate of approximately 80%, especially in high-grade serous ovarian cancer (HGSOC). Nevertheless, the disease of most patients recurs over time (5).
Recently, Poly (ADP-ribose) polymerase inhibitors (PARPi) were added to the therapeutic arsenal. Both the mainstay carboplatin/paclitaxel and these new strategies yield high responses in the overall population, which could be explained by homologous recombination deficiency (HRD) in a substantial proportion of OC (6).
Platinum compounds and PARPi exploit the HRD, by inducing DNA double-strand breaks or impeding its repair via synthetic lethality, leading to cell cycle arrest or death. The breast and ovarian cancer germline predisposition genes BRCA1 and BRCA2 play crucial roles in homologous recombination (7, 8), an essential, highly accurate DNA-repair process fixing double-strand breaks. Deleterious germline mutations with subsequent loss of heterozygosity (LOH) in BRCA1/2 can explain a subset of HR-deficient ovarian cancers, resulting in the registration of BRCA mutation analyses as companion diagnostics in specific OC (9) and metastasized breast cancer (10) settings. However, it was found that a larger proportion of OC displays a phenotype similar to germline (g) BRCA1/2-mutated cancers, so-called BRCAness (11). This is supported by preclinical analyses (12) and clinical trials of the three registered PARPi in OC (13-16) demonstrating survival benefits for a larger subgroup, and has led to their approval, independent of mutation status in most indications. Since these additional patients might benefit from a specific therapy, various potential biomarkers are under investigation, including single gene methylation, gene expression (profiles), copy number/ LOH based assays, mutational signatures, and combinations (17-21) as well as the identification of BRCAness status by analyzing whole-exome deep sequence data from wildtype OC cases; see Zhang et al., Gynecologic Oncology 141 (2016), 57-64.
For determining BRCA-deficient breast cancer tumors, mutational signatures have been established as described in Davies et al., Nature Medicine 23 (2017), 517-525, wherein for OC,, the LOH-score (15) (Foundation Medicine, Cambridge, U.S.) and MyChoice® (22) (Myriad, Salt Lake City, U.S.) were applied within the trials mentioned above and demonstrated the ability to narrow the respective subgroup. This led to the first approval of an HRD assay (MyChoice®) as a companion diagnostic for applying niraparib in heavily pretreated OC patients (23). Two recent clinical trials (24, 25) evaluated this biomarker in the first-line setting, showing a benefit of a PARPi predominantly in the HRD-positive, but also in the HRD-negative subgroup. In contrast, within the PAOLA-1 trial (26), the test defined a subpopulation beyond BRCA mutation-carriers benefitting most from the addition of olaparib to bevacizumab maintenance therapy after carboplatin and paclitaxel. Only recently, the EMA recommended the approval of this combination therapy for HR-deficient OC defined by the presence of BRCA-mutations or genomic instability for first-line maintenance treatment accordingly (27). Nonetheless, the exploration of further transparent tests that can easily be implemented and further elaborated on decentral platforms is still ongoing to improve the quest for predictive markers.

SUMMARY OF THE INVENTION

In general, the present invention relates to materials and methods involved in assessing cancer cells, in particular ovarian cancer cells, for homologous recombination deficiency (HRD). In particular, the present invention relates to a method of determining or predicting HRD status, *i.e.* the presence or absence of a HRD signature, in a subject having ovarian cancer and to a method of diagnosing the presence or absence of HRD in a patient sample, respectively. The method comprises the determination of copy number variation (CNV) of a genomic locus in a test sample from a subject comprising cancer cells, *e.g.* a DNA test sample of cancer cells, wherein the locus is selected from a pool of genomic loci comprising or consisting of the loci set forth in Table 1 and/or in Table 2; and wherein similarity between the CNVs of the pool of genomic loci in the test sample from the subject and the CNVs of a corresponding pool of genomic loci in the DNA of a reference sample of BRCA-like mutated cancer cells and a control sample of BRCA non-mutated cancer cells, is indicative for the HR deficiency status in the subject, wherein (i) similarity between the CNVs of the test sample and the CNVs of the reference sample identifies the subject as HR deficient; and/or (ii) similarity between the CNVs of the test sample and the CNVs of the control sample identifies the subject as not being HR deficient. CNVs include losses and gains of genomic segments. The similarity is defined as distance measure between the centroids of the two classes, *i.e.* the control and reference sample, and the centroid of the test sample.

A centroid of a class, which is in the present case the control and reference sample, respectively, is constructed by shrinking the class centroid by an optimization routine towards the overall centroid of both classes after standardizing by the within-class standard deviation for each CNVs.

Preferably, a similarity score, *i.e.* a posterior probability score, is computed by providing a shrunken centroid value which is derived from the CNV of the genomic locus and wherein the comparison of the shrunken centroids of the test sample from the subject with the shrunken centroids of the reference sample and the shrunken centroids of the control sample is converted into a posterior probability score according to Tibshirani PNAS 2002 (29). This score ranges from 0-1, with 0-0.49 being non-BRCA-like and 0.5-1 being BRCA-like, wherein this definition is set during the training, wherein it has been defined that the BRCA-like class is the one of interest.

Thus, in one embodiment the method of the present invention comprises (a) determining copy number variation (CNV) of a genomic locus in a test sample from a subject comprising cancer cells, *e.g.* a DNA test sample of cancer cells, wherein the locus is selected from a pool of genomic loci comprising or consisting of the loci set forth in Table 1 and/or Table 2; and (b) providing a shrunken centroid value which is derived from the CNV of the genomic locus, and wherein a posterior probability score of $\geq 0.5$ identifies the subject as being HRD; and/or wherein a posterior probability score of $< 0.5$ identifies the subject as not being HRD, wherein the posterior probability score is obtainable by comparison of the shrunken centroid values derived from the CNVs of the pool of genomic loci in the DNA of the cancer cells in the test sample from the subject with the shrunken centroid values derived from the CNVs of a reference set of samples of BRCA-like mutated cancer cells and of control samples of BRCA non-mutated cancer cells.

The method of the present invention optionally comprises the transmission of the result to the subject or to a third party like a physician or genetic counselor.

Furthermore, a posterior probability score of $\geq 0.5$ representing a higher similarity between the CNVs of the test sample and the CNVs of the reference sample than the control sample identifies the subject as being a likely responder to an anti-cancer therapy. A posterior probability score of $< 0.5$ representing a higher similarity between the CNVs of the test sample and the CNVs of the control sample than the reference sample, respectively identifies the subject as not being a likely responder to an anti-cancer therapy.

The present invention is also directed to a method of predicting the response of a subject having cancer, in particular ovarian cancer, to an anti-cancer therapy, *i.e.* to a cancer treatment regimen, wherein the method comprises the steps as defined above. In particular, a posterior probability score of $\geq 0.5$ and respectively a similarity between the CNVs of the test sample and the CNVs of the reference sample indicates an increased likelihood that the subject will respond to the anti-cancer therapy, and/or a posterior probability score of $< 0.5$ and respectively a similarity between the CNVs of the test sample and the CNVs of the control sample indicates an increased likelihood that the subj ect will not respond to the anti-cancer therapy. The method of the present optionally comprises the transmission of the result to the subject or to a third party like a physician or genetic counselor.

In a preferred embodiment, the anti-cancer therapy/the cancer treatment regime is selected from homologous recombination deficiency-targeting drugs, drugs that directly cause double strand DNA breaks, and drugs that indirectly cause double strand DNA breaks or where the drug or dose of the drug has been identified in mechanistic studies to target homologous recombination deficiency, like for example (but not limited to) interstrand crosslinking agents, platinum compounds, PARP inhibitors, anthracyclines, as well as topoisomerase I and II inhibitors, preferably the anti-cancer therapy/the cancer treatment regime comprises PARP inhibitors or DNA double strand break inducing agents, e.g. platinum compounds and interstrand crosslinking agents.

In one embodiment of the method of the present invention, the determination of the CNV is performed by DNA sequencing, preferably by low coverage whole genome sequencing (LG-WGS). In one embodiment of the method of the present invention, determination of the CNVs is performed prior to administration of the anti-cancer therapy.

Optionally, the method of the present invention further comprises recommending a subject which has been identified as being HRD and/or having an increased likelihood of responding to the anti-cancer therapy, a treatment regimen comprising homologous recombination deficiency-targeting drugs, drugs that directly cause double strand DNA breaks, drugs that indirectly cause double strand DNA breaks; and/or drugs which have been identified in mechanistic studies to target homologous recombination deficiency, for example interstrand crosslinking agents, platinum compounds, PARP inhibitors, anthracyclines, and topoisomerase I and II inhibitors, preferably PARP inhibitors or DNA double strand break inducing agents, e.g. platinum compounds and interstrand crosslinking agents. Alternatively, the method of the present invention may optionally comprise recommending a subject which has been identified as not being HRD and/or not having an increased likelihood of responding to the anti-cancer therapy, a standard treatment in the specific clinical setting of the patient, which might not include homologous recombination deficiency-targeting drugs, drugs that directly cause double strand DNA breaks, drugs that indirectly cause double strand DNA breaks, and/or drugs which have been identified in mechanistic studies to target homologous recombination deficiency.

The invention further relates to the above-mentioned drugs, namely PARP inhibitors, DNA double strand inducing agents, preferably platinum compounds or an interstrand crosslinking agents, anthracyclines, topoisomerase I inhibitors, or a topoisomerase II inhibitors for use in a method of treating cancer, in particular ovarian cancer. Such method is in general analogous to the methods described above and differs in that a particular treatment regimen is administered (recommended, prescribed, initiated, continued, etc.) based at least in part of the calculated posterior probability scores and the similarity determinations as explained above. The treatment regime is preferably the treatment regime as defined above.

Furthermore, the present invention relates to a treatment regime comprising homologous recombination deficiency-targeting drugs, drugs that directly cause double strand DNA breaks, drugs that indirectly cause double strand DNA breaks, or that otherwise have been identified as specifically targeting homologous recombination deficient tumors for use in treating ovarian cancer in a subject, wherein said subject has been identified as HRD and/or identified to have an increased likelihood of responding to the anti-cancer treatment regimen according to the method as described above.

Unless otherwise defined, all technical and scientific terms used herein have the same meaning as commonly understood by one of ordinary skill in the art to which this invention pertains. The details of one or more embodiments of the invention are set forth in the description and accompanying drawings as well as in the Examples below.

**Table 1: BRCA-1 like classifier.** The indicated locations are on genome version 'hg18'. Of course, in case a genome version changes, the person skilled in the art is able to assign the below mentioned positions to any new and/or different genome version. Thus, variations in the Start and End positions are also encompassed by the present invention.

| Order | Clone | chrom | Start | End | sc_control | sc_BRCA1 | sd |
|---|---|---|---|---|---|---|---|
| 3535 | GS-232-B23 | 1 | 125000 | 275000 | -0.1949 | -0.1766 | 0.5283 |
| 3537 | GS-62-L8 | 1 | 125050 | 275050 | -0.1949 | -0.1766 | 0.5283 |
| 2305 | RP4-785P20 | 1 | 3214521 | 3355092 | -0.1972 | -0.1322 | 0.4771 |
| 145 | RP1-37J18 | 1 | 4476787 | 4608114 | -0.1222 | -0.0946 | 0.4422 |
| 3 | RPII-484P7 | 1 | 9083937 | 9084661 | -0.0873 | -0.0568 | 0.4502 |
| 2313 | RP4-575L21 | 1 | 10075582 | 10170003 | -0.0480 | -0.0475 | 0.4569 |
| 153 | RP4-635E18 | 1 | 10986297 | 11099065 | -0.0480 | -0.0475 | 0.4569 |
| 2317 | RP4-539L13 | 1 | 11483207 | 11618991 | -0.0480 | -0.0475 | 0.4569 |
| 3358 | RP11-196P5 | 1 | 11765728 | 11932249 | -0.0480 | -0.0475 | 0.4569 |
| 57 | RP11-174G17 | 1 | 18255125 | 18370415 | -0.0880 | -0.0947 | 0.4135 |
| 2325 | RP4-540O3 | 1 | 19184476 | 19258712 | -0.0880 | -0.0947 | 0.4135 |
| 165 | RP4-745E8 | 1 | 20480049 | 20558818 | -0.0880 | -0.0947 | 0.4135 |
| 2221 | RP11-132G19 | 1 | 21921946 | 22097107 | -0.0586 | -0.0777 | 0.4213 |
| 2845 | RP1-184J9 | 1 | 23210636 | 23311487 | -0.0582 | -0.0646 | 0.4130 |
| 3491 | RP11-304H10 | 1 | 24599009 | 24781759 | -0.0580 | -0.0892 | 0.4081 |
| 685 | RP3-398I9 | 1 | 25064029 | 25159269 | -0.0580 | -0.0892 | 0.4081 |
| 689 | RP4-633N17 | 1 | 27233517 | 27352531 | -0.0580 | -0.0821 | 0.4097 |
| 2849 | RP1-144C9 | 1 | 27575323 | 27652644 | -0.0580 | -0.0821 | 0.4097 |
| 900 | RP1-159A19 | 1 | 27730312 | 27853599 | -0.0580 | -0.0821 | 0.4097 |
| 693 | RP4-669K10 | 1 | 28699604 | 28835423 | -0.0577 | -0.0643 | 0.4013 |
| 2853 | RP3-437I16 | 1 | 29481122 | 29601506 | -0.0577 | -0.0643 | 0.4013 |
| 2857 | RP5-893G23 | 1 | 30314279 | 30401538 | -0.0577 | -0.0643 | 0.4013 |

(continued)

| Order | Clone | chrom | Start | End | sc_control | sc_BRCA1 | sd |
|---|---|---|---|---|---|---|---|
| 2306 | RP4-739H11 | 1 | 40830672 | 40960522 | 0.0556 | 0.0493 | 0.3948 |
| 2761 | RP11-182I23 | 1 | 44522378 | 44608805 | 0.0554 | 0.0533 | 0.3974 |
| 3891 | RP 11-420M 12 | 1 | 45348865 | 45523040 | 0.0554 | 0.0533 | 0.3974 |
| 146 | RP4-534D 1 | 1 | 45524153 | 45623939 | 0.0554 | 0.0533 | 0.3974 |
| 605 | RP11-330M19 | 1 | 48002300 | 48202667 | -0.0583 | 0.0255 | 0.3611 |
| 2769 | RP11-428D12 | 1 | 48917309 | 49103008 | -0.0583 | 0.0255 | 0.3611 |
| 2310 | RP5-1013G21 | 1 | 50343064 | 50459755 | -0.0583 | 0.0255 | 0.3611 |
| 150 | RP1-86A18 | 1 | 50594537 | 50802796 | -0.0583 | 0.0255 | 0.3611 |
| 609 | RP11-253A20 | 1 | 51655989 | 51797658 | -0.0583 | 0.0255 | 0.3611 |
| 2210 | RP11-117D22 | 1 | 53582566 | 53632120 | -0.0583 | 0.0442 | 0.3617 |
| 2500 | RP11-243A18 | 1 | 53802593 | 53986331 | -0.0583 | 0.0442 | 0.3617 |
| 154 | RP5-1043G4 | 1 | 54894393 | 55023548 | -0.0583 | 0.0442 | 0.3617 |
| 2318 | RP5-1070D5 | 1 | 55675959 | 55690981 | -0.0583 | 0.0442 | 0.3617 |
| 158 | RP4-710M16 | 1 | 56595677 | 56724344 | -0.0432 | 0.0284 | 0.3684 |
| 1286 | RP11-20F20 | 1 | 57456434 | 57628847 | -0.0434 | 0.0355 | 0.3646 |
| 2139 | RP11-20F20 | 1 | 57456434 | 57628847 | -0.0434 | 0.0355 | 0.3646 |
| 50 | RP11-342F23 | 1 | 58740479 | 58790441 | -0.0421 | 0.0389 | 0.3917 |
| 2214 | RP11-37M11 | 1 | 59939363 | 60081655 | -0.0421 | 0.0389 | 0.3917 |
| 2322 | RP4-668G5 | 1 | 61044546 | 61191260 | -0.0424 | 0.0312 | 0.3857 |
| 162 | RP4-662P1 | 1 | 62131197 | 62274422 | -0.0424 | 0.0312 | 0.3857 |
| 2218 | RP11-5P4 | 1 | 63222826 | 63340665 | -0.0434 | 0.0050 | 0.3647 |
| 2326 | RP4-537F10 | 1 | 64206685 | 64303918 | -0.0434 | 0.0050 | 0.3647 |
| 166 | RP4-700A9 | 1 | 65371357 | 65460412 | -0.0432 | -0.0003 | 0.3674 |
| 2846 | RP4-759M20 | 1 | 66677274 | 66805768 | -0.0432 | -0.0003 | 0.3674 |
| 686 | RP4-547N15 | 1 | 67239504 | 67370748 | -0.0432 | -0.0003 | 0.3674 |
| 2850 | RP5-1033K19 | 1 | 68609845 | 68712983 | -0.0432 | -0.0003 | 0.3674 |
| 58 | RP11-412F21 | 1 | 69215584 | 69364440 | -0.0432 | -0.0003 | 0.3674 |
| 690 | RP5-944F13 | 1 | 69875730 | 69989244 | -0.0432 | -0.0003 | 0.3674 |
| 2680 | RP11-419I14 | 1 | 70178367 | 70348261 | -0.0432 | -0.0003 | 0.3674 |
| 2854 | RP5-1006B11 | 1 | 70717489 | 70814713 | -0.0432 | -0.0003 | 0.3674 |
| 2222 | RP11-175G14 | 1 | 71927563 | 72045513 | -0.0432 | -0.0003 | 0.3674 |
| 62 | RP11-261J10 | 1 | 72813123 | 72983939 | -0.0432 | -0.0003 | 0.3674 |
| 2858 | RP4-716B13 | 1 | 73625850 | 73764334 | -0.0432 | -0.0003 | 0.3674 |
| 2226 | RP11-250D8 | 1 | 74474909 | 74503104 | -0.0431 | -0.0065 | 0.3701 |
| 66 | RP11-25F16 | 1 | 75234702 | 75388843 | -0.0431 | -0.0065 | 0.3701 |
| 698 | RP5-963M5 | 1 | 76640776 | 76740455 | -0.0431 | -0.0065 | 0.3701 |
| 2230 | RP11-306I4 | 1 | 77815414 | 77880693 | -0.0431 | -0.0065 | 0.3701 |

(continued)

| Order | Clone | chrom | Start | End | sc_control | sc_BRCA1 | sd |
|---|---|---|---|---|---|---|---|
| 70 | RP11-143H12 | 1 | 78953798 | 79135725 | -0.0425 | -0.0019 | 0.3830 |
| 523 | RP11-111N8 | 1 | 78981093 | 79179795 | -0.0425 | -0.0019 | 0.3830 |
| 706 | RP4-653E17 | 1 | 81843695 | 81898453 | -0.0421 | 0.0044 | 0.3923 |
| 2750 | RP11-398N17 | 1 | 82504367 | 82595121 | -0.0423 | 0.0145 | 0.3883 |
| 590 | RP11-338F23 | 1 | 83494488 | 83678494 | -0.0423 | 0.0145 | 0.3883 |
| 1826 | RP11-28I21 | 1 | 83633989 | 83797164 | -0.0423 | 0.0145 | 0.3883 |
| 4296 | RPII-28I21 | 1 | 83633989 | 83797164 | -0.0423 | 0.0145 | 0.3883 |
| 2754 | RP11-118B23 | 1 | 84728027 | 84853591 | -0.0422 | 0.0199 | 0.3906 |
| 2307 | RP4-604P14 | 1 | 84964828 | 85107408 | -0.0422 | 0.0199 | 0.3906 |
| 147 | RP4-722L13 | 1 | 85912330 | 85939332 | -0.0422 | 0.0274 | 0.3892 |
| 2311 | RP4-612B15 | 1 | 86920706 | 87046530 | -0.0422 | 0.0274 | 0.3892 |
| 151 | RP5-1043L3 | 1 | 87450562 | 87550402 | -0.0422 | 0.0274 | 0.3892 |
| 594 | RP11-427B20 | 1 | 88626861 | 88735131 | -0.0424 | 0.0390 | 0.3858 |
| 2758 | RP11-413E1 | 1 | 89836299 | 90021480 | -0.0424 | 0.0390 | 0.3858 |
| 598 | RP11-298P9 | 1 | 90346809 | 90447017 | -0.0424 | 0.0390 | 0.3858 |
| 2315 | RP4-665J23 | 1 | 91067447 | 91151453 | -0.0424 | 0.0390 | 0.3858 |
| 155 | RP4-621B10 | 1 | 92289742 | 92432467 | -0.0426 | 0.0506 | 0.3824 |
| 2319 | RP5-1033H22 | 1 | 93768309 | 93910326 | -0.0425 | 0.0468 | 0.3844 |
| 2762 | RP11-366L18 | 1 | 94637145 | 94733145 | -0.0421 | 0.0420 | 0.3926 |
| 602 | RP11-14O19 | 1 | 95577488 | 95621690 | -0.0425 | 0.0311 | 0.3835 |
| 2766 | RP11-146P11 | 1 | 95756215 | 95929262 | -0.0425 | 0.0311 | 0.3835 |
| 606 | RP11-122C9 | 1 | 96868095 | 97055472 | -0.0425 | 0.0311 | 0.3835 |
| 2770 | RP11-128G13 | 1 | 97892513 | 98003512 | -0.0431 | 0.0390 | 0.3711 |
| 610 | RP11-17C2 | 1 | 98735430 | 98839846 | -0.0431 | 0.0390 | 0.3711 |
| 4184 | RP11-260K3 | 1 | 99670780 | 99835817 | -0.0431 | 0.0390 | 0.3711 |
| 2211 | RP11-413P11 | 1 | 99710406 | 99835827 | -0.0431 | 0.0390 | 0.3711 |
| 159 | RP4-549L20 | 1 | 101075342 | 101207111 | -0.0431 | 0.0390 | 0.3711 |
| 51 | RP11-421L21 | 1 | 101220661 | 101408985 | -0.0431 | 0.0390 | 0.3711 |
| 2215 | RP11-202K23 | 1 | 102366306 | 102506926 | -0.0431 | 0.0390 | 0.3711 |
| 55 | RP11-118G19 | 1 | 105468331 | 105581702 | -0.0431 | 0.0390 | 0.3711 |
| 2219 | RP11-90H3 | 1 | 106102370 | 106261386 | -0.0431 | 0.0390 | 0.3711 |
| 163 | RP5-916A15 | 1 | 106759849 | 106884451 | -0.0431 | 0.0390 | 0.3711 |
| 2327 | RP5-1077K16 | 1 | 107388307 | 107478600 | -0.0436 | 0.0503 | 0.3599 |
| 59 | RP11-28P8 | 1 | 108330015 | 108512967 | -0.0436 | 0.0503 | 0.3599 |
| 2847 | RP5-831G13 | 1 | 109816089 | 109885092 | -0.0436 | 0.0503 | 0.3599 |
| 2223 | RP11-284N8 | 1 | 110897276 | 111088458 | -0.0436 | 0.0503 | 0.3599 |
| 63 | RP11-88H9 | 1 | 112311566 | 112481164 | -0.0439 | 0.0442 | 0.3536 |

(continued)

| Order | Clone | chrom | Start | End | sc_ control | sc_ BRCA1 | sd |
|---|---|---|---|---|---|---|---|
| 687 | RP4-770C6 | 1 | 112861328 | 112960667 | -0.0439 | 0.0442 | 0.3536 |
| 2227 | RP11-31F15 | 1 | 113304869 | 113373751 | -0.0216 | 0.0442 | 0.3542 |
| 2525 | RP5-1000E10 | 1 | 115009146 | 115149253 | -0.0215 | 0.0501 | 0.3566 |
| 691 | RP4-663N10 | 1 | 115660445 | 115837510 | -0.0216 | 0.0625 | 0.3541 |
| 2859 | RP4-610L12 | 1 | 119405867 | 119480650 | 0.3336 | 0.1175 | 0.6047 |
| 71 | RP11-418J17 | 1 | 119501341 | 119658498 | 0.3336 | 0.1175 | 0.6047 |
| 591 | RP11-326G21 | 1 | 143613589 | 143768403 | 0.3503 | 0.1544 | 0.5030 |
| 2755 | RP11-315I20 | 1 | 144149933 | 144341526 | 0.1930 | 0.1765 | 0.3689 |
| 595 | RP11-533N14 | 1 | 145509311 | 145622017 | 0.1930 | 0.1765 | 0.3689 |
| 2759 | RP11-301M17 | 1 | 146153735 | 146204124 | 0.1930 | 0.1765 | 0.3689 |
| 2763 | RP11-98D18 | 1 | 149916726 | 150080114 | 0.1938 | 0.1755 | 0.3533 |
| 603 | RP11-216N14 | 1 | 151954673 | 152104838 | 0.1933 | 0.1840 | 0.3644 |
| 607 | RP11-422P24 | 1 | 152158475 | 152334301 | 0.1933 | 0.1840 | 0.3644 |
| 2767 | RP11-307C12 | 1 | 153110663 | 153292828 | 0.1933 | 0.1840 | 0.3644 |
| 2771 | RP11-98F1 | 1 | 153539661 | 153543654 | 0.1933 | 0.1840 | 0.3644 |
| 611 | RP11-172I6 | 1 | 154346199 | 154350727 | 0.1933 | 0.1840 | 0.3644 |
| 60 | RP11-430G6 | 1 | 161223665 | 161388993 | 0.1627 | 0.1619 | 0.3808 |
| 3336 | RP11-180L13 | 1 | 163383027 | 163567283 | 0.1627 | 0.1619 | 0.3808 |
| 64 | RP11-30611 | 1 | 163809021 | 163884934 | 0.1627 | 0.1619 | 0.3808 |
| 148 | RP1-97P20 | 1 | 168048730 | 168151811 | 0.1631 | 0.1473 | 0.3721 |
| 68 | RP11-277C14 | 1 | 170129727 | 170285667 | 0.1630 | 0.1462 | 0.3745 |
| 2232 | RP11-415M14 | 1 | 174374254 | 174386794 | 0.1629 | 0.1471 | 0.3772 |
| 2752 | RP11-12M5 | 1 | 177942453 | 178115483 | 0.1629 | 0.1419 | 0.3761 |
| 592 | RP11-46A10 | 1 | 179093654 | 179232659 | 0.1629 | 0.1419 | 0.3761 |
| 2756 | RP11-538D16 | 1 | 180286063 | 180360577 | 0.1629 | 0.1419 | 0.3761 |
| 596 | RP11-71D4 | 1 | 180974670 | 181046133 | 0.1628 | 0.1331 | 0.3776 |
| 3350 | RP11-181K3 | 1 | 181329737 | 181493820 | 0.1629 | 0.1412 | 0.3770 |
| 2760 | RP11-293B7 | 1 | 181919047 | 181949386 | 0.1624 | 0.1294 | 0.3864 |
| 164 | RP5-936P19 | 1 | 184949810 | 185050756 | 0.1926 | 0.1312 | 0.3804 |
| 2764 | RP11-108M21 | 1 | 185443394 | 185614032 | 0.1926 | 0.1312 | 0.3804 |
| 604 | RP11-336D15 | 1 | 187406225 | 187410218 | 0.1926 | 0.1312 | 0.3804 |
| 2768 | RP11-445K1 | 1 | 188252300 | 188442462 | 0.1929 | 0.1414 | 0.3727 |
| 612 | RP11-142L4 | 1 | 190417312 | 190511486 | 0.1929 | 0.1435 | 0.3726 |
| 2623 | RP11-239J11 | 1 | 191355372 | 191478235 | 0.1929 | 0.1390 | 0.3737 |
| 463 | RP11-435N12 | 1 | 193197924 | 193377705 | 0.1919 | 0.1487 | 0.3941 |
| 467 | RP11-75C23 | 1 | 195654060 | 195710013 | 0.1926 | 0.1344 | 0.3788 |
| 2631 | RP11-404H1 | 1 | 196603836 | 196750596 | 0.1926 | 0.1344 | 0.3788 |

(continued)

| Order | Clone | chrom | Start | End | sc_control | sc_BRCA1 | sd |
|-------|-------|-------|-------|-----|------------|----------|-----|
| 471 | RP11-152M20 | 1 | 197828613 | 197913713 | 0.1926 | 0.1344 | 0.3788 |
| 2635 | RP11-469A15 | 1 | 198548937 | 198714432 | 0.1926 | 0.1344 | 0.3788 |
| 2639 | RP11-572A16 | 1 | 200418733 | 200596076 | 0.1919 | 0.0979 | 0.3946 |
| 479 | RP11-480I12 | 1 | 200974582 | 201126597 | 0.1919 | 0.0979 | 0.3946 |
| 1988 | RP11-739N20 | 1 | 202583794 | 202750872 | 0.1908 | 0.0824 | 0.4181 |
| 2643 | RP11-563I16 | 1 | 202668576 | 202832483 | 0.1908 | 0.0824 | 0.4181 |
| 3895 | RP11-430C7 | 1 | 202750873 | 202882857 | 0.1908 | 0.0718 | 0.4189 |
| 3163 | RP 11-534L20 | 1 | 204706319 | 204814007 | 0.1562 | 0.0497 | 0.4209 |
| 1003 | RP11-564A8 | 1 | 205087940 | 205280504 | 0.1562 | 0.0497 | 0.4209 |
| 1007 | RP11-385M4 | 1 | 207338650 | 207512566 | 0.1556 | 0.0223 | 0.4334 |
| 3171 | RP11-354K1 | 1 | 209859608 | 209904751 | 0.1556 | 0.0223 | 0.4334 |
| 1011 | RP11-286E7 | 1 | 211304553 | 211481242 | 0.1556 | 0.0223 | 0.4334 |
| 3175 | RPII-434B7 | 1 | 211479243 | 211599451 | 0.1556 | 0.0182 | 0.4345 |
| 1015 | RP11-323K10 | 1 | 213140173 | 213231454 | 0.1560 | 0.0195 | 0.4249 |
| 3179 | RP11-438G15 | 1 | 213686067 | 213758612 | 0.1560 | 0.0195 | 0.4249 |
| 1019 | RP11-66M7 | 1 | 215209941 | 215373866 | 0.1560 | 0.0195 | 0.4249 |
| 3183 | RP11-241C9 | 1 | 215371867 | 215473408 | 0.1560 | 0.0195 | 0.4249 |
| 1718 | RP11-224O19 | 1 | 216532600 | 216705043 | 0.1561 | 0.0270 | 0.4233 |
| 1023 | RP11-392O17 | 1 | 217480858 | 217657906 | 0.1564 | 0.0419 | 0.4168 |
| 2624 | RP11-492K2 | 1 | 217542815 | 217743251 | 0.1564 | 0.0419 | 0.4168 |
| 464 | RP11-332J14 | 1 | 218698681 | 218866026 | 0.1564 | 0.0590 | 0.4167 |
| 2628 | RP11-308L13 | 1 | 219894313 | 219919554 | 0.2203 | 0.0689 | 0.4122 |
| 2632 | RP11-239E10 | 1 | 221352933 | 221534398 | 0.2203 | 0.0689 | 0.4122 |
| 472 | RP11-105I12 | 1 | 221890430 | 222001842 | 0.2203 | 0.0689 | 0.4122 |
| 2636 | RP11-100E13 | 1 | 222762747 | 222926492 | 0.2203 | 0.0689 | 0.4122 |
| 2640 | RP11-375H24 | 1 | 225133240 | 225194140 | 0.2604 | 0.0896 | 0.4223 |
| 480 | RP11-276J4 | 1 | 225649911 | 225807728 | 0.2604 | 0.0896 | 0.4223 |
| 2848 | RP4-799G3 | 1 | 226955298 | 227082936 | 0.2606 | 0.0982 | 0.4183 |
| 688 | RP4-679K16 | 1 | 227665287 | 227690355 | 0.2606 | 0.0982 | 0.4183 |
| 2644 | RP11-99J16 | 1 | 228957724 | 229009252 | 0.2606 | 0.0982 | 0.4183 |
| 484 | RP11-87P4 | 1 | 230784451 | 230922071 | 0.2603 | 0.0993 | 0.4253 |
| 3164 | RP11-528D17 | 1 | 231700764 | 231814183 | 0.2603 | 0.0993 | 0.4253 |
| 3168 | RP11-214M7 | 1 | 235542200 | 235725748 | 0.2606 | 0.0920 | 0.4181 |
| 1008 | RP11-433N10 | 1 | 236639314 | 236664614 | 0.2606 | 0.0920 | 0.4181 |
| 3172 | RP11-359A17 | 1 | 237591402 | 237780910 | 0.2606 | 0.0920 | 0.4181 |
| 1012 | RP 11-80B9 | 1 | 238957512 | 239131145 | 0.2606 | 0.0920 | 0.4181 |
| 3176 | RP11-553N16 | 1 | 240002343 | 240146309 | 0.2606 | 0.0920 | 0.4181 |

(continued)

| Order | Clone | chrom | Start | End | sc_ control | sc_ BRCA1 | sd |
|---|---|---|---|---|---|---|---|
| 1020 | RP11-399B15 | 1 | 243222295 | 243431391 | 0.2606 | 0.0920 | 0.4181 |
| 2625 | RP11-438H8 | 1 | 246013817 | 246181047 | 0.2606 | 0.0920 | 0.4181 |
| 1375 | GS-167-K11 | 1 | 246904719 | 247054719 | 0.2606 | 0.0920 | 0.4181 |
| 1377 | GS-160-H23 | 1 | 247124719 | 247274719 | 0.2606 | 0.0920 | 0.4181 |
| 3539 | GS-8-L3 | 2 | 255000 | 405000 | 0.1037 | 0.0763 | 0.4608 |
| 3541 | GS-892-G20 | 2 | 255050 | 405050 | 0.1037 | 0.0763 | 0.4608 |
| 3931 | RP11-352J11 | 2 | 2250133 | 2390017 | 0.1060 | 0.0999 | 0.4108 |
| 2484 | RP11-214N9 | 2 | 9588276 | 9685110 | 0.1068 | 0.1067 | 0.3946 |
| 648 | RP11-83M8 | 2 | 10189946 | 10392995 | 0.1061 | 0.1029 | 0.4094 |
| 3387 | RP11-345J13 | 2 | 11150863 | 11208496 | 0.1073 | 0.0818 | 0.3825 |
| 1226 | RP11-333O1 | 2 | 12700490 | 12904007 | 0.1078 | 0.0701 | 0.3708 |
| 293 | RP11-132H1 | 2 | 13501024 | 13636845 | 0.0674 | 0.0625 | 0.3712 |
| 3326 | RP11-163G14 | 2 | 14523373 | 14693180 | 0.0674 | 0.0625 | 0.3712 |
| 825 | RP11-136K18 | 2 | 14848829 | 14852339 | 0.0674 | 0.0625 | 0.3712 |
| 2019 | RP11-247H16 | 2 | 15119640 | 15208710 | 0.1015 | 0.0667 | 0.3590 |
| 4306 | RP11-247H16 | 2 | 15119640 | 15208710 | 0.1015 | 0.0667 | 0.3590 |
| 1199 | RP11-355H10 | 2 | 15932229 | 16129188 | 0.1015 | 0.0667 | 0.3590 |
| 4070 | RP11-549D18 | 2 | 16245950 | 16435643 | 0.1015 | 0.0667 | 0.3590 |
| 4192 | RP 11-262D22 | 2 | 17121302 | 17130519 | 0.1015 | 0.0667 | 0.3590 |
| 2058 | RP11-111J6 | 2 | 18490085 | 18666774 | 0.1015 | 0.0667 | 0.3590 |
| 2135 | RP11-17L8 | 2 | 20037859 | 20215537 | 0.0992 | 0.0900 | 0.4091 |
| 3422 | RP11-17L8 | 2 | 20037859 | 20215537 | 0.0992 | 0.0900 | 0.4091 |
| 3412 | RP11-23F10 | 2 | 21327465 | 21369660 | 0.1016 | 0.0689 | 0.3589 |
| 311 | RP11-368O18 | 2 | 22411645 | 22617616 | 0.1016 | 0.0747 | 0.3586 |
| 2929 | RP11-560C7 | 2 | 22998568 | 23181018 | 0.1018 | 0.0876 | 0.3524 |
| 761 | RP11-557N21 | 2 | 23723602 | 23881796 | 0.1020 | 0.0946 | 0.3497 |
| 3627 | RP11-557N21 | 2 | 23723602 | 23881796 | 0.1020 | 0.0946 | 0.3497 |
| 3335 | RP11-169L20 | 2 | 24704977 | 24873681 | 0.1021 | 0.0880 | 0.3465 |
| 306 | RP 11-404P 12 | 2 | 25696967 | 25870860 | 0.1021 | 0.0880 | 0.3465 |
| 3666 | RP11-106G13 | 2 | 26821201 | 26988191 | 0.1021 | 0.0880 | 0.3465 |
| 843 | RP 11-373D23 | 2 | 28414457 | 28572121 | 0.1021 | 0.0880 | 0.3465 |
| 3925 | RP11-328L16 | 2 | 29261112 | 29451760 | 0.1021 | 0.0880 | 0.3465 |
| 2477 | RP11-395B14 | 2 | 39539270 | 39691425 | 0.1339 | 0.1356 | 0.3440 |
| 3016 | RP11-389K20 | 2 | 52045693 | 52231824 | 0.1221 | 0.1286 | 0.3454 |
| 1234 | RP11-335O22 | 2 | 52193265 | 52373212 | 0.1224 | 0.1346 | 0.3525 |
| 2411 | RP11-7H13 | 2 | 52903958 | 53101085 | 0.1224 | 0.1317 | 0.3515 |
| 3527 | RP11-508L23 | 2 | 53793757 | 53906745 | 0.1242 | 0.1500 | 0.3906 |

(continued)

| Order | Clone | chrom | Start | End | sc_ control | sc_ BRCA1 | sd |
|---|---|---|---|---|---|---|---|
| 971 | RP11-30C22 | 2 | 54809162 | 54917397 | 0.1234 | 0.1439 | 0.3740 |
| 1173 | RP11-152O18 | 2 | 55792089 | 55796228 | 0.1234 | 0.1439 | 0.3740 |
| 546 | RP11-481J13 | 2 | 56202050 | 56343886 | 0.1234 | 0.1439 | 0.3740 |
| 1249 | RP 11-422B 1 | 2 | 58184911 | 58334911 | 0.1238 | 0.1244 | 0.3810 |
| 2024 | RP11-260K8 | 2 | 58959329 | 59141348 | 0.1238 | 0.1244 | 0.3810 |
| 14 | RP11-479F13 | 2 | 61286584 | 61378667 | 0.1226 | 0.1146 | 0.4215 |
| 4188 | RP11-261A24 | 2 | 63239468 | 63339572 | 0.1246 | 0.1211 | 0.3795 |
| 2922 | RP11-568N6 | 2 | 64478270 | 64676357 | 0.1246 | 0.1211 | 0.3795 |
| 1481 | RP11-263L17 | 2 | 65586797 | 65745895 | 0.1249 | 0.1152 | 0.3731 |
| 233 | RP11-554F14 | 2 | 66394108 | 66576570 | 0.1249 | 0.1152 | 0.3731 |
| 2814 | RP11-67K11 | 2 | 68007238 | 68161125 | 0.1260 | 0.0867 | 0.3483 |
| 1327 | RP11-304A15 | 2 | 68860250 | 69024244 | 0.1063 | 0.0805 | 0.3358 |
| 2095 | RP11-304A15 | 2 | 68860250 | 69024244 | 0.1063 | 0.0805 | 0.3358 |
| 864 | RP 11-245N4 | 2 | 70374522 | 70512358 | 0.1063 | 0.0772 | 0.3353 |
| 3613 | CTB-1011K1 | 2 | 70877487 | 70877988 | 0.1063 | 0.0772 | 0.3353 |
| 545 | RP 11-467P9 | 2 | 71113938 | 71283193 | 0.0825 | 0.0771 | 0.3285 |
| 1782 | RP11-343N14 | 2 | 72247343 | 72428558 | 0.0827 | 0.0811 | 0.3233 |
| 1569 | RP11-434P11 | 2 | 73670503 | 73883575 | 0.0827 | 0.0811 | 0.3233 |
| 257 | RP11-1P9 | 2 | 74335426 | 74424778 | 0.0827 | 0.0811 | 0.3233 |
| 1230 | RP11-335E8 | 2 | 76173054 | 76356807 | 0.0832 | 0.0780 | 0.3136 |
| 4065 | RP11-535E19 | 2 | 77131545 | 77193800 | 0.0832 | 0.0780 | 0.3136 |
| 2955 | RP11-9O10 | 2 | 77953060 | 77979896 | 0.0808 | 0.0597 | 0.3641 |
| 4267 | RP11-419E14 | 2 | 78739646 | 78927111 | 0.0825 | 0.0688 | 0.3277 |
| 1 | RP11-458O14 | 2 | 79819775 | 79920395 | 0.0825 | 0.0688 | 0.3277 |
| 1323 | RP11-30314 | 2 | 80657637 | 80808678 | 0.0825 | 0.0688 | 0.3277 |
| 2091 | RP11-30314 | 2 | 80657637 | 80808678 | 0.0825 | 0.0688 | 0.3277 |
| 2035 | RP11-89C12 | 2 | 81618971 | 81763529 | 0.0825 | 0.0688 | 0.3277 |
| 559 | RP11-495B16 | 2 | 82611679 | 82714465 | 0.0825 | 0.0688 | 0.3277 |
| 4067 | RP11-513O19 | 2 | 85513676 | 85688465 | 0.0832 | 0.0782 | 0.3136 |
| 4038 | RP11-301019 | 2 | 86132487 | 86301208 | 0.0832 | 0.0782 | 0.3136 |
| 3500 | RP11-316G9 | 2 | 89561552 | 89772753 | 0.0828 | 0.0655 | 0.3213 |
| 432 | RP11-34G16 | 2 | 95675761 | 95861540 | 0.1844 | 0.0860 | 0.3789 |
| 274 | RP11-139J6 | 2 | 96130286 | 96151127 | 0.1844 | 0.0882 | 0.3791 |
| 1780 | RP11-363D14 | 2 | 96531332 | 96597791 | 0.1840 | 0.0626 | 0.3885 |
| 3721 | RP11-363D14 | 2 | 96531332 | 96597791 | 0.1840 | 0.0626 | 0.3885 |
| 1366 | RP11-542D13 | 2 | 97472514 | 97700939 | 0.1840 | 0.0626 | 0.3885 |
| 4058 | RP11-547F10 | 2 | 98011952 | 98199250 | 0.1840 | 0.0626 | 0.3885 |

(continued)

| Order | Clone | chrom | Start | End | sc_control | sc_BRCA1 | sd |
|---|---|---|---|---|---|---|---|
| 417 | RP11-38C17 | 2 | 99112042 | 99179521 | 0.1840 | 0.0600 | 0.3881 |
| 4074 | RP11-549H5 | 2 | 99955224 | 100125797 | 0.1842 | 0.0685 | 0.3839 |
| 2928 | RP11-30G7 | 2 | 100125798 | 100273807 | 0.1842 | 0.0685 | 0.3839 |
| 1862 | RP 11-299H21 | 2 | 101071911 | 101277486 | 0.1842 | 0.0685 | 0.3839 |
| 4270 | RP11-451C2 | 2 | 101984766 | 102030378 | 0.0854 | 0.0679 | 0.3360 |
| 3007 | RP11-373G23 | 2 | 102712967 | 102891930 | 0.0850 | 0.0607 | 0.3455 |
| 2114 | RP11-451G1 | 2 | 103816333 | 103981851 | 0.0610 | 0.0488 | 0.3339 |
| 1239 | RP11-350B7 | 2 | 104912262 | 105047433 | 0.0610 | 0.0445 | 0.3344 |
| 4064 | RP11-526D2 | 2 | 105850219 | 106012104 | 0.0610 | 0.0445 | 0.3344 |
| 1360 | RP11-519H15 | 2 | 106847336 | 107035284 | 0.0612 | 0.0359 | 0.3302 |
| 234 | RP11-566O4 | 2 | 109113179 | 109319769 | 0.0612 | 0.0359 | 0.3302 |
| 1589 | RP11-438K19 | 2 | 111491772 | 111673269 | 0.0613 | 0.0397 | 0.3284 |
| 2467 | RP11-368A17 | 2 | 113091657 | 113230334 | 0.0613 | 0.0397 | 0.3284 |
| 560 | RP11-504G11 | 2 | 115568405 | 115730304 | 0.0523 | 0.0251 | 0.3316 |
| 2542 | RP 11-412A2 | 2 | 115793564 | 115936236 | 0.0528 | 0.0322 | 0.3198 |
| 1246 | RP11-339P1 | 2 | 116380901 | 116514763 | 0.0531 | 0.0376 | 0.3142 |
| 2813 | RP11-59P4 | 2 | 117162762 | 117348560 | 0.0531 | 0.0376 | 0.3142 |
| 1546 | RP11-425F6 | 2 | 118087040 | 118291673 | 0.0531 | 0.0376 | 0.3142 |
| 1588 | RP11-425F6 | 2 | 118087040 | 118291673 | 0.0531 | 0.0376 | 0.3142 |
| 1790 | RP11-19E11 | 2 | 119282245 | 119406283 | 0.0531 | 0.0376 | 0.3142 |
| 659 | RP11-77A13 | 2 | 119406284 | 119549525 | 0.0531 | 0.0376 | 0.3142 |
| 99 | RP11-69O6 | 2 | 121704118 | 121873929 | 0.0531 | 0.0376 | 0.3142 |
| 2703 | RP11-490M17 | 2 | 122905850 | 122950222 | 0.0528 | 0.0451 | 0.3196 |
| 4227 | RP11-202H24 | 2 | 122950223 | 122990154 | 0.0528 | 0.0451 | 0.3196 |
| 1338 | RP11-298E18 | 2 | 124928149 | 125078149 | 0.0520 | 0.0413 | 0.3365 |
| 958 | RP11-48K7 | 2 | 127180319 | 127253883 | 0.0518 | 0.0340 | 0.3410 |
| 3696 | RP11-207G14 | 2 | 129652992 | 129799323 | 0.0518 | 0.0340 | 0.3410 |
| 2588 | RP11-32C20 | 2 | 130418035 | 130583445 | 0.0518 | 0.0340 | 0.3410 |
| 3531 | RP11-510C1 | 2 | 131164571 | 131306971 | 0.0518 | 0.0340 | 0.3410 |
| 4224 | RP11-209H16 | 2 | 131497953 | 131669023 | 0.0518 | 0.0340 | 0.3410 |
| 1796 | RP11-24O13 | 2 | 131993057 | 131995050 | 0.0518 | 0.0340 | 0.3410 |
| 2417 | RP11-1L22 | 2 | 132997590 | 132999583 | 0.0513 | 0.0459 | 0.3531 |
| 1498 | RP11-105K20 | 2 | 133513096 | 133688729 | 0.0513 | 0.0459 | 0.3531 |
| 4191 | RP11-250H22 | 2 | 133791576 | 133959201 | 0.0511 | 0.0507 | 0.3562 |
| 3737 | RP11-436E24 | 2 | 134654310 | 134835354 | 0.0509 | 0.0495 | 0.3603 |
| 2096 | RP11-355I13 | 2 | 136875551 | 137087299 | 0.0515 | 0.0385 | 0.3486 |
| 3943 | RP11-355I13 | 2 | 136875551 | 137087299 | 0.0515 | 0.0385 | 0.3486 |

(continued)

| Order | Clone | chrom | Start | End | sc_control | sc_BRCA1 | sd |
|---|---|---|---|---|---|---|---|
| 858 | RP11-414I8 | 2 | 137366984 | 137538813 | 0.0613 | 0.0482 | 0.3541 |
| 113 | RP11-58C7 | 2 | 138125168 | 138212379 | 0.0613 | 0.0482 | 0.3541 |
| 2964 | RP11-12M21 | 2 | 139529620 | 139695761 | 0.0613 | 0.0482 | 0.3541 |
| 1252 | RP11-23H14 | 2 | 160688277 | 160856293 | 0.1116 | 0.1150 | 0.3743 |
| 4285 | RP11-440P12 | 2 | 161596464 | 161768144 | 0.1113 | 0.1408 | 0.3670 |
| 2383 | RP11-576I16 | 2 | 162682902 | 162846382 | 0.1113 | 0.1408 | 0.3670 |
| 798 | RP11-5J4 | 2 | 164165855 | 164240910 | 0.1112 | 0.1479 | 0.3650 |
| 4221 | RP11-218M2 | 2 | 165772205 | 165838175 | 0.1112 | 0.1479 | 0.3650 |
| 2953 | RP11-218 | 2 | 166510094 | 166698159 | 0.1110 | 0.1384 | 0.3591 |
| 2992 | RP11-125M15 | 2 | 167445843 | 167642208 | 0.1115 | 0.1469 | 0.3719 |
| 784 | RP11-11N16 | 2 | 168899618 | 169089882 | 0.1115 | 0.1469 | 0.3719 |
| 3481 | RP11-285F23 | 2 | 169206756 | 169395118 | 0.1115 | 0.1469 | 0.3719 |
| 1832 | RP11-141H20 | 2 | 169789426 | 169953565 | 0.1118 | 0.1542 | 0.3785 |
| 221 | RP11-551O2 | 2 | 169823899 | 169935265 | 0.1118 | 0.1542 | 0.3785 |
| 3623 | RP11-551O2 | 2 | 169823899 | 169935265 | 0.1118 | 0.1542 | 0.3785 |
| 3012 | RP11-389F18 | 2 | 170471086 | 170690261 | 0.1120 | 0.1559 | 0.3828 |
| 2011 | RP11-244E6 | 2 | 171080925 | 171261146 | 0.1120 | 0.1612 | 0.3824 |
| 3316 | RP11-176L20 | 2 | 172134584 | 172269397 | 0.1120 | 0.1612 | 0.3824 |
| 3942 | RP11-343H10 | 2 | 173168753 | 173325586 | 0.1126 | 0.1503 | 0.3949 |
| 1154 | RP11-158L8 | 2 | 173997059 | 174130810 | 0.1126 | 0.1503 | 0.3949 |
| 2718 | RP11-483E17 | 2 | 175049767 | 175231429 | 0.1140 | 0.1658 | 0.4258 |
| 3684 | RP11-205B19 | 2 | 175978396 | 176145371 | 0.1376 | 0.1658 | 0.4310 |
| 1713 | RP11-157E8 | 2 | 177052080 | 177205498 | 0.1376 | 0.1658 | 0.4310 |
| 2031 | RP11-250N10 | 2 | 178079879 | 178252093 | 0.1370 | 0.1576 | 0.4168 |
| 2802 | RP11-65L3 | 2 | 178966384 | 179139011 | 0.1368 | 0.1619 | 0.4126 |
| 1901 | RP11-535B12 | 2 | 179945353 | 180115969 | 0.1363 | 0.1588 | 0.4021 |
| 301 | RP11-391P1 | 2 | 180331396 | 180481646 | 0.1363 | 0.1588 | 0.4021 |
| 2720 | RP11-504G5 | 2 | 181817089 | 181987870 | 0.1363 | 0.1532 | 0.4028 |
| 421 | RP 11-3 8H6 | 2 | 183145945 | 183309874 | 0.1363 | 0.1532 | 0.4028 |
| 2938 | RP11-571N13 | 2 | 184429689 | 184467238 | 0.1363 | 0.1532 | 0.4028 |
| 1784 | RP11-363K21 | 2 | 185140061 | 185332568 | 0.1357 | 0.1673 | 0.3878 |
| 1830 | RP11-29E4 | 2 | 185796056 | 186001245 | 0.1360 | 0.1490 | 0.3962 |
| 857 | RP11-400O18 | 2 | 186322693 | 186426356 | 0.1496 | 0.1521 | 0.4013 |
| 839 | RP11-372A6 | 2 | 187410393 | 187617316 | 0.1290 | 0.1631 | 0.3818 |
| 4249 | RP11-372A6 | 2 | 187410393 | 187617316 | 0.1290 | 0.1631 | 0.3818 |
| 2980 | RP11-123G24 | 2 | 188197232 | 188357186 | 0.1290 | 0.1631 | 0.3818 |
| 1680 | RP11-172N20 | 2 | 188855759 | 189025686 | 0.1290 | 0.1631 | 0.3818 |

(continued)

| Order | Clone | chrom | Start | End | sc_control | sc_BRCA1 | sd |
|---|---|---|---|---|---|---|---|
| 2118 | RP11-455J20 | 2 | 190280560 | 190364670 | 0.1427 | 0.1735 | 0.3822 |
| 3609 | RP11-1021H16 | 2 | 190364671 | 190468806 | 0.1420 | 0.1669 | 0.3673 |
| 2074 | RP 11-235L22 | 2 | 190468807 | 190574136 | 0.1420 | 0.1669 | 0.3673 |
| 2030 | RP11-284E5 | 2 | 191161960 | 191354774 | 0.1420 | 0.1669 | 0.3673 |
| 649 | RP11-59L22 | 2 | 192914919 | 193076759 | 0.1421 | 0.1617 | 0.3703 |
| 3944 | RP11-363G3 | 2 | 193770313 | 193936253 | 0.1421 | 0.1617 | 0.3703 |
| 2401 | RP11-1A1 | 2 | 195231805 | 195411755 | 0.1415 | 0.1519 | 0.3572 |
| 3751 | RP11-1A1 | 2 | 195231805 | 195411755 | 0.1415 | 0.1519 | 0.3572 |
| 1157 | RP11-149P12 | 2 | 195826913 | 195994198 | 0.1409 | 0.1424 | 0.3438 |
| 1789 | RP11-15J24 | 2 | 205174820 | 205347296 | 0.1091 | 0.1047 | 0.3471 |
| 1233 | RP11-325M10 | 2 | 206054358 | 206242420 | 0.1091 | 0.1047 | 0.3471 |
| 553 | RP11-470J24 | 2 | 206846958 | 206994419 | 0.1089 | 0.1067 | 0.3513 |
| 841 | RP11-396J8 | 2 | 210627910 | 210820013 | 0.1315 | 0.1185 | 0.3884 |
| 4030 | RP11-300D24 | 2 | 211890302 | 212037638 | 0.1313 | 0.1148 | 0.3914 |
| 769 | RP11-560C24 | 2 | 213160973 | 213345198 | 0.1318 | 0.1168 | 0.3820 |
| 3662 | RP11-105N14 | 2 | 213713153 | 213872184 | 0.1318 | 0.1168 | 0.3820 |
| 3710 | RP11-427F22 | 2 | 214750215 | 214905472 | 0.1162 | 0.0810 | 0.3587 |
| 4272 | RP11-427F22 | 2 | 214750215 | 214905472 | 0.1162 | 0.0810 | 0.3587 |
| 1179 | RP11-307A11 | 2 | 217064016 | 217224225 | 0.1163 | 0.0620 | 0.3568 |
| 1351 | RP11-506C8 | 2 | 217224025 | 217403884 | 0.1163 | 0.0620 | 0.3568 |
| 3413 | RP11-423F9 | 2 | 217797627 | 218010176 | 0.1157 | 0.0707 | 0.3689 |
| 2456 | RP11-378A13 | 2 | 218827093 | 219018647 | 0.1153 | 0.0561 | 0.3784 |
| 4063 | RP11-512O7 | 2 | 220236911 | 220431668 | 0.1159 | 0.0512 | 0.3655 |
| 1570 | RP11-444B5 | 2 | 221335168 | 221420064 | 0.1159 | 0.0512 | 0.3655 |
| 119 | RP11-71J24 | 2 | 222493290 | 222674059 | 0.1159 | 0.0512 | 0.3655 |
| 3289 | RP11-397D2 | 2 | 222855303 | 223012639 | 0.1159 | 0.0512 | 0.3655 |
| 250 | RP11-3122 | 2 | 223189276 | 223293801 | 0.1159 | 0.0512 | 0.3655 |
| 2381 | RP11-551D18 | 2 | 224018057 | 224147409 | 0.1159 | 0.0512 | 0.3655 |
| 3512 | RP11-516C4 | 2 | 225556155 | 225739388 | 0.1159 | 0.0512 | 0.3655 |
| 1597 | RP11-86O17 | 2 | 226629534 | 226739370 | 0.1151 | 0.0628 | 0.3815 |
| 3655 | RP11-86O17 | 2 | 226629534 | 226739370 | 0.1151 | 0.0628 | 0.3815 |
| 4236 | RP11-211K17 | 2 | 227039788 | 227203287 | 0.1155 | 0.0435 | 0.3728 |
| 3317 | RP11-149O3 | 2 | 228366695 | 228540039 | 0.1165 | 0.0251 | 0.3505 |
| 107 | RP11-70L16 | 2 | 229316582 | 229434960 | 0.0954 | 0.0251 | 0.3519 |
| 4290 | RP11-457P23 | 2 | 230386956 | 230587338 | 0.0547 | 0.0252 | 0.3581 |
| 4271 | RP 11-419H23 | 2 | 231787899 | 231964428 | 0.0543 | 0.0119 | 0.3671 |
| 978 | RP11-52C8 | 2 | 232425628 | 232539910 | 0.0392 | 0.0119 | 0.3693 |

(continued)

| Order | Clone | chrom | Start | End | sc_control | sc_BRCA1 | sd |
|-------|-------|-------|-------|-----|------------|----------|-----|
| 2148 | RP11-52C8 | 2 | 232425628 | 232539910 | 0.0392 | 0.0119 | 0.3693 |
| 1512 | RP11-91N19 | 2 | 232987280 | 233143792 | 0.0390 | 0.0006 | 0.3751 |
| 3611 | RP11-534J17 | 2 | 233993547 | 234101158 | 0.0387 | 0.0082 | 0.3818 |
| 4057 | RP11-534J17 | 2 | 233993547 | 234101158 | 0.0387 | 0.0082 | 0.3818 |
| 1477 | RP11-263G22 | 2 | 234593055 | 234701766 | 0.0387 | 0.0082 | 0.3818 |
| 4314 | RP11-263G22 | 2 | 234593055 | 234701766 | 0.0387 | 0.0082 | 0.3818 |
| 2816 | RP11-84G18 | 2 | 235987110 | 236175494 | 0.0378 | -0.0116 | 0.4011 |
| 2168 | RP11-497D24 | 2 | 238251662 | 238463736 | 0.0376 | -0.0350 | 0.4053 |
| 3953 | RP11-15L18 | 2 | 238922713 | 239091229 | -0.0201 | -0.0578 | 0.4145 |
| 1894 | RP11-546M8 | 2 | 239997825 | 240140444 | -0.0201 | -0.0578 | 0.4145 |
| 9 | RP11-463B12 | 2 | 240559949 | 240731368 | -0.0201 | -0.0578 | 0.4145 |
| 1381 | GS-1011-O17 | 2 | 242636149 | 242786149 | -0.2528 | -0.2307 | 0.4603 |
| 3536 | RG-172-I13 | 2 | 242636199 | 242786199 | -0.2528 | -0.2307 | 0.4603 |
| 3545 | GS-1186-B18 | 3 | 225000 | 375000 | -0.0486 | -0.0119 | 0.5295 |
| 660 | RP11-86C13 | 3 | 768338 | 958276 | -0.0546 | -0.0427 | 0.3985 |
| 3661 | RP11-97C16 | 3 | 3117275 | 3274532 | -0.0546 | -0.0427 | 0.3985 |
| 4216 | RP11-95E11 | 3 | 3134172 | 3304965 | -0.0546 | -0.0427 | 0.3985 |
| 2404 | RP11-10H6 | 3 | 3156042 | 3326580 | -0.0546 | -0.0427 | 0.3985 |
| 67 | RP11-183N22 | 3 | 4492889 | 4493650 | -0.0546 | -0.0427 | 0.3985 |
| 4242 | RP11-238A9 | 3 | 4562897 | 4652965 | -0.0546 | -0.0427 | 0.3985 |
| 2445 | RP11-129J10 | 3 | 5125758 | 5180955 | -0.0546 | -0.0427 | 0.3985 |
| 2537 | RP11-277D17 | 3 | 6595704 | 6636734 | -0.0546 | -0.0322 | 0.3974 |
| 1714 | RP11-167K17 | 3 | 6749027 | 6942291 | -0.0546 | -0.0322 | 0.3974 |
| 3625 | CTB-1114A6 | 3 | 7498176 | 7537606 | -0.0546 | -0.0322 | 0.3974 |
| 3929 | RP11-329A2 | 3 | 7634301 | 7760384 | -0.0548 | -0.0238 | 0.3930 |
| 3658 | RP11-105K13 | 3 | 8869825 | 9037105 | -0.0547 | -0.0206 | 0.3955 |
| 2113 | RP11-439F4 | 3 | 9803852 | 9993473 | -0.0546 | -0.0161 | 0.3976 |
| 2093 | RP11-382A21 | 3 | 10066548 | 10067175 | -0.0530 | 0.0113 | 0.4325 |
| 3030 | RP11-382A21 | 3 | 10066548 | 10067175 | -0.0530 | 0.0113 | 0.4325 |
| 1739 | RP11-438J1 | 3 | 10137483 | 10347535 | -0.0544 | -0.0084 | 0.4035 |
| 2044 | RP11-94A14 | 3 | 10613350 | 10788431 | -0.0544 | -0.0084 | 0.4035 |
| 1804 | RP11-25C10 | 3 | 12420266 | 12590814 | -0.0534 | 0.0269 | 0.4247 |
| 3346 | RP11-163D23 | 3 | 12590779 | 12735990 | -0.0534 | 0.0269 | 0.4247 |
| 1698 | RP11-165B2 | 3 | 13797747 | 13964637 | -0.0534 | 0.0408 | 0.4246 |
| 1336 | RP11-316A10 | 3 | 14886274 | 15046975 | -0.0545 | 0.0213 | 0.4013 |
| 1335 | RP 11-305L22 | 3 | 15628045 | 15764879 | -0.0545 | 0.0213 | 0.4013 |
| 1488 | RP11-255O19 | 3 | 15780358 | 15940642 | -0.0545 | 0.0213 | 0.4013 |

(continued)

| Order | Clone | chrom | Start | End | sc_control | sc_BRCA1 | sd |
|---|---|---|---|---|---|---|---|
| 2280 | RP11-80D24 | 3 | 17181366 | 17345270 | -0.0546 | 0.0271 | 0.3974 |
| 259 | RP11-8D23 | 3 | 17814951 | 17960794 | -0.0546 | 0.0271 | 0.3974 |
| 961 | RP 11-44M22 | 3 | 19336535 | 19494336 | -0.0546 | 0.0271 | 0.3974 |
| 955 | RP 11-27J5 | 3 | 20636015 | 20828584 | -0.0546 | 0.0271 | 0.3974 |
| 1176 | RP11-180N14 | 3 | 21314358 | 21484066 | -0.0354 | 0.0272 | 0.3928 |
| 3434 | RP11-16E8 | 3 | 21574260 | 21742237 | -0.0354 | 0.0272 | 0.3928 |
| 1540 | RP11-208G16 | 3 | 22372629 | 22514821 | -0.0354 | 0.0272 | 0.3928 |
| 2043 | RP11-89F18 | 3 | 23404804 | 23575751 | -0.0354 | 0.0272 | 0.3928 |
| 1266 | RP11-18L17 | 3 | 24167259 | 24311442 | -0.0357 | 0.0359 | 0.3867 |
| 4287 | RP11-421F9 | 3 | 25447987 | 25518546 | -0.0356 | 0.0480 | 0.3885 |
| 4241 | RP11-226E22 | 3 | 26718003 | 26882790 | -0.0346 | 0.0301 | 0.4103 |
| 789 | RP 11-2G22 | 3 | 27349564 | 27531283 | -0.0337 | 0.0427 | 0.4295 |
| 2420 | RP11-11L6 | 3 | 28645912 | 28824358 | -0.0346 | 0.0310 | 0.4107 |
| 1185 | RP11-30G4 | 3 | 28813263 | 28966852 | -0.0345 | 0.0355 | 0.4113 |
| 2049 | RP11-103N21 | 3 | 29910388 | 30018478 | -0.0346 | 0.0329 | 0.4099 |
| 658 | RP11-69K20 | 3 | 30275511 | 30447593 | -0.0346 | 0.0329 | 0.4099 |
| 1177 | RP11-7I16 | 3 | 30704315 | 30705070 | -0.0347 | 0.0142 | 0.4066 |
| 438 | RP11-48E16 | 3 | 31634821 | 31812073 | -0.0259 | 0.0272 | 0.4072 |
| 4056 | RP11-524O15 | 3 | 32472740 | 32650842 | -0.0255 | 0.0483 | 0.4155 |
| 109 | RP11-56P22 | 3 | 34981197 | 35131137 | -0.0255 | 0.0483 | 0.4155 |
| 308 | RP11-380G10 | 3 | 35590144 | 35766135 | -0.0255 | 0.0483 | 0.4155 |
| 2977 | RP11-134C18 | 3 | 36562366 | 36744618 | -0.0265 | 0.0615 | 0.3951 |
| 4293 | RP11-134C18 | 3 | 36562366 | 36744618 | -0.0265 | 0.0615 | 0.3951 |
| 3897 | RP11-129K12 | 3 | 36866838 | 37036150 | -0.0265 | 0.0615 | 0.3951 |
| 2707 | RP11-491D6 | 3 | 37036151 | 37136521 | -0.0265 | 0.0615 | 0.3951 |
| 1777 | RP11-331G2 | 3 | 39137073 | 39311657 | -0.0266 | 0.0767 | 0.3915 |
| 2806 | RP11-66G8 | 3 | 39724379 | 39836467 | -0.0266 | 0.0767 | 0.3915 |
| 1580 | RP11-424L2 | 3 | 40986834 | 41155572 | -0.0266 | 0.0767 | 0.3915 |
| 2105 | RP11-424L2 | 3 | 40986834 | 41155572 | -0.0266 | 0.0767 | 0.3915 |
| 1353 | RP11-527M19 | 3 | 41172081 | 41354719 | -0.0266 | 0.0767 | 0.3915 |
| 3569 | RP4-613B23 | 3 | 42629196 | 42784970 | -0.0271 | 0.0508 | 0.3815 |
| 3758 | RP11-189H19 | 3 | 44567485 | 44737886 | -0.0274 | 0.0427 | 0.3748 |
| 3876 | RP11-189H19 | 3 | 44567485 | 44737886 | -0.0274 | 0.0427 | 0.3748 |
| 2431 | RP11-111P21 | 3 | 45438197 | 45497978 | -0.0263 | 0.0583 | 0.3999 |
| 1504 | RP11-91E8 | 3 | 46036466 | 46093400 | -0.0731 | -0.0103 | 0.4072 |
| 1948 | RP4-544D10 | 3 | 47295529 | 47384745 | -0.0068 | 0.0104 | 0.3893 |
| 2741 | RP11-88B8 | 3 | 47860083 | 47993747 | -0.0068 | 0.0104 | 0.3893 |

(continued)

| Order | Clone | chrom | Start | End | sc_ control | sc_ BRCA1 | sd |
|---|---|---|---|---|---|---|---|
| 2504 | RP1-289H18 | 3 | 48080483 | 48208886 | -0.0068 | 0.0104 | 0.3893 |
| 3424 | RP11-24C3 | 3 | 48357034 | 48512503 | -0.0068 | 0.0104 | 0.3893 |
| 242 | RP11-3B7 | 3 | 49256500 | 49393338 | -0.0068 | 0.0104 | 0.3893 |
| 108 | RP11-78O10 | 3 | 49843918 | 50006856 | -0.0069 | -0.0042 | 0.3880 |
| 2727 | RP11-314A5 | 3 | 51791953 | 51927169 | -0.0250 | -0.0233 | 0.3907 |
| 2728 | RP 11-447A21 | 3 | 52556292 | 52743025 | -0.0250 | -0.0233 | 0.3907 |
| 1030 | RP5-966M1 | 3 | 52743026 | 52839812 | -0.0248 | -0.0123 | 0.3946 |
| 630 | RP11-122d19 | 3 | 54007389 | 54188449 | -0.0247 | -0.0169 | 0.3959 |
| 2972 | RP11-122D19 | 3 | 54007389 | 54188449 | -0.0247 | -0.0169 | 0.3959 |
| 2397 | RP11-554O1 | 3 | 54623583 | 54778937 | -0.0247 | -0.0169 | 0.3959 |
| 2801 | RP11-58O15 | 3 | 55610036 | 55797581 | -0.0250 | -0.0071 | 0.3900 |
| 276 | RP11-120C2 | 3 | 57340288 | 57375009 | -0.0250 | -0.0032 | 0.3892 |
| 3694 | RP11-229A12 | 3 | 57370434 | 57534933 | -0.0250 | -0.0032 | 0.3892 |
| 120 | RP11-80H18 | 3 | 58149203 | 58327212 | -0.0253 | 0.0068 | 0.3834 |
| 1695 | RP11-170K19 | 3 | 59701342 | 59875950 | -0.0256 | -0.0009 | 0.3778 |
| 2786 | RP11-94d19 | 3 | 60756896 | 60927079 | -0.0256 | -0.0009 | 0.3778 |
| 4208 | RP11-94D19 | 3 | 60756896 | 60927079 | -0.0256 | -0.0009 | 0.3778 |
| 2487 | RP11-154D3 | 3 | 62016747 | 62182595 | -0.0258 | 0.0040 | 0.3736 |
| 1606 | RP11-204J18 | 3 | 62190612 | 62347194 | -0.0259 | -0.0078 | 0.3715 |
| 2079 | RP11-204J18 | 3 | 62190612 | 62347194 | -0.0259 | -0.0078 | 0.3715 |
| 1518 | RP11-108A8 | 3 | 63254921 | 63396321 | -0.0260 | 0.0025 | 0.3675 |
| 783 | RP 11-9A 1 | 3 | 64198694 | 64357759 | -0.0260 | 0.0103 | 0.3678 |
| 3960 | RP11-24O17 | 3 | 66890138 | 67018894 | -0.0563 | 0.0216 | 0.3867 |
| 2272 | RP11-79C12 | 3 | 68008697 | 68146379 | -0.0563 | 0.0216 | 0.3867 |
| 3640 | RP11-253K11 | 3 | 68472738 | 68646025 | -0.0563 | 0.0216 | 0.3867 |
| 3333 | RP11-152N21 | 3 | 69417957 | 69541169 | -0.0560 | 0.0420 | 0.3939 |
| 2432 | RP11-118O11 | 3 | 70741634 | 70792905 | -0.0560 | 0.0420 | 0.3939 |
| 3861 | RP11-154H23 | 3 | 71622956 | 71816508 | -0.0560 | 0.0339 | 0.3941 |
| 3532 | RP11-522N9 | 3 | 72457282 | 72633500 | -0.0564 | 0.0525 | 0.3848 |
| 1476 | RP11-252O10 | 3 | 73463946 | 73612108 | -0.0564 | 0.0543 | 0.3853 |
| 1802 | RP 11-20B7 | 3 | 73854898 | 74026003 | -0.0564 | 0.0543 | 0.3853 |
| 260 | RP11-11L10 | 3 | 74234096 | 74391234 | -0.0564 | 0.0543 | 0.3853 |
| 4071 | RP11-514J23 | 3 | 76310308 | 76477952 | -0.0562 | 0.0730 | 0.3885 |
| 4229 | RP11-220O14 | 3 | 77652537 | 77654544 | -0.0320 | 0.0860 | 0.3908 |
| 1809 | RP11-16M12 | 3 | 78395760 | 78564986 | 0.0076 | 0.0944 | 0.4012 |
| 1785 | RP11-333I20 | 3 | 78932852 | 79097134 | 0.0078 | 0.1077 | 0.4042 |
| 3720 | RP11-333I20 | 3 | 78932852 | 79097134 | 0.0078 | 0.1077 | 0.4042 |

(continued)

| Order | Clone | chrom | Start | End | sc_ control | sc_ BRCA1 | sd |
|---|---|---|---|---|---|---|---|
| 423 | RP11-26H10 | 3 | 80133749 | 80297117 | 0.0071 | 0.0928 | 0.3906 |
| 1367 | RP11-510B7 | 3 | 80611850 | 80795918 | 0.0071 | 0.0928 | 0.3906 |
| 4289 | RP 11-442C9 | 3 | 81649526 | 81740108 | 0.0069 | 0.0886 | 0.3857 |
| 3706 | RP11-425D6 | 3 | 81649532 | 81826325 | 0.0069 | 0.0886 | 0.3857 |
| 3748 | RP11-425D6 | 3 | 81649532 | 81826325 | 0.0069 | 0.0886 | 0.3857 |
| 1528 | RP11-206J21 | 3 | 82657795 | 82799473 | 0.0314 | 0.0936 | 0.3806 |
| 2476 | RP11-382L10 | 3 | 83811935 | 83922050 | 0.0311 | 0.1029 | 0.3752 |
| 2721 | RP11-474M18 | 3 | 84623842 | 84801874 | 0.0311 | 0.1029 | 0.3752 |
| 3750 | RP 11-447J 13 | 3 | 86090396 | 86268611 | 0.0308 | 0.0984 | 0.3692 |
| 640 | RP11-81P15 | 3 | 87044531 | 87223753 | 0.0308 | 0.0984 | 0.3692 |
| 2773 | RP11-81p15 | 3 | 87044531 | 87223753 | 0.0308 | 0.0984 | 0.3692 |
| 4039 | RP11-312H1 | 3 | 87569839 | 87730259 | 0.0308 | 0.0984 | 0.3692 |
| 1511 | RP11-8817 | 3 | 95519622 | 95706420 | 0.1107 | 0.1335 | 0.3578 |
| 788 | RP11-12A13 | 3 | 96093190 | 96250469 | 0.1107 | 0.1335 | 0.3578 |
| 796 | RP11-12J13 | 3 | 97024240 | 97176860 | 0.0750 | 0.1229 | 0.3843 |
| 3521 | RP11-529P9 | 3 | 98524225 | 98714854 | 0.0744 | 0.1391 | 0.3704 |
| 3685 | RP11-214N20 | 3 | 99258539 | 99288219 | 0.0744 | 0.1391 | 0.3704 |
| 2448 | RP11-121C1 | 3 | 100490741 | 100545594 | 0.0744 | 0.1391 | 0.3704 |
| 2443 | RP11-114I8 | 3 | 101480701 | 101632377 | 0.1208 | 0.1276 | 0.3789 |
| 3122 | RP11-4914 | 3 | 102993643 | 103144288 | 0.1214 | 0.1252 | 0.3933 |
| 562 | RP11-484119 | 3 | 103202967 | 103378300 | 0.1214 | 0.1252 | 0.3933 |
| 2094 | RP11-484119 | 3 | 103202967 | 103378300 | 0.1214 | 0.1252 | 0.3933 |
| 291 | RP11-115B22 | 3 | 105815245 | 105971443 | 0.1226 | 0.1375 | 0.4189 |
| 3664 | RP11-91B3 | 3 | 106787071 | 106966499 | 0.1226 | 0.1375 | 0.4189 |
| 2378 | RP11-561O4 | 3 | 108272967 | 108465288 | 0.1226 | 0.1375 | 0.4189 |
| 4204 | RP11-93N14 | 3 | 110118060 | 110272409 | 0.1226 | 0.1375 | 0.4189 |
| 1329 | RP11-286P15 | 3 | 111576106 | 111691384 | 0.1389 | 0.1466 | 0.4177 |
| 3855 | RP11-169N13 | 3 | 120928481 | 121091573 | 0.1343 | 0.1136 | 0.4165 |
| 3881 | RP11-73F12 | 3 | 121091573 | 121205101 | 0.1345 | 0.1207 | 0.4119 |
| 4156 | RP11-767L7 | 3 | 121148280 | 121326590 | 0.1345 | 0.1207 | 0.4119 |
| 1240 | RP11-359H3 | 3 | 121653170 | 121848109 | 0.1345 | 0.1207 | 0.4119 |
| 2182 | RP11-480B17 | 3 | 122380934 | 122541241 | 0.1345 | 0.1207 | 0.4119 |
| 4026 | RP 11-299J3 | 3 | 123486719 | 123632323 | 0.1345 | 0.1246 | 0.4113 |
| 2122 | RP11-457E6 | 3 | 123901753 | 124000617 | 0.1345 | 0.1246 | 0.4113 |
| 791 | RP11-9N20 | 3 | 124855434 | 125034609 | 0.1346 | 0.1039 | 0.4084 |
| 3528 | RP11-521J5 | 3 | 125238538 | 125434264 | 0.1348 | 0.0978 | 0.4046 |
| 2279 | RP11-71H17 | 3 | 125910478 | 126076548 | 0.1348 | 0.0978 | 0.4046 |

(continued)

| Order | Clone | chrom | Start | End | sc_control | sc_BRCA1 | sd |
|-------|-------|-------|-------|-----|-----------|----------|-----|
| 1520 | RP 11-205A6 | 3 | 127434149 | 127483270 | 0.1346 | 0.1175 | 0.4093 |
| 1868 | RP11-321A2 | 3 | 127987691 | 128108621 | 0.1347 | 0.1115 | 0.4072 |
| 645 | RP11-59J16 | 3 | 128599460 | 128752045 | 0.1347 | 0.1115 | 0.4072 |
| 2069 | RP11-221E20 | 3 | 129993751 | 130058432 | 0.1481 | 0.0984 | 0.4111 |
| 285 | RP11-129J11 | 3 | 131459233 | 131616358 | 0.1481 | 0.1157 | 0.4102 |
| 1352 | RP11-517B11 | 3 | 132615008 | 132784649 | 0.1973 | 0.1374 | 0.4063 |
| 3118 | RP11-48F15 | 3 | 133822599 | 133959231 | 0.2272 | 0.1454 | 0.4174 |
| 1508 | RP11-91K8 | 3 | 134620115 | 134771760 | 0.2272 | 0.1454 | 0.4174 |
| 3761 | RP11-91K8 | 3 | 134620115 | 134771760 | 0.2272 | 0.1454 | 0.4174 |
| 384 | RP 11-22E 12 | 3 | 135843620 | 136012651 | 0.2272 | 0.1454 | 0.4174 |
| 3676 | RP11-91O5 | 3 | 136539146 | 136692406 | 0.2274 | 0.1503 | 0.4121 |
| 1871 | RP11-311K2 | 3 | 137747908 | 137748612 | 0.2426 | 0.2230 | 0.4597 |
| 4263 | RP11-311K2 | 3 | 137747908 | 137748612 | 0.2426 | 0.2230 | 0.4597 |
| 4185 | RP11-269A14 | 3 | 138231890 | 138416930 | 0.2446 | 0.1863 | 0.4169 |
| 2421 | RP 11-2A4 | 3 | 138893123 | 139048143 | 0.2448 | 0.2033 | 0.4119 |
| 2673 | RP11-80p20 | 3 | 139548619 | 139696434 | 0.2446 | 0.2096 | 0.4160 |
| 2084 | RP11-349D24 | 3 | 140626727 | 140627481 | 0.2446 | 0.2096 | 0.4160 |
| 3399 | RP11-349D24 | 3 | 140626727 | 140627481 | 0.2446 | 0.2096 | 0.4160 |
| 2039 | RP11-89E16 | 3 | 141265360 | 141410458 | 0.2446 | 0.2096 | 0.4160 |
| 2062 | RP11-231L11 | 3 | 142174414 | 142328307 | 0.2445 | 0.1988 | 0.4198 |
| 4234 | RP11-235I18 | 3 | 143078415 | 143237513 | 0.2445 | 0.2043 | 0.4195 |
| 80 | RP11-160a13 | 3 | 144154813 | 144312325 | 0.2572 | 0.2351 | 0.4346 |
| 3322 | RP11-160A13 | 3 | 144154813 | 144312325 | 0.2572 | 0.2351 | 0.4346 |
| 3866 | RP11-165M11 | 3 | 144868248 | 144912572 | 0.2572 | 0.2351 | 0.4346 |
| 2059 | RP11-200O17 | 3 | 145885183 | 146013682 | 0.2572 | 0.2351 | 0.4346 |
| 1507 | RP11-88H10 | 3 | 147013411 | 147184823 | 0.2572 | 0.2351 | 0.4346 |
| 368 | RP11-21M4 | 3 | 147739246 | 147851434 | 0.2568 | 0.2518 | 0.4427 |
| 16 | RP11-500K7 | 3 | 149208178 | 149208692 | 0.2568 | 0.2518 | 0.4427 |
| 2073 | RP11-223L18 | 3 | 154955751 | 155086892 | 0.2458 | 0.2562 | 0.4413 |
| 827 | RP11-117L15 | 3 | 156644066 | 156810056 | 0.2458 | 0.2562 | 0.4413 |
| 4218 | RP11-111F10 | 3 | 157115243 | 157286192 | 0.2458 | 0.2562 | 0.4413 |
| 243 | RP11-6F2 | 3 | 158177800 | 158323315 | 0.2103 | 0.2562 | 0.4387 |
| 94 | RP 11-209h21 | 3 | 160726423 | 160903086 | 0.2101 | 0.2684 | 0.4348 |
| 2064 | RP 11-209H21 | 3 | 160726423 | 160903086 | 0.2101 | 0.2684 | 0.4348 |
| 3770 | RP11-209H21 | 3 | 160726423 | 160903086 | 0.2101 | 0.2684 | 0.4348 |
| 646 | RP 11-67F24 | 3 | 161144699 | 161320806 | 0.2097 | 0.2708 | 0.4250 |
| 2172 | RP11-498P15 | 3 | 163532324 | 163648094 | 0.3034 | 0.3100 | 0.4524 |

(continued)

| Order | Clone | chrom | Start | End | sc_control | sc_BRCA1 | sd |
|---|---|---|---|---|---|---|---|
| 1593 | RP11-81E19 | 3 | 166072602 | 166237067 | 0.3034 | 0.3100 | 0.4524 |
| 2800 | RP11-81E19 | 3 | 166072602 | 166237067 | 0.3034 | 0.3100 | 0.4524 |
| 1166 | RP11-163H6 | 3 | 173183497 | 173348027 | 0.3548 | 0.3673 | 0.4623 |
| 2593 | RP 11-44A 1 | 3 | 173688016 | 173855765 | 0.3548 | 0.3673 | 0.4623 |
| 2930 | RP11-569P10 | 3 | 174372436 | 174549467 | 0.3548 | 0.3673 | 0.4623 |
| 2 | RP11-477P16 | 3 | 175307349 | 175497951 | 0.3548 | 0.3673 | 0.4623 |
| 4246 | RP11-477P16 | 3 | 175307349 | 175497951 | 0.3548 | 0.3673 | 0.4623 |
| 115 | RP11-71G7 | 3 | 177686929 | 177849652 | 0.3551 | 0.3776 | 0.4694 |
| 2722 | RP11-484D18 | 3 | 178148582 | 178318214 | 0.3551 | 0.3776 | 0.4694 |
| 3672 | RP11-91K9 | 3 | 178971537 | 179129175 | 0.3551 | 0.3776 | 0.4694 |
| 1456 | RP11-682A21 | 3 | 180293412 | 180449253 | 0.3551 | 0.3776 | 0.4694 |
| 3137 | RP11-45I24 | 3 | 180867439 | 181028448 | 0.3549 | 0.3655 | 0.4658 |
| 1447 | RP11-534H15 | 3 | 181435699 | 181436285 | 0.3551 | 0.3565 | 0.4701 |
| 1893 | RP11-534H15 | 3 | 181435699 | 181436285 | 0.3551 | 0.3565 | 0.4701 |
| 638 | RP11-65J14 | 3 | 187299591 | 187370245 | 0.3122 | 0.3086 | 0.4639 |
| 272 | RP11-119E13 | 3 | 188090387 | 188091023 | 0.3111 | 0.2925 | 0.4868 |
| 1896 | RP11-525C11 | 3 | 192500451 | 192584204 | 0.2681 | 0.2401 | 0.5826 |
| 2590 | RP 11-47G2 | 3 | 193748157 | 193916452 | 0.2505 | 0.2586 | 0.5198 |
| 105 | RP11-56C4 | 3 | 193972699 | 194023813 | 0.2504 | 0.2540 | 0.5194 |
| 2549 | RP11-279P10 | 3 | 195537798 | 195629784 | 0.2505 | 0.2592 | 0.5197 |
| 1355 | RP11-506F8 | 3 | 196614673 | 196768568 | 0.2505 | 0.2786 | 0.5199 |
| 3636 | RP11-252K11 | 3 | 197391807 | 197555990 | 0.2502 | 0.2838 | 0.5148 |
| 279 | RP11-114F20 | 3 | 198339450 | 198528158 | 0.2516 | 0.2833 | 0.5449 |
| 1379 | GS-56-H22 | 3 | 198976827 | 199126827 | 0.2519 | 0.2751 | 0.5505 |
| 1385 | GS-196-F4 | 3 | 198976877 | 199126877 | 0.2519 | 0.2751 | 0.5505 |
| 3416 | RP 11-23 M2 | 3 | 198977374 | 199160103 | 0.2519 | 0.2751 | 0.5505 |
| 3764 | RP 11-23 M2 | 3 | 198977374 | 199160103 | 0.2519 | 0.2751 | 0.5505 |
| 3549 | GS-36-P21 | 4 | 15000 | 165000 | -0.0394 | -0.0302 | 0.3882 |
| 3543 | GS-118-B13 | 4 | 26000 | 176000 | -0.0394 | -0.0302 | 0.3882 |
| 4193 | RP11-270I3 | 4 | 10640517 | 10803372 | -0.0001 | 0.0028 | 0.3846 |
| 1159 | RP11-168E17 | 4 | 11490644 | 11562120 | -0.0001 | 0.0028 | 0.3846 |
| 2149 | RP11-168E17 | 4 | 11490644 | 11562120 | -0.0001 | 0.0028 | 0.3846 |
| 2540 | RP 11-22A3 | 4 | 12410325 | 12584750 | -0.0002 | 0.0098 | 0.3811 |
| 1582 | RP11-446J8 | 4 | 16019234 | 16183206 | -0.0002 | 0.0098 | 0.3811 |
| 2125 | RP11-441O12 | 4 | 18028014 | 18213766 | -0.0002 | 0.0098 | 0.3811 |
| 2414 | RP11-3J1 | 4 | 18737376 | 18858211 | -0.0005 | 0.0178 | 0.3752 |
| 3755 | RP11-3J1 | 4 | 18737376 | 18858211 | -0.0005 | 0.0178 | 0.3752 |

(continued)

| Order | Clone | chrom | Start | End | sc_ control | sc_ BRCA1 | sd |
|---|---|---|---|---|---|---|---|
| 1776 | RP 11-3 62J 17 | 4 | 20347899 | 20529941 | 0.0131 | 0.0178 | 0.3708 |
| 656 | RP11-84G22 | 4 | 21482302 | 21587657 | 0.0131 | 0.0178 | 0.3708 |
| 3757 | RP11-84G22 | 4 | 21482302 | 21587657 | 0.0131 | 0.0178 | 0.3708 |
| 803 | RP11-10G12 | 4 | 22163781 | 22288928 | 0.0131 | 0.0279 | 0.3704 |
| 2478 | RP11-406E22 | 4 | 22956884 | 22976809 | 0.0131 | 0.0279 | 0.3704 |
| 1356 | RP11-519A11 | 4 | 28748334 | 28909642 | 0.0133 | 0.0158 | 0.3750 |
| 856 | RP11-390C19 | 4 | 29381036 | 29556502 | 0.0133 | 0.0158 | 0.3750 |
| 10 | RP11-478O20 | 4 | 31129762 | 31286231 | 0.0133 | 0.0187 | 0.3761 |
| 389 | RP 11-280K20 | 4 | 31500912 | 31664000 | 0.0133 | 0.0187 | 0.3761 |
| 3707 | RP 11-280K20 | 4 | 31500912 | 31664000 | 0.0133 | 0.0187 | 0.3761 |
| 1806 | RP 11-20M7 | 4 | 32138110 | 32290383 | 0.0134 | 0.0359 | 0.3780 |
| 112 | RP11-79E3 | 4 | 33550507 | 33712878 | 0.0134 | 0.0359 | 0.3780 |
| 1268 | RP11-24H13 | 4 | 34561876 | 34578239 | 0.0134 | 0.0359 | 0.3780 |
| 2981 | RP11-135M12 | 4 | 35206808 | 35398317 | 0.0134 | 0.0359 | 0.3780 |
| 4209 | RP11-103J17 | 4 | 36674852 | 36836152 | 0.0134 | 0.0247 | 0.3775 |
| 3088 | RP11-36B15 | 4 | 37192086 | 37351155 | 0.0134 | 0.0247 | 0.3775 |
| 1560 | RP11-343C9 | 4 | 39430681 | 39629614 | 0.0193 | 0.0292 | 0.3753 |
| 1778 | RP11-343C9 | 4 | 39430681 | 39629614 | 0.0193 | 0.0292 | 0.3753 |
| 3686 | RP11-227F19 | 4 | 41340065 | 41525131 | 0.0193 | 0.0379 | 0.3754 |
| 4206 | RP11-109E24 | 4 | 42330834 | 42508587 | 0.0192 | 0.0466 | 0.3728 |
| 1534 | RP 11-229G4 | 4 | 43182054 | 43332054 | 0.0190 | 0.0358 | 0.3691 |
| 1451 | RP11-542I3 | 4 | 43837854 | 44039573 | 0.0197 | 0.0251 | 0.3830 |
| 3530 | RP11-542I3 | 4 | 43837854 | 44039573 | 0.0197 | 0.0251 | 0.3830 |
| 101 | RP11-55C6 | 4 | 44529011 | 44675684 | 0.0197 | 0.0251 | 0.3830 |
| 1575 | RP 11-416A5 | 4 | 45608185 | 45778656 | 0.0186 | 0.0469 | 0.3608 |
| 2037 | RP11-100N21 | 4 | 47264009 | 47432186 | 0.0186 | 0.0469 | 0.3608 |
| 3735 | RP11-415L23 | 4 | 47677783 | 47787969 | 0.0185 | 0.0545 | 0.3576 |
| 1794 | RP11-19F13 | 4 | 48565289 | 48589289 | 0.0181 | 0.0662 | 0.3495 |
| 2459 | RP11-365H22 | 4 | 52354875 | 52530859 | 0.0611 | 0.0822 | 0.3528 |
| 4245 | RP11-365H22 | 4 | 52354875 | 52530859 | 0.0611 | 0.0822 | 0.3528 |
| 3873 | RP11-157C8 | 4 | 53060046 | 53208038 | 0.0616 | 0.0644 | 0.3647 |
| 2956 | RP11-12J3 | 4 | 54523234 | 54684548 | 0.0616 | 0.0644 | 0.3647 |
| 4222 | RP11-231C18 | 4 | 54822092 | 54952255 | 0.0616 | 0.0644 | 0.3647 |
| 2994 | RP11-148K14 | 4 | 55856180 | 55928233 | 0.0616 | 0.0644 | 0.3647 |
| 4239 | RP11-204H9 | 4 | 64867896 | 65032899 | 0.0376 | 0.0384 | 0.3657 |
| 1576 | RP11-423P14 | 4 | 65766214 | 65968121 | 0.0376 | 0.0384 | 0.3657 |
| 549 | RP11-468N14 | 4 | 69696766 | 69912765 | -0.1156 | 0.0172 | 0.4193 |

(continued)

| Order | Clone | chrom | Start | End | sc_control | sc_BRCA1 | sd |
|---|---|---|---|---|---|---|---|
| 4196 | RP 1 1-92H22 | 4 | 71879338 | 72031750 | 0.0823 | 0.0273 | 0.4239 |
| 249 | RP11-1J11 | 4 | 72265535 | 72403640 | 0.0823 | 0.0273 | 0.4239 |
| 847 | RP 11-373J21 | 4 | 73321374 | 73496366 | 0.0823 | 0.0273 | 0.4239 |
| 1586 | RP11-447E20 | 4 | 74788409 | 74864817 | 0.0823 | 0.0234 | 0.4249 |
| 3426 | RP11-17P8 | 4 | 89621629 | 89807633 | -0.0452 | -0.0607 | 0.3650 |
| 24 | RP11-502A23 | 4 | 90029276 | 90198548 | -0.0452 | -0.0607 | 0.3650 |
| 2971 | RP11-115D19 | 4 | 90755173 | 90922656 | -0.0452 | -0.0607 | 0.3650 |
| 264 | RP11-11N6 | 4 | 91753755 | 91900370 | -0.0452 | -0.0607 | 0.3650 |
| 787 | RP11-9B6 | 4 | 93421437 | 93529296 | -0.0451 | -0.0672 | 0.3615 |
| 3125 | RP11-44P19 | 4 | 93630873 | 93775737 | -0.0482 | -0.0867 | 0.4288 |
| 1801 | RP11-16I17 | 4 | 94662870 | 94836378 | -0.0481 | -0.0947 | 0.4270 |
| 244 | RP11-10L11 | 4 | 96358705 | 96515910 | -0.0452 | -0.0873 | 0.3652 |
| 2092 | RP11-354C17 | 4 | 97073538 | 97124201 | -0.0456 | -0.0815 | 0.3725 |
| 3935 | RP11-354C17 | 4 | 97073538 | 97124201 | -0.0456 | -0.0815 | 0.3725 |
| 3643 | RP11-240J11 | 4 | 98832380 | 98895589 | -0.0456 | -0.0815 | 0.3725 |
| 2112 | RP11-428B4 | 4 | 99946600 | 100058902 | -0.0456 | -0.0815 | 0.3725 |
| 1990 | RP11-56D20 | 4 | 101150764 | 101325336 | -0.0456 | -0.0815 | 0.3725 |
| 911 | RP11-13F20 | 4 | 101510577 | 101670097 | -0.0456 | -0.0815 | 0.3725 |
| 3315 | RP11-167N19 | 4 | 101813198 | 101961364 | -0.0456 | -0.0815 | 0.3725 |
| 2050 | RP11-110C16 | 4 | 103067212 | 103220659 | -0.0456 | -0.0815 | 0.3725 |
| 4273 | RP11-438P8 | 4 | 104406898 | 104431241 | -0.0456 | -0.0815 | 0.3725 |
| 1707 | RP11-174B22 | 4 | 104431242 | 104513122 | -0.0456 | -0.0815 | 0.3725 |
| 292 | RP11-122B24 | 4 | 105288367 | 105355835 | -0.0454 | -0.0867 | 0.3694 |
| 1797 | RP11-16G16 | 4 | 106336237 | 106507303 | -0.0453 | -0.0967 | 0.3663 |
| 4220 | RP11-208O6 | 4 | 107027991 | 107064932 | -0.0457 | -0.0919 | 0.3750 |
| 3667 | RP11-88D10 | 4 | 107736653 | 107943820 | -0.0457 | -0.1002 | 0.3760 |
| 2046 | RP11-109F18 | 4 | 108646932 | 108795103 | -0.0457 | -0.1002 | 0.3760 |
| 651 | RP 11-75N20 | 4 | 109877028 | 110025600 | -0.0457 | -0.1069 | 0.3747 |
| 2273 | RP11-57A22 | 4 | 110057781 | 110225193 | -0.0457 | -0.1069 | 0.3747 |
| 2468 | RP11-380D23 | 4 | 111660447 | 111860073 | -0.0458 | -0.1017 | 0.3777 |
| 1237 | RP11-326N15 | 4 | 112359892 | 112502402 | -0.0458 | -0.1017 | 0.3777 |
| 2264 | RP 11-77N9 | 4 | 112853134 | 112892477 | -0.0467 | -0.1121 | 0.3971 |
| 3753 | RP 11-77N9 | 4 | 112853134 | 112892477 | -0.0467 | -0.1121 | 0.3971 |
| 298 | RP 11-402J6 | 4 | 113599819 | 113777515 | -0.0459 | -0.0928 | 0.3800 |
| 1587 | RP 11-419E3 | 4 | 116321032 | 116470420 | -0.0476 | -0.0672 | 0.4177 |
| 3964 | RP11-25B14 | 4 | 116748169 | 116935082 | -0.0476 | -0.0672 | 0.4177 |
| 2265 | RP11-55L3 | 4 | 117579708 | 117743758 | -0.0477 | -0.0607 | 0.4184 |

(continued)

| Order | Clone | chrom | Start | End | sc_control | sc_BRCA1 | sd |
|---|---|---|---|---|---|---|---|
| 1371 | RP11-510D4 | 4 | 118641765 | 118817790 | -0.0477 | -0.0607 | 0.4184 |
| 1513 | RP11-100C9 | 4 | 119888512 | 120033602 | -0.0467 | -0.0733 | 0.3979 |
| 3018 | RP11-414D7 | 4 | 121243300 | 121302803 | -0.0467 | -0.0733 | 0.3979 |
| 2180 | RP11-501E14 | 4 | 121715814 | 121904034 | -0.0467 | -0.0733 | 0.3979 |
| 1565 | RP11-364P2 | 4 | 122489952 | 122658178 | -0.0467 | -0.0733 | 0.3979 |
| 2455 | RP11-364P2 | 4 | 122489952 | 122658178 | -0.0467 | -0.0733 | 0.3979 |
| 2472 | RP11-381N20 | 4 | 124552339 | 124675489 | -0.0467 | -0.0733 | 0.3979 |
| 4200 | RP11-93I21 | 4 | 125513755 | 125668337 | -0.0467 | -0.0733 | 0.3979 |
| 1793 | RPII-15O17 | 4 | 126354712 | 126517758 | -0.0467 | -0.0826 | 0.3968 |
| 1547 | RP11-282B13 | 4 | 127250441 | 127434004 | -0.0467 | -0.0771 | 0.3966 |
| 4178 | RP11-282B13 | 4 | 127250441 | 127434004 | -0.0467 | -0.0771 | 0.3966 |
| 237 | RP11-555L17 | 4 | 129322994 | 129526144 | -0.0466 | -0.0732 | 0.3948 |
| 2060 | RP11-209D20 | 4 | 130001104 | 130120812 | -0.0466 | -0.0732 | 0.3948 |
| 1604 | RP11-24P18 | 4 | 131614434 | 131767271 | -0.0472 | -0.0613 | 0.4090 |
| 1800 | RP 11-24P 18 | 4 | 131614434 | 131767271 | -0.0472 | -0.0613 | 0.4090 |
| 3488 | RP11-314N14 | 4 | 132653555 | 132677577 | -0.0472 | -0.0613 | 0.4090 |
| 3313 | RP11-149A7 | 4 | 133664206 | 133834624 | -0.0473 | -0.0629 | 0.4098 |
| 3394 | RP11-335K21 | 4 | 134372074 | 134522870 | -0.0473 | -0.0629 | 0.4098 |
| 3017 | RP11-400D2 | 4 | 135464064 | 135638813 | -0.0473 | -0.0629 | 0.4098 |
| 2274 | RP 11-63 M2 | 4 | 138014415 | 138174314 | -0.0473 | -0.0605 | 0.4102 |
| 4268 | RP 11-425J20 | 4 | 138445740 | 138620766 | -0.0473 | -0.0605 | 0.4102 |
| 3665 | RP11-98O2 | 4 | 139829360 | 139995928 | -0.0468 | -0.0769 | 0.3984 |
| 2259 | RP11-69O1 | 4 | 141603563 | 141774290 | -0.0467 | -0.0691 | 0.3964 |
| 3936 | RP 11-3 62F 19 | 4 | 142401187 | 142585401 | -0.0467 | -0.0691 | 0.3964 |
| 3996 | RP11-141I1 | 4 | 143754328 | 143872121 | -0.0463 | -0.0574 | 0.3893 |
| 1788 | RP11-364L4 | 4 | 144367994 | 144531240 | -0.0463 | -0.0574 | 0.3893 |
| 3725 | RP11-364L4 | 4 | 144367994 | 144531240 | -0.0463 | -0.0574 | 0.3893 |
| 1870 | RP11-301H24 | 4 | 146558547 | 146722475 | -0.0467 | -0.0506 | 0.3973 |
| 1696 | RP11-181K12 | 4 | 146929833 | 147087801 | -0.0467 | -0.0506 | 0.3973 |
| 1584 | RP11-425A23 | 4 | 148754633 | 148949857 | -0.0467 | -0.0553 | 0.3980 |
| 552 | RP11-503L19 | 4 | 149764895 | 149896425 | -0.0467 | -0.0553 | 0.3980 |
| 550 | RP11-481K13 | 4 | 150581329 | 150736953 | -0.0467 | -0.0553 | 0.3980 |
| 118 | RP 11-64M20 | 4 | 151573175 | 151716754 | -0.0467 | -0.0553 | 0.3980 |
| 963 | RP11-28E24 | 4 | 151847129 | 151991147 | -0.0467 | -0.0553 | 0.3980 |
| 4292 | RP11-28E24 | 4 | 151847129 | 151991147 | -0.0467 | -0.0553 | 0.3980 |
| 2016 | RP11-259G7 | 4 | 154858444 | 155057548 | -0.0688 | -0.0946 | 0.3826 |
| 3115 | RP11-27G13 | 4 | 156373845 | 156550048 | -0.0688 | -0.0906 | 0.3826 |

(continued)

| Order | Clone | chrom | Start | End | sc_control | sc_BRCA1 | sd |
|---|---|---|---|---|---|---|---|
| 3040 | RP11-264E11 | 4 | 156624370 | 156778344 | -0.0688 | -0.0906 | 0.3826 |
| 1699 | RP11-171N4 | 4 | 157665354 | 157836342 | -0.0689 | -0.0940 | 0.3844 |
| 1250 | RP11-17C4 | 4 | 158576037 | 158701473 | -0.0694 | -0.1005 | 0.3940 |
| 2403 | RP11-6C14 | 4 | 160913163 | 161053242 | -0.0693 | -0.0973 | 0.3925 |
| 2409 | RP11-1G8 | 4 | 163467846 | 163611458 | -0.0693 | -0.0973 | 0.3925 |
| 3701 | RP11-218F10 | 4 | 164527241 | 164686093 | -0.0693 | -0.0973 | 0.3925 |
| 3398 | RP11-336N6 | 4 | 166240478 | 166417001 | -0.0693 | -0.0973 | 0.3925 |
| 2579 | RP11-25N12 | 4 | 166740143 | 166917702 | -0.0693 | -0.0973 | 0.3925 |
| 4281 | RP11-440L13 | 4 | 167847352 | 168019175 | -0.0695 | -0.1031 | 0.3974 |
| 325 | RP11-275K4 | 4 | 170624275 | 170779794 | -0.0727 | -0.0744 | 0.4662 |
| 3703 | RP11-275K4 | 4 | 170624275 | 170779794 | -0.0727 | -0.0744 | 0.4662 |
| 3681 | RP11-213L8 | 4 | 171956844 | 172113114 | -0.0707 | -0.0937 | 0.4238 |
| 919 | RP11-13N11 | 4 | 171958495 | 172124798 | -0.0707 | -0.0937 | 0.4238 |
| 840 | RP11-384D10 | 4 | 172313670 | 172377198 | -0.0693 | -0.1113 | 0.3915 |
| 2436 | RP11-119J20 | 4 | 173240623 | 173408179 | -0.0693 | -0.1113 | 0.3915 |
| 282 | RP11-140M23 | 4 | 173960100 | 174135022 | -0.0693 | -0.1113 | 0.3915 |
| 834 | RP11-148L24 | 4 | 175343072 | 175486570 | -0.0693 | -0.1113 | 0.3915 |
| 1559 | RP11-287F9 | 4 | 176347359 | 176566236 | -0.0693 | -0.1113 | 0.3915 |
| 3493 | RP11-287F9 | 4 | 176347359 | 176566236 | -0.0693 | -0.1113 | 0.3915 |
| 3659 | RP11-87F15 | 4 | 177405896 | 177623816 | -0.0693 | -0.1113 | 0.3915 |
| 4157 | CTC-1499I20 | 4 | 179303625 | 179403709 | -0.0460 | -0.1112 | 0.3942 |
| 2820 | RP11-84H6 | 4 | 179406094 | 179461528 | -0.0460 | -0.1112 | 0.3942 |
| 563 | RP11-495H13 | 4 | 179834806 | 180006551 | -0.0460 | -0.1112 | 0.3942 |
| 3001 | RP11-396I22 | 4 | 181215945 | 181420733 | -0.0058 | -0.1112 | 0.4019 |
| 2262 | RP11-62B7 | 4 | 182513493 | 182549904 | -0.0058 | -0.1112 | 0.4019 |
| 2805 | RP11-59I16 | 4 | 184232930 | 184404421 | -0.0060 | -0.1051 | 0.4052 |
| 4274 | RP11-451F20 | 4 | 184365840 | 184561883 | -0.0060 | -0.1051 | 0.4052 |
| 3690 | RP11-228F3 | 4 | 185872338 | 186040225 | -0.0468 | -0.1051 | 0.4069 |
| 1874 | RP11-301L8 | 4 | 186652578 | 186820398 | -0.0489 | -0.1368 | 0.4515 |
| 1543 | RP11-279K24 | 4 | 187170026 | 187291316 | -0.0489 | -0.1368 | 0.4515 |
| 2545 | RP11-279K24 | 4 | 187170026 | 187291316 | -0.0489 | -0.1368 | 0.4515 |
| 3668 | RP11-91J3 | 4 | 188522244 | 188674927 | -0.0980 | -0.1369 | 0.4445 |
| 3939 | RP11-354H17 | 4 | 189958830 | 190149921 | -0.0986 | -0.1330 | 0.4577 |
| 4256 | RP11-354H17 | 4 | 189958830 | 190149921 | -0.0986 | -0.1330 | 0.4577 |
| 973 | RP 11-45F23 | 4 | 190520006 | 190668424 | -0.0986 | -0.1330 | 0.4577 |
| 1383 | GS-31-J3 | 4 | 190698063 | 190848063 | -0.0970 | -0.1171 | 0.4238 |
| 1389 | GS-963-K6 | 4 | 190810563 | 190960563 | -0.0965 | -0.1271 | 0.4119 |

(continued)

| Order | Clone | chrom | Start | End | sc_control | sc_BRCA1 | sd |
|---|---|---|---|---|---|---|---|
| 1934 | GS-24-H17 | 5 | 1 | 125000 | 0.0086 | -0.0130 | 0.4405 |
| 3540 | 1129-C9 | 5 | 1 | 125050 | 0.0086 | -0.0130 | 0.4405 |
| 4090 | 189-N21 | 5 | 1 | 125100 | 0.0086 | -0.0130 | 0.4405 |
| 2843 | CTD-2265D9 | 5 | 2570683 | 2671745 | 0.0733 | 0.1924 | 0.4535 |
| 3450 | RP11-20K9 | 5 | 3172060 | 3340431 | 0.0733 | 0.1924 | 0.4535 |
| 1609 | RP11-121L11 | 5 | 3426806 | 3597254 | 0.0734 | 0.2032 | 0.4553 |
| 2452 | RP11-121L11 | 5 | 3426806 | 3597254 | 0.0734 | 0.2032 | 0.4553 |
| 1611 | RP11-13H10 | 5 | 4351733 | 4533614 | 0.0442 | 0.2081 | 0.4476 |
| 3981 | RP11-13H10 | 5 | 4351733 | 4533614 | 0.0442 | 0.2081 | 0.4476 |
| 1828 | RP11-137P5 | 5 | 5500098 | 5687008 | 0.0442 | 0.2081 | 0.4476 |
| 3024 | RP11-245L6 | 5 | 5879129 | 5995464 | 0.0442 | 0.2056 | 0.4479 |
| 2292 | CTD-2324F15 | 5 | 6350940 | 6430016 | 0.0442 | 0.2056 | 0.4479 |
| 287 | RP11-114M17 | 5 | 6515876 | 6708632 | 0.0442 | 0.2056 | 0.4479 |
| 2300 | CTD-2346M20 | 5 | 7449057 | 7530186 | 0.0443 | 0.2172 | 0.4495 |
| 2973 | RP 11-132J20 | 5 | 8413255 | 8600646 | 0.0446 | 0.2294 | 0.4564 |
| 1834 | RP11-32D12 | 5 | 9436026 | 9606181 | 0.0437 | 0.2214 | 0.4374 |
| 2284 | CTD-2274H20 | 5 | 10359620 | 10495937 | 0.0433 | 0.2144 | 0.4293 |
| 2488 | RP11-215G15 | 5 | 10644705 | 10722894 | 0.0433 | 0.2144 | 0.4293 |
| 1944 | RP3-502L6 | 5 | 10925533 | 11148940 | 0.0433 | 0.2089 | 0.4288 |
| 2205 | RP11-44C2 | 5 | 12130015 | 12216435 | 0.0438 | 0.1970 | 0.4389 |
| 1411 | RP1-64M18 | 5 | 12216436 | 12262940 | 0.0431 | 0.1842 | 0.4240 |
| 1407 | RP1-61C2 | 5 | 13138380 | 13288380 | 0.0430 | 0.1761 | 0.4225 |
| 44 | RP11-415K6 | 5 | 14596376 | 14781538 | 0.0433 | 0.1845 | 0.4280 |
| 4099 | RP1-137K24 | 5 | 15191411 | 15315356 | 0.0433 | 0.1845 | 0.4280 |
| 1947 | RP1-167G20 | 5 | 16354087 | 16529263 | 0.0573 | 0.1845 | 0.4321 |
| 1263 | RP11-21H5 | 5 | 16365822 | 16546966 | 0.0573 | 0.1845 | 0.4321 |
| 31 | RP11-260E18 | 5 | 16623433 | 16726662 | 0.0711 | 0.1844 | 0.4330 |
| 2140 | RP11-28P24 | 5 | 19642833 | 19781435 | 0.0710 | 0.1882 | 0.4303 |
| 3990 | RP11-28P24 | 5 | 19642833 | 19781435 | 0.0710 | 0.1882 | 0.4303 |
| 1141 | RP11-414I19 | 5 | 20429524 | 20595579 | 0.0710 | 0.1882 | 0.4303 |
| 866 | RP11-374E21 | 5 | 21573338 | 21757179 | 0.0710 | 0.1882 | 0.4303 |
| 2089 | RP11-374E21 | 5 | 21573338 | 21757179 | 0.0710 | 0.1882 | 0.4303 |
| 1608 | RP11-26L18 | 5 | 22309858 | 22468875 | 0.0710 | 0.1882 | 0.4303 |
| 2591 | RP11-26L18 | 5 | 22309858 | 22468875 | 0.0710 | 0.1882 | 0.4303 |
| 1287 | RP11-57I6 | 5 | 23644140 | 23795207 | 0.0702 | 0.1574 | 0.4138 |
| 4312 | RP11-57I6 | 5 | 23644140 | 23795207 | 0.0702 | 0.1574 | 0.4138 |
| 3679 | RP11-192H6 | 5 | 25187395 | 25333181 | 0.0869 | 0.2154 | 0.4458 |

(continued)

| Order | Clone | chrom | Start | End | sc_control | sc_BRCA1 | sd |
|---|---|---|---|---|---|---|---|
| 2498 | RP11-351N6 | 5 | 26353438 | 26539899 | 0.1042 | 0.2154 | 0.4455 |
| 2839 | CTD-2219P 12 | 5 | 27359951 | 27463814 | 0.1668 | 0.2468 | 0.4779 |
| 2578 | RP11-46C20 | 5 | 27524331 | 27641965 | 0.1668 | 0.2468 | 0.4779 |
| 3435 | RP11-37M16 | 5 | 28851494 | 28918008 | 0.1634 | 0.1829 | 0.4041 |
| 1829 | RP11-16A6 | 5 | 31326937 | 31504934 | 0.1626 | 0.1679 | 0.3866 |
| 3447 | RP11-53L13 | 5 | 32742919 | 32916357 | 0.1626 | 0.1679 | 0.3866 |
| 3989 | RP11-15G6 | 5 | 33280772 | 33449384 | 0.1626 | 0.1679 | 0.3866 |
| 4291 | RP11-15G6 | 5 | 33280772 | 33449384 | 0.1626 | 0.1679 | 0.3866 |
| 2001 | CTD-2052F19 | 5 | 34971009 | 35043293 | 0.1626 | 0.1679 | 0.3866 |
| 2288 | CTD-2291F22 | 5 | 35388192 | 35513594 | 0.1624 | 0.1634 | 0.3807 |
| 683 | CTD-2267H19 | 5 | 36275837 | 36380871 | 0.1618 | 0.1740 | 0.3689 |
| 251 | RP11-7M4 | 5 | 36988555 | 37177051 | 0.1615 | 0.1674 | 0.3616 |
| 2379 | RP11-575G10 | 5 | 42897645 | 42898147 | 0.1249 | 0.1266 | 0.3595 |
| 2507 | RP4-592P18 | 5 | 51083837 | 51232444 | 0.1058 | 0.1300 | 0.4406 |
| 3850 | RP11-506H20 | 5 | 54562681 | 54725678 | -0.1204 | -0.0332 | 0.4188 |
| 862 | RP11-364C6 | 5 | 55408298 | 55588124 | -0.1206 | -0.0803 | 0.4151 |
| 1561 | RP11-364C6 | 5 | 55408298 | 55588124 | -0.1206 | -0.0803 | 0.4151 |
| 3297 | RP 11-412L4 | 5 | 55546666 | 55547446 | -0.1207 | -0.0954 | 0.4116 |
| 1048 | RP4-572A3 | 5 | 56921507 | 57065064 | -0.1377 | -0.1291 | 0.4086 |
| 3846 | RP11-495K20 | 5 | 58128952 | 58280997 | -0.1377 | -0.1291 | 0.4086 |
| 3029 | RP11-313P15 | 5 | 60735461 | 60791039 | -0.1218 | -0.1621 | 0.3925 |
| 2596 | RP 11-34J 15 | 5 | 61659573 | 61769004 | -0.1219 | -0.1745 | 0.3945 |
| 3841 | RP11-432A10 | 5 | 62041315 | 62191844 | -0.1219 | -0.1745 | 0.3945 |
| 1282 | RP11-19B7 | 5 | 64142336 | 64300795 | -0.1219 | -0.1745 | 0.3945 |
| 22 | RP11-480H11 | 5 | 65059150 | 65239020 | -0.1466 | -0.1746 | 0.3906 |
| 2295 | CTC-536A23 | 5 | 65352862 | 65454024 | -0.1466 | -0.1746 | 0.3906 |
| 1133 | RP11-402F5 | 5 | 66695993 | 66888015 | -0.1087 | -0.1746 | 0.3842 |
| 1682 | RP 11-494P23 | 5 | 67322915 | 67501695 | -0.1091 | -0.1964 | 0.3937 |
| 217 | RP11-551B22 | 5 | 70361715 | 70517014 | -0.1114 | -0.2206 | 0.4429 |
| 3976 | RP11-115I6 | 5 | 71659788 | 71823446 | -0.0608 | -0.1972 | 0.3747 |
| 2291 | CTC-533D18 | 5 | 72794427 | 72920172 | -0.0610 | -0.1872 | 0.3792 |
| 853 | RP11-399M5 | 5 | 73126031 | 73314954 | -0.0610 | -0.1872 | 0.3792 |
| 1989 | CTC-1347N9 | 5 | 73872906 | 73873453 | -0.0613 | -0.1802 | 0.3854 |
| 1835 | RP11-97L2 | 5 | 73817310 | 73968588 | -0.0613 | -0.1802 | 0.3854 |
| 675 | CTD-2200O3 | 5 | 75600638 | 75734191 | -0.0612 | -0.1858 | 0.3832 |
| 3616 | RP11-667P13 | 5 | 78198045 | 78198547 | -0.0612 | -0.1782 | 0.3845 |
| 1294 | RP11-21K15 | 5 | 78203689 | 78284615 | -0.0612 | -0.1782 | 0.3845 |

(continued)

| Order | Clone | chrom | Start | End | sc_control | sc_BRCA1 | sd |
|-------|-------|-------|-------|-----|------------|----------|-----|
| 666 | CTC-431G16 | 5 | 79093741 | 79219539 | -0.0612 | -0.1658 | 0.3839 |
| 910 | RP11-30D15 | 5 | 79172773 | 79351477 | -0.0612 | -0.1658 | 0.3839 |
| 4069 | RP11-538B23 | 5 | 79936331 | 80082792 | -0.0612 | -0.1658 | 0.3839 |
| 1722 | RP11-241J12 | 5 | 80075105 | 80216216 | -0.0617 | -0.1589 | 0.3954 |
| 2774 | RP11-241j12 | 5 | 80075105 | 80216216 | -0.0617 | -0.1589 | 0.3954 |
| 2104 | RP 11-356D23 | 5 | 81265606 | 81410507 | -0.0618 | -0.1546 | 0.3974 |
| 3022 | RP11-356D23 | 5 | 81265606 | 81410507 | -0.0618 | -0.1546 | 0.3974 |
| 2173 | RP11-463C5 | 5 | 82570102 | 82742889 | -0.0618 | -0.1546 | 0.3974 |
| 2283 | CTC-480C2 | 5 | 84860884 | 85040367 | -0.0624 | -0.1473 | 0.4106 |
| 3979 | RP 11-72L22 | 5 | 86341428 | 86472400 | -0.0626 | -0.1512 | 0.4139 |
| 1285 | RP11-3H15 | 5 | 87297507 | 87443969 | -0.0626 | -0.1512 | 0.4139 |
| 4059 | RP11-512I16 | 5 | 87676680 | 87711396 | -0.0626 | -0.1512 | 0.4139 |
| 792 | RP11-12D3 | 5 | 90806344 | 90964362 | -0.0321 | -0.1449 | 0.4263 |
| 143 | CTD-2011L22 | 5 | 92074742 | 92092165 | -0.0316 | -0.1638 | 0.4143 |
| 1290 | RP11-20O13 | 5 | 93129507 | 93173459 | -0.0317 | -0.1582 | 0.4160 |
| 2585 | RP11-39B14 | 5 | 93540085 | 93709247 | -0.0317 | -0.1540 | 0.4168 |
| 142 | CTC-366N20 | 5 | 94527791 | 94663165 | -0.0318 | -0.1439 | 0.4186 |
| 1904 | RP11-526D16 | 5 | 95842138 | 96007440 | -0.0323 | -0.1125 | 0.4291 |
| 2299 | CTC-564E20 | 5 | 98073104 | 98073634 | -0.0321 | -0.0833 | 0.4247 |
| 3687 | RP11-195A20 | 5 | 98954115 | 99111442 | -0.0321 | -0.0833 | 0.4247 |
| 2827 | CTD-2068C11 | 5 | 99850207 | 100001668 | -0.0321 | -0.0833 | 0.4247 |
| 3747 | RP11-418P19 | 5 | 100715359 | 100906849 | -0.0318 | -0.0738 | 0.4196 |
| 2933 | RP11-560F8 | 5 | 100991080 | 101181927 | -0.0318 | -0.0738 | 0.4196 |
| 3847 | RP11-565F4 | 5 | 119956077 | 120134621 | -0.0436 | -0.0351 | 0.4618 |
| 2293 | CTB-54G2 | 5 | 126657706 | 126865341 | -0.0462 | -0.0201 | 0.4053 |
| 2298 | CTC-352M6 | 5 | 127597190 | 127759109 | -0.0462 | -0.0201 | 0.4053 |
| 3196 | RP 1-117N3 | 5 | 128354467 | 128454909 | -0.0463 | -0.0145 | 0.4040 |
| 1040 | RP1-179P12 | 5 | 129269732 | 129450042 | -0.0464 | -0.0063 | 0.4001 |
| 1955 | RP1-236L2 | 5 | 129569617 | 129599992 | -0.0464 | -0.0063 | 0.4001 |
| 2198 | RP11-114H7 | 5 | 130306745 | 130412531 | -0.0464 | -0.0063 | 0.4001 |
| 4119 | RP1-247F3 | 5 | 130616593 | 130630517 | -0.0465 | -0.0023 | 0.3993 |
| 3575 | RP1-66P19 | 5 | 131009149 | 131090780 | -0.0465 | -0.0023 | 0.3993 |
| 2506 | RP1-241C15 | 5 | 131365774 | 131577988 | -0.0463 | -0.0161 | 0.4038 |
| 141 | CTB-104P14 | 5 | 131686843 | 131731311 | -0.0585 | -0.0161 | 0.4013 |
| 3628 | RP11-729C24 | 5 | 131817042 | 131977001 | -0.0590 | -0.0031 | 0.3907 |
| 129 | CTB-49A3 | 5 | 132506140 | 132704549 | -0.0590 | -0.0058 | 0.3898 |
| 2285 | CTB-28J9 | 5 | 135549762 | 135738309 | -0.0372 | 0.0110 | 0.3700 |

(continued)

| Order | Clone | chrom | Start | End | sc_control | sc_BRCA1 | sd |
|-------|-------|-------|-------|-----|-----------|----------|-----|
| 38 | RP11-114H21 | 5 | 135739990 | 135899842 | -0.0368 | 0.0160 | 0.3797 |
| 3204 | RP 1-244J5 | 5 | 136365521 | 136366234 | -0.0368 | 0.0160 | 0.3797 |
| 4111 | RP1-186K10 | 5 | 136892127 | 137064639 | -0.0367 | 0.0277 | 0.3822 |
| 2191 | RP11-256P1 | 5 | 137635258 | 137734281 | -0.0367 | 0.0277 | 0.3822 |
| 3192 | RP1-98O22 | 5 | 137758103 | 137758844 | -0.0370 | 0.0214 | 0.3761 |
| 2289 | CTB-46B19 | 5 | 138230413 | 138414953 | -0.0370 | 0.0214 | 0.3761 |
| 2825 | CTB-133C10 | 5 | 139529321 | 139663275 | -0.0371 | 0.0301 | 0.3736 |
| 1131 | RP11-515C16 | 5 | 139904059 | 140124593 | -0.0371 | 0.0301 | 0.3736 |
| 128 | CTD-2323H12 | 5 | 141041469 | 141198799 | -0.0367 | 0.0363 | 0.3807 |
| 2296 | CTD-2332G20 | 5 | 141852268 | 141998278 | -0.0371 | 0.0543 | 0.3735 |
| 2835 | CTD-2185H17 | 5 | 142505106 | 142742827 | -0.0371 | 0.0543 | 0.3735 |
| 3682 | RP11-226O9 | 5 | 143298228 | 143480824 | -0.0371 | 0.0543 | 0.3735 |
| 2822 | CTC-370H24 | 5 | 145164596 | 145347857 | -0.0371 | 0.0543 | 0.3735 |
| 3310 | RP11-481E16 | 5 | 146313344 | 146482537 | -0.0595 | 0.0603 | 0.3696 |
| 2821 | CTB-107A22 | 5 | 146972544 | 147089688 | -0.0592 | 0.0505 | 0.3751 |
| 2829 | CTB-176I15 | 5 | 147869999 | 147926171 | -0.0592 | 0.0505 | 0.3751 |
| 882 | RP11-394O4 | 5 | 148643204 | 148821858 | -0.0589 | 0.0575 | 0.3823 |
| 2290 | CTC-307M15 | 5 | 149174490 | 149349990 | -0.0589 | 0.0575 | 0.3823 |
| 1453 | CTB-1013H5 | 5 | 149407439 | 149494253 | -0.0589 | 0.0575 | 0.3823 |
| 665 | CTB-137O22 | 5 | 149978532 | 150055332 | -0.0589 | 0.0575 | 0.3823 |
| 677 | CTC-209K13 | 5 | 151319679 | 151506795 | -0.0595 | 0.0455 | 0.3692 |
| 3986 | RP 11-26B2 | 5 | 152539684 | 152728888 | -0.0431 | 0.0377 | 0.3670 |
| 2715 | RP11-494C5 | 5 | 152817260 | 152985165 | -0.0431 | 0.0377 | 0.3670 |
| 2286 | CTC-263A14 | 5 | 154417750 | 154418252 | -0.0431 | 0.0377 | 0.3670 |
| 2281 | CTB-3C20 | 5 | 155606865 | 155624831 | -0.0437 | 0.0308 | 0.3553 |
| 661 | CTB-120L21 | 5 | 156441856 | 156481323 | -0.0437 | 0.0308 | 0.3553 |
| 1280 | RP11-102A1 | 5 | 157180096 | 157349365 | -0.0436 | 0.0343 | 0.3572 |
| 878 | RP11-391B7 | 5 | 157384965 | 157573351 | -0.0436 | 0.0343 | 0.3572 |
| 126 | CTC-279E3 | 5 | 159195207 | 159278867 | -0.0436 | 0.0504 | 0.3569 |
| 3511 | RP11-505G12 | 5 | 162295646 | 162296436 | -0.0436 | 0.0504 | 0.3569 |
| 4262 | RP11-505G12 | 5 | 162295646 | 162296436 | -0.0436 | 0.0504 | 0.3569 |
| 130 | CTC-320C6 | 5 | 163176794 | 163287813 | -0.0436 | 0.0504 | 0.3569 |
| 3306 | RP11-469G19 | 5 | 164752357 | 164922098 | -0.0436 | 0.0504 | 0.3569 |
| 3019 | RP11-170P5 | 5 | 165677723 | 165841050 | -0.0727 | 0.0400 | 0.3608 |
| 588 | RP11-511M9 | 5 | 165789662 | 165925473 | -0.0727 | 0.0400 | 0.3608 |
| 669 | CTB-180C19 | 5 | 166550287 | 166647640 | -0.0730 | 0.0346 | 0.3550 |
| 2297 | CTB-55A14 | 5 | 167592450 | 167807599 | -0.0730 | 0.0346 | 0.3550 |

(continued)

| Order | Clone | chrom | Start | End | sc_control | sc_BRCA1 | sd |
|---|---|---|---|---|---|---|---|
| 1149 | RP11-420L4 | 5 | 169138790 | 169139510 | -0.0731 | 0.0577 | 0.3519 |
| 1869 | RP11-292M11 | 5 | 169915012 | 170086458 | -0.0726 | 0.0489 | 0.3638 |
| 1672 | RP11-20O22 | 5 | 171046969 | 171113733 | -0.0318 | 0.0374 | 0.3728 |
| 2143 | RP11-20O22 | 5 | 171046969 | 171113733 | -0.0318 | 0.0374 | 0.3728 |
| 133 | CTB-54I1 | 5 | 171714383 | 171955339 | -0.0317 | 0.0427 | 0.3757 |
| 3838 | RP11-489P1 | 5 | 173800904 | 173985872 | -0.0317 | 0.0427 | 0.3757 |
| 137 | CTB-73D21 | 5 | 174509436 | 174643725 | -0.0317 | 0.0427 | 0.3757 |
| 138 | CTC-355H1 | 5 | 175139848 | 175176805 | -0.0317 | 0.0427 | 0.3757 |
| 2301 | CTB-87L24 | 5 | 175835967 | 175892322 | -0.0312 | 0.0506 | 0.3856 |
| 2386 | RP11-564G9 | 5 | 176797689 | 176798343 | 0.0353 | 0.0505 | 0.3955 |
| 2014 | RP11-281O15 | 5 | 178243599 | 178455573 | -0.0332 | 0.0506 | 0.3862 |
| 3553 | GS-240-G13 | 5 | 180537866 | 180687866 | -0.1077 | -0.0768 | 0.4313 |
| 1930 | 84-C11 | 5 | 180689187 | 180839187 | -0.3190 | -0.3097 | 0.6022 |
| 1393 | GS-62-L11 | 6 | 110996 | 260996 | 0.0298 | 0.1440 | 0.4207 |
| 1927 | GS-196-15 | 6 | 225000 | 375000 | 0.0295 | 0.1845 | 0.4135 |
| 3809 | RP11-13J16 | 6 | 1240996 | 1301756 | 0.1256 | 0.1486 | 0.3986 |
| 3817 | RP11-15N12 | 6 | 3292049 | 3425043 | 0.1256 | 0.1486 | 0.3986 |
| 1646 | RP11-174B19 | 6 | 5151674 | 5170849 | 0.1256 | 0.1486 | 0.3986 |
| 3818 | RP11-232H4 | 6 | 6151264 | 6169479 | 0.1256 | 0.1486 | 0.3986 |
| 3330 | RP11-163I22 | 6 | 6217514 | 6382300 | 0.1256 | 0.1486 | 0.3986 |
| 3271 | RP11-320C15 | 6 | 6705338 | 6770147 | 0.1256 | 0.1486 | 0.3986 |
| 3252 | RP3-336K20 | 6 | 7544150 | 7575683 | 0.1256 | 0.1681 | 0.3972 |
| 1527 | RP11-196B15 | 6 | 7898162 | 8078796 | 0.1257 | 0.1759 | 0.4006 |
| 3807 | RP11-385L2 | 6 | 8598346 | 8599694 | 0.1257 | 0.1759 | 0.4006 |
| 1119 | RP11-360O19 | 6 | 10597237 | 10684872 | 0.1254 | 0.1663 | 0.3932 |
| 1623 | RP3-510L9 | 6 | 11352273 | 11482926 | 0.1260 | 0.1625 | 0.4060 |
| 1114 | RP11-97A19 | 6 | 12430744 | 12449045 | 0.1046 | 0.1625 | 0.4014 |
| 3242 | RP1-257A7 | 6 | 13285332 | 13413248 | 0.1045 | 0.1559 | 0.4000 |
| 3262 | RP3-365E2 | 6 | 14536103 | 14673832 | 0.1044 | 0.1513 | 0.3972 |
| 1637 | RP1-232K4 | 6 | 15434571 | 15630732 | 0.1041 | 0.1434 | 0.3903 |
| 3241 | RP1-13D10 | 6 | 16165681 | 16318728 | 0.1041 | 0.1434 | 0.3903 |
| 1106 | RP11-68J15 | 6 | 17735571 | 17805613 | 0.1041 | 0.1386 | 0.3909 |
| 3811 | RP11-408C8 | 6 | 18937804 | 19079559 | 0.1043 | 0.1318 | 0.3961 |
| 1087 | RP4-625H18 | 6 | 19863670 | 20018592 | 0.1046 | 0.1361 | 0.4023 |
| 1634 | RP3-444C7 | 6 | 20630894 | 20759064 | 0.1046 | 0.1361 | 0.4023 |
| 1650 | RP11-204E9 | 6 | 21543541 | 21694512 | 0.1051 | 0.1456 | 0.4128 |
| 3825 | RP11-33I5 | 6 | 22580401 | 22666188 | 0.1051 | 0.1456 | 0.4128 |

(continued)

| Order | Clone | chrom | Start | End | sc_control | sc_BRCA1 | sd |
|---|---|---|---|---|---|---|---|
| 1666 | RP11-289M23 | 6 | 23499374 | 23534047 | 0.1051 | 0.1456 | 0.4128 |
| 1116 | RP11-176J5 | 6 | 24254612 | 24267701 | 0.1054 | 0.1384 | 0.4196 |
| 3826 | RP11-289G11 | 6 | 25494592 | 25630305 | 0.1342 | 0.1383 | 0.4307 |
| 1093 | RP1-86C11 | 6 | 27152454 | 27241469 | 0.1330 | 0.1332 | 0.4307 |
| 3789 | RP1-153G14 | 6 | 27444496 | 27526951 | 0.1330 | 0.1332 | 0.4307 |
| 2505 | RP3-431A14 | 6 | 36751257 | 36944619 | 0.0957 | 0.1404 | 0.4767 |
| 1635 | RP1-90K10 | 6 | 36944620 | 37077773 | 0.0957 | 0.1404 | 0.4767 |
| 3260 | RP3-441A12 | 6 | 37889292 | 38009626 | 0.0953 | 0.1367 | 0.4697 |
| 1092 | RP3-350J21 | 6 | 39022180 | 39078688 | 0.0953 | 0.1367 | 0.4697 |
| 3246 | RP1-278E11 | 6 | 39977164 | 40076642 | 0.1233 | 0.1366 | 0.4735 |
| 1126 | RP11-162O6 | 6 | 40860313 | 41030209 | 0.1212 | 0.1249 | 0.4270 |
| 3272 | RP11-533O20 | 6 | 42026550 | 42131628 | 0.1049 | 0.1441 | 0.4115 |
| 3823 | RP11-501I18 | 6 | 42815771 | 42914110 | 0.1049 | 0.1441 | 0.4115 |
| 1654 | RP 11-227E22 | 6 | 43965908 | 44050879 | 0.1039 | 0.1212 | 0.3906 |
| 1112 | RP11-554O14 | 6 | 44987769 | 45164736 | 0.0597 | 0.1009 | 0.3808 |
| 3808 | RP11-546O15 | 6 | 45777368 | 45960349 | 0.0601 | 0.0842 | 0.3888 |
| 1102 | RP3-3650 12 | 6 | 46918495 | 47036031 | 0.0388 | 0.1027 | 0.3835 |
| 1626 | RP3-402H5 | 6 | 47870439 | 48002405 | 0.0379 | 0.0954 | 0.3649 |
| 1111 | RP11-334H12 | 6 | 48824592 | 48956050 | 0.0381 | 0.0916 | 0.3676 |
| 1100 | RP3-442L6 | 6 | 49686791 | 49805314 | 0.0397 | 0.1202 | 0.4028 |
| 1094 | RP3-336H9 | 6 | 50775801 | 50907374 | 0.0375 | 0.0961 | 0.3550 |
| 3268 | RP11-529A21 | 6 | 51408727 | 51472574 | 0.0374 | 0.0899 | 0.3532 |
| 3284 | RP11-362K18 | 6 | 54103953 | 54130898 | 0.0006 | 0.0632 | 0.3637 |
| 1667 | RP11-524H19 | 6 | 54743356 | 54856430 | 0.0415 | 0.0633 | 0.3559 |
| 1659 | RP11-472M19 | 6 | 56747375 | 56905292 | 0.0414 | 0.0586 | 0.3543 |
| 1630 | RP3-422B11 | 6 | 57292500 | 57422028 | 0.0407 | 0.0765 | 0.3398 |
| 3810 | RP11-199A24 | 6 | 58610220 | 58788604 | 0.0407 | 0.0765 | 0.3398 |
| 1124 | RP11-506N21 | 6 | 62639102 | 62725300 | 0.0419 | 0.0672 | 0.3642 |
| 3281 | RP11-767J14 | 6 | 63764581 | 63875481 | 0.0420 | 0.0562 | 0.3681 |
| 1120 | RP11-349P19 | 6 | 65158448 | 65208779 | 0.0426 | 0.0652 | 0.3814 |
| 1627 | RP 1-40C9 | 6 | 65839972 | 65862416 | 0.0426 | 0.0652 | 0.3814 |
| 1090 | RP3-324B8 | 6 | 66901062 | 67021596 | 0.0435 | 0.0733 | 0.3992 |
| 1651 | RP11-409K15 | 6 | 68213162 | 68318818 | -0.0054 | 0.0654 | 0.3838 |
| 1089 | RP1-46B1 | 6 | 69438689 | 69596892 | -0.0054 | 0.0654 | 0.3838 |
| 1633 | RP1-160B9 | 6 | 69937981 | 70007502 | -0.0469 | 0.0571 | 0.3952 |
| 3819 | RP 11-462G2 | 6 | 71121392 | 71183718 | -0.0220 | 0.0576 | 0.3906 |
| 3282 | RP11-111D8 | 6 | 72159825 | 72169003 | -0.0193 | 0.0725 | 0.4478 |

(continued)

| Order | Clone | chrom | Start | End | sc_control | sc_BRCA1 | sd |
|---|---|---|---|---|---|---|---|
| 3822 | RP11-256L9 | 6 | 73180824 | 73217550 | -0.0210 | 0.0406 | 0.4113 |
| 3801 | RP1-234P15 | 6 | 75947840 | 76073480 | -0.0219 | 0.0134 | 0.3918 |
| 1642 | RP3-472A9 | 6 | 76600011 | 76685205 | -0.4201 | -0.2326 | 0.7162 |
| 3275 | RP11-343P23 | 6 | 77623996 | 77788369 | -0.0777 | 0.0213 | 0.3952 |
| 3806 | RP11-173D14 | 6 | 79534513 | 79708816 | -0.0777 | 0.0213 | 0.3952 |
| 3793 | RP1-159G19 | 6 | 80482703 | 80571211 | -0.0777 | 0.0213 | 0.3952 |
| 1881 | RP11-296E7 | 6 | 80815400 | 80852964 | -0.0777 | 0.0213 | 0.3952 |
| 3821 | RP11-25O6 | 6 | 83405494 | 83562457 | -0.0771 | 0.0094 | 0.4071 |
| 3781 | RP 1-120N9 | 6 | 84309049 | 84476066 | -0.0774 | 0.0187 | 0.4027 |
| 3814 | RP11-223L24 | 6 | 85028178 | 85089451 | -0.0777 | 0.0103 | 0.3944 |
| 1661 | RP11-30P6 | 6 | 86051994 | 86222225 | -0.0777 | 0.0103 | 0.3944 |
| 1118 | RP11-106G15 | 6 | 88309107 | 88415806 | -0.0768 | 0.0008 | 0.4149 |
| 1081 | RP1-23D17 | 6 | 88811014 | 88923225 | -0.0768 | 0.0008 | 0.4149 |
| 3791 | RP1-61F10 | 6 | 89632167 | 89689630 | -0.0768 | 0.0008 | 0.4149 |
| 1621 | RP1-122O8 | 6 | 90373901 | 90514111 | -0.0767 | -0.0158 | 0.4175 |
| 1629 | RP1-154G14 | 6 | 91251386 | 91352280 | -0.1041 | -0.0282 | 0.4204 |
| 3288 | RP11-538A16 | 6 | 93629576 | 93823839 | -0.1041 | -0.0486 | 0.4208 |
| 3269 | RP11-572N15 | 6 | 96322017 | 96469450 | -0.1041 | -0.0486 | 0.4208 |
| 782 | RP11-4D24 | 6 | 96958620 | 97139258 | -0.1041 | -0.0486 | 0.4208 |
| 1622 | RP3-393D12 | 6 | 97092087 | 97184903 | -0.1041 | -0.0486 | 0.4208 |
| 1082 | RP1-273N12 | 6 | 99385962 | 99512744 | -0.1035 | -0.0509 | 0.4323 |
| 3278 | RP11-98I9 | 6 | 99909276 | 100037180 | -0.1033 | -0.0785 | 0.4364 |
| 1122 | RP11-117M4 | 6 | 100808566 | 100935179 | -0.0852 | -0.0725 | 0.4305 |
| 3803 | RP1-167P23 | 6 | 101487105 | 101513554 | -0.0852 | -0.0725 | 0.4305 |
| 3279 | RP11-347H8 | 6 | 102119070 | 102294657 | -0.0852 | -0.0725 | 0.4305 |
| 1104 | RP3-449G2 | 6 | 103440994 | 103493022 | -0.0852 | -0.0725 | 0.4305 |
| 3812 | RP11-721G11 | 6 | 116166720 | 116274627 | -0.0826 | -0.0581 | 0.4867 |
| 3251 | RP4-726A1 | 6 | 124382750 | 124503648 | -0.0864 | -0.0730 | 0.4035 |
| 3795 | RP1-84N20 | 6 | 125389373 | 125541247 | -0.0864 | -0.0730 | 0.4035 |
| 3827 | RP11-524A17 | 6 | 126458967 | 126536599 | -0.0863 | -0.0633 | 0.4054 |
| 3274 | RP11-95M15 | 6 | 137962575 | 138077798 | -0.0527 | -0.0783 | 0.4087 |
| 1653 | RP11-15H7 | 6 | 139976115 | 140131794 | -0.0528 | -0.0817 | 0.4110 |
| 1638 | RP3-460G2 | 6 | 141124534 | 141227085 | -0.0535 | -0.0658 | 0.4272 |
| 3283 | RP11-368P1 | 6 | 142222367 | 142361452 | -0.0910 | -0.1020 | 0.3848 |
| 3270 | RP11-86O4 | 6 | 143501404 | 143622432 | -0.0910 | -0.1020 | 0.3848 |
| 3813 | RP11-3B11 | 6 | 144509482 | 144514258 | -0.0910 | -0.1020 | 0.3848 |
| 1647 | RP11-386H19 | 6 | 145089782 | 145171242 | -0.0909 | -0.1192 | 0.3814 |

(continued)

| Order | Clone | chrom | Start | End | sc_control | sc_BRCA1 | sd |
|---|---|---|---|---|---|---|---|
| 1128 | RP11-545I5 | 6 | 146157896 | 146266194 | -0.0903 | -0.1236 | 0.3700 |
| 3273 | RP11-631F7 | 6 | 148426158 | 148609993 | -0.0903 | -0.1236 | 0.3700 |
| 1101 | RP1-111D6 | 6 | 149547579 | 149673910 | -0.0902 | -0.1305 | 0.3674 |
| 1099 | RP5-1179L24 | 6 | 150539735 | 150646838 | -0.0902 | -0.1305 | 0.3674 |
| 1084 | RP1-297M16 | 6 | 151496433 | 151562014 | -0.0902 | -0.1305 | 0.3674 |
| 345 | RP3-443C4 | 6 | 152121130 | 152188475 | -0.0917 | -0.1010 | 0.3986 |
| 3344 | RP11-450E24 | 6 | 152186476 | 152326162 | -0.0917 | -0.1010 | 0.3986 |
| 888 | RP1-130E4 | 6 | 152411768 | 152520660 | -0.0910 | -0.1174 | 0.3831 |
| 3787 | RP4-546K19 | 6 | 153104985 | 153131413 | -0.0904 | -0.1377 | 0.3711 |
| 1645 | RP11-495B4 | 6 | 154042907 | 154157510 | -0.0905 | -0.1378 | 0.3729 |
| 3263 | RP1-66H9 | 6 | 155289734 | 155381577 | -0.0907 | -0.1570 | 0.3767 |
| 1125 | RP11-100E6 | 6 | 155961812 | 156120703 | -0.0907 | -0.1570 | 0.3767 |
| 3254 | RP3-336G18 | 6 | 156885767 | 156988048 | -0.0907 | -0.1570 | 0.3767 |
| 3280 | RP11-266C7 | 6 | 158279904 | 158440778 | -0.0910 | -0.1798 | 0.3847 |
| 3243 | RP3-442A17 | 6 | 158818064 | 158854414 | -0.0910 | -0.1798 | 0.3847 |
| 1121 | RP11-13P5 | 6 | 159413709 | 159520135 | -0.0910 | -0.1798 | 0.3847 |
| 1103 | RP1-249F5 | 6 | 160224241 | 160354109 | -0.0908 | -0.1841 | 0.3808 |
| 3894 | RP11-288H12 | 6 | 160354009 | 160500259 | -0.0908 | -0.1841 | 0.3808 |
| 3782 | RP3-366M24 | 6 | 160621320 | 160702239 | -0.0908 | -0.1841 | 0.3808 |
| 3783 | RP3-473J16 | 6 | 161450523 | 161626739 | -0.0910 | -0.1897 | 0.3848 |
| 3250 | RP1-292F10 | 6 | 162710104 | 162803740 | -0.0912 | -0.1793 | 0.3892 |
| 1083 | RP1-257A15 | 6 | 163355878 | 163476865 | -0.0912 | -0.1793 | 0.3892 |
| 3247 | RP3-471D13 | 6 | 164296629 | 164338799 | -0.0912 | -0.1869 | 0.3874 |
| 3805 | RP11-300M24 | 6 | 165061358 | 165244699 | -0.0912 | -0.1869 | 0.3874 |
| 1662 | RP11-289E16 | 6 | 165840859 | 165928214 | -0.0912 | -0.1869 | 0.3874 |
| 3285 | RP11-351J23 | 6 | 167790640 | 167835277 | -0.0912 | -0.1869 | 0.3874 |
| 3802 | RP3-470B24 | 6 | 168036070 | 168121862 | -0.0912 | -0.1869 | 0.3874 |
| 1625 | RP1-137D17 | 6 | 169488216 | 169576118 | -0.0914 | -0.1858 | 0.3931 |
| 3557 | GS-57-H24 | 6 | 170544992 | 170694992 | -0.0914 | -0.1858 | 0.3931 |
| 1397 | GS-164-D18 | 7 | 180000 | 330000 | -0.0858 | -0.1343 | 0.4297 |
| 1891 | RP11-510K8 | 7 | 1611828 | 1805609 | -0.0842 | -0.1944 | 0.3942 |
| 2653 | RP4-607J2 | 7 | 2370558 | 2519955 | -0.0840 | -0.2089 | 0.3898 |
| 1564 | RP11-348A21 | 7 | 3526556 | 3613603 | -0.0840 | -0.2089 | 0.3898 |
| 3395 | RP11-348A21 | 7 | 3526556 | 3613603 | -0.0840 | -0.2089 | 0.3898 |
| 570 | RP11-172O13 | 7 | 5704716 | 5847079 | -0.0835 | -0.1980 | 0.3794 |
| 1403 | RP1-42M2 | 7 | 5969700 | 6049710 | -0.0835 | -0.1980 | 0.3794 |
| 1029 | RP4-810E6 | 7 | 6049510 | 6202437 | -0.0835 | -0.1980 | 0.3794 |

(continued)

| Order | Clone | chrom | Start | End | sc_control | sc_BRCA1 | sd |
|---|---|---|---|---|---|---|---|
| 505 | RP4-733B9 | 7 | 7176436 | 7292189 | -0.0835 | -0.1902 | 0.3799 |
| 564 | RP11-505D17 | 7 | 7947759 | 8125919 | -0.0836 | -0.1851 | 0.3817 |
| 3487 | RP11-304A10 | 7 | 8950512 | 9040173 | -0.0836 | -0.1851 | 0.3817 |
| 4255 | RP11-304A10 | 7 | 8950512 | 9040173 | -0.0836 | -0.1851 | 0.3817 |
| 4114 | RP5-959C21 | 7 | 9924891 | 10064840 | -0.0836 | -0.1851 | 0.3817 |
| 583 | RP11-352E12 | 7 | 10064640 | 10151963 | -0.0836 | -0.1851 | 0.3817 |
| 2200 | RP11-392P1 | 7 | 10372373 | 10429780 | -0.0834 | -0.1656 | 0.3768 |
| 1402 | RP5-855F16 | 7 | 10904962 | 11000307 | -0.0834 | -0.1566 | 0.3784 |
| 2712 | RP11-502P9 | 7 | 11782368 | 11938707 | -0.0834 | -0.1566 | 0.3784 |
| 2738 | RP11-195L14 | 7 | 12951352 | 13077542 | -0.0835 | -0.1502 | 0.3806 |
| 501 | RP4-685A2 | 7 | 13887063 | 13996422 | -0.0838 | -0.1356 | 0.3865 |
| 1720 | RP11-512E16 | 7 | 14128072 | 14279388 | -0.0838 | -0.1356 | 0.3865 |
| 1455 | RP11-547G15 | 7 | 14863402 | 15056327 | -0.0838 | -0.1356 | 0.3865 |
| 1671 | RP11-547G15 | 7 | 14863402 | 15056327 | -0.0838 | -0.1356 | 0.3865 |
| 875 | RP11-196O16 | 7 | 16023294 | 16205465 | -0.0299 | -0.0836 | 0.3780 |
| 4040 | RP11-323K15 | 7 | 17781327 | 17929240 | -0.0296 | -0.0825 | 0.3713 |
| 111 | RP11-71F18 | 7 | 19406242 | 19580569 | -0.0294 | -0.0729 | 0.3675 |
| 1670 | RP11-486P11 | 7 | 20042179 | 20150597 | -0.0295 | -0.0646 | 0.3682 |
| 1578 | RP11-445O1 | 7 | 21588234 | 21669042 | -0.0291 | -0.0410 | 0.3614 |
| 2490 | RP 11-343P21 | 7 | 24511504 | 24521807 | -0.0310 | -0.0310 | 0.4010 |
| 3436 | RP11-99O17 | 7 | 25824267 | 25925677 | -0.0310 | -0.0310 | 0.4010 |
| 3865 | RP11-155G7 | 7 | 36168081 | 36309869 | 0.0047 | 0.0114 | 0.3741 |
| 1882 | RP11-302L6 | 7 | 37482196 | 37597750 | 0.0050 | 0.0187 | 0.3819 |
| 3223 | RP11-243E12 | 7 | 37825117 | 37999358 | 0.0050 | 0.0187 | 0.3819 |
| 3227 | RP11-273L18 | 7 | 38356844 | 38489780 | 0.0049 | 0.0256 | 0.3789 |
| 114 | RP11-6412 | 7 | 39496099 | 39613863 | 0.0049 | 0.0256 | 0.3789 |
| 1037 | RP5-1032B10 | 7 | 43150163 | 43228683 | 0.0050 | 0.0123 | 0.3811 |
| 2201 | RP11-36H20 | 7 | 43453095 | 43594872 | 0.0050 | 0.0074 | 0.3804 |
| 2657 | RP4-647J21 | 7 | 44926600 | 45048880 | 0.0050 | 0.0074 | 0.3804 |
| 30 | RP11-111G20 | 7 | 45308102 | 45400347 | 0.0050 | 0.0074 | 0.3804 |
| 1737 | RP11-132L11 | 7 | 45887168 | 45956854 | 0.0050 | 0.0074 | 0.3804 |
| 774 | RP11-571N3 | 7 | 45956855 | 46124185 | 0.0050 | 0.0074 | 0.3804 |
| 2056 | RP11-95J15 | 7 | 46567503 | 46734218 | 0.0050 | 0.0074 | 0.3804 |
| 889 | RP5-1091E12 | 7 | 55045081 | 55222878 | 0.0043 | 0.0059 | 0.3671 |
| 1191 | RP11-339F13 | 7 | 55222879 | 55347931 | 0.0043 | 0.0059 | 0.3671 |
| 799 | RP11-10F11 | 7 | 56639424 | 56803089 | -0.0131 | -0.0195 | 0.3525 |
| 47 | RP11-313P13 | 7 | 71970679 | 72156415 | 0.0007 | 0.0035 | 0.3482 |

(continued)

| Order | Clone | chrom | Start | End | sc_control | sc_BRCA1 | sd |
|---|---|---|---|---|---|---|---|
| 3294 | RP11-450O3 | 7 | 72073705 | 72269192 | 0.0007 | 0.0035 | 0.3482 |
| 1945 | RP4-771P4 | 7 | 73732805 | 73733598 | 0.0314 | 0.0635 | 0.3328 |
| 1949 | RP4-799O8 | 7 | 76190005 | 76361341 | 0.0318 | 0.0499 | 0.3408 |
| 2736 | RP11-467H10 | 7 | 76490656 | 76687315 | 0.0321 | 0.0543 | 0.3472 |
| 4120 | RP4-562A11 | 7 | 76971221 | 77115186 | 0.0324 | 0.0620 | 0.3557 |
| 1909 | RP11-538D15 | 7 | 77710860 | 77899695 | 0.0327 | 0.0681 | 0.3614 |
| 579 | RP11-343J14 | 7 | 78415998 | 78422030 | 0.0327 | 0.0681 | 0.3614 |
| 3206 | RP5-1164F5 | 7 | 78422031 | 78579140 | 0.0327 | 0.0681 | 0.3614 |
| 343 | RP5-1057M1 | 7 | 79539131 | 79644969 | 0.0333 | 0.0574 | 0.3739 |
| 3892 | RP11-575G1 | 7 | 81501227 | 81558313 | 0.0338 | 0.0388 | 0.3861 |
| 876 | RP11-263N12 | 7 | 89329383 | 89449178 | 0.0514 | 0.0537 | 0.3963 |
| 2650 | RP5-1099C19 | 7 | 92000501 | 92074199 | 0.0509 | 0.0534 | 0.3850 |
| 3298 | RP11-45519 | 7 | 93400348 | 93502745 | 0.0509 | 0.0534 | 0.3850 |
| 3440 | RP11-101N13 | 7 | 94315003 | 94427370 | 0.0509 | 0.0534 | 0.3850 |
| 2192 | RP11-380G21 | 7 | 97314556 | 97410111 | 0.0360 | 0.0339 | 0.3826 |
| 2483 | RP11-148A10 | 7 | 103964266 | 104082406 | 0.0705 | 0.0758 | 0.3680 |
| 388 | RP11-22N19 | 7 | 105622027 | 105748629 | 0.0703 | 0.0829 | 0.3640 |
| 1274 | RP11-17I10 | 7 | 105828598 | 105875439 | 0.0936 | 0.0916 | 0.3656 |
| 4295 | RP11-17I10 | 7 | 105828598 | 105875439 | 0.0936 | 0.0916 | 0.3656 |
| 3443 | RP 11-45 K3 | 7 | 114973552 | 115099387 | 0.0669 | 0.0895 | 0.3935 |
| 342 | RP11-354H2 | 7 | 115743960 | 115770524 | 0.0668 | 0.0934 | 0.3912 |
| 3617 | CTB-1013N12 | 7 | 116079831 | 116304165 | 0.0666 | 0.1072 | 0.3869 |
| 587 | RP11-374M7 | 7 | 116365845 | 116463440 | 0.0666 | 0.1072 | 0.3869 |
| 1033 | RP5-866N18 | 7 | 116651982 | 116688781 | 0.0666 | 0.1072 | 0.3869 |
| 1824 | RP11-126C19 | 7 | 117988169 | 118140112 | 0.0670 | 0.0988 | 0.3970 |
| 3773 | RP11-126C19 | 7 | 117988169 | 118140112 | 0.0670 | 0.0988 | 0.3970 |
| 4217 | RP11-105B19 | 7 | 118629304 | 118806607 | 0.0670 | 0.0988 | 0.3970 |
| 3015 | RP11-376O14 | 7 | 119703959 | 119857235 | 0.0670 | 0.0988 | 0.3970 |
| 2669 | RP4-745K6 | 7 | 120979600 | 121110006 | 0.0670 | 0.0988 | 0.3970 |
| 32 | RP11-384A20 | 7 | 121371260 | 121438335 | 0.0687 | 0.0816 | 0.4325 |
| 773 | RP11-560I19 | 7 | 121590947 | 121768222 | 0.0673 | 0.1086 | 0.4025 |
| 1467 | RP11-560I19 | 7 | 121590947 | 121768222 | 0.0673 | 0.1086 | 0.4025 |
| 1502 | RP11-106F1 | 7 | 122549026 | 122703244 | 0.0673 | 0.1086 | 0.4025 |
| 1605 | RP11-106F1 | 7 | 122549026 | 122703244 | 0.0673 | 0.1086 | 0.4025 |
| 3582 | RP5-902E20 | 7 | 123592768 | 123711877 | 0.0674 | 0.1141 | 0.4039 |
| 2195 | RP 11-264K23 | 7 | 123711878 | 123721155 | 0.0672 | 0.1098 | 0.4016 |
| 2119 | RP11-420H19 | 7 | 124677369 | 124832004 | 0.0672 | 0.1098 | 0.4016 |

(continued)

| Order | Clone | chrom | Start | End | sc_control | sc_BRCA1 | sd |
|-------|-------|-------|-------|-----|------------|----------|-----|
| 1264 | RP11-24C13 | 7 | 126157554 | 126225780 | 0.0668 | 0.1223 | 0.3922 |
| 4237 | RP 11-224A 1 | 7 | 127107823 | 127230031 | 0.0666 | 0.1328 | 0.3877 |
| 2461 | RP11-391H5 | 7 | 127389011 | 127586356 | 0.0661 | 0.1397 | 0.3768 |
| 281 | RP11-128A6 | 7 | 128410251 | 128501561 | 0.0661 | 0.1509 | 0.3764 |
| 1027 | RP5-999D10 | 7 | 129238727 | 129329133 | 0.0661 | 0.1509 | 0.3764 |
| 924 | RP11-36B6 | 7 | 130078069 | 130270836 | 0.0673 | 0.1827 | 0.4025 |
| 1556 | RP11-329I5 | 7 | 130598383 | 130792906 | 0.0676 | 0.1729 | 0.4098 |
| 3933 | RP11-329I5 | 7 | 130598383 | 130792906 | 0.0676 | 0.1729 | 0.4098 |
| 3683 | RP11-193I17 | 7 | 131217474 | 131308648 | 0.0676 | 0.1729 | 0.4098 |
| 1783 | RP11-355K3 | 7 | 131972230 | 132193724 | 0.0676 | 0.1729 | 0.4098 |
| 4260 | RP11-355K3 | 7 | 131972230 | 132193724 | 0.0676 | 0.1729 | 0.4098 |
| 1475 | RP11-239G19 | 7 | 133858770 | 134022400 | 0.0673 | 0.1622 | 0.4017 |
| 4233 | RP11-221G19 | 7 | 134079168 | 134234824 | 0.0673 | 0.1622 | 0.4017 |
| 4294 | RP11-221G19 | 7 | 134079168 | 134234824 | 0.0673 | 0.1622 | 0.4017 |
| 2999 | RP11-371N6 | 7 | 134684519 | 134842817 | 0.0673 | 0.1622 | 0.4017 |
| 3675 | RP11-88K4 | 7 | 136263936 | 136416924 | 0.0673 | 0.1622 | 0.4017 |
| 2045 | RP11-102G17 | 7 | 136606302 | 136766865 | 0.1143 | 0.1621 | 0.4112 |
| 263 | RP11-8P6 | 7 | 136684139 | 136846035 | 0.1143 | 0.1621 | 0.4112 |
| 2029 | RP11-269N18 | 7 | 137919496 | 138093873 | 0.1354 | 0.1621 | 0.4139 |
| 836 | RP11-383F6 | 7 | 138384237 | 138486575 | 0.1350 | 0.1585 | 0.4052 |
| 1046 | RP5-1173P7 | 7 | 140392201 | 140521423 | 0.1350 | 0.1585 | 0.4052 |
| 27 | RP11-237G17 | 7 | 140911726 | 140979677 | 0.1350 | 0.1581 | 0.4059 |
| 3578 | RP5-894A10 | 7 | 140977682 | 141092852 | 0.1350 | 0.1581 | 0.4059 |
| 520 | RP11-426J23 | 7 | 142042929 | 142222513 | 0.1351 | 0.1385 | 0.4077 |
| 1953 | RP4-811H12 | 7 | 146787501 | 146949716 | 0.1150 | 0.1224 | 0.4158 |
| 2733 | RP11-69O3 | 7 | 155193647 | 155346385 | 0.1155 | 0.1169 | 0.4253 |
| 1958 | RP5-982E9 | 7 | 156044134 | 156202353 | 0.1155 | 0.1169 | 0.4253 |
| 1917 | GS-580-L5 | 8 | 175000 | 325000 | -0.1945 | -0.2122 | 0.4143 |
| 3547 | GS-77-L23 | 8 | 275000 | 425000 | -0.1945 | -0.2228 | 0.4138 |
| 2486 | RP11-338B22 | 8 | 477644 | 658409 | -0.1945 | -0.2228 | 0.4138 |
| 3561 | RP4-593A12 | 8 | 1528552 | 1680296 | -0.1945 | -0.2228 | 0.4138 |
| 489 | RP4-605G11 | 8 | 1610535 | 1706678 | -0.1945 | -0.2228 | 0.4138 |
| 1238 | RP11-336N16 | 8 | 2898584 | 3068882 | -0.1977 | -0.2279 | 0.4836 |
| 1283 | RP11-45M12 | 8 | 3899636 | 4066807 | -0.1985 | -0.2435 | 0.5009 |
| 3961 | RP11-16H11 | 8 | 4187855 | 4386741 | -0.1977 | -0.2289 | 0.4837 |
| 2413 | RP11-1K11 | 8 | 4596112 | 4755808 | -0.1977 | -0.2289 | 0.4837 |
| 33 | RP 11-29A2 | 8 | 5176646 | 5256106 | -0.1977 | -0.2289 | 0.4837 |

(continued)

| Order | Clone | chrom | Start | End | sc_control | sc_BRCA1 | sd |
|---|---|---|---|---|---|---|---|
| 1034 | RP5-991O23 | 8 | 5326020 | 5459277 | -0.1977 | -0.2289 | 0.4837 |
| 2832 | CTD-2629I16 | 8 | 6684740 | 6685317 | -0.1957 | -0.2074 | 0.4394 |
| 3514 | RP11-540E4 | 8 | 8029024 | 8179923 | -0.1961 | -0.2107 | 0.4479 |
| 2068 | RP11-211C9 | 8 | 8504285 | 8677721 | -0.1961 | -0.2107 | 0.4479 |
| 3647 | RP11-241P12 | 8 | 9788890 | 9958420 | -0.1217 | -0.1978 | 0.4468 |
| 1156 | RP11-177H2 | 8 | 10696470 | 10796498 | -0.1217 | -0.1978 | 0.4468 |
| 1330 | RP 11-297N6 | 8 | 11571002 | 11740080 | -0.0592 | -0.1972 | 0.5011 |
| 1448 | RP11-589N15 | 8 | 11627380 | 11803128 | -0.0592 | -0.1972 | 0.5011 |
| 2124 | RP11-433L7 | 8 | 14278096 | 14461154 | -0.1428 | -0.2008 | 0.4096 |
| 653 | RP11-60C8 | 8 | 15279356 | 15445712 | -0.1428 | -0.2008 | 0.4096 |
| 957 | RP11-44L18 | 8 | 15557561 | 15699688 | -0.1428 | -0.2008 | 0.4096 |
| 3644 | RP11-255E13 | 8 | 16333693 | 16472754 | -0.1427 | -0.2092 | 0.4072 |
| 1139 | RP11-525O22 | 8 | 17784184 | 17950943 | -0.1425 | -0.2167 | 0.4039 |
| 2000 | RP11-809L8 | 8 | 18302963 | 18450086 | -0.1425 | -0.2167 | 0.4039 |
| 1162 | RP11-161I2 | 8 | 18447534 | 18644382 | -0.1425 | -0.2167 | 0.4039 |
| 1361 | RP11-529P14 | 8 | 21756478 | 21872595 | -0.1423 | -0.2379 | 0.3974 |
| 863 | RP11-177H13 | 8 | 23107310 | 23286631 | -0.1423 | -0.2379 | 0.3974 |
| 777 | RP11-561E1 | 8 | 24385106 | 24469397 | -0.1425 | -0.2298 | 0.4031 |
| 2997 | RP11-395I14 | 8 | 25159703 | 25340100 | -0.1425 | -0.2298 | 0.4031 |
| 833 | RP11-138J2 | 8 | 27277246 | 27304557 | -0.1168 | -0.1979 | 0.3969 |
| 3388 | RP11-356F24 | 8 | 28432319 | 28631588 | -0.1169 | -0.1899 | 0.4006 |
| 3705 | RP11-356F24 | 8 | 28432319 | 28631588 | -0.1169 | -0.1899 | 0.4006 |
| 1473 | RP11-263C6 | 8 | 28927769 | 29094558 | -0.1169 | -0.1899 | 0.4006 |
| 299 | RP11-366G13 | 8 | 29818492 | 30006914 | -0.1173 | -0.1728 | 0.4083 |
| 4033 | RP11-293D9 | 8 | 30923281 | 30990492 | -0.1174 | -0.1636 | 0.4108 |
| 1184 | RP11-473A17 | 8 | 30945705 | 31125802 | -0.0853 | -0.1636 | 0.4094 |
| 3879 | RP 11-363L24 | 8 | 31125803 | 31243057 | -0.0853 | -0.1636 | 0.4094 |
| 1738 | RP11-301H15 | 8 | 32530006 | 32715284 | -0.0851 | -0.1117 | 0.4067 |
| 3973 | RP11-11N9 | 8 | 32855440 | 33028630 | -0.0851 | -0.1117 | 0.4067 |
| 573 | RP11-75P13 | 8 | 34287764 | 34455012 | -0.0316 | -0.1025 | 0.4322 |
| 4103 | RP1-144M5 | 8 | 34483916 | 34660804 | -0.0316 | -0.1025 | 0.4322 |
| 1943 | RP1-155L11 | 8 | 35234984 | 35343974 | -0.0316 | -0.1025 | 0.4322 |
| 3889 | RP11-98I12 | 8 | 36363042 | 36516657 | -0.0135 | -0.0841 | 0.4308 |
| 1535 | RP11-197P20 | 8 | 37204481 | 37371241 | -0.0136 | -0.0487 | 0.4338 |
| 3762 | RP11-197P20 | 8 | 37204481 | 37371241 | -0.0136 | -0.0487 | 0.4338 |
| 3927 | RP11-350N15 | 8 | 38358847 | 38488999 | 0.2443 | 0.0056 | 0.5921 |
| 3439 | RP 11-44K6 | 8 | 39836600 | 39985607 | 0.2983 | 0.0785 | 0.6093 |

(continued)

| Order | Clone | chrom | Start | End | sc_control | sc_BRCA1 | sd |
|-------|-------|-------|-------|-----|------------|----------|-----|
| 974 | RP11-51K12 | 8 | 40501066 | 40649254 | 0.2425 | 0.0520 | 0.4954 |
| 25 | RP11-15G16 | 8 | 41287409 | 41424589 | 0.2425 | 0.0564 | 0.4937 |
| 1951 | RP1-198M21 | 8 | 41516869 | 41617043 | 0.3371 | 0.0773 | 0.4982 |
| 3882 | RP11-231D20 | 8 | 42155380 | 42333717 | 0.2687 | 0.0774 | 0.4896 |
| 1686 | RP 11-503E24 | 8 | 42503724 | 42674303 | 0.2689 | 0.0825 | 0.4861 |
| 2831 | CTD-2115H11 | 8 | 43315811 | 43438715 | 0.2907 | 0.0907 | 0.4868 |
| 3403 | RP11-350F16 | 8 | 47816603 | 47982207 | 0.0546 | 0.0800 | 0.4071 |
| 1293 | RP11-11C20 | 8 | 52786052 | 52920538 | 0.1624 | 0.1099 | 0.4082 |
| 1531 | RP11-197I11 | 8 | 53135835 | 53175020 | 0.1613 | 0.1300 | 0.4319 |
| 3860 | RP11-182E14 | 8 | 53938355 | 54105603 | 0.1615 | 0.1292 | 0.4269 |
| 97 | RP11-53M11 | 8 | 55513791 | 55667180 | 0.1615 | 0.1240 | 0.4267 |
| 3938 | RP11-342K10 | 8 | 57944550 | 58097264 | 0.1373 | 0.1373 | 0.4038 |
| 3452 | RP11-114M5 | 8 | 59452734 | 59619718 | 0.1373 | 0.1373 | 0.4038 |
| 1567 | RP 11-414L 17 | 8 | 61675923 | 61851185 | 0.1376 | 0.1511 | 0.4104 |
| 3072 | RP11-35A5 | 8 | 61829586 | 62011027 | 0.1383 | 0.1629 | 0.4255 |
| 4104 | RP3-491L6 | 8 | 61918628 | 61993775 | 0.1383 | 0.1678 | 0.4243 |
| 2492 | RP 11-227F6 | 8 | 62411158 | 62572801 | 0.1377 | 0.1598 | 0.4113 |
| 431 | RP 11-26L21 | 8 | 63219972 | 63361585 | 0.1377 | 0.1598 | 0.4113 |
| 977 | RP11-45K10 | 8 | 64465942 | 64651178 | 0.1378 | 0.1561 | 0.4152 |
| 3415 | RP11-21C5 | 8 | 69451201 | 69616969 | 0.1383 | 0.1401 | 0.4248 |
| 3454 | RP11-21C17 | 8 | 70222710 | 70378941 | 0.1383 | 0.1401 | 0.4248 |
| 261 | RP 11-2B 15 | 8 | 72635961 | 72767666 | 0.1388 | 0.1450 | 0.4351 |
| 1767 | RP11-351E7 | 8 | 73498816 | 73677520 | 0.1388 | 0.1450 | 0.4351 |
| 1879 | RP11-313C15 | 8 | 73722091 | 73910845 | 0.1388 | 0.1393 | 0.4367 |
| 1515 | RP11-88N8 | 8 | 75573453 | 75722946 | 0.1056 | 0.1251 | 0.4168 |
| 2418 | RP11-3N13 | 8 | 75850330 | 76026072 | 0.1318 | 0.1374 | 0.4342 |
| 2067 | RP11-202L1 | 8 | 77234275 | 77394656 | 0.1321 | 0.1463 | 0.4407 |
| 2598 | RP11-48D4 | 8 | 77936444 | 78029382 | 0.1325 | 0.1559 | 0.4487 |
| 800 | RP11-12L20 | 8 | 78929594 | 79062039 | 0.1325 | 0.1559 | 0.4487 |
| 4052 | RP11-523D2 | 8 | 79633094 | 79802906 | 0.1325 | 0.1559 | 0.4487 |
| 959 | RP 11-27N21 | 8 | 80736766 | 80893803 | 0.1321 | 0.1543 | 0.4391 |
| 2594 | RP11-48B3 | 8 | 81538449 | 81648851 | 0.1323 | 0.1623 | 0.4437 |
| 102 | RP11-62E9 | 8 | 81904332 | 82095689 | 0.1327 | 0.1523 | 0.4533 |
| 3068 | RP11-34M16 | 8 | 82640038 | 82768430 | 0.1323 | 0.1420 | 0.4452 |
| 3138 | RP11-51M18 | 8 | 84421672 | 84550079 | 0.1353 | 0.1725 | 0.5103 |
| 2656 | RP5-1098O20 | 8 | 90947388 | 91066955 | 0.1322 | 0.1967 | 0.4433 |
| 338 | RP11-353D5 | 8 | 92425104 | 92553416 | 0.1321 | 0.2042 | 0.4410 |

(continued)

| Order | Clone | chrom | Start | End | sc_control | sc_BRCA1 | sd |
|---|---|---|---|---|---|---|---|
| 254 | RP11-3J21 | 8 | 93160371 | 93308372 | 0.1321 | 0.2042 | 0.4410 |
| 874 | RP11-388K12 | 8 | 94214289 | 94424296 | 0.1456 | 0.2150 | 0.4450 |
| 1489 | RP11-266D22 | 8 | 95335790 | 95508534 | 0.1654 | 0.2150 | 0.4440 |
| 4028 | RP11-320N21 | 8 | 96081539 | 96178047 | 0.1819 | 0.2062 | 0.4635 |
| 246 | RP11-3D19 | 8 | 97773101 | 97944401 | 0.1197 | 0.2267 | 0.4470 |
| 3428 | RP11-24H3 | 8 | 99215537 | 99366374 | 0.1199 | 0.2331 | 0.4512 |
| 2963 | RP11-10G10 | 8 | 101279027 | 101431748 | 0.1212 | 0.2647 | 0.4794 |
| 1259 | RP11-21E8 | 8 | 102295847 | 102476213 | 0.1222 | 0.2921 | 0.5007 |
| 3011 | RP 11-373L22 | 8 | 102544799 | 102710032 | 0.1221 | 0.2998 | 0.4988 |
| 1161 | RP11-150P21 | 8 | 103802267 | 103959662 | 0.1221 | 0.3151 | 0.5001 |
| 4027 | RP11-310H18 | 8 | 104441333 | 104600167 | 0.1214 | 0.3180 | 0.4836 |
| 3872 | RP11-188I6 | 8 | 104875738 | 105015142 | 0.1226 | 0.2991 | 0.5103 |
| 2453 | RP11-132E3 | 8 | 105218041 | 105402499 | 0.1213 | 0.3247 | 0.4817 |
| 1539 | RP11-200A13 | 8 | 106039025 | 106200563 | 0.1211 | 0.3370 | 0.4777 |
| 2276 | RP11-79F7 | 8 | 107820491 | 107973246 | 0.1215 | 0.3321 | 0.4875 |
| 1810 | RP11-20P9 | 8 | 108006920 | 108167162 | 0.1217 | 0.3378 | 0.4916 |
| 819 | RP11-115M13 | 8 | 108980084 | 109137354 | 0.1599 | 0.3379 | 0.4844 |
| 2115 | RP 11-419L20 | 8 | 110461319 | 110612552 | 0.1604 | 0.3957 | 0.4963 |
| 4037 | RP 11-294M6 | 8 | 111740331 | 111910034 | 0.1809 | 0.4074 | 0.4927 |
| 2451 | RP11-114O8 | 8 | 112187912 | 112266168 | 0.1809 | 0.4074 | 0.4927 |
| 1374 | RP11-544H16 | 8 | 112566055 | 112683962 | 0.1809 | 0.4074 | 0.4927 |
| 248 | RP11-11A18 | 8 | 113492780 | 113659918 | 0.1809 | 0.3994 | 0.4919 |
| 1472 | RP11-252H14 | 8 | 114428898 | 114574464 | 0.1809 | 0.3994 | 0.4919 |
| 801 | RP11-2K18 | 8 | 115760314 | 115915271 | 0.2136 | 0.4416 | 0.5040 |
| 3307 | RP11-536K17 | 8 | 117379793 | 117484202 | 0.2134 | 0.4466 | 0.4992 |
| 654 | RP11-67N21 | 8 | 118297084 | 118467725 | 0.2141 | 0.4352 | 0.5150 |
| 555 | RP11-494N20 | 8 | 118573210 | 118737745 | 0.2141 | 0.4352 | 0.5150 |
| 2803 | RP11-72M5 | 8 | 119798302 | 119961178 | 0.2142 | 0.4288 | 0.5171 |
| 2979 | RP11-115M9 | 8 | 122743719 | 122893632 | 0.2526 | 0.4608 | 0.5385 |
| 3957 | RP11-16G11 | 8 | 122793257 | 122875995 | 0.2526 | 0.4608 | 0.5385 |
| 2677 | CTB49g22 | 8 | 122849778 | 122999778 | 0.2526 | 0.4608 | 0.5385 |
| 3657 | RP11-96B2 | 8 | 123538667 | 123697997 | 0.2526 | 0.4608 | 0.5385 |
| 3871 | RP11-174I12 | 8 | 124550978 | 124718434 | 0.2528 | 0.4576 | 0.5428 |
| 1369 | RP 11-532M24 | 8 | 125633453 | 125840521 | 0.2910 | 0.4661 | 0.5465 |
| 2723 | RP11-495D4 | 8 | 126599186 | 126798853 | 0.2910 | 0.4799 | 0.5469 |
| 3127 | RP11-28I2 | 8 | 127563614 | 127764311 | 0.2909 | 0.4870 | 0.5437 |
| 312 | RP11-382A18 | 8 | 128313668 | 128490427 | 0.2909 | 0.4870 | 0.5437 |

(continued)

| Order | Clone | chrom | Start | End | sc_control | sc_BRCA1 | sd |
|---|---|---|---|---|---|---|---|
| 3044 | RP1-80K22 | 8 | 128735637 | 128884770 | 0.2913 | 0.4824 | 0.5520 |
| 258 | RP11-3O20 | 8 | 130380459 | 130458325 | 0.2915 | 0.4762 | 0.5562 |
| 1595 | RP11-3O20 | 8 | 130380459 | 130458325 | 0.2915 | 0.4762 | 0.5562 |
| 3414 | RP11-17E16 | 8 | 130562446 | 130578066 | 0.2917 | 0.4806 | 0.5606 |
| 2942 | RP11-4C17 | 8 | 132474524 | 132638132 | 0.2917 | 0.4806 | 0.5606 |
| 2799 | RP11-71N3 | 8 | 133156771 | 133318034 | 0.2908 | 0.4621 | 0.5426 |
| 886 | RP6-98A24 | 8 | 134125496 | 134255043 | 0.2910 | 0.4568 | 0.5464 |
| 2441 | RP11-127J8 | 8 | 134871608 | 135047479 | 0.2910 | 0.4568 | 0.5464 |
| 330 | RP 11-343P9 | 8 | 136470355 | 136653421 | 0.2120 | 0.4616 | 0.5205 |
| 1703 | RP11-172M18 | 8 | 139307361 | 139453265 | 0.2120 | 0.4616 | 0.5205 |
| 1912 | RP11-526P7 | 8 | 140285419 | 140443685 | 0.2113 | 0.4316 | 0.5052 |
| 1295 | RP 11-65A5 | 8 | 141431413 | 141551818 | 0.2113 | 0.4316 | 0.5052 |
| 315 | RP11-370K2 | 8 | 142620100 | 142790558 | 0.2113 | 0.4316 | 0.5052 |
| 576 | RP11-472K18 | 8 | 144183954 | 144333954 | 0.2320 | 0.4407 | 0.5218 |
| 500 | RP5-1118A7 | 8 | 144404204 | 144527057 | 0.2603 | 0.4633 | 0.5534 |
| 2743 | RP11-349C2 | 8 | 145582815 | 145779789 | 0.2606 | 0.4334 | 0.5612 |
| 1387 | GS-261-I1 | 8 | 146029826 | 146179826 | 0.2201 | 0.4334 | 0.5648 |
| 4081 | GS-489-D 14 | 8 | 146029876 | 146179876 | 0.2201 | 0.4334 | 0.5648 |
| 3551 | RG-41-L13 | 9 | 115000 | 265000 | -0.1362 | -0.0273 | 0.4484 |
| 1921 | GS-43-N6 | 9 | 525000 | 675000 | -0.1362 | -0.0273 | 0.4484 |
| 992 | RP11-147I11 | 9 | 991053 | 1101150 | -0.1362 | -0.0273 | 0.4484 |
| 991 | RP11-48M17 | 9 | 2136330 | 2296367 | -0.1362 | -0.0273 | 0.4484 |
| 997 | RP11-320E16 | 9 | 2583689 | 2658649 | -0.1356 | -0.0121 | 0.4619 |
| 3595 | RP11-509J21 | 9 | 3533196 | 3696642 | -0.1356 | -0.0121 | 0.4619 |
| 3144 | RP11-125K10 | 9 | 4819656 | 4991841 | -0.1356 | -0.0121 | 0.4619 |
| 1435 | RP11-509D8 | 9 | 4991742 | 5008809 | -0.1347 | -0.0019 | 0.4817 |
| 2621 | RP11-218I7 | 9 | 5993678 | 6062142 | -0.0510 | 0.0272 | 0.5936 |
| 456 | RP11-106A1 | 9 | 6599727 | 6659690 | -0.1258 | -0.0142 | 0.4584 |
| 979 | RP 11-29B9 | 9 | 7904421 | 8056890 | -0.1258 | -0.0142 | 0.4584 |
| 445 | RP11-175E13 | 9 | 8398602 | 8490985 | -0.1258 | -0.0142 | 0.4584 |
| 1439 | RP11-527D15 | 9 | 9657591 | 9823759 | -0.1261 | -0.0212 | 0.4511 |
| 447 | RP11-19G1 | 9 | 9999831 | 10094473 | -0.1261 | -0.0212 | 0.4511 |
| 2615 | RP11-23D5 | 9 | 11227455 | 11303482 | -0.1261 | -0.0262 | 0.4514 |
| 442 | RP11-352F21 | 9 | 11389654 | 11588151 | -0.1261 | -0.0212 | 0.4511 |
| 3162 | RP 11-446F 13 | 9 | 12281538 | 12346848 | -0.1262 | -0.0130 | 0.4502 |
| 449 | RP11-187K14 | 9 | 12872920 | 12952270 | -0.1262 | -0.0130 | 0.4502 |
| 3142 | RP11-413D24 | 9 | 13729563 | 13912972 | -0.1268 | -0.0214 | 0.4375 |

(continued)

| Order | Clone | chrom | Start | End | sc_ control | sc_ BRCA1 | sd |
|---|---|---|---|---|---|---|---|
| 462 | RP11-408A13 | 9 | 14500602 | 14586756 | -0.1268 | -0.0214 | 0.4375 |
| 3587 | RP11-490C5 | 9 | 15298239 | 15399992 | -0.1268 | -0.0214 | 0.4375 |
| 460 | RP11-109M15 | 9 | 16171750 | 16325494 | -0.1268 | -0.0419 | 0.4371 |
| 984 | RP11-132E11 | 9 | 16995146 | 17138368 | -0.1268 | -0.0419 | 0.4371 |
| 980 | RP11-123J20 | 9 | 17933998 | 18013852 | -0.1269 | -0.0366 | 0.4347 |
| 1431 | RP11-503K16 | 9 | 18579960 | 18743122 | -0.1269 | -0.0366 | 0.4347 |
| 3599 | RP11-513M16 | 9 | 19310506 | 19423218 | -0.1269 | -0.0366 | 0.4347 |
| 2607 | RP11-15P13 | 9 | 20172502 | 20351132 | -0.1270 | -0.0446 | 0.4320 |
| 3140 | RP11-113D19 | 9 | 20996401 | 21158465 | -0.1271 | -0.0386 | 0.4303 |
| 3156 | RP11-14912 | 9 | 21899259 | 22000413 | -0.1271 | -0.0386 | 0.4303 |
| 443 | RP11-11J1 | 9 | 22479496 | 22579721 | -0.1269 | -0.0502 | 0.4353 |
| 3591 | RP11-495L19 | 9 | 23461740 | 23557472 | -0.1269 | -0.0502 | 0.4353 |
| 3147 | RP11-33K8 | 9 | 24090706 | 24243453 | -0.1029 | -0.0468 | 0.4324 |
| 3583 | RP11-468C2 | 9 | 24937707 | 25069411 | -0.1029 | -0.0468 | 0.4324 |
| 987 | RP11-33G16 | 9 | 25690183 | 25853240 | -0.1029 | -0.0468 | 0.4324 |
| 2599 | RP11-5P15 | 9 | 26516496 | 26681794 | -0.0752 | -0.0468 | 0.4303 |
| 459 | RP 11-27J8 | 9 | 27449441 | 27590267 | -0.0752 | -0.0468 | 0.4303 |
| 451 | RP11-20P5 | 9 | 28040167 | 28204457 | -0.0752 | -0.0468 | 0.4303 |
| 3141 | RP11-264J11 | 9 | 28670842 | 28755649 | -0.0745 | -0.0640 | 0.4461 |
| 3151 | RP11-48L13 | 9 | 29567030 | 29639069 | -0.0755 | -0.0377 | 0.4237 |
| 532 | RP11-630H9 | 9 | 30404961 | 30535678 | -0.0712 | 0.0095 | 0.5178 |
| 516 | RP11-402B2 | 9 | 31599160 | 31764898 | -0.0761 | -0.0171 | 0.4118 |
| 1055 | RP 11-205M20 | 9 | 32452045 | 32607629 | -0.0761 | -0.0171 | 0.4118 |
| 1071 | RP 11-326F20 | 9 | 33086353 | 33247752 | -0.0765 | -0.0069 | 0.4028 |
| 3215 | RP11-195F19 | 9 | 34634468 | 34820900 | -0.0411 | -0.0169 | 0.3855 |
| 2687 | RP11-112J3 | 9 | 35670117 | 35865495 | -0.0412 | -0.0110 | 0.3840 |
| 511 | RP11-8N6 | 9 | 36632473 | 36774305 | -0.0410 | -0.0174 | 0.3875 |
| 2676 | RP11-397D12 | 9 | 37263640 | 37455975 | -0.0410 | -0.0174 | 0.3875 |
| 2671 | RP11-3J10 | 9 | 37747915 | 37935186 | -0.0410 | -0.0174 | 0.3875 |
| 527 | RP11-113O24 | 9 | 38263089 | 38427296 | -0.0408 | -0.0274 | 0.3910 |
| 3231 | RP 11-290L7 | 9 | 38983970 | 39177643 | -0.2143 | -0.1120 | 0.4990 |
| 512 | RP11-395E19 | 9 | 40645668 | 40774565 | -0.2134 | -0.1327 | 0.5192 |
| 2675 | RP11-15E1 | 9 | 42890129 | 43009824 | -0.3049 | -0.2053 | 0.5779 |
| 2688 | RP11-475I24 | 9 | 44245620 | 44448510 | -0.3049 | -0.2053 | 0.5779 |
| 1067 | RP11-268E1 | 9 | 45843103 | 46009277 | -0.3037 | -0.2316 | 0.6038 |
| 3219 | RP11-211N8 | 9 | 46630312 | 46771375 | -0.3035 | -0.1792 | 0.6076 |
| 515 | RP11-38P6 | 9 | 67106117 | 67192942 | -0.1310 | -0.0154 | 0.4316 |

(continued)

| Order | Clone | chrom | Start | End | sc_ control | sc_ BRCA1 | sd |
|-------|-------|-------|-------|-----|-------------|-----------|-----|
| 985 | RP11-274B18 | 9 | 70318668 | 70488660 | -0.1241 | -0.0184 | 0.3984 |
| 981 | RP11-265B8 | 9 | 70756001 | 70927381 | -0.1241 | -0.0184 | 0.3984 |
| 2620 | RP11-109D9 | 9 | 71447567 | 71636843 | -0.0941 | -0.0184 | 0.3993 |
| 988 | RP11-141J10 | 9 | 72488784 | 72637616 | -0.0939 | -0.0184 | 0.4032 |
| 4131 | RP11-563H8 | 9 | 73274802 | 73425572 | -0.0939 | -0.0184 | 0.4032 |
| 990 | RP 11-429L21 | 9 | 74281669 | 74401046 | -0.0939 | -0.0262 | 0.4038 |
| 440 | RP11-71A24 | 9 | 74808540 | 74977641 | -0.0939 | -0.0338 | 0.4042 |
| 2622 | RP11-401G5 | 9 | 75584335 | 75757100 | -0.0937 | -0.0441 | 0.4089 |
| 441 | RP11-174B4 | 9 | 76502362 | 76697651 | -0.0937 | -0.0441 | 0.4089 |
| 3159 | RP11-66O21 | 9 | 77399675 | 77508705 | -0.0937 | -0.0441 | 0.4089 |
| 986 | RP11-422N19 | 9 | 78111661 | 78213797 | -0.0937 | -0.0441 | 0.4089 |
| 1427 | RP11-490H9 | 9 | 78861728 | 78928696 | -0.0937 | -0.0441 | 0.4089 |
| 3161 | RP11-336N8 | 9 | 79930194 | 80096565 | -0.0939 | -0.0551 | 0.4042 |
| 2605 | RP11-174K23 | 9 | 80495074 | 80676552 | -0.0941 | -0.0422 | 0.3990 |
| 446 | RP11-362L2 | 9 | 81425152 | 81446829 | -0.0941 | -0.0422 | 0.3990 |
| 450 | RP11-384P5 | 9 | 82178554 | 82315521 | -0.0943 | -0.0463 | 0.3952 |
| 999 | RP11-66D1 | 9 | 82951739 | 83064769 | -0.0943 | -0.0463 | 0.3952 |
| 3154 | RP11-432M2 | 9 | 84030884 | 84146321 | -0.0944 | -0.0386 | 0.3924 |
| 4123 | RP11-541F16 | 9 | 84667634 | 84783005 | -0.0944 | -0.0451 | 0.3932 |
| 3158 | RP11-439A18 | 9 | 85290903 | 85375534 | -0.0944 | -0.0451 | 0.3932 |
| 995 | RP11-59M22 | 9 | 86180525 | 86337480 | -0.0718 | -0.0451 | 0.3966 |
| 2601 | RP11-172F7 | 9 | 87074588 | 87248611 | -0.0718 | -0.0381 | 0.3961 |
| 3153 | RP11-280P22 | 9 | 87941616 | 87990836 | -0.0703 | -0.0377 | 0.4292 |
| 3149 | RP 11-276H19 | 9 | 88787386 | 88940677 | -0.0703 | -0.0377 | 0.4292 |
| 3736 | RP11-423O13 | 9 | 88883454 | 89058511 | -0.0703 | -0.0377 | 0.4292 |
| 1189 | RP11-40C6 | 9 | 89255601 | 89375411 | -0.0703 | -0.0377 | 0.4292 |
| 1726 | RP11-249H20 | 9 | 89373412 | 89446315 | -0.0703 | -0.0377 | 0.4292 |
| 1717 | RP11-65B23 | 9 | 89444561 | 89614800 | -0.0703 | -0.0377 | 0.4292 |
| 2602 | RP11-345K9 | 9 | 89912028 | 89983781 | -0.0702 | -0.0581 | 0.4306 |
| 2609 | RP11-176L21 | 9 | 90659199 | 90759709 | -0.0702 | -0.0581 | 0.4306 |
| 2603 | RP11-8B23 | 9 | 91718617 | 91756894 | -0.0673 | -0.0330 | 0.4947 |
| 1967 | RP11-555F9 | 9 | 92185900 | 92362321 | -0.0703 | -0.0598 | 0.4299 |
| 998 | RP11-440G5 | 9 | 93170710 | 93341607 | -0.0697 | -0.0666 | 0.4418 |
| 2611 | RP 11-19J3 | 9 | 94281893 | 94449626 | -0.0838 | -0.0814 | 0.4397 |
| 3143 | RP11-30L4 | 9 | 95248566 | 95419410 | -0.0838 | -0.0814 | 0.4397 |
| 3945 | RP11-33317 | 9 | 96375866 | 96551155 | -0.0835 | -0.0729 | 0.4470 |
| 989 | RP11-279I21 | 9 | 96493468 | 96616027 | -0.0835 | -0.0729 | 0.4470 |

(continued)

| Order | Clone | chrom | Start | End | sc_ control | sc_ BRCA1 | sd |
|-------|-------|-------|-------|-----|-------------|-----------|-----|
| 3899 | RP11-435O5 | 9 | 97175312 | 97362955 | -0.0841 | -0.0640 | 0.4334 |
| 3160 | RP11-160D19 | 9 | 97447876 | 97448518 | -0.0841 | -0.0640 | 0.4334 |
| 461 | RP 11-240L7 | 9 | 98020511 | 98190157 | -0.0843 | -0.0586 | 0.4305 |
| 455 | RP 11-23J9 | 9 | 99080845 | 99246276 | -0.0843 | -0.0524 | 0.4299 |
| 2619 | RP11-23B15 | 9 | 99583820 | 99744615 | -0.0853 | -0.0653 | 0.4073 |
| 452 | RP11-92C4 | 9 | 100604517 | 100754446 | -0.0852 | -0.0717 | 0.4097 |
| 1194 | RP11-192E23 | 9 | 100754347 | 100889311 | -0.0852 | -0.0717 | 0.4097 |
| 1725 | RP11-96L7 | 9 | 100889312 | 100995443 | -0.0852 | -0.0717 | 0.4097 |
| 4127 | RP11-547C13 | 9 | 101231132 | 101374570 | -0.0852 | -0.0717 | 0.4097 |
| 457 | RP11-208F1 | 9 | 102096861 | 102138010 | -0.0853 | -0.0671 | 0.4077 |
| 2608 | RP11-80H12 | 9 | 102748901 | 102915948 | -0.0853 | -0.0671 | 0.4077 |
| 1001 | RP11-342F21 | 9 | 104230495 | 104390758 | -0.0853 | -0.0671 | 0.4077 |
| 994 | RP11-438P9 | 9 | 109317528 | 109517128 | -0.0620 | -0.0587 | 0.4033 |
| 458 | RP11-400A24 | 9 | 110244896 | 110428437 | -0.0620 | -0.0587 | 0.4033 |
| 454 | RP11-388N6 | 9 | 111218628 | 111227230 | -0.0620 | -0.0587 | 0.4033 |
| 1423 | RP 11-470J20 | 9 | 111963321 | 112039788 | -0.0620 | -0.0587 | 0.4033 |
| 2613 | RP11-202G18 | 9 | 112915770 | 113092458 | -0.0620 | -0.0587 | 0.4033 |
| 1971 | RP11-570D4 | 9 | 113691272 | 113781581 | -0.0620 | -0.0587 | 0.4033 |
| 2612 | RP11-88M9 | 9 | 114552190 | 114649546 | -0.0620 | -0.0587 | 0.4033 |
| 3603 | RP11-53418 | 9 | 115621750 | 115806051 | -0.0620 | -0.0587 | 0.4033 |
| 1000 | RP11-165P4 | 9 | 122882717 | 123029745 | -0.0791 | -0.0778 | 0.3884 |
| 3348 | RP11-477J21 | 9 | 123029646 | 123138575 | -0.0791 | -0.0778 | 0.3884 |
| 3150 | RP 11-429D3 | 9 | 123651637 | 123824698 | -0.0656 | -0.0683 | 0.4064 |
| 2610 | RP11-373J8 | 9 | 129369677 | 129390787 | 0.1265 | -0.0148 | 0.5034 |
| 1963 | RP11-545E17 | 9 | 130501515 | 130668178 | -0.1621 | -0.0970 | 0.4226 |
| 453 | RP11-202H3 | 9 | 131896794 | 131932670 | -0.1629 | -0.1172 | 0.4053 |
| 4154 | RP11-57C19 | 9 | 132470366 | 132643828 | -0.1629 | -0.1172 | 0.4053 |
| 1721 | RP11-83J21 | 9 | 132641829 | 132818294 | -0.1626 | -0.1337 | 0.4119 |
| 3152 | RP11-143H20 | 9 | 132906902 | 132957071 | -0.1626 | -0.1337 | 0.4119 |
| 439 | RP11-5N16 | 9 | 133982008 | 134077119 | -0.1626 | -0.1337 | 0.4119 |
| 1734 | RP11-295G24 | 9 | 134621360 | 134820472 | -0.1626 | -0.1337 | 0.4119 |
| 996 | RP11-153P4 | 9 | 135531562 | 135650232 | -0.1626 | -0.1337 | 0.4119 |
| 2618 | RP11-399H11 | 9 | 137112170 | 137333442 | -0.1626 | -0.1337 | 0.4119 |
| 448 | RP11-83N9 | 9 | 138121793 | 138276710 | -0.1626 | -0.1337 | 0.4119 |
| 982 | RP 11-417A4 | 9 | 139523184 | 139715973 | -0.1619 | -0.1102 | 0.4264 |
| 1391 | GS-135-I17 | 9 | 140133252 | 140283252 | -0.1603 | -0.0742 | 0.4630 |
| 4085 | GS-112-N13 | 9 | 140133302 | 140283302 | -0.1603 | -0.0742 | 0.4630 |

(continued)

| Order | Clone | chrom | Start | End | sc_control | sc_BRCA1 | sd |
|-------|-------|-------|-------|-----|-----------|----------|-----|
| 1925 | GS-306-F7 | 10 | 245000 | 395000 | 0.0003 | -0.0321 | 0.4794 |
| 3555 | GS-23-B11 | 10 | 245050 | 395050 | 0.0003 | -0.0321 | 0.4794 |
| 2691 | RP11-118K6 | 10 | 3035538 | 3133935 | 0.0376 | 0.0314 | 0.4184 |
| 2890 | RP11-189M8 | 10 | 10064792 | 10122105 | 0.0762 | 0.0816 | 0.4207 |
| 1728 | RP11-566K1 | 10 | 10830787 | 11020034 | 0.0759 | 0.1017 | 0.4139 |
| 1856 | RP1-251M9 | 10 | 10983504 | 11094455 | 0.0759 | 0.1017 | 0.4139 |
| 732 | RP11-401F24 | 10 | 11805219 | 12011805 | 0.0758 | 0.1170 | 0.4111 |
| 2638 | RP11-730A19 | 10 | 13058479 | 13254681 | 0.0758 | 0.1120 | 0.4111 |
| 187 | RP11-275E20 | 10 | 14321741 | 14514319 | 0.0758 | 0.1120 | 0.4111 |
| 2874 | RP11-165O3 | 10 | 21382229 | 21495264 | 0.0499 | 0.0712 | 0.4266 |
| 170 | RP11-108B14 | 10 | 22399352 | 22575928 | 0.0499 | 0.0712 | 0.4266 |
| 2880 | RP11-379L21 | 10 | 23123769 | 23317773 | 0.0499 | 0.0712 | 0.4266 |
| 190 | RP11-162E8 | 10 | 24196692 | 24341441 | 0.0494 | 0.0538 | 0.4152 |
| 2342 | RP11-129O7 | 10 | 25140960 | 25271606 | 0.0497 | 0.0653 | 0.4219 |
| 715 | RP11-307B23 | 10 | 25971696 | 26132567 | 0.0494 | 0.0799 | 0.4150 |
| 178 | RP11-128B16 | 10 | 26797312 | 26941977 | 0.0479 | 0.0652 | 0.3838 |
| 2646 | RP13-355A21 | 10 | 27935285 | 28141734 | 0.0479 | 0.0652 | 0.3838 |
| 1005 | RP11-478H13 | 10 | 29030096 | 29145351 | 0.0263 | 0.0652 | 0.3864 |
| 179 | RP11-254A5 | 10 | 36972908 | 37144255 | 0.0518 | 0.0608 | 0.3547 |
| 3166 | RP13-445N5 | 10 | 38601694 | 38812575 | -0.0678 | -0.0540 | 0.3740 |
| 2338 | RP11-124O11 | 10 | 42660862 | 42819196 | -0.0172 | -0.0157 | 0.3367 |
| 2869 | RP11-38B21 | 10 | 43864788 | 44024289 | -0.0172 | -0.0157 | 0.3367 |
| 481 | RP11-432I13 | 10 | 44988687 | 45157159 | -0.0172 | -0.0157 | 0.3367 |
| 713 | RP11-47O13 | 10 | 52979753 | 53069434 | 0.0249 | -0.0010 | 0.3599 |
| 1025 | RP11-556E13 | 10 | 53912334 | 54101584 | 0.0249 | -0.0010 | 0.3599 |
| 3185 | RP11-548F5 | 10 | 54798024 | 54938956 | 0.0251 | 0.0166 | 0.3560 |
| 2879 | RP11-313B15 | 10 | 55595990 | 55799248 | 0.0251 | 0.0071 | 0.3553 |
| 2641 | RP11-430K23 | 10 | 56709824 | 56882190 | 0.0251 | 0.0071 | 0.3553 |
| 2886 | RP11-183D3 | 10 | 57554198 | 57668808 | 0.0251 | 0.0071 | 0.3553 |
| 2876 | RP11-371O16 | 10 | 59005947 | 59241663 | 0.0711 | 0.0122 | 0.3565 |
| 721 | RP11-79I23 | 10 | 60095350 | 60270673 | 0.0711 | 0.0122 | 0.3565 |
| 2352 | RP11-357A18 | 10 | 60813485 | 60976636 | 0.0711 | 0.0122 | 0.3565 |
| 718 | RP11-166B18 | 10 | 61902197 | 62069022 | 0.0712 | 0.0173 | 0.3536 |
| 482 | RP11-809M12 | 10 | 62866153 | 62996102 | 0.0712 | 0.0173 | 0.3536 |
| 173 | RP11-13A2 | 10 | 63820049 | 63955343 | 0.0709 | 0.0156 | 0.3602 |
| 722 | RP11-176H12 | 10 | 65709954 | 65899759 | 0.0370 | 0.0157 | 0.3674 |
| 186 | RP11-161L14 | 10 | 66811668 | 66948580 | 0.0367 | 0.0239 | 0.3743 |

(continued)

| Order | Clone | chrom | Start | End | sc_ control | sc_ BRCA1 | sd |
|---|---|---|---|---|---|---|---|
| 3174 | RP11-210G22 | 10 | 67400045 | 67579279 | 0.0367 | 0.0239 | 0.3743 |
| 2642 | RP11-778O10 | 10 | 67577279 | 67645677 | 0.0367 | 0.0239 | 0.3743 |
| 2637 | RP11-428N21 | 10 | 68138624 | 68290770 | 0.0367 | 0.0239 | 0.3743 |
| 2871 | RP11-297N15 | 10 | 69441097 | 69601754 | 0.0769 | 0.0239 | 0.3681 |
| 2883 | RP11-314J18 | 10 | 70288201 | 70457568 | 0.0769 | 0.0239 | 0.3681 |
| 712 | RP11-367H5 | 10 | 71487221 | 71672097 | 0.0298 | 0.0240 | 0.3792 |
| 2877 | RP11-58O1 | 10 | 72645813 | 72768100 | 0.0298 | 0.0240 | 0.3792 |
| 2340 | RP 11-345K20 | 10 | 74818637 | 74895321 | 0.2188 | 0.1553 | 0.5798 |
| 728 | RP11-399K21 | 10 | 76694924 | 76882564 | 0.1151 | 0.0643 | 0.4252 |
| 727 | RP11-325D15 | 10 | 77881141 | 78010383 | 0.1149 | 0.0720 | 0.4290 |
| 1010 | RP11-328K22 | 10 | 78687185 | 78783396 | 0.1147 | 0.0646 | 0.4329 |
| 2630 | RP11-619F23 | 10 | 78781397 | 78833935 | 0.1147 | 0.0646 | 0.4329 |
| 725 | RP11-90J7 | 10 | 79687950 | 79792544 | 0.1148 | 0.0506 | 0.4315 |
| 2626 | RP11-574P20 | 10 | 80580496 | 80624367 | 0.1148 | 0.0506 | 0.4315 |
| 709 | RP 11-40F6 | 10 | 81915872 | 81996355 | 0.1148 | 0.0506 | 0.4315 |
| 185 | RP 11-20E23 | 10 | 82479159 | 82648710 | 0.0749 | 0.0452 | 0.4312 |
| 729 | RP11-95M17 | 10 | 84424513 | 84577760 | 0.0047 | -0.0004 | 0.4609 |
| 2335 | RP 11-219F 10 | 10 | 85603794 | 85708505 | 0.0047 | -0.0004 | 0.4609 |
| 465 | RP11-470J18 | 10 | 86321573 | 86390139 | 0.0052 | -0.0168 | 0.4503 |
| 2334 | RP11-113E21 | 10 | 87212538 | 87312980 | 0.0052 | -0.0253 | 0.4506 |
| 2888 | RP11-396M20 | 10 | 88054883 | 88222662 | 0.0053 | -0.0463 | 0.4482 |
| 714 | RP11-165M8 | 10 | 89531916 | 89596957 | 0.0001 | -0.1301 | 0.5603 |
| 1723 | RP11-380G5 | 10 | 89596958 | 89798494 | 0.0044 | -0.1029 | 0.4679 |
| 478 | RP11-765C10 | 10 | 89796494 | 89839511 | 0.0044 | -0.1029 | 0.4679 |
| 2252 | RP11-765c10 | 10 | 89796494 | 89839511 | 0.0044 | -0.1029 | 0.4679 |
| 2889 | RP11-92H8 | 10 | 92926791 | 93134225 | -0.0353 | -0.0837 | 0.3727 |
| 2344 | RP11-348J12 | 10 | 94800116 | 94832262 | 0.0351 | -0.0262 | 0.5178 |
| 2350 | RP11-162K11 | 10 | 95842632 | 95886385 | -0.0382 | -0.1174 | 0.4083 |
| 710 | RP11-164I17 | 10 | 97222399 | 97318079 | -0.0382 | -0.1401 | 0.4096 |
| 2339 | RP11-248J23 | 10 | 97667188 | 97780399 | -0.0382 | -0.1565 | 0.4080 |
| 2331 | RP11-196N24 | 10 | 98419168 | 98525673 | -0.0382 | -0.1587 | 0.4092 |
| 2345 | RP11-19C6 | 10 | 99620296 | 99781530 | -0.0079 | -0.1701 | 0.4045 |
| 2634 | RP11-704L16 | 10 | 100941233 | 101064714 | -0.0078 | -0.1708 | 0.4017 |
| 2351 | RP11-287G8 | 10 | 101701714 | 101837645 | -0.0078 | -0.1708 | 0.4017 |
| 2887 | RP11-324L3 | 10 | 102895109 | 102985100 | -0.0078 | -0.1708 | 0.4017 |
| 1730 | RP11-264H19 | 10 | 103139013 | 103282090 | -0.0078 | -0.1708 | 0.4017 |
| 3356 | RP11-529I10 | 10 | 103280091 | 103386596 | -0.0078 | -0.1708 | 0.4017 |

(continued)

| Order | Clone | chrom | Start | End | sc_control | sc_BRCA1 | sd |
|-------|-------|-------|-------|-----|-----------|----------|-----|
| 716 | RP11-373N18 | 10 | 105866280 | 105921006 | -0.0071 | -0.1548 | 0.3858 |
| 2878 | RP11-165P9 | 10 | 106789661 | 106827519 | -0.0069 | -0.1628 | 0.3814 |
| 466 | RP11-596L14 | 10 | 107741530 | 107812754 | -0.0067 | -0.1359 | 0.3772 |
| 470 | RP11-699H2 | 10 | 108487790 | 108574828 | -0.0065 | -0.1264 | 0.3741 |
| 3169 | RP11-478K18 | 10 | 109290757 | 109428292 | -0.0075 | -0.1128 | 0.3945 |
| 2870 | RP11-163F15 | 10 | 110243435 | 110340531 | -0.0074 | -0.1090 | 0.3932 |
| 2347 | RP11-271I13 | 10 | 111497654 | 111539088 | -0.0072 | -0.1037 | 0.3891 |
| 2872 | RP11-364E8 | 10 | 112423849 | 112498372 | -0.0072 | -0.1037 | 0.3891 |
| 720 | RP11-381K7 | 10 | 112987340 | 113136937 | -0.0065 | -0.0893 | 0.3734 |
| 2633 | RP11-426E5 | 10 | 113860100 | 114056756 | -0.0199 | -0.0893 | 0.3714 |
| 188 | RP11-357H24 | 10 | 114874395 | 115058209 | -0.0199 | -0.0893 | 0.3714 |
| 2629 | RP11-411P18 | 10 | 115833956 | 115994376 | -0.0193 | -0.0966 | 0.3583 |
| 2336 | RP11-338L11 | 10 | 116774286 | 116971219 | -0.0193 | -0.0966 | 0.3583 |
| 2330 | RP11-96N16 | 10 | 117796864 | 117962983 | -0.0208 | -0.0900 | 0.3918 |
| 169 | RP11-5G18 | 10 | 118760709 | 118853817 | -0.0208 | -0.0900 | 0.3918 |
| 2348 | RP11-355F22 | 10 | 119495410 | 119624604 | -0.0208 | -0.0900 | 0.3918 |
| 473 | RP11-427L15 | 10 | 120419970 | 120485859 | -0.0209 | -0.0990 | 0.3930 |
| 192 | RP11-359E7 | 10 | 121545719 | 121577827 | -0.0210 | -0.1096 | 0.3960 |
| 723 | RP11-323P17 | 10 | 122467430 | 122626379 | -0.0210 | -0.1096 | 0.3960 |
| 717 | RP 11-62L 18 | 10 | 123214091 | 123388488 | -0.0212 | -0.0925 | 0.4006 |
| 3353 | RP 11-45D20 | 10 | 123333380 | 123408484 | -0.0212 | -0.0925 | 0.4006 |
| 2645 | RP11-436O19 | 10 | 124157652 | 124268654 | -0.0212 | -0.0925 | 0.4006 |
| 176 | RP11-338O1 | 10 | 125057753 | 125259149 | -0.0416 | -0.0917 | 0.4009 |
| 175 | RP13-238F13 | 10 | 126007440 | 126190394 | -0.0416 | -0.0856 | 0.4011 |
| 2983 | RP11-115N19 | 10 | 126605954 | 126760588 | -0.0413 | -0.0983 | 0.3943 |
| 182 | RP11-151A10 | 10 | 126760588 | 126955018 | -0.0413 | -0.0983 | 0.3943 |
| 2341 | RP11-16P8 | 10 | 128180750 | 128268594 | -0.0417 | -0.1061 | 0.4034 |
| 724 | RP11-384P18 | 10 | 128798515 | 128966887 | -0.0772 | -0.1060 | 0.4091 |
| 183 | RP11-264E18 | 10 | 129933086 | 130095129 | -0.0772 | -0.1060 | 0.4091 |
| 2882 | RP11-168C9 | 10 | 130845700 | 130997880 | -0.0778 | -0.0977 | 0.4216 |
| 2892 | RP11-400F15 | 10 | 131923441 | 131970517 | -0.0774 | -0.0860 | 0.4131 |
| 719 | RP11-314D6 | 10 | 132451032 | 132635359 | -0.0774 | -0.0860 | 0.4131 |
| 2873 | RP11-45A17 | 10 | 133629568 | 133778458 | -0.0774 | -0.0860 | 0.4131 |
| 1931 | GS-261-B16 | 10 | 135029737 | 135179737 | -0.2825 | -0.2058 | 0.5492 |
| 4089 | GS-137-E24 | 10 | 135029787 | 135179787 | -0.2825 | -0.2058 | 0.5492 |
| 1929 | GS-908-H22 | 11 | 50000 | 200000 | -0.0777 | -0.1197 | 0.4574 |
| 4098 | GS-44-H18 | 11 | 50050 | 200050 | -0.0777 | -0.1197 | 0.4574 |

(continued)

| Order | Clone | chrom | Start | End | sc_control | sc_BRCA1 | sd |
|---|---|---|---|---|---|---|---|
| 3352 | RP11-49619 | 11 | 531284 | 635941 | -0.0738 | -0.2314 | 0.3727 |
| 2941 | RP11-2B17 | 11 | 1687193 | 1687713 | -0.0738 | -0.2314 | 0.3727 |
| 333 | RP11-295K3 | 11 | 1672662 | 1785148 | -0.0738 | -0.2314 | 0.3727 |
| 904 | RP3-416J11 | 11 | 2061361 | 2140325 | -0.0734 | -0.2333 | 0.3637 |
| 1733 | RP11-113A6 | 11 | 2261568 | 2430797 | -0.0734 | -0.2333 | 0.3637 |
| 1798 | RP11-19N21 | 11 | 2745620 | 2920188 | -0.0734 | -0.2333 | 0.3637 |
| 3341 | RP11-19N21 | 11 | 2745620 | 2920188 | -0.0734 | -0.2333 | 0.3637 |
| 2440 | RP11-120E20 | 11 | 3573895 | 3758001 | -0.0734 | -0.2240 | 0.3628 |
| 4269 | RP11-438N5 | 11 | 3854180 | 4005785 | -0.0734 | -0.2240 | 0.3628 |
| 1343 | RP11-309J20 | 11 | 4826420 | 4895678 | -0.0734 | -0.2336 | 0.3624 |
| 3948 | RP11-364G22 | 11 | 5753680 | 5927293 | -0.0734 | -0.2336 | 0.3624 |
| 4199 | RP11-89D4 | 11 | 7249883 | 7402046 | -0.0342 | -0.1967 | 0.3918 |
| 300 | RP11-379P15 | 11 | 8144634 | 8368375 | -0.0342 | -0.1967 | 0.3918 |
| 1169 | RP11-152H18 | 11 | 8634957 | 8795697 | -0.0342 | -0.1967 | 0.3918 |
| 4171 | RP11-243M7 | 11 | 9666315 | 9667022 | -0.0340 | -0.1725 | 0.3882 |
| 3405 | RP11-327O2 | 11 | 10289439 | 10454001 | -0.0337 | -0.1777 | 0.3812 |
| 3692 | RP11-206L19 | 11 | 11681593 | 11828351 | -0.0339 | -0.1641 | 0.3861 |
| 3134 | RP11-51B23 | 11 | 12368547 | 12543770 | -0.0339 | -0.1641 | 0.3861 |
| 544 | RP11-502G22 | 11 | 13920040 | 14078651 | -0.0345 | -0.1388 | 0.3985 |
| 255 | RP11-7O20 | 11 | 14830547 | 14997984 | -0.0345 | -0.1388 | 0.3985 |
| 915 | RP11-13J19 | 11 | 15778995 | 15940672 | -0.0345 | -0.1388 | 0.3985 |
| 262 | RP11-4B7 | 11 | 16626917 | 16774089 | -0.0356 | -0.1092 | 0.4216 |
| 4149 | CTC-1277H1 | 11 | 17512270 | 17608985 | -0.0354 | -0.1051 | 0.4183 |
| 3688 | RP11-206C1 | 11 | 18095206 | 18291271 | -0.0354 | -0.0956 | 0.4180 |
| 556 | RP11-504G3 | 11 | 18545502 | 18615506 | -0.0355 | -0.0847 | 0.4198 |
| 2407 | RP11-6K5 | 11 | 20206577 | 20366384 | -0.0346 | -0.0964 | 0.3998 |
| 288 | RP11-121C18 | 11 | 21566335 | 21747083 | -0.0379 | -0.0659 | 0.4734 |
| 908 | RP11-34N19 | 11 | 22392002 | 22530139 | -0.0006 | -0.0950 | 0.3949 |
| 639 | RP 11-72C9 | 11 | 24362907 | 24551978 | -0.0015 | -0.0623 | 0.4131 |
| 4198 | RP11-108G3 | 11 | 25458832 | 25606731 | -0.0015 | -0.0517 | 0.4137 |
| 2116 | RP11-430L3 | 11 | 26201313 | 26402986 | -0.0017 | -0.0334 | 0.4173 |
| 3522 | RP11-541L7 | 11 | 27223810 | 27371257 | -0.0014 | -0.0253 | 0.4123 |
| 572 | RP11-466I1 | 11 | 29080323 | 29253188 | -0.0057 | -0.0545 | 0.5064 |
| 3566 | RP5-859D17 | 11 | 29439625 | 29480700 | -0.0013 | -0.0213 | 0.4102 |
| 2740 | RP11-472K20 | 11 | 35698571 | 35767187 | 0.0039 | 0.0103 | 0.4017 |
| 2206 | RP11-115P8 | 11 | 35657471 | 35810959 | 0.0039 | 0.0103 | 0.4017 |
| 1225 | RP11-324K6 | 11 | 37673711 | 37779927 | 0.0040 | 0.0160 | 0.4045 |

(continued)

| Order | Clone | chrom | Start | End | sc_control | sc_BRCA1 | sd |
|---|---|---|---|---|---|---|---|
| 2541 | RP 11-277K23 | 11 | 38558445 | 38673899 | 0.0038 | 0.0099 | 0.3995 |
| 3854 | RP11-164L18 | 11 | 46216229 | 46383009 | 0.0020 | 0.0000 | 0.3597 |
| 289 | RP11-131J4 | 11 | 54839304 | 55007605 | 0.0352 | 0.0296 | 0.3450 |
| 3190 | RP3-466A11 | 11 | 61394968 | 61395556 | 0.0116 | 0.0118 | 0.3462 |
| 832 | RP11-126P21 | 11 | 66841191 | 66872614 | -0.0237 | -0.0300 | 0.4225 |
| 2726 | RP11-138N3 | 11 | 67395614 | 67616066 | -0.0261 | -0.1007 | 0.4740 |
| 766 | RP11-569N5 | 11 | 68072319 | 68278585 | 0.0134 | 0.0436 | 0.3863 |
| 3839 | RP11-554A11 | 11 | 68509551 | 68701448 | 0.0134 | 0.0436 | 0.3863 |
| 4160 | RP11-804L21 | 11 | 69297663 | 69494896 | 0.0139 | 0.0585 | 0.3971 |
| 3419 | RP11-21D20 | 11 | 70236623 | 70391405 | 0.0139 | 0.0585 | 0.3971 |
| 3612 | RP11-598K3 | 11 | 70306687 | 70472869 | 0.0139 | 0.0585 | 0.3971 |
| 1899 | RP11-512I24 | 11 | 70596594 | 70766596 | 0.0139 | 0.0585 | 0.3971 |
| 2584 | RP11-31L22 | 11 | 71992654 | 72170769 | 0.0139 | 0.0585 | 0.3971 |
| 2040 | RP11-93M11 | 11 | 72509790 | 72552991 | 0.0146 | 0.1337 | 0.4124 |
| 2136 | RP11-28L18 | 11 | 73676966 | 73848622 | 0.0143 | 0.1629 | 0.4038 |
| 3131 | RP11-28L18 | 11 | 73676966 | 73848622 | 0.0143 | 0.1629 | 0.4038 |
| 3874 | RP11-167F22 | 11 | 73706065 | 73867546 | 0.0143 | 0.1629 | 0.4038 |
| 4061 | RP11-535A19 | 11 | 75177922 | 75265229 | 0.0141 | 0.1573 | 0.4010 |
| 1748 | RP11-25F7 | 11 | 75965024 | 76130350 | 0.1511 | 0.1565 | 0.4666 |
| 1822 | RP11-25F7 | 11 | 75965024 | 76130350 | 0.1511 | 0.1565 | 0.4666 |
| 2580 | RP 11-30J7 | 11 | 76074447 | 76232373 | 0.1511 | 0.1710 | 0.4668 |
| 1548 | RP11-321B9 | 11 | 77210154 | 77360154 | 0.1508 | 0.1654 | 0.4606 |
| 4032 | RP11-321B9 | 11 | 77210204 | 77360204 | 0.1508 | 0.1654 | 0.4606 |
| 247 | RP 11-7H7 | 11 | 78034362 | 78206791 | 0.0548 | 0.1591 | 0.3924 |
| 1693 | RP11-153F6 | 11 | 78641698 | 78791854 | 0.0549 | 0.1649 | 0.3938 |
| 422 | RP11-46D24 | 11 | 79636818 | 79817932 | 0.0087 | 0.1700 | 0.3970 |
| 1708 | RP11-187P2 | 11 | 80418018 | 80501017 | 0.0087 | 0.1700 | 0.3970 |
| 3027 | RP11-179A16 | 11 | 81301982 | 81405303 | 0.0087 | 0.1700 | 0.3970 |
| 2439 | RP11-113K21 | 11 | 82426834 | 82518598 | 0.0087 | 0.1700 | 0.3970 |
| 831 | RP11-118L16 | 11 | 83021047 | 83205297 | -0.0073 | 0.1523 | 0.3774 |
| 554 | RP11-482L11 | 11 | 84737802 | 84904751 | -0.0071 | 0.1450 | 0.3815 |
| 3656 | RP11-90K17 | 11 | 85263545 | 85320757 | -0.0065 | 0.1577 | 0.3938 |
| 1864 | RP11-320L11 | 11 | 85613336 | 85682335 | -0.0065 | 0.1577 | 0.3938 |
| 829 | RP11-137O10 | 11 | 86920457 | 87028461 | -0.0180 | 0.1255 | 0.4010 |
| 3389 | RP11-325I16 | 11 | 90903371 | 91076086 | -0.0159 | 0.1009 | 0.4465 |
| 4025 | RP11-291N1 | 11 | 90957432 | 91163512 | -0.0159 | 0.1009 | 0.4465 |
| 1373 | RP11-533H15 | 11 | 91893716 | 92071858 | -0.0175 | 0.1121 | 0.4115 |

(continued)

| Order | Clone | chrom | Start | End | sc_control | sc_BRCA1 | sd |
|---|---|---|---|---|---|---|---|
| 644 | RP11-83E23 | 11 | 92720473 | 92905772 | -0.0175 | 0.1121 | 0.4115 |
| 4238 | RP11-236L3 | 11 | 93040962 | 93189086 | -0.0175 | 0.1121 | 0.4115 |
| 2004 | RP11-816P15 | 11 | 93724208 | 93796118 | -0.0180 | 0.1345 | 0.3997 |
| 3620 | RP11-685N10 | 11 | 93796119 | 93869551 | -0.0180 | 0.1345 | 0.3997 |
| 334 | RP11-349I16 | 11 | 94000378 | 94161783 | -0.0180 | 0.1345 | 0.3997 |
| 3402 | RP11-338H14 | 11 | 94849629 | 94936449 | -0.0180 | 0.1413 | 0.4003 |
| 1805 | RP11-16K5 | 11 | 95569628 | 95712661 | -0.0180 | 0.1413 | 0.4003 |
| 419 | RP 11-25 P2 | 11 | 96155646 | 96278738 | -0.0182 | 0.1373 | 0.3970 |
| 2460 | RP11-379J13 | 11 | 97102773 | 97328943 | -0.0182 | 0.1373 | 0.3970 |
| 1601 | RP11-99C10 | 11 | 98312645 | 98420558 | -0.0174 | 0.1476 | 0.4129 |
| 3669 | RP11-99C10 | 11 | 98312645 | 98420558 | -0.0174 | 0.1476 | 0.4129 |
| 962 | RP11-49M9 | 11 | 99613167 | 99747975 | -0.0183 | 0.1322 | 0.3941 |
| 969 | RP11-45C5 | 11 | 100145392 | 100299550 | -0.0568 | 0.1322 | 0.3996 |
| 3423 | RP11-21G19 | 11 | 101247704 | 101397612 | -0.0568 | 0.1322 | 0.3996 |
| 3343 | RP11-315O6 | 11 | 101724655 | 101922841 | -0.0568 | 0.1322 | 0.3996 |
| 1992 | RP11-750P5 | 11 | 102010617 | 102011178 | -0.0564 | 0.1222 | 0.4083 |
| 2957 | RP11-2I22 | 11 | 102613695 | 102771597 | -0.0563 | 0.1073 | 0.4111 |
| 1464 | RP11-725J15 | 11 | 102741000 | 102891000 | -0.0563 | 0.1073 | 0.4111 |
| 222 | RP11-563P16 | 11 | 102966300 | 103066089 | -0.0563 | 0.1041 | 0.4120 |
| 1168 | RP11-179B7 | 11 | 103803543 | 103965030 | -0.0292 | 0.1042 | 0.4065 |
| 3966 | RP11-20H14 | 11 | 104950107 | 105093266 | -0.0292 | 0.1042 | 0.4065 |
| 762 | RP11-569A20 | 11 | 106083757 | 106252007 | -0.0294 | 0.0985 | 0.4035 |
| 1193 | RP11-56J3 | 11 | 107487025 | 107646360 | -0.0296 | 0.0692 | 0.3983 |
| 2242 | RP11-56j3 | 11 | 107487025 | 107646360 | -0.0296 | 0.0692 | 0.3983 |
| 1483 | RP11-241D13 | 11 | 107541895 | 107699828 | -0.0296 | 0.0692 | 0.3983 |
| 1812 | RP11-25I9 | 11 | 108226252 | 108391319 | -0.0294 | 0.0508 | 0.4022 |
| 2944 | RP11-11N15 | 11 | 110352104 | 110510107 | -0.0292 | 0.0548 | 0.4066 |
| 2042 | RP11-108O10 | 11 | 111095144 | 111274317 | -0.0282 | 0.0658 | 0.4295 |
| 3678 | RP11-107P10 | 11 | 111797481 | 111856589 | -0.0282 | 0.0658 | 0.4295 |
| 3663 | RP11-87N22 | 11 | 112570898 | 112764095 | -0.0285 | 0.0577 | 0.4231 |
| 4240 | RP11-212D19 | 11 | 113740432 | 113877494 | -0.0285 | 0.0577 | 0.4231 |
| 2985 | RP11-136I14 | 11 | 114823728 | 114977828 | -0.0285 | 0.0524 | 0.4221 |
| 1288 | RP11-114K7 | 11 | 115593822 | 115721329 | -0.0285 | 0.0524 | 0.4221 |
| 790 | RP11-4N9 | 11 | 116204676 | 116296560 | -0.0285 | 0.0524 | 0.4221 |
| 920 | RP11-35P15 | 11 | 117022825 | 117131495 | -0.0285 | 0.0524 | 0.4221 |
| 2840 | CTD-3245B9 | 11 | 118148996 | 118395819 | -0.0285 | 0.0524 | 0.4221 |
| 2582 | RP11-46D5 | 11 | 118726259 | 118898358 | -0.0285 | 0.0524 | 0.4221 |

(continued)

| Order | Clone | chrom | Start | End | sc_control | sc_BRCA1 | sd |
|---|---|---|---|---|---|---|---|
| 324 | RP11-215D10 | 11 | 119196989 | 119349896 | -0.0285 | 0.0524 | 0.4221 |
| 2454 | RP11-142I2 | 11 | 120790886 | 120821699 | -0.0281 | 0.0516 | 0.4306 |
| 1706 | RP11-166D19 | 11 | 121326321 | 121516680 | -0.0281 | 0.0516 | 0.4306 |
| 2036 | RP11-93E4 | 11 | 122124382 | 122299430 | -0.0284 | 0.0603 | 0.4250 |
| 2167 | RP11-485A5 | 11 | 123265105 | 123449312 | -0.0284 | 0.0539 | 0.4257 |
| 2408 | RP11-10N17 | 11 | 124487806 | 124646248 | -0.0284 | 0.0539 | 0.4257 |
| 4201 | RP11-100P11 | 11 | 124585409 | 124761537 | -0.0284 | 0.0539 | 0.4257 |
| 2120 | RP11-432I22 | 11 | 125827474 | 126006340 | -0.0282 | 0.0456 | 0.4301 |
| 3670 | RP11-106O22 | 11 | 125962083 | 126160028 | -0.0282 | 0.0456 | 0.4301 |
| 3327 | RP11-168K9 | 11 | 126420601 | 126506792 | -0.0282 | 0.0456 | 0.4301 |
| 1485 | RP 11-264E20 | 11 | 127930569 | 128023639 | -0.0274 | 0.0285 | 0.4470 |
| 238 | RP 11-567M21 | 11 | 129326430 | 129514601 | -0.0254 | 0.0585 | 0.4892 |
| 1987 | RP 11-567M21 | 11 | 129326430 | 129514601 | -0.0254 | 0.0585 | 0.4892 |
| 3930 | RP11-340L13 | 11 | 130972996 | 131149195 | -0.0255 | 0.0261 | 0.4887 |
| 2107 | RP11-419F8 | 11 | 132221425 | 132391430 | -0.0255 | 0.0261 | 0.4887 |
| 4054 | RP11-545G16 | 11 | 132451658 | 132626345 | -0.0255 | 0.0261 | 0.4887 |
| 1153 | RP11-149G17 | 11 | 133514734 | 133706144 | -0.0255 | 0.0261 | 0.4887 |
| 2713 | RP 11-469N6 | 11 | 133983990 | 134148703 | -0.0255 | 0.0261 | 0.4887 |
| 1933 | GS-26-N8 | 11 | 134312384 | 134462384 | -0.2840 | -0.1199 | 0.6160 |
| 4093 | GS-770-G7 | 11 | 134312434 | 134462434 | -0.2840 | -0.1199 | 0.6160 |
| 1395 | GS-8-M16 | 12 | 25000 | 175000 | 0.1956 | 0.3179 | 0.5357 |
| 4075 | GS-124-K20 | 12 | 25050 | 175050 | 0.1956 | 0.3179 | 0.5357 |
| 329 | RP11-28313 | 12 | 152505 | 329906 | 0.1393 | 0.2882 | 0.4929 |
| 1555 | RP11-28313 | 12 | 152505 | 329906 | 0.1393 | 0.2882 | 0.4929 |
| 4097 | GS-496-A11 | 12 | 640787 | 790787 | 0.1398 | 0.2893 | 0.5030 |
| 513 | RP11-359b12 | 12 | 858588 | 1012956 | 0.1397 | 0.2953 | 0.5018 |
| 1236 | RP11-359B12 | 12 | 858588 | 1012956 | 0.1397 | 0.2953 | 0.5018 |
| 29 | RP11-21K20 | 12 | 1719983 | 1855190 | 0.1382 | 0.2356 | 0.4690 |
| 3202 | RP5-1096D14 | 12 | 1855112 | 2075367 | 0.1382 | 0.2356 | 0.4690 |
| 3534 | RP11-543P15 | 12 | 3101423 | 3265129 | 0.1378 | 0.2277 | 0.4601 |
| 2731 | RP11-320N7 | 12 | 3997826 | 4189702 | 0.1378 | 0.2277 | 0.4601 |
| 3890 | RP11-264F23 | 12 | 4189703 | 4315660 | 0.1378 | 0.2277 | 0.4601 |
| 3504 | RP11-319E16 | 12 | 5163922 | 5344301 | 0.1814 | 0.2228 | 0.4420 |
| 568 | RP11-451H11 | 12 | 5802494 | 5927041 | 0.1814 | 0.2228 | 0.4420 |
| 4100 | RP3-467F14 | 12 | 5990176 | 6100072 | 0.1812 | 0.2143 | 0.4375 |
| 2954 | RP 1 1-4N23 | 12 | 13505605 | 13592296 | 0.2607 | 0.1961 | 0.4749 |
| 2708 | RP11-502N13 | 12 | 14521905 | 14648407 | 0.2608 | 0.2069 | 0.4737 |

(continued)

| Order | Clone | chrom | Start | End | sc_control | sc_BRCA1 | sd |
|---|---|---|---|---|---|---|---|
| 4024 | RP 11-320J20 | 12 | 15496292 | 15658372 | 0.2608 | 0.2069 | 0.4737 |
| 1613 | RP11-127B1 | 12 | 18497855 | 18558864 | 0.2401 | 0.1881 | 0.4210 |
| 2437 | RP11-127B1 | 12 | 18497855 | 18558864 | 0.2401 | 0.1881 | 0.4210 |
| 1765 | RP11-328P13 | 12 | 19403674 | 19416674 | 0.2395 | 0.1667 | 0.4344 |
| 2146 | RP11-248B13 | 12 | 21303649 | 21412934 | 0.2390 | 0.2298 | 0.4469 |
| 4183 | RP11-248B13 | 12 | 21303649 | 21412934 | 0.2390 | 0.2298 | 0.4469 |
| 1244 | RP11-359J14 | 12 | 22472217 | 22628174 | 0.2390 | 0.2058 | 0.4469 |
| 3745 | RP11-437F6 | 12 | 23668225 | 23784534 | 0.2393 | 0.1929 | 0.4386 |
| 3624 | RP11-707G18 | 12 | 25271849 | 25447854 | 0.2398 | 0.1743 | 0.4278 |
| 3033 | RP11-318G8 | 12 | 25445849 | 25520153 | 0.2398 | 0.1743 | 0.4278 |
| 1368 | RP11-522D14 | 12 | 25691330 | 25738010 | 0.2398 | 0.1743 | 0.4278 |
| 1243 | RP11-350G2 | 12 | 26494858 | 26676686 | 0.2183 | 0.1707 | 0.4275 |
| 1669 | RP11-425D17 | 12 | 28228331 | 28409325 | 0.1968 | 0.1780 | 0.4219 |
| 3702 | RP11-230B21 | 12 | 28623872 | 28774457 | 0.1679 | 0.1627 | 0.4122 |
| 1867 | RP11-310I24 | 12 | 29551976 | 29607136 | 0.1679 | 0.1627 | 0.4122 |
| 4259 | RP11-310I24 | 12 | 29551976 | 29607136 | 0.1679 | 0.1627 | 0.4122 |
| 2809 | RP11-59L15 | 12 | 30396155 | 30548869 | 0.1677 | 0.1496 | 0.4151 |
| 2260 | RP11-77I22 | 12 | 30742591 | 30939660 | 0.1674 | 0.1409 | 0.4216 |
| 3130 | RP11-50I19 | 12 | 32002852 | 32053594 | 0.1693 | 0.1143 | 0.3801 |
| 3008 | RP11-388G12 | 12 | 32552936 | 32687563 | 0.1695 | 0.1169 | 0.3760 |
| 5 | RP11-460N10 | 12 | 33170516 | 33333493 | 0.2033 | 0.1183 | 0.4112 |
| 1781 | RP11-333D23 | 12 | 37199957 | 37369589 | 0.1926 | 0.0600 | 0.3668 |
| 1487 | RP 11-242B24 | 12 | 37329345 | 37489083 | 0.1926 | 0.0600 | 0.3668 |
| 811 | RP11-115F18 | 12 | 38991138 | 39141490 | 0.1926 | 0.0600 | 0.3668 |
| 1910 | RP11-549E9 | 12 | 39661383 | 39833150 | 0.1924 | 0.0658 | 0.3706 |
| 2117 | RP 11-440G2 | 12 | 40718187 | 40834128 | 0.1337 | 0.0653 | 0.3318 |
| 1895 | RP11-510P12 | 12 | 41495094 | 41624443 | 0.1337 | 0.0653 | 0.3318 |
| 4228 | RP11-210N13 | 12 | 42413120 | 42531691 | 0.1337 | 0.0653 | 0.3318 |
| 3719 | RP11-283I17 | 12 | 43152685 | 43346494 | 0.1339 | 0.0611 | 0.3281 |
| 4190 | RP11-283I17 | 12 | 43152685 | 43346494 | 0.1339 | 0.0611 | 0.3281 |
| 3875 | RP11-176J16 | 12 | 43511484 | 43548358 | 0.1339 | 0.0611 | 0.3281 |
| 309 | RP11-394C19 | 12 | 44741501 | 44903830 | 0.1339 | 0.0611 | 0.3281 |
| 580 | RP11-493L12 | 12 | 45864297 | 46054344 | 0.1338 | 0.0505 | 0.3307 |
| 1044 | RP3-432E18 | 12 | 46156911 | 46299979 | 0.1336 | 0.0556 | 0.3353 |
| 2503 | RP5-1057I20 | 12 | 46451113 | 46571184 | 0.1336 | 0.0556 | 0.3353 |
| 2745 | RP11-89H19 | 12 | 46569188 | 46627459 | 0.1336 | 0.0556 | 0.3353 |
| 1878 | RP11-302B13 | 12 | 47561979 | 47666571 | 0.1336 | 0.0556 | 0.3353 |

(continued)

| Order | Clone | chrom | Start | End | sc_control | sc_BRCA1 | sd |
|---|---|---|---|---|---|---|---|
| 1165 | RP11-152A18 | 12 | 48258682 | 48259463 | 0.1336 | 0.0556 | 0.3353 |
| 3572 | RP3-405J10 | 12 | 48854951 | 48970294 | 0.1336 | 0.0556 | 0.3353 |
| 34 | RP11-112N23 | 12 | 49017640 | 49198741 | 0.1339 | 0.0614 | 0.3295 |
| 4041 | RP11-295B8 | 12 | 49924002 | 50099834 | 0.1341 | 0.0573 | 0.3247 |
| 1497 | RP11-96P3 | 12 | 51027854 | 51174171 | 0.1341 | 0.0573 | 0.3247 |
| 2129 | RP11-443I3 | 12 | 51649634 | 51653639 | 0.3219 | 0.0822 | 0.4801 |
| 3718 | RP11-443I3 | 12 | 51649634 | 51653639 | 0.3219 | 0.0822 | 0.4801 |
| 1449 | RP11-1136G11 | 12 | 51745455 | 51903261 | 0.0911 | 0.0638 | 0.3465 |
| 578 | RP11-202G24 | 12 | 76592229 | 76660224 | 0.0262 | 0.0226 | 0.3803 |
| 427 | RP11-26L7 | 12 | 76975277 | 77145211 | 0.0259 | 0.0126 | 0.3857 |
| 437 | RP11-44H8 | 12 | 78580511 | 78745903 | 0.0259 | 0.0126 | 0.3857 |
| 4175 | RP11-244J10 | 12 | 78967744 | 79140545 | 0.0386 | 0.0126 | 0.3878 |
| 3932 | RP11-362A1 | 12 | 80826960 | 80924954 | 0.0382 | -0.0220 | 0.3962 |
| 1503 | RP11-87P13 | 12 | 81990475 | 82172372 | 0.0381 | -0.0355 | 0.3977 |
| 2960 | RP11-12K13 | 12 | 82690966 | 82845137 | 0.0381 | -0.0355 | 0.3977 |
| 2595 | RP11-26O20 | 12 | 83409425 | 83566636 | 0.0381 | -0.0355 | 0.3977 |
| 2719 | RP11-494O6 | 12 | 83887442 | 83970441 | -0.0073 | -0.0400 | 0.4081 |
| 2063 | RP11-202H2 | 12 | 85524877 | 85686118 | -0.0079 | -0.0596 | 0.4226 |
| 1573 | RP11-435O22 | 12 | 86171716 | 86310791 | -0.0611 | -0.0739 | 0.4422 |
| 1877 | RP11-295A20 | 12 | 87680771 | 87694735 | 0.0342 | -0.0571 | 0.4171 |
| 2424 | RP11-11M4 | 12 | 88092437 | 88268617 | 0.0342 | -0.0571 | 0.4171 |
| 3635 | RP11-239F20 | 12 | 90443259 | 90550237 | 0.0236 | -0.0613 | 0.4156 |
| 2961 | RP11-2K12 | 12 | 91204898 | 91367961 | 0.0236 | -0.0613 | 0.4156 |
| 1792 | RP11-24119 | 12 | 92994984 | 93158945 | 0.0237 | -0.0759 | 0.4130 |
| 2811 | RP11-74K11 | 12 | 93046134 | 93160933 | 0.0237 | -0.0759 | 0.4130 |
| 3733 | RP11-435E3 | 12 | 94119164 | 94318610 | 0.0236 | -0.0848 | 0.4166 |
| 374 | RP11-410A13 | 12 | 94483197 | 94607734 | 0.0241 | -0.1022 | 0.4043 |
| 4051 | RP11-510I5 | 12 | 95366386 | 95513503 | 0.0241 | -0.1022 | 0.4043 |
| 1358 | RP11-541G9 | 12 | 96065354 | 96245207 | 0.0241 | -0.1022 | 0.4043 |
| 2710 | RP11-481K9 | 12 | 97471300 | 97567505 | 0.0230 | -0.0897 | 0.4283 |
| 318 | RP11-406H4 | 12 | 97941617 | 98048716 | 0.0242 | -0.1074 | 0.4024 |
| 219 | RP11-575G13 | 12 | 99371399 | 99527544 | 0.0242 | -0.1043 | 0.4035 |
| 2108 | RP11-426H24 | 12 | 99942639 | 100140740 | 0.0242 | -0.1043 | 0.4035 |
| 2034 | RP11-285E23 | 12 | 100590405 | 100640299 | 0.0242 | -0.1043 | 0.4035 |
| 3878 | RP11-210L7 | 12 | 101312683 | 101482488 | 0.0242 | -0.0939 | 0.4015 |
| 907 | RP11-13C3 | 12 | 101566355 | 101678102 | 0.0242 | -0.0939 | 0.4015 |
| 1524 | RP11-205I24 | 12 | 102490337 | 102647688 | -0.0171 | -0.0939 | 0.3991 |

(continued)

| Order | Clone | chrom | Start | End | sc_control | sc_BRCA1 | sd |
|---|---|---|---|---|---|---|---|
| 1571 | RP11-415D21 | 12 | 103552321 | 103714150 | -0.0171 | -0.0939 | 0.3991 |
| 2086 | RP11-478H3 | 12 | 104827123 | 104902202 | -0.0177 | -0.0885 | 0.4107 |
| 2166 | RP11-478H3 | 12 | 104827123 | 104902202 | -0.0177 | -0.0885 | 0.4107 |
| 2041 | RP11-101A9 | 12 | 105069953 | 105070704 | -0.0177 | -0.0885 | 0.4107 |
| 3660 | RP11-90N16 | 12 | 106096622 | 106265244 | -0.0177 | -0.0945 | 0.4122 |
| 241 | RP11-1C11 | 12 | 106901904 | 107044260 | -0.0177 | -0.0945 | 0.4122 |
| 3652 | RP11-256L11 | 12 | 108191164 | 108360951 | -0.0177 | -0.0945 | 0.4122 |
| 3564 | RP1-305I20 | 12 | 109161542 | 109284146 | 0.0052 | -0.0945 | 0.4094 |
| 3567 | RP1-46F2 | 12 | 109800084 | 109949322 | 0.0052 | -0.1036 | 0.4094 |
| 566 | RP11-162P23 | 12 | 110600796 | 110713632 | 0.0052 | -0.1036 | 0.4094 |
| 3560 | RP1-261P5 | 12 | 111103270 | 111175773 | 0.0052 | -0.1036 | 0.4094 |
| 1408 | RP3-363I18 | 12 | 111697722 | 111800274 | 0.0052 | -0.1036 | 0.4094 |
| 2109 | RP11-438N16 | 12 | 112653966 | 112826540 | 0.0060 | -0.1069 | 0.3935 |
| 1808 | RP11-25E2 | 12 | 113938111 | 114127189 | 0.0064 | -0.0960 | 0.3837 |
| 2419 | RP11-8A1 | 12 | 114694711 | 114865871 | 0.0064 | -0.0960 | 0.3837 |
| 8 | RP11-497G19 | 12 | 115487313 | 115638423 | 0.0061 | -0.0805 | 0.3909 |
| 2054 | RP11-110J12 | 12 | 116586716 | 116748345 | 0.0061 | -0.0805 | 0.3909 |
| 2818 | RP11-68H18 | 12 | 117108405 | 117263887 | 0.0060 | -0.0763 | 0.3919 |
| 1872 | RP11-322N7 | 12 | 118059529 | 118238008 | 0.0060 | -0.0763 | 0.3919 |
| 1400 | RP1-267D11 | 12 | 118976136 | 119114667 | 0.0060 | -0.0763 | 0.3919 |
| 2974 | RP11-144B2 | 12 | 119062348 | 119282179 | 0.0060 | -0.0763 | 0.3919 |
| 2189 | RP11-18C24 | 12 | 119397641 | 119534882 | 0.0237 | -0.0763 | 0.3929 |
| 2597 | RP11-44F24 | 12 | 120351695 | 120440866 | 0.0237 | -0.0763 | 0.3929 |
| 1495 | RP11-87C12 | 12 | 120795462 | 120980024 | 0.0237 | -0.0763 | 0.3929 |
| 428 | RP11-32O4 | 12 | 121815155 | 121982522 | 0.0237 | -0.0763 | 0.3929 |
| 326 | RP11-338K17 | 12 | 122658537 | 122877009 | 0.0237 | -0.0763 | 0.3929 |
| 1521 | RP11-214K3 | 12 | 122994584 | 123193120 | 0.0237 | -0.0763 | 0.3929 |
| 1946 | RP5-916L7 | 12 | 124975368 | 125074019 | 0.0238 | -0.0690 | 0.3902 |
| 2412 | RP11-11C16 | 12 | 126779004 | 126932042 | 0.0238 | -0.0690 | 0.3902 |
| 3320 | RP11-178A2 | 12 | 127840870 | 127841450 | 0.0238 | -0.0690 | 0.3902 |
| 3431 | RP11-21K12 | 12 | 127784854 | 127902285 | 0.0238 | -0.0690 | 0.3902 |
| 1340 | RP11-317A20 | 12 | 129082274 | 129082806 | 0.0245 | -0.0767 | 0.3761 |
| 1178 | RP11-143E21 | 12 | 129169568 | 129337249 | 0.0245 | -0.0767 | 0.3761 |
| 253 | RP11-1K22 | 12 | 129648137 | 129839417 | 0.0245 | -0.0767 | 0.3761 |
| 2164 | RP 11-495K9 | 12 | 130657863 | 130834848 | 0.0238 | -0.0887 | 0.3907 |
| 426 | RP11-46H11 | 12 | 131857464 | 132036477 | 0.0238 | -0.0887 | 0.3907 |
| 1937 | GS-221-K18 | 12 | 132084534 | 132234534 | 0.0238 | -0.0887 | 0.3907 |

(continued)

| Order | Clone | chrom | Start | End | sc_ control | sc_ BRCA1 | sd |
|---|---|---|---|---|---|---|---|
| 931 | RP11-76K19 | 13 | 19007559 | 19137337 | 0.0095 | -0.0541 | 0.4080 |
| 3092 | RP11-187L3 | 13 | 19832836 | 19920914 | 0.0097 | -0.0675 | 0.4036 |
| 943 | RP11-110K8 | 13 | 20994607 | 21089699 | 0.0050 | -0.0692 | 0.5056 |
| 399 | RP11-26A3 | 13 | 21944537 | 22068982 | 0.0096 | -0.0752 | 0.4060 |
| 1983 | RP11-760M1 | 13 | 22887525 | 23056187 | 0.0096 | -0.0752 | 0.4060 |
| 950 | RP11-556N21 | 13 | 23994474 | 24103118 | -0.0177 | -0.0752 | 0.4051 |
| 3107 | RP11-111G7 | 13 | 25124500 | 25300819 | -0.0177 | -0.0804 | 0.4036 |
| 1979 | RP11-570F6 | 13 | 25898592 | 26065378 | -0.0177 | -0.0804 | 0.4036 |
| 1275 | RP 11-44J9 | 13 | 26088616 | 26258539 | -0.0177 | -0.0804 | 0.4036 |
| 2556 | RP11-125I23 | 13 | 26843991 | 27010032 | -0.0182 | -0.0876 | 0.4156 |
| 3342 | RP11-153M24 | 13 | 27414162 | 27490882 | -0.0182 | -0.0876 | 0.4156 |
| 3099 | RP11-95G6 | 13 | 27868844 | 27970962 | 0.0283 | -0.0827 | 0.4242 |
| 936 | RP11-218E6 | 13 | 28837843 | 29008462 | 0.0283 | -0.0741 | 0.4236 |
| 946 | RP11-550P23 | 13 | 29914762 | 30048628 | 0.0898 | -0.0312 | 0.4195 |
| 939 | RP11-95N14 | 13 | 31136680 | 31227403 | 0.0903 | -0.0501 | 0.4084 |
| 3349 | RP11-37E23 | 13 | 31703950 | 31870606 | 0.0903 | -0.0501 | 0.4084 |
| 2564 | RP11-141M1 | 13 | 32790709 | 32842539 | 0.0903 | -0.0501 | 0.4084 |
| 3095 | RP11-87G1 | 13 | 32842440 | 33025356 | 0.0903 | -0.0501 | 0.4084 |
| 2553 | RP11-266E6 | 13 | 33995013 | 34159357 | 0.0903 | -0.0544 | 0.4097 |
| 3103 | RP11-98D3 | 13 | 34909905 | 35080902 | 0.0530 | -0.0545 | 0.3985 |
| 2555 | RP11-10M8 | 13 | 37051980 | 37156004 | 0.0149 | -0.0742 | 0.4204 |
| 400 | RP11-131P10 | 13 | 37797278 | 37920029 | 0.0149 | -0.0742 | 0.4204 |
| 2560 | RP11-131F1 | 13 | 38577172 | 38608597 | 0.0150 | -0.0949 | 0.4172 |
| 2554 | RP11-407E23 | 13 | 39306578 | 39498144 | -0.0184 | -0.1008 | 0.4195 |
| 2551 | RP11-2P5 | 13 | 40367434 | 40566000 | -0.0189 | -0.1092 | 0.4305 |
| 392 | RP11-125A7 | 13 | 41105696 | 41263001 | -0.0189 | -0.1092 | 0.4305 |
| 3111 | RP11-117I13 | 13 | 42155411 | 42253968 | -0.0185 | -0.1392 | 0.4211 |
| 2568 | RP11-168P13 | 13 | 43148292 | 43291406 | -0.0184 | -0.1476 | 0.4193 |
| 406 | RP11-442J21 | 13 | 44121277 | 44178227 | -0.0184 | -0.1435 | 0.4195 |
| 413 | RP11-351K3 | 13 | 44939380 | 45114967 | -0.0185 | -0.1385 | 0.4226 |
| 394 | RP11-408L13 | 13 | 46981023 | 47110676 | -0.0186 | -0.1323 | 0.4241 |
| 3339 | RP11-305D15 | 13 | 47760580 | 47897921 | -0.0206 | -0.1513 | 0.4681 |
| 408 | RP11-174I10 | 13 | 47897822 | 47960646 | -0.0206 | -0.1513 | 0.4681 |
| 412 | RP11-185C18 | 13 | 48960782 | 49068912 | -0.0185 | -0.1317 | 0.4217 |
| 2563 | RP11-40A8 | 13 | 50328519 | 50471842 | -0.0188 | -0.0988 | 0.4296 |
| 409 | RP11-327P2 | 13 | 51243102 | 51412205 | -0.0191 | -0.0924 | 0.4346 |
| 2562 | RP11-431O22 | 13 | 52183619 | 52356910 | -0.0189 | -0.1042 | 0.4319 |

(continued)

| Order | Clone | chrom | Start | End | sc_control | sc_BRCA1 | sd |
|---|---|---|---|---|---|---|---|
| 3105 | RP11-384G23 | 13 | 52856276 | 52955532 | -0.0189 | -0.1042 | 0.4319 |
| 3096 | RP11-200F15 | 13 | 53772767 | 53839190 | -0.0189 | -0.1109 | 0.4317 |
| 410 | RP11-478B20 | 13 | 55372289 | 55430761 | -0.0190 | -0.1061 | 0.4339 |
| 3098 | RP11-516G5 | 13 | 55715878 | 55803708 | -0.0190 | -0.1061 | 0.4339 |
| 3106 | RP11-538C21 | 13 | 57629368 | 57780304 | -0.0190 | -0.1061 | 0.4339 |
| 3102 | RP11-524F1 | 13 | 58618142 | 58784577 | -0.0190 | -0.1061 | 0.4339 |
| 3093 | RP11-359P14 | 13 | 59662064 | 59888923 | -0.0190 | -0.1061 | 0.4339 |
| 2565 | RP11-310K10 | 13 | 60719182 | 60882412 | -0.0190 | -0.1061 | 0.4339 |
| 3104 | RP11-234O23 | 13 | 61999010 | 62167077 | -0.0190 | -0.1061 | 0.4339 |
| 411 | RP11-67L17 | 13 | 62944452 | 63070144 | 0.0082 | -0.1242 | 0.4515 |
| 396 | RP11-129M14 | 13 | 64739934 | 64788659 | 0.0411 | -0.0991 | 0.4396 |
| 398 | RP11-424E21 | 13 | 65400727 | 65415270 | 0.0411 | -0.0991 | 0.4396 |
| 391 | RP11-10M21 | 13 | 66264238 | 66378527 | 0.0411 | -0.0991 | 0.4396 |
| 938 | RP11-521L15 | 13 | 67416969 | 67493555 | 0.0411 | -0.0991 | 0.4396 |
| 937 | RP11-370A2 | 13 | 68205057 | 68256830 | 0.0411 | -0.0991 | 0.4396 |
| 402 | RP11-436I5 | 13 | 69221540 | 69315439 | 0.0411 | -0.0991 | 0.4396 |
| 2572 | RP11-184L18 | 13 | 70296335 | 70417830 | 0.0167 | -0.0897 | 0.4425 |
| 949 | RP11-393H6 | 13 | 71124055 | 71226425 | 0.0167 | -0.0810 | 0.4417 |
| 2561 | RP11-309H15 | 13 | 72606894 | 72687491 | 0.0167 | -0.0810 | 0.4417 |
| 3110 | RP11-552M6 | 13 | 73180719 | 73302614 | 0.0413 | -0.0810 | 0.4478 |
| 3113 | RP11-394J19 | 13 | 74458403 | 74519114 | 0.0192 | -0.0843 | 0.4441 |
| 2573 | RP11-332E3 | 13 | 75289517 | 75440004 | 0.0192 | -0.0843 | 0.4441 |
| 3100 | RP11-226E21 | 13 | 76523033 | 76620509 | 0.0192 | -0.0883 | 0.4446 |
| 3109 | RP11-388E20 | 13 | 77789419 | 77956914 | 0.0196 | -0.1040 | 0.4361 |
| 2567 | RP11-52L5 | 13 | 77954915 | 78112216 | 0.0194 | -0.1093 | 0.4404 |
| 2558 | RP11-421K11 | 13 | 80711170 | 80743754 | -0.0298 | -0.1093 | 0.4334 |
| 942 | RP11-533P8 | 13 | 80910204 | 80958002 | -0.0298 | -0.1093 | 0.4334 |
| 947 | RP11-115N13 | 13 | 82035430 | 82201311 | -0.0298 | -0.1093 | 0.4334 |
| 2570 | RP11-46414 | 13 | 82592802 | 82779067 | -0.0298 | -0.1093 | 0.4334 |
| 2552 | RP11-120L14 | 13 | 83766477 | 83924559 | -0.0298 | -0.1130 | 0.4354 |
| 3101 | RP11-376H15 | 13 | 84781264 | 84909801 | -0.0298 | -0.1130 | 0.4354 |
| 3097 | RP11-366K1 | 13 | 86241567 | 86431667 | -0.0295 | -0.1227 | 0.4269 |
| 933 | RP11-365J7 | 13 | 86903182 | 86956601 | -0.0295 | -0.1227 | 0.4269 |
| 2574 | RP11-478H12 | 13 | 87930105 | 87985085 | -0.0295 | -0.1227 | 0.4269 |
| 393 | RP11-275J18 | 13 | 88476233 | 88642741 | -0.0295 | -0.1227 | 0.4269 |
| 945 | RP11-388D4 | 13 | 89314344 | 89451286 | -0.0801 | -0.0865 | 0.5394 |
| 2569 | RP11-319L6 | 13 | 90396132 | 90476548 | -0.0757 | -0.1242 | 0.4427 |

(continued)

| Order | Clone | chrom | Start | End | sc_control | sc_BRCA1 | sd |
|---|---|---|---|---|---|---|---|
| 935 | RP11-95C14 | 13 | 91284428 | 91449527 | -0.0757 | -0.1153 | 0.4428 |
| 4143 | RP11-632L2 | 13 | 92499761 | 92681327 | -0.0755 | -0.1035 | 0.4400 |
| 2571 | RP11-62D23 | 13 | 93229515 | 93403250 | -0.0512 | -0.1034 | 0.4415 |
| 3091 | RP11-74A12 | 13 | 94378172 | 94494192 | -0.0513 | -0.0967 | 0.4436 |
| 405 | RP11-318K19 | 13 | 95333171 | 95461724 | -0.0513 | -0.0967 | 0.4436 |
| 944 | RP11-235O20 | 13 | 96143662 | 96286081 | -0.0278 | -0.0969 | 0.4292 |
| 941 | RP11-383H17 | 13 | 97306085 | 97467156 | -0.0278 | -0.0969 | 0.4292 |
| 2566 | RP11-44219 | 13 | 98901053 | 98920998 | -0.0280 | -0.0856 | 0.4325 |
| 2557 | RP11-279D17 | 13 | 99118125 | 99217923 | -0.0279 | -0.0913 | 0.4319 |
| 951 | RP11-118F16 | 13 | 100348990 | 100402525 | -0.0284 | -0.0948 | 0.4426 |
| 954 | RP11-564N10 | 13 | 101318689 | 101507384 | -0.0284 | -0.0890 | 0.4420 |
| 3112 | RP11-255P5 | 13 | 102337585 | 102377477 | -0.0286 | -0.0841 | 0.4468 |
| 3114 | RP11-562E17 | 13 | 104304190 | 104442099 | -0.0286 | -0.0841 | 0.4468 |
| 953 | RP11-406G20 | 13 | 105286188 | 105459683 | -0.0286 | -0.0841 | 0.4468 |
| 940 | RP11-232K22 | 13 | 106527417 | 106657339 | -0.0277 | -0.0720 | 0.4259 |
| 934 | RP11-513N16 | 13 | 107333660 | 107395169 | -0.0277 | -0.0720 | 0.4259 |
| 404 | RP11-141M24 | 13 | 108167626 | 108341364 | -0.0468 | -0.1081 | 0.4339 |
| 403 | RP 11-40E6 | 13 | 109253824 | 109357572 | -0.0671 | -0.1267 | 0.4371 |
| 395 | RP11-17E4 | 13 | 110602913 | 110681747 | -0.0671 | -0.1334 | 0.4367 |
| 401 | RP11-310D8 | 13 | 111638256 | 111785821 | -0.0671 | -0.1334 | 0.4367 |
| 414 | RP11-480K16 | 13 | 112025897 | 112208258 | -0.0671 | -0.1288 | 0.4369 |
| 952 | RP11-265C7 | 13 | 112776669 | 112779673 | -0.0675 | -0.1432 | 0.4453 |
| 3108 | RP11-245B11 | 13 | 113770511 | 113930806 | -0.0675 | -0.1432 | 0.4453 |
| 1378 | GS-1-L16 | 13 | 113897980 | 114047980 | -0.0675 | -0.1432 | 0.4453 |
| 3538 | GS-163-C9 | 13 | 114047980 | 114197980 | -0.0675 | -0.1432 | 0.4453 |
| 1501 | RP11-98N22 | 14 | 19570792 | 19728992 | 0.0893 | 0.0746 | 0.4290 |
| 1059 | RP 11-242H9 | 14 | 20949376 | 21092316 | 0.0574 | 0.0725 | 0.4130 |
| 2047 | RP11-89K22 | 14 | 24548289 | 24713726 | 0.0282 | 0.0478 | 0.3802 |
| 3937 | RP11-330O19 | 14 | 25538833 | 25757239 | 0.0282 | 0.0478 | 0.3802 |
| 814 | RP11-144C18 | 14 | 26574257 | 26745001 | 0.0285 | 0.0625 | 0.3860 |
| 280 | RP11-120I18 | 14 | 27534082 | 27695894 | 0.0288 | 0.0477 | 0.3923 |
| 2576 | RP11-30H9 | 14 | 28381631 | 28530540 | 0.0288 | 0.0477 | 0.3923 |
| 3309 | RP11-419C10 | 14 | 29355162 | 29403141 | 0.2903 | 0.0751 | 0.7324 |
| 1158 | RP11-159L20 | 14 | 30160191 | 30325327 | 0.0025 | 0.0636 | 0.4397 |
| 3868 | RP11-187E13 | 14 | 31443208 | 31600340 | 0.0025 | 0.0585 | 0.4391 |
| 20 | RP11-501E21 | 14 | 32427439 | 32540812 | 0.0067 | 0.0659 | 0.5293 |
| 2447 | RP11-114L8 | 14 | 32835734 | 32999496 | 0.0038 | 0.0484 | 0.4682 |

(continued)

| Order | Clone | chrom | Start | End | sc_ control | sc_ BRCA1 | sd |
|---|---|---|---|---|---|---|---|
| 2937 | RP11-561B11 | 14 | 34839169 | 35020664 | 0.0037 | 0.0412 | 0.4644 |
| 3844 | RP11-259K15 | 14 | 35613605 | 35777003 | 0.0035 | 0.0243 | 0.4614 |
| 3392 | RP11-356O9 | 14 | 37010388 | 37212627 | 0.0013 | 0.0042 | 0.4135 |
| 2993 | RP11-138H18 | 14 | 37351226 | 37487778 | 0.0013 | 0.0042 | 0.4135 |
| 3941 | RP11-332O9 | 14 | 40363695 | 40563204 | -0.0224 | -0.0070 | 0.4147 |
| 3319 | RP11-168D12 | 14 | 41353854 | 41500293 | -0.0508 | -0.0069 | 0.4092 |
| 3386 | RP11-333K19 | 14 | 41996958 | 42171562 | -0.0508 | -0.0069 | 0.4092 |
| 817 | RP11-134J10 | 14 | 43288612 | 43453124 | -0.0510 | 0.0005 | 0.4058 |
| 3673 | RP11-99L13 | 14 | 43907856 | 44043642 | -0.0510 | 0.0005 | 0.4058 |
| 912 | RP11-35B20 | 14 | 44799277 | 44957674 | -0.0510 | 0.0005 | 0.4058 |
| 2390 | RP11-565J15 | 14 | 45964782 | 46138593 | -0.0876 | -0.0719 | 0.5117 |
| 2494 | RP11-346L24 | 14 | 48879617 | 49050961 | -0.0163 | 0.0217 | 0.4021 |
| 117 | RP11-58E21 | 14 | 49439912 | 49597311 | -0.0163 | 0.0217 | 0.4021 |
| 1484 | RP11-255G12 | 14 | 50856205 | 51015468 | -0.0170 | 0.0119 | 0.3854 |
| 1562 | RP11-463J10 | 14 | 51390055 | 51556954 | -0.0170 | 0.0119 | 0.3854 |
| 2177 | RP11-463J10 | 14 | 51390055 | 51556954 | -0.0170 | 0.0119 | 0.3854 |
| 2032 | RP 11-262M8 | 14 | 51907834 | 51964775 | -0.0170 | 0.0119 | 0.3854 |
| 4053 | RP11-533L7 | 14 | 53101869 | 53262854 | -0.0662 | 0.0222 | 0.3894 |
| 3451 | RP11-60K23 | 14 | 53577087 | 53721826 | -0.0660 | 0.0020 | 0.3940 |
| 1331 | RP11-304L20 | 14 | 54378845 | 54570403 | -0.0660 | 0.0020 | 0.3940 |
| 3830 | RP11-484F16 | 14 | 55649062 | 55827391 | -0.0659 | -0.0197 | 0.3958 |
| 4202 | RP11-108M12 | 14 | 56456898 | 56622286 | -0.0659 | -0.0271 | 0.3952 |
| 1459 | RP11-550M19 | 14 | 57270635 | 57420134 | -0.0659 | -0.0347 | 0.3965 |
| 2377 | RP11-550M19 | 14 | 57270635 | 57420134 | -0.0659 | -0.0347 | 0.3965 |
| 271 | RP11-112J1 | 14 | 58322732 | 58470270 | -0.0664 | -0.0525 | 0.3840 |
| 3769 | RP11-112J1 | 14 | 58322732 | 58470270 | -0.0664 | -0.0525 | 0.3840 |
| 1339 | RP11-307P22 | 14 | 60238356 | 60397779 | -0.0662 | -0.0626 | 0.3881 |
| 430 | RP11-47I22 | 14 | 61037391 | 61208390 | -0.0662 | -0.0597 | 0.3887 |
| 1183 | RP11-316E14 | 14 | 74386055 | 74503657 | -0.0665 | -0.0620 | 0.3834 |
| 307 | RP11-368K8 | 14 | 75210598 | 75354768 | -0.0665 | -0.0620 | 0.3834 |
| 2085 | RP11-368K8 | 14 | 75210598 | 75354768 | -0.0665 | -0.0620 | 0.3834 |
| 1248 | RP11-361H10 | 14 | 75653309 | 75832542 | -0.0667 | -0.0561 | 0.3790 |
| 3713 | RP11-361H10 | 14 | 75653309 | 75832542 | -0.0667 | -0.0561 | 0.3790 |
| 3302 | RP11-463C8 | 14 | 76653300 | 76831630 | -0.0662 | -0.0448 | 0.3886 |
| 657 | RP11-61F4 | 14 | 77244986 | 77511223 | -0.0973 | -0.0457 | 0.3939 |
| 1325 | RP11-285P21 | 14 | 77458135 | 77664678 | -0.0973 | -0.0457 | 0.3939 |
| 4072 | RP11-526N18 | 14 | 79340663 | 79518766 | -0.0965 | -0.0233 | 0.4098 |

(continued)

| Order | Clone | chrom | Start | End | sc_ control | sc_ BRCA1 | sd |
|---|---|---|---|---|---|---|---|
| 1292 | RP11-114N19 | 14 | 80497720 | 80657456 | -0.0965 | -0.0233 | 0.4098 |
| 1522 | RP 11-226P 1 | 14 | 81191225 | 81376452 | -0.0965 | -0.0233 | 0.4098 |
| 3069 | RP11-406A9 | 14 | 82169980 | 82316561 | -0.0959 | -0.0448 | 0.4240 |
| 1172 | RP11-179O11 | 14 | 83056583 | 83215764 | -0.0959 | -0.0448 | 0.4240 |
| 3754 | RP11-179O11 | 14 | 83056583 | 83215764 | -0.0959 | -0.0448 | 0.4240 |
| 2071 | RP11-203D9 | 14 | 83853577 | 84001147 | -0.0959 | -0.0448 | 0.4240 |
| 2724 | RP11-50411 | 14 | 84168823 | 84169632 | -0.0959 | -0.0448 | 0.4240 |
| 3995 | RP11-96H16 | 14 | 86077244 | 86231535 | -0.0969 | -0.0532 | 0.4011 |
| 3496 | RP11-315O17 | 14 | 86413307 | 86581958 | -0.0969 | -0.0532 | 0.4011 |
| 3708 | RP11-315O17 | 14 | 86413307 | 86581958 | -0.0969 | -0.0532 | 0.4011 |
| 3437 | RP11-2E15 | 14 | 86833840 | 86986583 | -0.0973 | -0.0436 | 0.3937 |
| 341 | RP11-300J18 | 14 | 87489467 | 87634856 | -0.0969 | -0.0530 | 0.4024 |
| 2087 | RP11-300J18 | 14 | 87489467 | 87634856 | -0.0969 | -0.0530 | 0.4024 |
| 1170 | RP11-164H13 | 14 | 95199486 | 95364094 | -0.0638 | -0.0619 | 0.4206 |
| 1051 | RP11-185P18 | 14 | 95364095 | 95527190 | -0.0638 | -0.0619 | 0.4206 |
| 3083 | RP11-13O24 | 15 | 8632 | 331718 | -0.2556 | -0.0820 | 0.5068 |
| 3501 | RP11-289D12 | 15 | 20428073 | 20542381 | -0.2237 | -0.0629 | 0.3779 |
| 2449 | RP11-131I21 | 15 | 22736020 | 22894878 | -0.1874 | -0.0688 | 0.3806 |
| 3746 | RP11-446P9 | 15 | 23661828 | 23823376 | -0.1874 | -0.0688 | 0.3806 |
| 770 | RP11-570N16 | 15 | 24960279 | 25130030 | -0.1875 | -0.0740 | 0.3781 |
| 4036 | RP11-322N14 | 15 | 25800215 | 25980080 | -0.1875 | -0.0740 | 0.3781 |
| 370 | RP11-408F10 | 15 | 27500519 | 27613844 | -0.1875 | -0.0740 | 0.3781 |
| 2581 | RP11-38E12 | 15 | 28255532 | 28543404 | -0.1658 | -0.0740 | 0.3745 |
| 1174 | RP11-164K24 | 15 | 29207958 | 29366727 | -0.1424 | -0.0587 | 0.3690 |
| 2027 | RP11-250G15 | 15 | 31026423 | 31138717 | -0.1399 | -0.0285 | 0.4216 |
| 2406 | RP11-3D4 | 15 | 31826214 | 31975488 | -0.1422 | -0.0502 | 0.3719 |
| 2808 | RP11-83J16 | 15 | 32893104 | 33055200 | -0.1418 | -0.0718 | 0.3818 |
| 2263 | RP11-70A1 | 15 | 34700382 | 34860406 | -0.1418 | -0.0751 | 0.3823 |
| 2028 | RP11-261P5 | 15 | 35740031 | 35906900 | -0.1418 | -0.0751 | 0.3823 |
| 4165 | CTD-2033D 15 | 15 | 37614149 | 37799843 | -0.1236 | -0.0751 | 0.3855 |
| 3455 | RP11-64K12 | 15 | 38393770 | 38570669 | -0.1236 | -0.0751 | 0.3855 |
| 3529 | RP11-532F12 | 15 | 38766143 | 38944390 | -0.1236 | -0.0751 | 0.3855 |
| 304 | RP11-380D11 | 15 | 39583713 | 39754407 | -0.1235 | -0.0826 | 0.3867 |
| 3334 | RP11-164J13 | 15 | 40399163 | 40544395 | -0.0989 | -0.0825 | 0.3806 |
| 1779 | RP11-355D13 | 15 | 41409158 | 41591921 | -0.0986 | -0.0883 | 0.3871 |
| 3641 | RP11-263I19 | 15 | 41680206 | 41851733 | -0.0986 | -0.0883 | 0.3871 |
| 3325 | RP11-151N17 | 15 | 42593665 | 42756159 | -0.0986 | -0.0883 | 0.3871 |

(continued)

| Order | Clone | chrom | Start | End | sc_ control | sc_ BRCA1 | sd |
|---|---|---|---|---|---|---|---|
| 3520 | RP11-519G16 | 15 | 43413001 | 43613803 | -0.0986 | -0.0883 | 0.3871 |
| 1332 | RP11-315O8 | 15 | 44046010 | 44246699 | -0.0986 | -0.0883 | 0.3871 |
| 4226 | RP11-232J12 | 15 | 51579153 | 51736194 | -0.0921 | -0.0951 | 0.3849 |
| 2137 | RP11-141F2 | 15 | 52559590 | 52719684 | -0.0921 | -0.0951 | 0.3849 |
| 2936 | RP11-141F2 | 15 | 52559590 | 52719684 | -0.0921 | -0.0951 | 0.3849 |
| 3693 | RP11-215J7 | 15 | 52652476 | 52815253 | -0.0921 | -0.0951 | 0.3849 |
| 1542 | RP11-231A23 | 15 | 58182960 | 58247408 | -0.0972 | -0.0888 | 0.3650 |
| 1175 | RP11-169M2 | 15 | 60717917 | 60895399 | -0.0972 | -0.0888 | 0.3650 |
| 3956 | RP 11-24N 10 | 15 | 60932059 | 61119643 | -0.0978 | -0.0960 | 0.3517 |
| 2078 | RP11-236P11 | 15 | 62379521 | 62510410 | -0.0977 | -0.0881 | 0.3544 |
| 3393 | RP11-325L12 | 15 | 63070257 | 63255120 | -0.0975 | -0.0841 | 0.3586 |
| 4042 | RP11-302G2 | 15 | 63873345 | 64073088 | -0.0975 | -0.0867 | 0.3589 |
| 1235 | RP11-348J6 | 15 | 63987575 | 64164144 | -0.0975 | -0.0867 | 0.3589 |
| 4244 | RP11-348J6 | 15 | 63987575 | 64164144 | -0.0975 | -0.0867 | 0.3589 |
| 3347 | RP11-321F6 | 15 | 64661286 | 64838835 | -0.0975 | -0.0867 | 0.3589 |
| 3893 | RP11-100A11 | 15 | 64818125 | 64818855 | -0.0975 | -0.0867 | 0.3589 |
| 3518 | RP11-540N15 | 15 | 64985110 | 65179655 | -0.0975 | -0.0867 | 0.3589 |
| 1195 | RP11-342M21 | 15 | 65164138 | 65330922 | -0.0975 | -0.0867 | 0.3589 |
| 793 | RP11-2I17 | 15 | 70142505 | 70313627 | -0.0542 | -0.0723 | 0.3942 |
| 1553 | RP11-361M10 | 15 | 70693862 | 70907606 | -0.0542 | -0.0723 | 0.3945 |
| 1768 | RP11-361M10 | 15 | 70693862 | 70907606 | -0.0542 | -0.0723 | 0.3945 |
| 2033 | RP11-272D12 | 15 | 71270952 | 71440843 | -0.0542 | -0.0723 | 0.3945 |
| 2943 | RP11-8P11 | 15 | 71764663 | 71904838 | -0.0542 | -0.0801 | 0.3937 |
| 3727 | RP11-414J4 | 15 | 72768830 | 72953361 | -0.0549 | -0.0614 | 0.4097 |
| 4212 | RP 11-94P 14 | 15 | 75185320 | 75348639 | 0.0065 | 0.0048 | 0.5147 |
| 12 | RP11-499F3 | 15 | 79676203 | 79756580 | -0.0130 | 0.0248 | 0.4952 |
| 3329 | RP11-152F13 | 15 | 80887175 | 81024207 | -0.0130 | 0.0319 | 0.4961 |
| 2051 | RP11-90B9 | 15 | 81348690 | 81488675 | -0.0130 | 0.0319 | 0.4961 |
| 295 | RP11-365F16 | 15 | 82005246 | 82186756 | -0.0116 | 0.0644 | 0.5251 |
| 230 | RP11-565O12 | 15 | 83040659 | 83070787 | -0.0122 | 0.0789 | 0.5131 |
| 1905 | RP11-535P8 | 15 | 83685716 | 83883618 | -0.0134 | 0.0625 | 0.4867 |
| 813 | RP11-133L19 | 15 | 85186168 | 85362806 | -0.0317 | 0.0503 | 0.4923 |
| 1787 | RP11-356B18 | 15 | 86472170 | 86639789 | -0.0317 | 0.0503 | 0.4923 |
| 4264 | RP11-356B18 | 15 | 86472170 | 86639789 | -0.0317 | 0.0503 | 0.4923 |
| 3397 | RP11-326A19 | 15 | 87338936 | 87527181 | -0.0226 | 0.0686 | 0.4939 |
| 2066 | RP11-233C13 | 15 | 88091170 | 88207896 | -0.0219 | 0.0787 | 0.5089 |
| 4298 | RP11-233C13 | 15 | 88091170 | 88207896 | -0.0219 | 0.0787 | 0.5089 |

(continued)

| Order | Clone | chrom | Start | End | sc_control | sc_BRCA1 | sd |
|---|---|---|---|---|---|---|---|
| 3576 | RP3-443N8 | 15 | 88522448 | 88672448 | -0.0219 | 0.0781 | 0.5110 |
| 3056 | RP1-138O23 | 15 | 88798992 | 88948992 | -0.0220 | 0.0949 | 0.5071 |
| 1727 | RP11-405A15 | 15 | 89241954 | 89260209 | 0.0087 | 0.0991 | 0.5082 |
| 1493 | RP11-266O8 | 15 | 91550182 | 91714423 | 0.0074 | 0.1041 | 0.4800 |
| 429 | RP11-41P8 | 15 | 92324496 | 92491964 | 0.0075 | 0.0949 | 0.4835 |
| 436 | RP11-34L4 | 15 | 93038001 | 93169755 | 0.0075 | 0.0949 | 0.4835 |
| 2144 | RP11-34L4 | 15 | 93038001 | 93169755 | 0.0075 | 0.0949 | 0.4835 |
| 4308 | RP 11-44B21 | 15 | 93987934 | 94138221 | -0.0099 | 0.0949 | 0.4830 |
| 1328 | RP11-315L6 | 15 | 95508704 | 95699734 | -0.0099 | 0.0949 | 0.4830 |
| 1700 | RP11-183E24 | 15 | 96460021 | 96572341 | -0.0092 | 0.1174 | 0.4982 |
| 3355 | RP11-342L10 | 15 | 96860057 | 97061845 | -0.0092 | 0.1174 | 0.4982 |
| 3633 | RP11-262P8 | 15 | 97047742 | 97225942 | -0.0092 | 0.1174 | 0.4982 |
| 4215 | RP11-90E5 | 15 | 98388046 | 98574562 | -0.0092 | 0.1174 | 0.4982 |
| 3323 | RP11-168G16 | 15 | 98677983 | 98814208 | -0.0092 | 0.1174 | 0.4982 |
| 4022 | RP 11-299G20 | 15 | 99550339 | 99734009 | -0.0092 | 0.1174 | 0.4982 |
| 1382 | GS-124-5 | 15 | 99963915 | 100113915 | -0.0079 | 0.0925 | 0.5276 |
| 3546 | GS-154-P1 | 15 | 99963965 | 100113965 | -0.0079 | 0.0925 | 0.5276 |
| 3886 | RP11-243K18 | 16 | 266993 | 431793 | -0.0447 | -0.0548 | 0.3994 |
| 1182 | RP11-161M6 | 16 | 919651 | 1081992 | -0.0435 | -0.0717 | 0.3735 |
| 4158 | RP11-304L19 | 16 | 2081103 | 2267414 | -0.0435 | -0.0717 | 0.3735 |
| 4288 | RP11-433P17 | 16 | 3292433 | 3471943 | -0.0433 | -0.0823 | 0.3693 |
| 2169 | RP11-462G12 | 16 | 3918289 | 4131849 | -0.0433 | -0.0625 | 0.3690 |
| 3084 | RP11-35P16 | 16 | 4441438 | 4520536 | -0.0432 | -0.0577 | 0.3687 |
| 1710 | RP11-167B4 | 16 | 6171414 | 6339845 | -0.0629 | -0.0617 | 0.3523 |
| 2990 | RP11-148F10 | 16 | 7933143 | 8120601 | -0.0629 | -0.0617 | 0.3523 |
| 3853 | RP 11-152P23 | 16 | 8777495 | 8852419 | -0.0625 | -0.0380 | 0.3604 |
| 4309 | RP 11-152P23 | 16 | 8777495 | 8852419 | -0.0625 | -0.0380 | 0.3604 |
| 3490 | RP 11-297M9 | 16 | 9634532 | 9827751 | -0.0626 | -0.0319 | 0.3583 |
| 3119 | RP 11-27M24 | 16 | 10507665 | 10596534 | -0.0626 | -0.0319 | 0.3583 |
| 543 | RP11-490O6 | 16 | 11635175 | 11796320 | -0.0608 | -0.0251 | 0.3976 |
| 424 | RP11-31O11 | 16 | 11848758 | 12014145 | -0.0608 | -0.0251 | 0.3976 |
| 4304 | RP11-31O11 | 16 | 11848758 | 12014145 | -0.0608 | -0.0251 | 0.3976 |
| 373 | RP11-276H1 | 16 | 12014799 | 12176022 | -0.0623 | -0.0391 | 0.3647 |
| 2804 | RP11-82O18 | 16 | 14602445 | 14723189 | -0.0625 | -0.0375 | 0.3617 |
| 1674 | RP11-489O1 | 16 | 15411955 | 15573494 | -0.0625 | -0.0375 | 0.3617 |
| 2009 | CTD-2504F3 | 16 | 15986518 | 16192266 | -0.0625 | -0.0375 | 0.3617 |
| 296 | RP11-378B23 | 16 | 16783566 | 16933566 | -0.0626 | -0.0409 | 0.3598 |

(continued)

| Order | Clone | chrom | Start | End | sc_control | sc_BRCA1 | sd |
|-------|-------|-------|-------|-----|------------|----------|-----|
| 3497 | RP11-288I13 | 16 | 17137869 | 17316758 | -0.0626 | -0.0409 | 0.3598 |
| 2692 | RP11-626G11 | 16 | 18850659 | 19066922 | -0.0625 | -0.0379 | 0.3605 |
| 2684 | RP11-429K17 | 16 | 20151302 | 20315827 | -0.0625 | -0.0379 | 0.3605 |
| 3834 | RP11-489A11 | 16 | 21304276 | 21473433 | -0.0628 | -0.0301 | 0.3544 |
| 4205 | RP11-101E7 | 16 | 21936632 | 22116416 | -0.0628 | -0.0301 | 0.3544 |
| 1676 | RP11-105C19 | 16 | 22508689 | 22679811 | -0.0628 | -0.0301 | 0.3544 |
| 3765 | RP11-105C19 | 16 | 22508689 | 22679811 | -0.0628 | -0.0301 | 0.3544 |
| 650 | RP11-67I10 | 16 | 26107482 | 26264124 | -0.0236 | -0.0249 | 0.3537 |
| 1836 | RP11-142A12 | 16 | 26595064 | 26727359 | -0.0236 | -0.0249 | 0.3537 |
| 4301 | RP11-142A12 | 16 | 26595064 | 26727359 | -0.0236 | -0.0249 | 0.3537 |
| 4094 | F21283 | 16 | 28579078 | 28729078 | -0.0236 | -0.0371 | 0.3547 |
| 2962 | RP11-5L1 | 16 | 45067244 | 45231075 | -0.0267 | -0.0829 | 0.3739 |
| 2022 | RP11-283C7 | 16 | 45627546 | 45688113 | -0.0568 | -0.0923 | 0.3856 |
| 3711 | RP11-283C7 | 16 | 45627546 | 45688113 | -0.0568 | -0.0923 | 0.3856 |
| 822 | RP11-147B17 | 16 | 49090412 | 49252652 | -0.1116 | -0.1251 | 0.3714 |
| 3740 | RP 11-424K7 | 16 | 49618230 | 49794206 | -0.1116 | -0.1294 | 0.3725 |
| 812 | RP11-122K22 | 16 | 50431358 | 50611758 | -0.1119 | -0.1369 | 0.3783 |
| 2701 | RP11-467J12 | 16 | 51466013 | 51629836 | -0.1120 | -0.1450 | 0.3812 |
| 1232 | RP11-357N13 | 16 | 52448777 | 52626499 | -0.1120 | -0.1450 | 0.3812 |
| 3709 | RP11-357N13 | 16 | 52448777 | 52626499 | -0.1120 | -0.1450 | 0.3812 |
| 2415 | RP11-7O2 | 16 | 53823781 | 53975887 | -0.1120 | -0.1585 | 0.3818 |
| 1702 | RP11-165M2 | 16 | 54486638 | 54559962 | -0.1118 | -0.1703 | 0.3771 |
| 1229 | RP11-325K4 | 16 | 55378542 | 55559305 | -0.1120 | -0.1793 | 0.3809 |
| 2470 | RP 11-405F3 | 16 | 56181076 | 56331523 | -0.1122 | -0.1848 | 0.3850 |
| 2090 | RP11-481J2 | 16 | 56965501 | 57139976 | -0.1120 | -0.1928 | 0.3807 |
| 2706 | RP11-481J2 | 16 | 56965501 | 57139976 | -0.1120 | -0.1928 | 0.3807 |
| 2057 | RP11-105C20 | 16 | 58235437 | 58349316 | -0.1122 | -0.1984 | 0.3859 |
| 4282 | RP11-457D20 | 16 | 58776524 | 58993500 | -0.1122 | -0.1984 | 0.3859 |
| 3314 | RP11-157H19 | 16 | 60426260 | 60527522 | -0.1131 | -0.2146 | 0.4044 |
| 2705 | RP11-467O15 | 16 | 61098231 | 61286993 | -0.1131 | -0.2146 | 0.4044 |
| 830 | RP11-148F12 | 16 | 61943888 | 62080690 | -0.1130 | -0.2200 | 0.4018 |
| 2995 | RP11-370P15 | 16 | 62221465 | 62396983 | -0.1124 | -0.2102 | 0.3898 |
| 541 | RP11-467L24 | 16 | 62921648 | 63071733 | -0.1124 | -0.2102 | 0.3898 |
| 1538 | RP11-229O3 | 16 | 63462867 | 63616454 | -0.1124 | -0.2102 | 0.3898 |
| 3498 | RP11-298C15 | 16 | 63934753 | 64142097 | -0.0844 | -0.2185 | 0.3897 |
| 4243 | RP11-298C15 | 16 | 63934753 | 64142097 | -0.0844 | -0.2185 | 0.3897 |
| 1291 | RP11-63M22 | 16 | 65152748 | 65333456 | -0.1265 | -0.2227 | 0.3957 |

(continued)

| Order | Clone | chrom | Start | End | sc_ control | sc_ BRCA1 | sd |
|---|---|---|---|---|---|---|---|
| 1155 | RP11-167P11 | 16 | 66238838 | 66393297 | -0.1265 | -0.2227 | 0.3957 |
| 2819 | RP 11-76H6 | 16 | 67078490 | 67134347 | -0.1262 | -0.2189 | 0.3896 |
| 3359 | RP11-354N7 | 16 | 67318526 | 67478802 | -0.0749 | -0.2061 | 0.3893 |
| 816 | RP11-123C5 | 16 | 67554082 | 67731555 | -0.0749 | -0.2061 | 0.3893 |
| 4031 | RP11-311C24 | 16 | 68224900 | 68296605 | -0.0750 | -0.1990 | 0.3912 |
| 1563 | RP11-296I10 | 16 | 68690128 | 68870723 | -0.0753 | -0.2044 | 0.3984 |
| 3486 | RP11-296I10 | 16 | 68690128 | 68870723 | -0.0753 | -0.2044 | 0.3984 |
| 2469 | RP11-394B2 | 16 | 69203922 | 69365103 | -0.0752 | -0.2120 | 0.3967 |
| 3305 | RP 11-417N 10 | 16 | 70330095 | 70453950 | -0.0978 | -0.2121 | 0.3931 |
| 2267 | RP 11-70E3 | 16 | 70619026 | 70788093 | -0.0978 | -0.2121 | 0.3931 |
| 2735 | RP11-328J14 | 16 | 70783654 | 70973636 | -0.0978 | -0.2121 | 0.3931 |
| 3194 | RP5-991G20 | 16 | 71316768 | 71457881 | -0.0986 | -0.2008 | 0.4107 |
| 3485 | RP11-285K4 | 16 | 72300075 | 72453658 | -0.0986 | -0.2008 | 0.4107 |
| 100 | RP11-77K12 | 16 | 73977115 | 74134472 | -0.0986 | -0.2008 | 0.4107 |
| 4219 | RP11-200H5 | 16 | 74775676 | 74819173 | -0.0987 | -0.2104 | 0.4136 |
| 3516 | RP11-518I8 | 16 | 75914617 | 76024261 | -0.0987 | -0.2104 | 0.4136 |
| 3432 | RP11-24I3 | 16 | 76343711 | 76539056 | -0.1210 | -0.2104 | 0.4102 |
| 4174 | RP11-281J9 | 16 | 76447923 | 76606529 | -0.1210 | -0.2104 | 0.4102 |
| 2917 | RP11-556H2 | 16 | 77599698 | 77781811 | -0.1214 | -0.2258 | 0.4182 |
| 3483 | RP11-303E16 | 16 | 79571775 | 79773939 | -0.1214 | -0.2258 | 0.4182 |
| 4251 | RP11-303E16 | 16 | 79571775 | 79773939 | -0.1214 | -0.2258 | 0.4182 |
| 3290 | RP11-446C5 | 16 | 79834964 | 80033098 | -0.1216 | -0.2167 | 0.4229 |
| 3849 | RP11-437L22 | 16 | 80148499 | 80307562 | -0.1216 | -0.2167 | 0.4229 |
| 2402 | RP11-2L4 | 16 | 81162708 | 81279605 | -0.1217 | -0.2113 | 0.4242 |
| 558 | RP11-483P21 | 16 | 82361608 | 82534818 | -0.1219 | -0.1943 | 0.4288 |
| 4254 | RP11-483P21 | 16 | 82361608 | 82534818 | -0.1219 | -0.1943 | 0.4288 |
| 1480 | RP11-254F19 | 16 | 83357615 | 83531735 | -0.1215 | -0.1926 | 0.4197 |
| 2170 | RP11-478M13 | 16 | 84379908 | 84540021 | -0.1213 | -0.1876 | 0.4158 |
| 1907 | RP11-514D23 | 16 | 84812502 | 84922043 | -0.1422 | -0.2044 | 0.3923 |
| 1251 | RP11-21B21 | 16 | 87086505 | 87260489 | -0.1421 | -0.1974 | 0.3909 |
| 1401 | RP4-597G12 | 16 | 88403055 | 88495451 | -0.1429 | -0.1873 | 0.4071 |
| 3533 | RP11-533D19 | 16 | 88421159 | 88643456 | -0.1429 | -0.1873 | 0.4071 |
| 3550 | GS-191-P24 | 16 | 88552254 | 88702254 | -0.1429 | -0.1873 | 0.4071 |
| 3697 | RP11-216P6 | 17 | 800495 | 1008155 | -0.1480 | -0.1417 | 0.4192 |
| 786 | RP11-4F24 | 17 | 1564767 | 1565500 | -0.1480 | -0.1417 | 0.4192 |
| 821 | RP11-135N5 | 17 | 2316192 | 2492179 | -0.1480 | -0.1417 | 0.4192 |
| 1198 | RP11-199F11 | 17 | 7516212 | 7599153 | -0.1691 | -0.1925 | 0.4146 |

(continued)

| Order | Clone | chrom | Start | End | sc_control | sc_BRCA1 | sd |
|---|---|---|---|---|---|---|---|
| 2466 | RP 11-404G1 | 17 | 7563684 | 7682367 | -0.1691 | -0.1925 | 0.4146 |
| 3977 | RP11-12H18 | 17 | 8446409 | 8574345 | -0.1691 | -0.1925 | 0.4146 |
| 3700 | RP11-208F13 | 17 | 9466335 | 9611536 | -0.1683 | -0.2213 | 0.3969 |
| 3293 | RP11-401O9 | 17 | 10055057 | 10253638 | -0.1683 | -0.2213 | 0.3969 |
| 2717 | RP11-471L13 | 17 | 11940105 | 12065840 | -0.1681 | -0.2304 | 0.3928 |
| 15 | RP11-488L1 | 17 | 12940551 | 13028533 | -0.1677 | -0.2429 | 0.3828 |
| 3034 | RP11-388F14 | 17 | 13226766 | 13375795 | -0.1677 | -0.2429 | 0.3828 |
| 3559 | RP1-27J12 | 17 | 14285924 | 14360892 | -0.1677 | -0.2429 | 0.3828 |
| 569 | RP11-64B12 | 17 | 14531958 | 14598528 | -0.1677 | -0.2429 | 0.3828 |
| 3004 | RP11-385D13 | 17 | 15374763 | 15435531 | -0.1676 | -0.2461 | 0.3805 |
| 2061 | RP11-219A15 | 17 | 16646746 | 16718827 | -0.2025 | -0.2553 | 0.3820 |
| 3299 | RP11-524F11 | 17 | 17338138 | 17521471 | -0.2022 | -0.2478 | 0.3767 |
| 1712 | RP11-189D22 | 17 | 17940393 | 18114679 | -0.2022 | -0.2478 | 0.3767 |
| 3200 | RP1-162E17 | 17 | 19126535 | 19251679 | -0.2022 | -0.2478 | 0.3767 |
| 3629 | CTB-1187M2 | 17 | 19211694 | 19360796 | -0.2022 | -0.2478 | 0.3767 |
| 2737 | RP11-78O7 | 17 | 19614001 | 19748680 | -0.2016 | -0.2400 | 0.3625 |
| 1961 | RP5-836L9 | 17 | 20014357 | 20134155 | -0.2016 | -0.2400 | 0.3625 |
| 46 | RP11-121A13 | 17 | 20134151 | 20230382 | -0.2016 | -0.2400 | 0.3625 |
| 519 | RP11-64J19 | 17 | 21055083 | 21191541 | -0.2016 | -0.2400 | 0.3625 |
| 3216 | RP11-744K17 | 17 | 21822900 | 21916486 | -0.2014 | -0.2465 | 0.3581 |
| 4180 | RP11-260A9 | 17 | 22287134 | 22465691 | -0.1545 | -0.2397 | 0.3515 |
| 2434 | RP11-138P22 | 17 | 23133770 | 23308272 | -0.1545 | -0.2397 | 0.3515 |
| 3035 | RP11-192H23 | 17 | 23864147 | 24008957 | -0.0864 | -0.2291 | 0.3995 |
| 4213 | RP11-104120 | 17 | 25164209 | 25184918 | -0.0864 | -0.2291 | 0.3995 |
| 302 | RP11-403E9 | 17 | 25591017 | 25693302 | -0.0237 | -0.2201 | 0.4841 |
| 3698 | RP11-229K15 | 17 | 26294586 | 26453072 | -0.1541 | -0.2123 | 0.3622 |
| 3338 | RP11-142O6 | 17 | 26429957 | 26575104 | -0.1541 | -0.2123 | 0.3622 |
| 557 | RP11-474K4 | 17 | 27416322 | 27596064 | -0.1541 | -0.2123 | 0.3622 |
| 1399 | RP1-29G21 | 17 | 28308287 | 28423825 | -0.1566 | -0.2124 | 0.4185 |
| 3840 | RP11-139K6 | 17 | 28664934 | 28816067 | -0.1566 | -0.2124 | 0.4185 |
| 1529 | RP11-215E13 | 17 | 29522483 | 29667899 | -0.1567 | -0.2012 | 0.4193 |
| 3562 | RP5-837J1 | 17 | 30346236 | 30441459 | -0.1315 | -0.1844 | 0.3738 |
| 45 | RP11-47L3 | 17 | 30523516 | 30685989 | -0.1313 | -0.1933 | 0.3712 |
| 3738 | RP11-445F12 | 17 | 32246319 | 32371528 | -0.0897 | -0.1894 | 0.3865 |
| 4122 | RP5-986F12 | 17 | 33099924 | 33206361 | -0.0903 | -0.1804 | 0.4011 |
| 4280 | RP11-432121 | 17 | 33794727 | 33977073 | -0.0903 | -0.1804 | 0.4011 |
| 3621 | CTB-1058E17 | 17 | 33964845 | 34251503 | -0.0906 | -0.1688 | 0.4064 |

(continued)

| Order | Clone | chrom | Start | End | sc_ control | sc_ BRCA1 | sd |
|---|---|---|---|---|---|---|---|
| 4106 | RP5-906A24 | 17 | 34510775 | 34624022 | -0.0906 | -0.1688 | 0.4064 |
| 2196 | RP11-390P24 | 17 | 34803854 | 34979173 | -0.0904 | -0.1802 | 0.4026 |
| 3885 | RP11-94L15 | 17 | 35065321 | 35227135 | -0.0906 | -0.1632 | 0.4062 |
| 3053 | RP5-1112G21 | 17 | 35682018 | 35682629 | -0.1429 | -0.1634 | 0.3939 |
| 637 | RP11-58O9 | 17 | 35754712 | 35920945 | -0.1429 | -0.1634 | 0.3939 |
| 2660 | RP5-1110E20 | 17 | 36260511 | 36373721 | -0.1430 | -0.1529 | 0.3960 |
| 3869 | RP11-156E6 | 17 | 37192545 | 37370889 | -0.1430 | -0.1494 | 0.3956 |
| 2744 | RP11-506G7 | 17 | 38139932 | 38327654 | -0.1077 | -0.1495 | 0.3889 |
| 3883 | RP11-376M2 | 17 | 38221253 | 38425998 | -0.1077 | -0.1495 | 0.3889 |
| 2008 | RP11-948G15 | 17 | 38425359 | 38425924 | -0.1079 | -0.1382 | 0.3925 |
| 36 | RP11-392O1 | 17 | 38932046 | 39091576 | -0.1078 | -0.1325 | 0.3907 |
| 1942 | RP5-905N1 | 17 | 39182384 | 39277131 | -0.1078 | -0.1325 | 0.3907 |
| 1045 | RP5-1067M6 | 17 | 39683991 | 39821481 | -0.0361 | -0.1261 | 0.4186 |
| 2654 | RP5-1169K15 | 17 | 40698240 | 40742647 | -0.0363 | -0.1084 | 0.4235 |
| 3193 | RP5-843B9 | 17 | 41441703 | 41565982 | -0.0363 | -0.1084 | 0.4235 |
| 4118 | RP5-971F3 | 17 | 41818827 | 41939592 | -0.0420 | -0.2518 | 0.5467 |
| 2065 | RP 11-220N20 | 17 | 42226771 | 42268630 | -0.0375 | -0.1378 | 0.4492 |
| 3739 | RP11-416K7 | 17 | 42875373 | 43043115 | -0.0373 | -0.1060 | 0.4454 |
| 3928 | RP11-361K8 | 17 | 43957672 | 44125738 | -0.0030 | -0.0579 | 0.4155 |
| 3571 | RP1-6209 | 17 | 44647599 | 44753692 | -0.0030 | -0.0579 | 0.4155 |
| 577 | RP11-81K2 | 17 | 44908966 | 44942436 | -0.0030 | -0.0579 | 0.4155 |
| 1410 | RP5-875H18 | 17 | 45492779 | 45587911 | -0.0030 | -0.0579 | 0.4155 |
| 585 | RP11-94C24 | 17 | 45874046 | 46041875 | -0.0035 | -0.0497 | 0.4258 |
| 3515 | RP11-506D12 | 17 | 46263624 | 46333071 | -0.0035 | -0.0449 | 0.4255 |
| 2702 | RP11-481C4 | 17 | 46505878 | 46634205 | -0.0048 | -0.0305 | 0.4542 |
| 4250 | RP11-481C4 | 17 | 46505878 | 46634205 | -0.0048 | -0.0305 | 0.4542 |
| 2023 | RP11-248L3 | 17 | 48434263 | 48595726 | -0.0297 | -0.0533 | 0.4083 |
| 3201 | RP5-1043H15 | 17 | 48606238 | 48664580 | -0.0296 | -0.0451 | 0.4056 |
| 3025 | RP11-312B18 | 17 | 49013794 | 49212728 | -0.0296 | -0.0451 | 0.4056 |
| 1537 | RP11-217N19 | 17 | 49753469 | 49922957 | -0.0291 | -0.0547 | 0.3936 |
| 3003 | RP11-372K20 | 17 | 50112652 | 50268101 | -0.0291 | -0.0547 | 0.3936 |
| 1911 | RP11-515O17 | 17 | 50675072 | 50836282 | -0.0291 | -0.0547 | 0.3936 |
| 2102 | RP11-515O17 | 17 | 50675072 | 50836282 | -0.0291 | -0.0547 | 0.3936 |
| 2435 | RP11-112J9 | 17 | 52088163 | 52172786 | -0.0291 | -0.0547 | 0.3936 |
| 3958 | RP11-19F16 | 17 | 52616475 | 52760769 | -0.0291 | -0.0547 | 0.3936 |
| 3608 | RP11-579A4 | 17 | 54149949 | 54280513 | -0.0291 | -0.0547 | 0.3936 |
| 3070 | RP11-567L7 | 17 | 54696369 | 54872935 | -0.0291 | -0.0402 | 0.3935 |

(continued)

| Order | Clone | chrom | Start | End | sc_control | sc_BRCA1 | sd |
|---|---|---|---|---|---|---|---|
| 1160 | RP11-178C3 | 17 | 55387410 | 55498115 | -0.0291 | -0.0402 | 0.3935 |
| 3351 | RP11-332H18 | 17 | 56659719 | 56847075 | -0.0290 | -0.0329 | 0.3916 |
| 1774 | RP11-342K2 | 17 | 57418528 | 57473362 | -0.0290 | -0.0329 | 0.3916 |
| 1701 | RP11-155C2 | 17 | 69446939 | 69639765 | -0.0206 | 0.0071 | 0.3923 |
| 2174 | RP11-478P5 | 17 | 69778939 | 69931286 | -0.0206 | 0.0071 | 0.3923 |
| 3691 | RP11-196E5 | 17 | 71063165 | 71247066 | -0.0209 | -0.0116 | 0.3863 |
| 1499 | RP11-87G24 | 17 | 72342642 | 72523921 | -0.0207 | -0.0108 | 0.3908 |
| 286 | RP11-141D15 | 17 | 73636230 | 73813841 | -0.0206 | -0.0044 | 0.3943 |
| 1880 | RP11-323N12 | 17 | 74200806 | 74384944 | -0.0206 | -0.0044 | 0.3943 |
| 849 | RP11-398J5 | 17 | 74610889 | 74792977 | -0.0206 | -0.0044 | 0.3943 |
| 4043 | RP11-313F15 | 17 | 76183534 | 76354885 | -0.0203 | 0.0026 | 0.3996 |
| 1900 | RP11-525L23 | 17 | 77988926 | 78221351 | -0.0199 | 0.0090 | 0.4074 |
| 2106 | RP11-525L23 | 17 | 77988926 | 78221351 | -0.0199 | 0.0090 | 0.4074 |
| 758 | RP11-567O16 | 17 | 78311474 | 78374827 | -0.0199 | 0.0090 | 0.4074 |
| 1919 | GS-50-C4 | 17 | 78499742 | 78649742 | -0.0199 | 0.0090 | 0.4074 |
| 3554 | GS-362-K4 | 17 | 78609742 | 78759742 | -0.0202 | -0.0146 | 0.4030 |
| 1394 | GS-52-M11 | 18 | 1 | 125000 | -0.1875 | -0.1621 | 0.5212 |
| 1380 | GS-74-G18 | 18 | 137949 | 287949 | 0.0361 | -0.0161 | 0.4287 |
| 4044 | RP11-324G2 | 18 | 168428 | 340445 | 0.0361 | -0.0161 | 0.4287 |
| 4173 | RP 11-267C19 | 18 | 957554 | 1114347 | 0.0361 | -0.0161 | 0.4287 |
| 1861 | RP11-291G24 | 18 | 1551101 | 1734182 | 0.0361 | -0.0161 | 0.4287 |
| 4279 | RP11-419P8 | 18 | 2479734 | 2665807 | 0.0365 | -0.0050 | 0.4203 |
| 1506 | RP11-106J7 | 18 | 3268738 | 3442992 | 0.0367 | 0.0019 | 0.4169 |
| 548 | RP11-502P1 | 18 | 3863227 | 4046692 | 0.0369 | 0.0115 | 0.4131 |
| 1516 | RP11-92G19 | 18 | 4926254 | 5083821 | 0.0370 | 0.0071 | 0.4109 |
| 3946 | RP11-344B7 | 18 | 5260008 | 5444854 | 0.0370 | 0.0071 | 0.4109 |
| 1998 | RP11-39D15 | 18 | 6041920 | 6213636 | 0.0378 | 0.0159 | 0.3923 |
| 2982 | RP11-146G7 | 18 | 6752909 | 6933645 | 0.0378 | 0.0159 | 0.3923 |
| 970 | RP11-51B9 | 18 | 7827406 | 7985296 | 0.0381 | 0.0232 | 0.3869 |
| 3418 | RP11-17J14 | 18 | 19024430 | 19181612 | -0.0408 | 0.0076 | 0.4007 |
| 1164 | RP11-178F10 | 18 | 20259694 | 20371250 | 0.0369 | 0.0183 | 0.4893 |
| 1514 | RP11-107K17 | 18 | 22172290 | 22273859 | 0.1597 | 0.0275 | 0.4851 |
| 1908 | RP11-52618 | 18 | 22781318 | 22931013 | 0.1418 | -0.0033 | 0.4911 |
| 1673 | RP11-430E17 | 18 | 23970883 | 24165115 | 0.1418 | -0.0033 | 0.4911 |
| 3962 | RP 11-20A 14 | 18 | 25599565 | 25763888 | 0.0105 | -0.0177 | 0.4313 |
| 1337 | RP11-289A1 | 18 | 26696058 | 26878315 | 0.0386 | -0.0092 | 0.4378 |
| 1906 | RP11-549B18 | 18 | 27447010 | 27569489 | 0.0383 | -0.0218 | 0.4445 |

(continued)

| Order | Clone | chrom | Start | End | sc_ control | sc_ BRCA1 | sd |
|---|---|---|---|---|---|---|---|
| 850 | RP11-413I9 | 18 | 28294303 | 28415096 | 0.0387 | -0.0363 | 0.4347 |
| 2048 | RP11-94E2 | 18 | 28808364 | 28955525 | 0.0387 | -0.0363 | 0.4347 |
| 218 | RP11-562H1 | 18 | 29409484 | 29472060 | 0.0365 | -0.0534 | 0.4824 |
| 542 | RP11-481E24 | 18 | 30773825 | 30859605 | 0.0011 | -0.0373 | 0.4073 |
| 316 | RP11-383C19 | 18 | 32335236 | 32502050 | 0.0011 | -0.0373 | 0.4073 |
| 2797 | RP11-58G13 | 18 | 32721707 | 32885642 | -0.0096 | -0.0373 | 0.4092 |
| 106 | RP 11-62M21 | 18 | 34297490 | 34473929 | -0.0465 | -0.0539 | 0.4156 |
| 1694 | RP11-164M8 | 18 | 37363566 | 37482575 | -0.0467 | -0.0637 | 0.4202 |
| 1510 | RP11-107G7 | 18 | 38035420 | 38205929 | -0.0470 | -0.0582 | 0.4247 |
| 11 | RP11-486C18 | 18 | 40044271 | 40244003 | -0.0584 | -0.0631 | 0.4272 |
| 13 | RP11-463D17 | 18 | 41216566 | 41408713 | -0.0585 | -0.0839 | 0.4287 |
| 2423 | RP 11-8H2 | 18 | 41851567 | 41984947 | -0.0585 | -0.0839 | 0.4287 |
| 865 | RP11-313C14 | 18 | 42350383 | 42350954 | -0.0585 | -0.0839 | 0.4287 |
| 2275 | RP11-71F23 | 18 | 43069061 | 43274200 | -0.0585 | -0.0839 | 0.4287 |
| 4152 | RP11-748M14 | 18 | 43589519 | 43745379 | -0.0584 | -0.0873 | 0.4274 |
| 418 | RP11-46D1 | 18 | 44393176 | 44552924 | -0.0585 | -0.1103 | 0.4276 |
| 655 | RP 11-76E22 | 18 | 46462730 | 46633371 | -0.0585 | -0.1311 | 0.4293 |
| 1468 | RP 11-729G3 | 18 | 46732284 | 46888494 | -0.0585 | -0.1311 | 0.4293 |
| 245 | RP11-1E21 | 18 | 47274828 | 47443303 | -0.0585 | -0.1311 | 0.4293 |
| 1181 | RP11-25O3 | 18 | 47958320 | 48119508 | -0.0585 | -0.1311 | 0.4293 |
| 643 | RP11-73I6 | 18 | 48386193 | 48496258 | -0.0588 | -0.1368 | 0.4361 |
| 823 | RP11-116K4 | 18 | 49795841 | 49971830 | -0.1009 | -0.1492 | 0.4232 |
| 1505 | RP11-99A1 | 18 | 50563151 | 50702093 | -0.1009 | -0.1492 | 0.4232 |
| 845 | RP11-397A16 | 18 | 51445553 | 51648118 | -0.1013 | -0.1653 | 0.4311 |
| 2996 | RP11-383D22 | 18 | 52656265 | 52867730 | -0.1017 | -0.1442 | 0.4408 |
| 3080 | RP11-35G9 | 18 | 53447744 | 53561700 | -0.1017 | -0.1442 | 0.4408 |
| 98 | RP11-61J14 | 18 | 54567051 | 54747660 | -0.1013 | -0.1505 | 0.4308 |
| 1247 | RP11-350K6 | 18 | 54867252 | 55027999 | -0.1013 | -0.1642 | 0.4328 |
| 4248 | RP11-350K6 | 18 | 54867252 | 55027999 | -0.1013 | -0.1642 | 0.4328 |
| 3005 | RP11-396N11 | 18 | 56063594 | 56151592 | -0.1013 | -0.1642 | 0.4328 |
| 3524 | RP11-520K18 | 18 | 56874822 | 57034619 | -0.1018 | -0.1729 | 0.4418 |
| 1525 | RP 11-215A20 | 18 | 58572412 | 58756503 | -0.1018 | -0.1729 | 0.4418 |
| 3345 | RP11-28F1 | 18 | 59074167 | 59175738 | -0.1018 | -0.1729 | 0.4418 |
| 4230 | RP11-233O10 | 18 | 59886252 | 59971318 | -0.1158 | -0.1958 | 0.4352 |
| 852 | RP11-389J22 | 18 | 61594898 | 61752947 | -0.1158 | -0.1958 | 0.4352 |
| 3420 | RP11-23N5 | 18 | 62491384 | 62645196 | -0.1478 | -0.2044 | 0.4402 |
| 2528 | RP11-21L20 | 18 | 64827903 | 64947832 | -0.1481 | -0.2354 | 0.4476 |

(continued)

| Order | Clone | chrom | Start | End | sc_ control | sc_ BRCA1 | sd |
|-------|-------|-------|-------|-----|-------------|-----------|-----|
| 3934 | RP11-342G3 | 18 | 64985278 | 65144962 | -0.1481 | -0.2354 | 0.4476 |
| 2163 | RP11-484N16 | 18 | 66000833 | 66160916 | -0.1481 | -0.2354 | 0.4476 |
| 4276 | RP11-430H7 | 18 | 66753077 | 66929602 | -0.1481 | -0.2354 | 0.4476 |
| 965 | RP11-45A1 | 18 | 67916649 | 68041627 | -0.1481 | -0.2354 | 0.4476 |
| 3331 | RP11-169F17 | 18 | 68809458 | 69000813 | -0.1481 | -0.2354 | 0.4476 |
| 415 | RP11-25L3 | 18 | 69588036 | 69755177 | -0.1481 | -0.2354 | 0.4476 |
| 3631 | RP11-238P13 | 18 | 70484649 | 70635265 | -0.1479 | -0.2467 | 0.4422 |
| 837 | RP11-396D4 | 18 | 71168342 | 71337306 | -0.1423 | -0.2615 | 0.4347 |
| 2070 | RP11-234N1 | 18 | 72266630 | 72448118 | -0.1423 | -0.2615 | 0.4347 |
| 2991 | RP11-118I2 | 18 | 73613846 | 73764173 | -0.1418 | -0.2813 | 0.4254 |
| 3969 | RP11-16L7 | 18 | 73908671 | 74017409 | -0.1418 | -0.2813 | 0.4254 |
| 2382 | RP11-563B11 | 18 | 74707626 | 74870951 | -0.1418 | -0.2813 | 0.4254 |
| 4147 | RP11-563B11 | 18 | 74707626 | 74870951 | -0.1418 | -0.2813 | 0.4254 |
| 1544 | RP11-315M18 | 18 | 75519491 | 75520038 | -0.1418 | -0.2813 | 0.4254 |
| 3492 | RP11-315M18 | 18 | 75519491 | 75520038 | -0.1418 | -0.2813 | 0.4254 |
| 1697 | RP11-154H12 | 18 | 75586355 | 75701258 | -0.1418 | -0.2813 | 0.4254 |
| 3558 | GS-964-M9 | 18 | 75752153 | 75902153 | -0.1422 | -0.2723 | 0.4337 |
| 4083 | GS-75-F20 | 18 | 75752203 | 75902203 | -0.1422 | -0.2723 | 0.4337 |
| 672 | CTD-3113P16 | 19 | 183069 | 327646 | -0.2224 | -0.2798 | 0.4038 |
| 1398 | GS-546-C11 | 19 | 300000 | 450000 | -0.2224 | -0.2798 | 0.4038 |
| 3544 | RG-129-F16 | 19 | 300050 | 450050 | -0.2224 | -0.2798 | 0.4038 |
| 125 | CTB-31C16 | 19 | 2595842 | 2773344 | -0.2216 | -0.2897 | 0.3868 |
| 2021 | RP11-268O21 | 19 | 2928774 | 3109041 | -0.2216 | -0.2897 | 0.3868 |
| 3848 | RP11-500M22 | 19 | 4806367 | 5009897 | -0.2224 | -0.2475 | 0.4033 |
| 123 | CTC-482H14 | 19 | 4998784 | 5154792 | -0.2225 | -0.2395 | 0.4054 |
| 1406 | RP5-859H16 | 19 | 5549421 | 5722324 | -0.2225 | -0.2338 | 0.4059 |
| 1687 | RP11-565J3 | 19 | 5713441 | 5893445 | -0.2225 | -0.2338 | 0.4059 |
| 1187 | RP11-330I7 | 19 | 6523485 | 6701760 | -0.2224 | -0.2277 | 0.4046 |
| 1457 | CTB-1025J19 | 19 | 7075600 | 7076170 | -0.2233 | -0.2310 | 0.4225 |
| 1450 | CTD-2623N2 | 19 | 9725333 | 9826741 | -0.0216 | -0.0447 | 0.4551 |
| 3039 | RP11-197O4 | 19 | 10248852 | 10410584 | 0.0053 | -0.0206 | 0.4540 |
| 135 | CTC-539A10 | 19 | 10528447 | 10699602 | 0.0051 | -0.0015 | 0.4589 |
| 2302 | CTC-359D24 | 19 | 11978969 | 12159478 | 0.0750 | 0.0119 | 0.4593 |
| 1454 | CTD-2659N19 | 19 | 12627096 | 12774845 | 0.1671 | 0.0286 | 0.4501 |
| 681 | CTC-250I14 | 19 | 13100858 | 13216789 | 0.3636 | 0.0441 | 0.5152 |
| 1684 | RP11-285H8 | 19 | 13815739 | 13816285 | 0.4097 | 0.0565 | 0.5062 |
| 2679 | RP11-56K21 | 19 | 14326904 | 14476904 | 0.3575 | 0.0559 | 0.4556 |

(continued)

| Order | Clone | chrom | Start | End | sc_control | sc_BRCA1 | sd |
|---|---|---|---|---|---|---|---|
| 2837 | CTB-187L3 | 19 | 15415833 | 15568896 | 0.1500 | 0.0559 | 0.4149 |
| 679 | CTD-2231E14 | 19 | 16012544 | 16124610 | 0.1500 | 0.0559 | 0.4149 |
| 1137 | RP11-413M18 | 19 | 16712801 | 16899931 | 0.1509 | 0.0694 | 0.3944 |
| 676 | CTD-3149D2 | 19 | 17561413 | 17787582 | 0.1514 | 0.0226 | 0.3826 |
| 3220 | RP11-943H6 | 19 | 17677637 | 17827637 | 0.1514 | 0.0226 | 0.3826 |
| 2282 | CTC-251H24 | 19 | 18202507 | 18412025 | 0.1108 | 0.0226 | 0.3782 |
| 528 | RP11-520G3 | 19 | 19024191 | 19194051 | 0.1111 | -0.0181 | 0.3719 |
| 122 | CTC-260F20 | 19 | 19409711 | 19574832 | 0.1114 | -0.0283 | 0.3635 |
| 132 | CTD-2332E11 | 19 | 20532129 | 20637787 | 0.0167 | -0.0280 | 0.3848 |
| 2672 | RP11-352D15 | 19 | 20860912 | 21010912 | 0.0167 | -0.0280 | 0.3848 |
| 2583 | RP 11-26D24 | 19 | 21345149 | 21504328 | 0.0167 | -0.0280 | 0.3848 |
| 2396 | RP11-165F1 | 19 | 22133724 | 22288871 | -0.0295 | -0.0428 | 0.3717 |
| 3404 | RP11-359H18 | 19 | 23555009 | 23732307 | 0.0072 | -0.0181 | 0.3823 |
| 678 | CTC-459F4 | 19 | 32889410 | 33099546 | 0.2281 | 0.0647 | 0.5117 |
| 663 | CTD-2043I16 | 19 | 33293108 | 33399253 | 0.3636 | 0.0523 | 0.5555 |
| 667 | CTD-2081K17 | 19 | 34149277 | 34316693 | 0.3636 | 0.0523 | 0.5555 |
| 4169 | CTD-2057D4 | 19 | 34956732 | 35092062 | 0.4170 | 0.0428 | 0.6081 |
| 139 | CTD-2001J20 | 19 | 35629515 | 35766589 | 0.1208 | 0.0409 | 0.4471 |
| 668 | CTD-2583G20 | 19 | 36159014 | 36322238 | 0.0606 | 0.0465 | 0.4469 |
| 2294 | CTC-325L16 | 19 | 37623634 | 37664852 | 0.0708 | 0.0354 | 0.4340 |
| 337 | RP11-298M15 | 19 | 38091322 | 38282890 | 0.0713 | 0.0261 | 0.4241 |
| 2083 | RP11-298M15 | 19 | 38091322 | 38282890 | 0.0713 | 0.0261 | 0.4241 |
| 2101 | RP11-413M10 | 19 | 39226664 | 39427144 | 0.0716 | 0.0362 | 0.4172 |
| 3014 | RP11-413M10 | 19 | 39226664 | 39427144 | 0.0716 | 0.0362 | 0.4172 |
| 144 | CTD-2527I21 | 19 | 40169242 | 40339132 | 0.0716 | 0.0362 | 0.4172 |
| 2577 | RP11-38C1 | 19 | 40876527 | 41059171 | 0.0716 | 0.0362 | 0.4172 |
| 2304 | CTD-2525J15 | 19 | 42010271 | 42199756 | 0.2261 | 0.0371 | 0.4944 |
| 2951 | RP11-9B17 | 19 | 42852412 | 43004473 | 0.1780 | 0.0262 | 0.4950 |
| 673 | CTB-186G2 | 19 | 43766631 | 43893312 | 0.2084 | 0.0184 | 0.4966 |
| 2841 | CTC-246B18 | 19 | 44401379 | 44539916 | 0.2084 | 0.0184 | 0.4966 |
| 682 | CTC-471F3 | 19 | 45079506 | 45253514 | 0.0329 | 0.0176 | 0.4259 |
| 2830 | CTC-435M10 | 19 | 46498597 | 46628707 | 0.0359 | -0.0331 | 0.3616 |
| 3292 | RP11-569M1 | 19 | 47423994 | 47633625 | 0.0357 | -0.0410 | 0.3655 |
| 127 | CTC-490G23 | 19 | 48347697 | 48590893 | 0.1787 | -0.0397 | 0.4705 |
| 3949 | RP11-15A1 | 19 | 49084683 | 49206647 | 0.1787 | -0.0397 | 0.4705 |
| 652 | RP 11-84C16 | 19 | 49912622 | 50122984 | 0.0330 | -0.0634 | 0.3809 |
| 121 | CTB-14D10 | 19 | 51027521 | 51151415 | -0.0006 | -0.0809 | 0.3863 |

(continued)

| Order | Clone | chrom | Start | End | sc_control | sc_BRCA1 | sd |
|---|---|---|---|---|---|---|---|
| 797 | RP 11-2J 15 | 19 | 51665795 | 51835146 | -0.0003 | -0.0818 | 0.3788 |
| 551 | RP11-492P7 | 19 | 51988000 | 52169534 | -0.0003 | -0.0818 | 0.3788 |
| 2287 | CTC-483I11 | 19 | 52341328 | 52496454 | -0.0003 | -0.0818 | 0.3788 |
| 3449 | RP11-3N16 | 19 | 53269624 | 53431702 | 0.0000 | -0.0812 | 0.3733 |
| 3614 | CTD-2639E6 | 19 | 53957736 | 54182006 | -0.0004 | -0.0716 | 0.3813 |
| 1135 | RP11-521120 | 19 | 54525652 | 54718270 | -0.0004 | -0.0716 | 0.3813 |
| 3610 | CTD-2545M3 | 19 | 55596764 | 55697589 | -0.0011 | -0.0572 | 0.3982 |
| 868 | RP11-256B9 | 19 | 57561867 | 57749886 | -0.0007 | -0.0397 | 0.3891 |
| 1279 | RP 11-44L20 | 19 | 58164197 | 58336597 | -0.0007 | -0.0397 | 0.3891 |
| 2495 | RP11-158G19 | 19 | 59041063 | 59235473 | -0.0007 | -0.0397 | 0.3891 |
| 136 | CTD-2337J16 | 19 | 59383667 | 59464093 | -0.0007 | -0.0397 | 0.3891 |
| 3042 | RP11-394L10 | 19 | 60914385 | 61088444 | -0.0007 | -0.0397 | 0.3891 |
| 2834 | CTC-444N24 | 19 | 62375405 | 62566112 | 0.0252 | -0.0396 | 0.3943 |
| 2836 | CTD-3138B18 | 19 | 63355845 | 63516696 | 0.0253 | -0.0323 | 0.3923 |
| 1384 | GS-325-I23 | 19 | 63361651 | 63511651 | 0.0253 | -0.0323 | 0.3923 |
| 1938 | GS-82-O2 | 20 | 105000 | 255000 | -0.0962 | -0.1245 | 0.6558 |
| 4078 | GS-1061-L1 | 20 | 105050 | 255050 | -0.0962 | -0.1245 | 0.6558 |
| 2910 | RP5-852M4 | 20 | 327037 | 385563 | 0.2181 | 0.0964 | 0.5006 |
| 1432 | RP11-314N13 | 20 | 1186672 | 1305743 | 0.2203 | 0.1149 | 0.4537 |
| 733 | RP4-686C3 | 20 | 2441424 | 2600595 | 0.2203 | 0.1149 | 0.4537 |
| 741 | RP4-741H3 | 20 | 3475976 | 3580882 | 0.2201 | 0.1102 | 0.4571 |
| 2373 | RP4-599I11 | 20 | 4706647 | 4766372 | 0.2204 | 0.1218 | 0.4498 |
| 745 | RP4-764O22 | 20 | 7012215 | 7095026 | 0.2204 | 0.1218 | 0.4498 |
| 2902 | RP5-1140M3 | 20 | 8198435 | 8214296 | 0.2205 | 0.1272 | 0.4484 |
| 2354 | RP4-811H13 | 20 | 9197701 | 9335479 | 0.2208 | 0.1385 | 0.4416 |
| 1968 | RP 11-204H22 | 20 | 10398740 | 10537181 | 0.2199 | 0.1454 | 0.4615 |
| 2905 | RP4-742J24 | 20 | 11812345 | 11979419 | 0.2202 | 0.1337 | 0.4546 |
| 3588 | RP11-104O6 | 20 | 12592830 | 12650613 | 0.2202 | 0.1337 | 0.4546 |
| 4124 | RP11-526K24 | 20 | 13618307 | 13720095 | 0.2202 | 0.1337 | 0.4546 |
| 194 | RP5-822J19 | 20 | 15512828 | 15602699 | 0.2202 | 0.1337 | 0.4546 |
| 205 | RP3-348M17 | 20 | 16588690 | 16635277 | 0.2205 | 0.1018 | 0.4495 |
| 1444 | RP11-504H3 | 20 | 17822016 | 17961860 | 0.2223 | 0.0780 | 0.4085 |
| 746 | RP1-167O22 | 20 | 21492993 | 21567966 | 0.2228 | 0.0805 | 0.3991 |
| 749 | RP4-788L20 | 20 | 22476757 | 22597625 | 0.2240 | 0.0654 | 0.3731 |
| 197 | RP1-234M6 | 20 | 23600046 | 23638470 | 0.2240 | 0.0654 | 0.3731 |
| 210 | RP5-1025A1 | 20 | 24956308 | 25050971 | 0.2241 | 0.0805 | 0.3699 |
| 1436 | RP11-348I14 | 20 | 28197652 | 28267569 | 0.2217 | 0.1152 | 0.4222 |

(continued)

| Order | Clone | chrom | Start | End | sc_ control | sc_ BRCA1 | sd |
|---|---|---|---|---|---|---|---|
| 3064 | RP3-324O17 | 20 | 29577867 | 29657285 | 0.2247 | 0.0942 | 0.3584 |
| 357 | RP5-857M17 | 20 | 29657186 | 29756778 | 0.2247 | 0.0942 | 0.3584 |
| 201 | RP1-310O13 | 20 | 29939119 | 30102094 | 0.2247 | 0.0942 | 0.3584 |
| 3604 | RP11-410N8 | 20 | 30605464 | 30669072 | 0.2247 | 0.0942 | 0.3584 |
| 346 | RP5-1085F17 | 20 | 30791562 | 30910460 | 0.2455 | 0.0997 | 0.3625 |
| 2901 | RP4-733O23 | 20 | 31169190 | 31184191 | 0.2455 | 0.0997 | 0.3625 |
| 738 | RP5-1137F22 | 20 | 31582324 | 31675130 | 0.2455 | 0.0997 | 0.3625 |
| 3600 | RP11-353C18 | 20 | 33744285 | 33824849 | 0.2448 | 0.0726 | 0.3788 |
| 3592 | RP11-234K24 | 20 | 34193483 | 34346674 | 0.2448 | 0.0680 | 0.3788 |
| 2369 | RP3-469A13 | 20 | 34762708 | 34886536 | 0.2448 | 0.0680 | 0.3788 |
| 2893 | RP4-633O20 | 20 | 35834704 | 35931163 | 0.2448 | 0.0680 | 0.3788 |
| 1428 | RP11-122O1 | 20 | 36744259 | 36840557 | 0.2449 | 0.0619 | 0.3768 |
| 213 | RP4-600E6 | 20 | 37565916 | 37666119 | 0.2449 | 0.0619 | 0.3768 |
| 750 | RP5-892M9 | 20 | 38231698 | 38293618 | 0.2449 | 0.0619 | 0.3768 |
| 753 | RP4-796I11 | 20 | 39313326 | 39390727 | 0.2444 | 0.0675 | 0.3856 |
| 2353 | RP1-128O17 | 20 | 39934889 | 40041261 | 0.2444 | 0.0675 | 0.3856 |
| 2357 | RP1-232N11 | 20 | 40680769 | 40792409 | 0.2441 | 0.0899 | 0.3932 |
| 193 | RP1-138B7 | 20 | 41584658 | 41668980 | 0.2669 | 0.0958 | 0.4005 |
| 3045 | RP5-1028D15 | 20 | 41668881 | 41804999 | 0.2668 | 0.1208 | 0.4023 |
| 2365 | RP3-337O18 | 20 | 43889651 | 43975730 | 0.2658 | 0.1557 | 0.4242 |
| 206 | RP5-1005L2 | 20 | 44369220 | 44380337 | 0.2658 | 0.1557 | 0.4242 |
| 3596 | RP11-347D21 | 20 | 46001209 | 46064435 | 0.2651 | 0.1621 | 0.4380 |
| 3060 | RP1-155G6 | 20 | 47049373 | 47129972 | 0.2651 | 0.1621 | 0.4380 |
| 2509 | RP3-470L14 | 20 | 47129873 | 47243708 | 0.2651 | 0.1621 | 0.4380 |
| 2913 | RP4-791K14 | 20 | 47382984 | 47538301 | 0.2648 | 0.1631 | 0.4453 |
| 2906 | RP5-1185N5 | 20 | 48091851 | 48205439 | 0.2648 | 0.1631 | 0.4453 |
| 349 | RP4-530I15 | 20 | 48590356 | 48701394 | 0.2648 | 0.1631 | 0.4453 |
| 2366 | RP5-994O24 | 20 | 49519272 | 49575686 | 0.2635 | 0.1822 | 0.4737 |
| 2897 | RP4-715N 11 | 20 | 50717369 | 50828741 | 0.2637 | 0.1779 | 0.4702 |
| 353 | RP4-724E16 | 20 | 51561511 | 51648112 | 0.2638 | 0.1907 | 0.4680 |
| 2894 | RP5-1075G21 | 20 | 52147783 | 52227376 | 0.2638 | 0.1830 | 0.4663 |
| 350 | RP5-1162C3 | 20 | 52227277 | 52329237 | 0.2638 | 0.1830 | 0.4663 |
| 3584 | RP11-6L15 | 20 | 52483567 | 52583948 | 0.2638 | 0.1830 | 0.4663 |
| 893 | RP5-1167H4 | 20 | 54336458 | 54472150 | 0.2652 | 0.1681 | 0.4360 |
| 742 | RP5-1153D9 | 20 | 54472051 | 54566171 | 0.2652 | 0.1681 | 0.4360 |
| 1424 | RP11-4603 | 20 | 54804991 | 54897122 | 0.2014 | 0.1685 | 0.4660 |
| 4128 | RP13-379L11 | 20 | 55913984 | 56121677 | 0.2822 | 0.1854 | 0.4137 |

(continued)

| Order | Clone | chrom | Start | End | sc_control | sc_BRCA1 | sd |
|---|---|---|---|---|---|---|---|
| 2361 | RP1-309F20 | 20 | 56868317 | 56911123 | 0.2822 | 0.1854 | 0.4137 |
| 2358 | RP5-827E24 | 20 | 59427000 | 59538724 | 0.2822 | 0.1854 | 0.4137 |
| 2898 | RP5-1107C24 | 20 | 59894657 | 60016638 | 0.2800 | 0.2158 | 0.4623 |
| 209 | RP4-563E14 | 20 | 61012762 | 61041280 | 0.2816 | 0.2046 | 0.4263 |
| 4132 | RP13-152O15 | 20 | 62108666 | 62163452 | 0.2815 | 0.1949 | 0.4297 |
| 1918 | GS-81-F12 | 20 | 62310964 | 62460964 | 0.2815 | 0.1949 | 0.4297 |
| 3051 | RP1-126N20 | 21 | 14634832 | 14731794 | -0.0823 | -0.3210 | 0.5819 |
| 760 | RP11-304D2 | 21 | 18224008 | 18383964 | -0.0366 | -0.0329 | 0.4108 |
| 2392 | RP11-49B5 | 21 | 20224452 | 20292462 | -0.0366 | -0.0329 | 0.4108 |
| 2748 | RP11-509A1 | 21 | 21128294 | 21149667 | -0.0366 | -0.0329 | 0.4108 |
| 3047 | RP1-50A12 | 21 | 21237180 | 21411332 | -0.0051 | -0.0043 | 0.4208 |
| 2940 | RP11-410P24 | 21 | 32849565 | 33019511 | -0.0197 | -0.0239 | 0.3592 |
| 3059 | RP1-245P17 | 21 | 34483960 | 34602938 | -0.0197 | -0.0239 | 0.3592 |
| 899 | RP1-255P7 | 21 | 35282309 | 35532845 | -0.0202 | -0.0524 | 0.3711 |
| 2932 | RP11-102E10 | 21 | 36765627 | 36936688 | -0.0198 | -0.0819 | 0.3609 |
| 768 | RP11-98O13 | 21 | 37915905 | 38078949 | -0.0209 | -0.0659 | 0.3865 |
| 776 | RP11-164E1 | 21 | 38601021 | 38740380 | -0.0209 | -0.0659 | 0.3865 |
| 891 | RP1-128M19 | 21 | 39567315 | 39698959 | -0.0209 | -0.0659 | 0.3865 |
| 344 | RP5-1031P17 | 21 | 39699017 | 39788341 | -0.0209 | -0.0659 | 0.3865 |
| 895 | RP1-171F15 | 21 | 40630421 | 40630982 | -0.0209 | -0.0733 | 0.3863 |
| 3063 | RP1-265B9 | 21 | 41559315 | 41716976 | -0.0207 | -0.0656 | 0.3824 |
| 232 | RP11-113F1 | 21 | 42507240 | 42689355 | -0.0209 | -0.0611 | 0.3854 |
| 2924 | RP11-351D2 | 21 | 43550744 | 43727862 | -0.0209 | -0.0528 | 0.3868 |
| 764 | RP11-397E9 | 21 | 44672693 | 44846917 | -0.0210 | -0.0434 | 0.3871 |
| 2097 | RP11-397E9 | 21 | 44672693 | 44846917 | -0.0210 | -0.0434 | 0.3871 |
| 2400 | RP11-178H12 | 21 | 46424050 | 46607897 | -0.0210 | -0.0434 | 0.3871 |
| 1922 | GS-2-H14 | 21 | 46694323 | 46844323 | -0.0210 | -0.0434 | 0.3871 |
| 4082 | GS-63-H24 | 21 | 46694373 | 46844373 | -0.0210 | -0.0434 | 0.3871 |
| 4008 | XX-p8708 | 22 | 15621248 | 15745966 | -0.0073 | -0.0574 | 0.3928 |
| 2911 | XX-bac32 | 22 | 16899186 | 17026897 | -0.0073 | -0.0574 | 0.3928 |
| 2903 | XX-461k10 | 22 | 17368953 | 17412487 | -0.0073 | -0.0574 | 0.3928 |
| 3211 | RP11-179H3 | 22 | 20578709 | 20719542 | -0.1023 | -0.0666 | 0.3808 |
| 1056 | RP11-757F24 | 22 | 21112875 | 21296725 | -0.1023 | -0.0666 | 0.3808 |
| 2899 | RP11-50L23 | 22 | 21400636 | 21552718 | -0.1029 | -0.0640 | 0.3672 |
| 739 | RP11-80O7 | 22 | 22510132 | 22789754 | -0.1017 | -0.0637 | 0.3933 |
| 3468 | LL22NC03-95F 10 | 22 | 23387722 | 23427691 | -0.1017 | -0.0637 | 0.3933 |
| 2356 | CTA-221G9 | 22 | 23879817 | 23982359 | -0.1004 | -0.0893 | 0.4224 |

(continued)

| Order | Clone | chrom | Start | End | sc_control | sc_BRCA1 | sd |
|-------|-------|-------|-------|-----|------------|----------|-----|
| 2915 | CTA-125H2 | 22 | 24560701 | 24734213 | -0.1004 | -0.0893 | 0.4224 |
| 2896 | CTA-445C9 | 22 | 25228499 | 25359896 | -0.1005 | -0.0947 | 0.4195 |
| 2907 | XXbac-677f7 | 22 | 31498418 | 31627100 | -0.1154 | -0.1380 | 0.4127 |
| 2376 | CTA-415G2 | 22 | 31695938 | 31825894 | -0.1154 | -0.1380 | 0.4127 |
| 1304 | LL22NC01-95B1 | 22 | 32340168 | 32367572 | -0.1150 | -0.1641 | 0.4026 |
| 200 | CTA-221H1 | 22 | 32901471 | 32904493 | -0.1146 | -0.1464 | 0.3952 |
| 1308 | RP1-215F16 | 22 | 33798722 | 33828353 | -0.1145 | -0.1538 | 0.3920 |
| 196 | CTA-212A2 | 22 | 34741486 | 34954238 | -0.1144 | -0.1585 | 0.3899 |
| 3460 | LL22NC01-132D12 | 22 | 35478908 | 35505203 | -0.1144 | -0.1585 | 0.3899 |
| 208 | CTA-390B3 | 22 | 36120349 | 36212755 | -0.1146 | -0.1707 | 0.3937 |
| 204 | CTA-228A9 | 22 | 36811298 | 36906672 | -0.1465 | -0.1770 | 0.3966 |
| 755 | CTA-150C2 | 22 | 37610145 | 37811287 | -0.1465 | -0.1722 | 0.3973 |
| 2514 | LL22NC03-10C3 | 22 | 37938484 | 37976499 | -0.1465 | -0.1678 | 0.3966 |
| 1852 | RP1-172B20 | 22 | 38345395 | 38559115 | -0.1465 | -0.1722 | 0.3969 |
| 2368 | CTA-229A8 | 22 | 39305277 | 39427833 | -0.1462 | -0.1810 | 0.3915 |
| 2006 | RP11-12M9 | 22 | 39701569 | 39877855 | -0.1462 | -0.1810 | 0.3915 |
| 1731 | RP11-422A16 | 22 | 39877756 | 39890808 | -0.1462 | -0.1888 | 0.3906 |
| 884 | RP1-85F18 | 22 | 39890708 | 39932624 | -0.1462 | -0.1888 | 0.3906 |
| 1844 | RP5-979N1 | 22 | 40022373 | 40124523 | -0.1462 | -0.1888 | 0.3906 |
| 2360 | CTA-250D10 | 22 | 40582711 | 40803605 | -0.1698 | -0.1883 | 0.4036 |
| 3476 | RP3-437M21 | 22 | 41529049 | 41616450 | -0.1697 | -0.1954 | 0.4017 |
| 1316 | RP3-388M5 | 22 | 42473525 | 42651092 | -0.1697 | -0.1954 | 0.4017 |
| 2372 | CTA-397C4 | 22 | 43212696 | 43260941 | -0.1697 | -0.1954 | 0.4017 |
| 2364 | CTA-268H5 | 22 | 43952888 | 44141720 | -0.1697 | -0.1954 | 0.4017 |
| 3480 | RP3-398C22 | 22 | 44454571 | 44543898 | -0.1697 | -0.1954 | 0.4017 |
| 736 | CTA-29F 11 | 22 | 45493480 | 45582798 | -0.1703 | -0.2084 | 0.4155 |
| 1300 | LL22NC03-75H12 | 22 | 46190401 | 46236544 | -0.1696 | -0.2242 | 0.4003 |
| 1848 | RP5-925J7 | 22 | 47740349 | 47838795 | -0.2419 | -0.2580 | 0.4401 |
| 1312 | RP3-355C18 | 22 | 48767848 | 48869117 | -0.1833 | -0.1980 | 0.4019 |
| 743 | CTA-799F10 | 22 | 49425750 | 49449200 | -0.1831 | -0.1896 | 0.3974 |
| 4091 | GS-3018-K1 | 22 | 49461432 | 49611432 | -0.1450 | -0.1652 | 0.3899 |
| 4086 | GS-99-K24 | 22 | 49496432 | 49646432 | -0.1021 | -0.2078 | 0.5619 |
| 1923 | GS-839-D20 | 23 | 85000 | 235000 | -0.1468 | -0.1884 | 0.5611 |
| 1583 | RP 11-418N20 | 23 | 2939509 | 3122882 | -0.3227 | -0.2103 | 0.4587 |
| 1260 | RP11-23N11 | 23 | 3707732 | 3754886 | -0.3227 | -0.2103 | 0.4587 |
| 2270 | RP 11-62N 12 | 23 | 4616007 | 4758929 | -0.3227 | -0.2103 | 0.4587 |
| 2817 | RP11-60N3 | 23 | 5403637 | 5566017 | -0.3227 | -0.2103 | 0.4587 |

(continued)

| Order | Clone | chrom | Start | End | sc_ control | sc_ BRCA1 | sd |
|---|---|---|---|---|---|---|---|
| 1150 | RP11-483M24 | 23 | 6957719 | 7136905 | -0.3227 | -0.2103 | 0.4587 |
| 571 | RP11-323F16 | 23 | 7455550 | 7633882 | -0.3227 | -0.2103 | 0.4587 |
| 3041 | RP11-323F16 | 23 | 7455550 | 7633882 | -0.3227 | -0.2103 | 0.4587 |
| 1032 | RP1-108M6 | 23 | 9561147 | 9684179 | -0.3777 | -0.2286 | 0.4472 |
| 3046 | RP6-1O2 | 23 | 10270814 | 10437697 | -0.3495 | -0.2285 | 0.4408 |
| 3188 | RP 1-27C22 | 23 | 11152958 | 11412159 | -0.3495 | -0.2285 | 0.4408 |
| 1591 | RP11-1J4 | 23 | 12448478 | 12542635 | -0.3514 | -0.2311 | 0.3994 |
| 3433 | RP11-1J4 | 23 | 12448478 | 12542635 | -0.3514 | -0.2311 | 0.3994 |
| 4107 | RP1-164K3 | 23 | 12952343 | 13058902 | -0.3514 | -0.2311 | 0.3994 |
| 1036 | RP1-122K4 | 23 | 13838730 | 14015083 | -0.3514 | -0.2311 | 0.3994 |
| 1028 | RP1-93D11 | 23 | 14768520 | 14808635 | -0.3516 | -0.2443 | 0.3934 |
| 1677 | RP11-431J24 | 23 | 16028650 | 16242650 | -0.3516 | -0.2443 | 0.3934 |
| 1042 | RP5-1129A6 | 23 | 18089214 | 18198231 | -0.3516 | -0.2443 | 0.3934 |
| 3888 | RP11-558P14 | 23 | 18352566 | 18447658 | -0.3516 | -0.2443 | 0.3934 |
| 3728 | RP11-421K1 | 23 | 18964945 | 19156262 | -0.3516 | -0.2443 | 0.3934 |
| 2474 | RP11-406A18 | 23 | 21448265 | 21601094 | -0.3516 | -0.2443 | 0.3934 |
| 872 | RP11-261M11 | 23 | 24176952 | 24341732 | -0.3349 | -0.2488 | 0.3758 |
| 369 | RP11-272N24 | 23 | 25247627 | 25248382 | -0.3349 | -0.2488 | 0.3758 |
| 2587 | RP11-26L4 | 23 | 26803849 | 26971177 | -0.3349 | -0.2488 | 0.3758 |
| 3768 | RP11-26L4 | 23 | 26803849 | 26971177 | -0.3349 | -0.2488 | 0.3758 |
| 2017 | RP11-268G12 | 23 | 27060718 | 27210570 | -0.3349 | -0.2488 | 0.3758 |
| 19 | RP11-489K4 | 23 | 28029871 | 28187101 | -0.3349 | -0.2488 | 0.3758 |
| 1171 | RP11-169J21 | 23 | 28257706 | 28427554 | -0.3344 | -0.2608 | 0.3868 |
| 41 | RP11-37E19 | 23 | 28928507 | 28929130 | -0.3344 | -0.2608 | 0.3868 |
| 1301 | RP6-27C10 | 23 | 29091437 | 29143794 | -0.3590 | -0.2609 | 0.3938 |
| 497 | RP4-662N3 | 23 | 29347326 | 29491215 | -0.3590 | -0.2609 | 0.3938 |
| 2976 | RP11-122N14 | 23 | 31429070 | 31590268 | -0.3588 | -0.2432 | 0.3966 |
| 3467 | RP4-639D23 | 23 | 32142380 | 32238245 | -0.3588 | -0.2432 | 0.3966 |
| 1843 | RP5-1147O16 | 23 | 32347144 | 32479433 | -0.3588 | -0.2432 | 0.3966 |
| 3602 | RP13-46M24 | 23 | 36337423 | 36531334 | -0.3588 | -0.2432 | 0.3966 |
| 1817 | RP11-12J5 | 23 | 37553875 | 37716467 | -0.3589 | -0.2381 | 0.3955 |
| 3771 | RP11-12J5 | 23 | 37553875 | 37716467 | -0.3589 | -0.2381 | 0.3955 |
| 1985 | RP11-469E19 | 23 | 40755108 | 40918627 | -0.3582 | -0.2445 | 0.4096 |
| 1849 | RP1-169I5 | 23 | 41107931 | 41241498 | -0.3582 | -0.2445 | 0.4096 |
| 3462 | RP1-308O1 | 23 | 42106089 | 42272704 | -0.3582 | -0.2445 | 0.4096 |
| 3479 | RP5-879N19 | 23 | 43352635 | 43432926 | -0.2571 | -0.2450 | 0.4504 |
| 1973 | RP11-386N14 | 23 | 44547837 | 44688553 | -0.3379 | -0.2205 | 0.4097 |

(continued)

| Order | Clone | chrom | Start | End | sc_control | sc_BRCA1 | sd |
|-------|-------|-------|-------|-----|------------|----------|-----|
| 3477 | RP1-54B20 | 23 | 47651854 | 47861472 | -0.1876 | -0.2047 | 0.4851 |
| 1434 | RP11-576P23 | 23 | 50147678 | 50302524 | -0.2160 | -0.1973 | 0.4934 |
| 1425 | RP11-56H2 | 23 | 51259019 | 51394297 | -0.3483 | -0.1963 | 0.4130 |
| 1446 | RP13-188A5 | 23 | 56037801 | 56140557 | -0.2911 | -0.1821 | 0.3904 |
| 1981 | RP 11-445 O 16 | 23 | 56976937 | 57113775 | -0.2911 | -0.1780 | 0.3904 |
| 1839 | RP5-966K21 | 23 | 57905135 | 57932055 | -0.2911 | -0.1832 | 0.3899 |
| 3598 | RP13-34C21 | 23 | 62281936 | 62462786 | -0.3099 | -0.1867 | 0.3915 |
| 1969 | RP11-284B18 | 23 | 63351090 | 63455623 | -0.3094 | -0.1985 | 0.4019 |
| 1306 | RP3-323B6 | 23 | 64514210 | 64626872 | -0.3094 | -0.1985 | 0.4019 |
| 1442 | RP13-130F17 | 23 | 65362816 | 65515922 | -0.2844 | -0.1816 | 0.4041 |
| 3461 | RP6-22P16 | 23 | 66474359 | 66577725 | -0.3334 | -0.1818 | 0.4237 |
| 2517 | RP4-808O4 | 23 | 66577626 | 66716658 | -0.3334 | -0.1818 | 0.4237 |
| 1314 | RP3-360E18 | 23 | 67342258 | 67474160 | -0.3334 | -0.1818 | 0.4237 |
| 4145 | RP11-446J19 | 23 | 68510619 | 68634148 | -0.2948 | -0.1894 | 0.4066 |
| 3463 | RP4-583H20 | 23 | 70619662 | 70620234 | -0.2949 | -0.1949 | 0.4056 |
| 4126 | RP13-260P4 | 23 | 72691735 | 72766161 | -0.2949 | -0.1808 | 0.4058 |
| 1965 | RP11-253L3 | 23 | 73522518 | 73533894 | -0.2949 | -0.1808 | 0.4058 |
| 4125 | RP11-236O12 | 23 | 74730746 | 74851000 | -0.2949 | -0.1808 | 0.4058 |
| 1315 | RP5-875J14 | 23 | 76640561 | 76788983 | -0.2949 | -0.1808 | 0.4058 |
| 4014 | RP3-465G10 | 23 | 77111566 | 77195728 | -0.2949 | -0.1808 | 0.4058 |
| 1437 | RP11-159L8 | 23 | 78252504 | 78419313 | -0.2949 | -0.1808 | 0.4058 |
| 3605 | RP 11-217H19 | 23 | 79352997 | 79447858 | -0.2949 | -0.1808 | 0.4058 |
| 3593 | RP11-102P23 | 23 | 80307276 | 80471287 | -0.2949 | -0.1808 | 0.4058 |
| 4007 | RP5-1156N12 | 23 | 81388801 | 81535160 | -0.2948 | -0.1899 | 0.4070 |
| 4005 | RP1-105K17 | 23 | 82482372 | 82563001 | -0.2948 | -0.1899 | 0.4070 |
| 3585 | RP11-54F22 | 23 | 83231140 | 83374359 | -0.2948 | -0.1899 | 0.4070 |
| 4003 | RP5-1109K10 | 23 | 83845334 | 83846061 | -0.2948 | -0.1899 | 0.4070 |
| 1841 | RP1-93L7 | 23 | 84848591 | 85055374 | -0.2948 | -0.1899 | 0.4070 |
| 3458 | RP1-225D2 | 23 | 85407563 | 85482124 | -0.2948 | -0.1899 | 0.4070 |
| 1441 | RP11-192B18 | 23 | 86196880 | 86368726 | -0.2948 | -0.1899 | 0.4070 |
| 1297 | RP6-22D12 | 23 | 86741677 | 86811503 | -0.2948 | -0.1899 | 0.4070 |
| 4018 | RP4-542O23 | 23 | 87708917 | 87785193 | -0.2944 | -0.1837 | 0.4169 |
| 3606 | RP13-166C10 | 23 | 90182084 | 90304132 | -0.2944 | -0.1837 | 0.4169 |
| 4133 | RP11-361B11 | 23 | 93061893 | 93118369 | -0.2943 | -0.1939 | 0.4180 |
| 1318 | RP3-380C13 | 23 | 94580184 | 94717628 | -0.2943 | -0.1939 | 0.4180 |
| 1857 | RP1-199L16 | 23 | 95213815 | 95348777 | -0.2943 | -0.1939 | 0.4180 |
| 4129 | RP11-274M8 | 23 | 96356903 | 96509340 | -0.2943 | -0.1939 | 0.4180 |

(continued)

| Order | Clone | chrom | Start | End | sc_control | sc_BRCA1 | sd |
|---|---|---|---|---|---|---|---|
| 4141 | RP11-400P10 | 23 | 97508539 | 97716150 | -0.2943 | -0.1939 | 0.4180 |
| 1433 | RP11-151N12 | 23 | 98412392 | 98497852 | -0.2943 | -0.1939 | 0.4180 |
| 1310 | RP3-341D10 | 23 | 100082182 | 100164698 | -0.2946 | -0.1932 | 0.4118 |
| 1858 | RP4-545K15 | 23 | 100732251 | 100789070 | -0.2644 | -0.1933 | 0.4218 |
| 1837 | RP1-77O19 | 23 | 101631035 | 101660222 | -0.2644 | -0.1933 | 0.4218 |
| 4006 | RP3-421I20 | 23 | 102569986 | 102622582 | -0.2641 | -0.1872 | 0.4302 |
| 1445 | RP11-230E14 | 23 | 103557065 | 103623441 | -0.2641 | -0.1858 | 0.4285 |
| 1430 | RP 11-539A6 | 23 | 104447295 | 104458809 | -0.2641 | -0.1858 | 0.4285 |
| 1302 | RP1-312P4 | 23 | 104929751 | 104940096 | -0.2641 | -0.1858 | 0.4285 |
| 3997 | RP1-75H8 | 23 | 106053097 | 106162561 | -0.2642 | -0.1976 | 0.4270 |
| 1311 | RP5-820B18 | 23 | 106921582 | 107015662 | -0.2642 | -0.1976 | 0.4270 |
| 1313 | RP1-31B8 | 23 | 108431925 | 108500258 | -0.2642 | -0.1976 | 0.4270 |
| 3052 | RP1-136J15 | 23 | 108678922 | 108804386 | -0.2642 | -0.1976 | 0.4270 |
| 1299 | RP4-557A17 | 23 | 109509387 | 109525239 | -0.2643 | -0.2066 | 0.4244 |
| 1319 | RP5-914P14 | 23 | 110343476 | 110498850 | -0.2643 | -0.2066 | 0.4244 |
| 1845 | RP1-115K14 | 23 | 111801639 | 111820528 | -0.2644 | -0.1981 | 0.4233 |
| 4001 | RP1-93I3 | 23 | 114632105 | 114737667 | -0.2642 | -0.1783 | 0.4273 |
| 1838 | RP3-394H4 | 23 | 116516765 | 116589055 | -0.2646 | -0.1698 | 0.4175 |
| 1305 | RP6-155F9 | 23 | 117482470 | 117605044 | -0.2651 | -0.1732 | 0.4082 |
| 4002 | RP3-404F18 | 23 | 118438874 | 118566990 | -0.2651 | -0.1695 | 0.4076 |
| 3466 | RP1-318C15 | 23 | 119444046 | 119591147 | -0.2651 | -0.1695 | 0.4076 |
| 3478 | RP3-368G6 | 23 | 120476625 | 120534500 | -0.2651 | -0.1695 | 0.4076 |
| 1853 | RP1-181N1 | 23 | 122218383 | 122344757 | -0.2651 | -0.1695 | 0.4076 |
| 3998 | RP3-394F12 | 23 | 123854482 | 123963507 | -0.2651 | -0.1695 | 0.4076 |
| 1307 | RP4-657J8 | 23 | 124726405 | 124825259 | -0.2651 | -0.1695 | 0.4076 |
| 1846 | RP3-428A13 | 23 | 126290610 | 126444844 | -0.2651 | -0.1695 | 0.4076 |
| 3465 | RP6-109P7 | 23 | 127817947 | 127921935 | -0.2651 | -0.1695 | 0.4076 |
| 3474 | RP3-345B16 | 23 | 128151281 | 128272312 | -0.2651 | -0.1695 | 0.4076 |
| 4013 | RP1-179D3 | 23 | 129036262 | 129085478 | -0.2650 | -0.1668 | 0.4093 |
| 1850 | RP3-463A9 | 23 | 129672525 | 129773621 | -0.2650 | -0.1668 | 0.4093 |
| 3999 | RP5-965E19 | 23 | 131271371 | 131294729 | -0.2650 | -0.1668 | 0.4093 |
| 3469 | RP6-198C21 | 23 | 132223249 | 132365010 | -0.2652 | -0.1584 | 0.4051 |
| 3586 | RP11-481F23 | 23 | 134479916 | 134510981 | -0.2652 | -0.1584 | 0.4051 |
| 4017 | RP1-196E23 | 23 | 135361435 | 135484339 | -0.2652 | -0.1432 | 0.4045 |
| 1309 | RP1-20J23 | 23 | 136272111 | 136409221 | -0.2652 | -0.1432 | 0.4045 |
| 1429 | RP 11-65G20 | 23 | 137322239 | 137377012 | -0.2652 | -0.1432 | 0.4045 |
| 3475 | RP5-833B2 | 23 | 137835938 | 137909069 | -0.2652 | -0.1354 | 0.4060 |

(continued)

| Order | Clone | chrom | Start | End | sc_ control | sc_ BRCA1 | sd |
|---|---|---|---|---|---|---|---|
| 1438 | RP13-34G21 | 23 | 138587336 | 138777284 | -0.2652 | -0.1354 | 0.4060 |
| 1303 | RP4-595A18 | 23 | 139456147 | 139571994 | -0.2652 | -0.1354 | 0.4060 |
| 1426 | RP11-518F7 | 23 | 140211892 | 140377252 | -0.2652 | -0.1354 | 0.4060 |
| 3459 | RP4-552K20 | 23 | 140718870 | 140770787 | -0.2652 | -0.1354 | 0.4060 |
| 3470 | RP3-324C6 | 23 | 141360527 | 141476821 | -0.2646 | -0.1300 | 0.4182 |
| 1966 | GS1-91O18 | 23 | 142126632 | 142173638 | -0.2637 | -0.1363 | 0.4375 |
| 3473 | RP1-29A6 | 23 | 143420952 | 143534939 | -0.2637 | -0.1363 | 0.4375 |
| 1977 | RP11-390O24 | 23 | 143983780 | 144162387 | -0.2637 | -0.1363 | 0.4375 |
| 4137 | RP11-387H19 | 23 | 144906312 | 145003054 | -0.2637 | -0.1363 | 0.4375 |
| 1317 | RP1-73A14 | 23 | 145778695 | 145884842 | -0.2637 | -0.1363 | 0.4375 |
| 4010 | RP3-433G13 | 23 | 146471558 | 146503967 | -0.2631 | -0.1467 | 0.4499 |
| 487 | RP6-224C24 | 23 | 146503968 | 146682511 | -0.2631 | -0.1467 | 0.4499 |
| 4110 | RP5-937E21 | 23 | 148437100 | 148548659 | -0.2634 | -0.1354 | 0.4448 |
| 1414 | RP5-892C22 | 23 | 150185779 | 150298000 | -0.2631 | -0.1297 | 0.4515 |
| 3133 | RP11-45D17 | 23 | 150493592 | 150625362 | -0.2631 | -0.1297 | 0.4515 |
| 2261 | RP11-54I20 | 23 | 152359276 | 152538530 | -0.2626 | -0.1458 | 0.4611 |
| 2729 | RP11-54I20 | 23 | 152359276 | 152538530 | -0.2626 | -0.1458 | 0.4611 |
| 488 | RP5-1087L19 | 23 | 153404680 | 153547872 | -0.2626 | -0.1458 | 0.4611 |
| 40 | RP11-402H20 | 23 | 153861611 | 153862276 | -0.2626 | -0.1458 | 0.4611 |
| 1326 | RP11-296N8 | 23 | 153783896 | 153962641 | -0.2626 | -0.1458 | 0.4611 |
| 1541 | RP11-218L14 | 23 | 154334463 | 154482340 | -0.2626 | -0.1458 | 0.4611 |
| 4087 | GS-225-F6 | 23 | 154738754 | 154888754 | -0.2626 | -0.1458 | 0.4611 |

**Table 2: BRCA-2 like classifiers.** The indicated locations are on genome version 'hg18'. Of course, in case a genome version changes, the person skilled in the art is able to assign the below mentioned positions to any new and/or different genome version. Thus, variations in the Start and End positions are also encompassed by the present invention.

| Order | Clone | chrom | Start | End | sc_ control | sc_ BRCA2 | sd |
|---|---|---|---|---|---|---|---|
| 2112 | RP11-428B4 | 4 | 99946600 | 100058902 | -0.0456 | -0.0815 | 0.3725 |
| 1990 | RP11-56D20 | 4 | 101150764 | 101325336 | -0.0456 | -0.0815 | 0.3725 |
| 911 | RP11-13F20 | 4 | 101510577 | 101670097 | -0.0456 | -0.0815 | 0.3725 |
| 3315 | RP11-167N19 | 4 | 101813198 | 101961364 | -0.0456 | -0.0815 | 0.3725 |
| 2050 | RP11-110C16 | 4 | 103067212 | 103220659 | -0.0456 | -0.0815 | 0.3725 |
| 4273 | RP11-438P8 | 4 | 104406898 | 104431241 | -0.0456 | -0.0815 | 0.3725 |
| 1707 | RP11-174B22 | 4 | 104431242 | 104513122 | -0.0456 | -0.0815 | 0.3725 |
| 292 | RP11-122B24 | 4 | 105288367 | 105355835 | -0.0454 | -0.0867 | 0.3694 |
| 1797 | RP11-16G16 | 4 | 106336237 | 106507303 | -0.0453 | -0.0967 | 0.3663 |

(continued)

| Order | Clone | chrom | Start | End | sc_control | sc_BRCA2 | sd |
|---|---|---|---|---|---|---|---|
| 4220 | RP11-208O6 | 4 | 107027991 | 107064932 | -0.0457 | -0.0919 | 0.3750 |
| 3667 | RP11-88D10 | 4 | 107736653 | 107943820 | -0.0457 | -0.1002 | 0.3760 |
| 2046 | RP11-109F18 | 4 | 108646932 | 108795103 | -0.0457 | -0.1002 | 0.3760 |
| 651 | RP11-75N20 | 4 | 109877028 | 110025600 | -0.0457 | -0.1069 | 0.3747 |
| 2273 | RP11-57A22 | 4 | 110057781 | 110225193 | -0.0457 | -0.1069 | 0.3747 |
| 2468 | RP11-380D23 | 4 | 111660447 | 111860073 | -0.0458 | -0.1017 | 0.3777 |
| 1237 | RP11-326N15 | 4 | 112359892 | 112502402 | -0.0458 | -0.1017 | 0.3777 |
| 2264 | RP 11-77N9 | 4 | 112853134 | 112892477 | -0.0467 | -0.1121 | 0.3971 |
| 3753 | RP 11-77N9 | 4 | 112853134 | 112892477 | -0.0467 | -0.1121 | 0.3971 |
| 298 | RP11-402J6 | 4 | 113599819 | 113777515 | -0.0459 | -0.0928 | 0.3800 |
| 1604 | RP11-24P18 | 4 | 131614434 | 131767271 | -0.0472 | -0.0613 | 0.4090 |
| 1800 | RP11-24P18 | 4 | 131614434 | 131767271 | -0.0472 | -0.0613 | 0.4090 |
| 3488 | RP11-314N14 | 4 | 132653555 | 132677577 | -0.0472 | -0.0613 | 0.4090 |
| 1611 | RP11-13H10 | 5 | 4351733 | 4533614 | 0.0442 | 0.2081 | 0.4476 |
| 3981 | RP11-13H10 | 5 | 4351733 | 4533614 | 0.0442 | 0.2081 | 0.4476 |
| 1828 | RP11-137P5 | 5 | 5500098 | 5687008 | 0.0442 | 0.2081 | 0.4476 |
| 3024 | RP11-245L6 | 5 | 5879129 | 5995464 | 0.0442 | 0.2056 | 0.4479 |
| 2292 | CTD-2324F15 | 5 | 6350940 | 6430016 | 0.0442 | 0.2056 | 0.4479 |
| 287 | RP11-114M17 | 5 | 6515876 | 6708632 | 0.0442 | 0.2056 | 0.4479 |
| 2300 | CTD-2346M20 | 5 | 7449057 | 7530186 | 0.0443 | 0.2172 | 0.4495 |
| 2973 | RP 11-132J20 | 5 | 8413255 | 8600646 | 0.0446 | 0.2294 | 0.4564 |
| 1834 | RP 11-32D 12 | 5 | 9436026 | 9606181 | 0.0437 | 0.2214 | 0.4374 |
| 2284 | CTD-2274H20 | 5 | 10359620 | 10495937 | 0.0433 | 0.2144 | 0.4293 |
| 2488 | RP11-215G15 | 5 | 10644705 | 10722894 | 0.0433 | 0.2144 | 0.4293 |
| 1393 | GS-62-L11 | 6 | 110996 | 260996 | 0.0298 | 0.1440 | 0.4207 |
| 1927 | GS-196-I5 | 6 | 225000 | 375000 | 0.0295 | 0.1845 | 0.4135 |
| 3809 | RP11-13J16 | 6 | 1240996 | 1301756 | 0.1256 | 0.1486 | 0.3986 |
| 3817 | RP11-15N12 | 6 | 3292049 | 3425043 | 0.1256 | 0.1486 | 0.3986 |
| 1646 | RP11-174B19 | 6 | 5151674 | 5170849 | 0.1256 | 0.1486 | 0.3986 |
| 3818 | RP11-232H4 | 6 | 6151264 | 6169479 | 0.1256 | 0.1486 | 0.3986 |
| 3330 | RP11-163I22 | 6 | 6217514 | 6382300 | 0.1256 | 0.1486 | 0.3986 |
| 3271 | RP11-320C15 | 6 | 6705338 | 6770147 | 0.1256 | 0.1486 | 0.3986 |
| 3252 | RP3-336K20 | 6 | 7544150 | 7575683 | 0.1256 | 0.1681 | 0.3972 |
| 1527 | RP11-196B15 | 6 | 7898162 | 8078796 | 0.1257 | 0.1759 | 0.4006 |
| 3807 | RP11-385L2 | 6 | 8598346 | 8599694 | 0.1257 | 0.1759 | 0.4006 |
| 1119 | RPII-360O19 | 6 | 10597237 | 10684872 | 0.1254 | 0.1663 | 0.3932 |
| 1623 | RP3-510L9 | 6 | 11352273 | 11482926 | 0.1260 | 0.1625 | 0.4060 |

(continued)

| Order | Clone | chrom | Start | End | sc_control | sc_BRCA2 | sd |
|---|---|---|---|---|---|---|---|
| 1114 | RP11-97A19 | 6 | 12430744 | 12449045 | 0.1046 | 0.1625 | 0.4014 |
| 3242 | RP1-257A7 | 6 | 13285332 | 13413248 | 0.1045 | 0.1559 | 0.4000 |
| 3262 | RP3-365E2 | 6 | 14536103 | 14673832 | 0.1044 | 0.1513 | 0.3972 |
| 1637 | RP1-232K4 | 6 | 15434571 | 15630732 | 0.1041 | 0.1434 | 0.3903 |
| 3241 | RP1-13D10 | 6 | 16165681 | 16318728 | 0.1041 | 0.1434 | 0.3903 |
| 1106 | RP11-68J15 | 6 | 17735571 | 17805613 | 0.1041 | 0.1386 | 0.3909 |
| 3811 | RP11-408C8 | 6 | 18937804 | 19079559 | 0.1043 | 0.1318 | 0.3961 |
| 1087 | RP4-625H18 | 6 | 19863670 | 20018592 | 0.1046 | 0.1361 | 0.4023 |
| 1634 | RP3-444C7 | 6 | 20630894 | 20759064 | 0.1046 | 0.1361 | 0.4023 |
| 1650 | RP11-204E9 | 6 | 21543541 | 21694512 | 0.1051 | 0.1456 | 0.4128 |
| 3825 | RP11-33I5 | 6 | 22580401 | 22666188 | 0.1051 | 0.1456 | 0.4128 |
| 1666 | RP11-289M23 | 6 | 23499374 | 23534047 | 0.1051 | 0.1456 | 0.4128 |
| 1116 | RP11-176J5 | 6 | 24254612 | 24267701 | 0.1054 | 0.1384 | 0.4196 |
| 3826 | RP11-289G11 | 6 | 25494592 | 25630305 | 0.1342 | 0.1383 | 0.4307 |
| 3286 | RP11-150A6 | 6 | 29321010 | 29495872 | 0.0895 | 0.0895 | 0.4020 |
| 3249 | RP1-34F7 | 6 | 32031923 | 32200821 | 0.0823 | 0.0823 | 0.3915 |
| 3255 | RP5-1077I5 | 6 | 32404070 | 32498505 | 0.0606 | 0.0606 | 0.4543 |
| 991 | RP11-48M17 | 9 | 2136330 | 2296367 | -0.1362 | -0.0273 | 0.4484 |
| 997 | RP11-320E16 | 9 | 2583689 | 2658649 | -0.1356 | -0.0121 | 0.4619 |
| 3595 | RP11-509J21 | 9 | 3533196 | 3696642 | -0.1356 | -0.0121 | 0.4619 |
| 3144 | RP11-125K10 | 9 | 4819656 | 4991841 | -0.1356 | -0.0121 | 0.4619 |
| 1435 | RP11-509D8 | 9 | 4991742 | 5008809 | -0.1347 | -0.0019 | 0.4817 |
| 456 | RP11-106A1 | 9 | 6599727 | 6659690 | -0.1258 | -0.0142 | 0.4584 |
| 979 | RP11-29B9 | 9 | 7904421 | 8056890 | -0.1258 | -0.0142 | 0.4584 |
| 445 | RP11-175E13 | 9 | 8398602 | 8490985 | -0.1258 | -0.0142 | 0.4584 |
| 1439 | RP11-527D15 | 9 | 9657591 | 9823759 | -0.1261 | -0.0212 | 0.4511 |
| 447 | RP11-19G1 | 9 | 9999831 | 10094473 | -0.1261 | -0.0212 | 0.4511 |
| 2615 | RP11-23D5 | 9 | 11227455 | 11303482 | -0.1261 | -0.0262 | 0.4514 |
| 442 | RP11-352F21 | 9 | 11389654 | 11588151 | -0.1261 | -0.0212 | 0.4511 |
| 3162 | RP11-446F 13 | 9 | 12281538 | 12346848 | -0.1262 | -0.0130 | 0.4502 |
| 449 | RP11-187K14 | 9 | 12872920 | 12952270 | -0.1262 | -0.0130 | 0.4502 |
| 3142 | RP11-413D24 | 9 | 13729563 | 13912972 | -0.1268 | -0.0214 | 0.4375 |
| 462 | RP11-408A13 | 9 | 14500602 | 14586756 | -0.1268 | -0.0214 | 0.4375 |
| 3587 | RP11-490C5 | 9 | 15298239 | 15399992 | -0.1268 | -0.0214 | 0.4375 |
| 460 | RP11-109M15 | 9 | 16171750 | 16325494 | -0.1268 | -0.0419 | 0.4371 |
| 984 | RP11-132E11 | 9 | 16995146 | 17138368 | -0.1268 | -0.0419 | 0.4371 |
| 980 | RP11-123J20 | 9 | 17933998 | 18013852 | -0.1269 | -0.0366 | 0.4347 |

(continued)

| Order | Clone | chrom | Start | End | sc_control | sc_BRCA2 | sd |
|---|---|---|---|---|---|---|---|
| 1431 | RP11-503K16 | 9 | 18579960 | 18743122 | -0.1269 | -0.0366 | 0.4347 |
| 3599 | RP11-513M16 | 9 | 19310506 | 19423218 | -0.1269 | -0.0366 | 0.4347 |
| 2607 | RP11-15P13 | 9 | 20172502 | 20351132 | -0.1270 | -0.0446 | 0.4320 |
| 3140 | RP11-113D19 | 9 | 20996401 | 21158465 | -0.1271 | -0.0386 | 0.4303 |
| 3156 | RP11-14912 | 9 | 21899259 | 22000413 | -0.1271 | -0.0386 | 0.4303 |
| 443 | RP11-11J1 | 9 | 22479496 | 22579721 | -0.1269 | -0.0502 | 0.4353 |
| 3591 | RP11-495L19 | 9 | 23461740 | 23557472 | -0.1269 | -0.0502 | 0.4353 |
| 3147 | RP11-33K8 | 9 | 24090706 | 24243453 | -0.1029 | -0.0468 | 0.4324 |
| 3583 | RP11-468C2 | 9 | 24937707 | 25069411 | -0.1029 | -0.0468 | 0.4324 |
| 3219 | RP11-211N8 | 9 | 46630312 | 46771375 | -0.3035 | -0.1792 | 0.6076 |
| 1931 | GS-261-B16 | 10 | 135029737 | 135179737 | -0.2825 | -0.2058 | 0.5492 |
| 4089 | GS-137-E24 | 10 | 135029787 | 135179787 | -0.2825 | -0.2058 | 0.5492 |
| 969 | RP11-45C5 | 11 | 100145392 | 100299550 | -0.0568 | 0.1322 | 0.3996 |
| 3423 | RP11-21G19 | 11 | 101247704 | 101397612 | -0.0568 | 0.1322 | 0.3996 |
| 3343 | RP11-315O6 | 11 | 101724655 | 101922841 | -0.0568 | 0.1322 | 0.3996 |
| 1992 | RP11-750P5 | 11 | 102010617 | 102011178 | -0.0564 | 0.1222 | 0.4083 |
| 2957 | RP11-2I22 | 11 | 102613695 | 102771597 | -0.0563 | 0.1073 | 0.4111 |
| 1464 | RP11-725J15 | 11 | 102741000 | 102891000 | -0.0563 | 0.1073 | 0.4111 |
| 222 | RP11-563P16 | 11 | 102966300 | 103066089 | -0.0563 | 0.1041 | 0.4120 |
| 931 | RP11-76K19 | 13 | 19007559 | 19137337 | 0.0095 | -0.0541 | 0.4080 |
| 3092 | RP11-187L3 | 13 | 19832836 | 19920914 | 0.0097 | -0.0675 | 0.4036 |
| 943 | RP11-110K8 | 13 | 20994607 | 21089699 | 0.0050 | -0.0692 | 0.5056 |
| 399 | RP11-26A3 | 13 | 21944537 | 22068982 | 0.0096 | -0.0752 | 0.4060 |
| 1983 | RP11-760M1 | 13 | 22887525 | 23056187 | 0.0096 | -0.0752 | 0.4060 |
| 950 | RP11-556N21 | 13 | 23994474 | 24103118 | -0.0177 | -0.0752 | 0.4051 |
| 3107 | RP11-111G7 | 13 | 25124500 | 25300819 | -0.0177 | -0.0804 | 0.4036 |
| 1979 | RP11-570F6 | 13 | 25898592 | 26065378 | -0.0177 | -0.0804 | 0.4036 |
| 1275 | RP11-44J9 | 13 | 26088616 | 26258539 | -0.0177 | -0.0804 | 0.4036 |
| 2556 | RP11-125I23 | 13 | 26843991 | 27010032 | -0.0182 | -0.0876 | 0.4156 |
| 3342 | RP11-153M24 | 13 | 27414162 | 27490882 | -0.0182 | -0.0876 | 0.4156 |
| 3099 | RP11-95G6 | 13 | 27868844 | 27970962 | 0.0283 | -0.0827 | 0.4242 |
| 936 | RP11-218E6 | 13 | 28837843 | 29008462 | 0.0283 | -0.0741 | 0.4236 |
| 946 | RP11-550P23 | 13 | 29914762 | 30048628 | 0.0898 | -0.0312 | 0.4195 |
| 939 | RP11-95N14 | 13 | 31136680 | 31227403 | 0.0903 | -0.0501 | 0.4084 |
| 3349 | RP11-37E23 | 13 | 31703950 | 31870606 | 0.0903 | -0.0501 | 0.4084 |
| 2564 | RP11-141M1 | 13 | 32790709 | 32842539 | 0.0903 | -0.0501 | 0.4084 |
| 3095 | RP11-87G1 | 13 | 32842440 | 33025356 | 0.0903 | -0.0501 | 0.4084 |

(continued)

| Order | Clone | chrom | Start | End | sc_control | sc_BRCA2 | sd |
|-------|-------|-------|-------|-----|------------|----------|-----|
| 2553 | RP11-266E6 | 13 | 33995013 | 34159357 | 0.0903 | -0.0544 | 0.4097 |
| 3103 | RP11-98D3 | 13 | 34909905 | 35080902 | 0.0530 | -0.0545 | 0.3985 |
| 2555 | RP11-10M8 | 13 | 37051980 | 37156004 | 0.0149 | -0.0742 | 0.4204 |
| 400 | RP11-131P10 | 13 | 37797278 | 37920029 | 0.0149 | -0.0742 | 0.4204 |
| 2560 | RP11-131F1 | 13 | 38577172 | 38608597 | 0.0150 | -0.0949 | 0.4172 |
| 2554 | RP11-407E23 | 13 | 39306578 | 39498144 | -0.0184 | -0.1008 | 0.4195 |
| 2551 | RP11-2P5 | 13 | 40367434 | 40566000 | -0.0189 | -0.1092 | 0.4305 |
| 392 | RP11-125A7 | 13 | 41105696 | 41263001 | -0.0189 | -0.1092 | 0.4305 |
| 3111 | RP11-117I13 | 13 | 42155411 | 42253968 | -0.0185 | -0.1392 | 0.4211 |
| 2568 | RP11-168P13 | 13 | 43148292 | 43291406 | -0.0184 | -0.1476 | 0.4193 |
| 406 | RP11-442J21 | 13 | 44121277 | 44178227 | -0.0184 | -0.1435 | 0.4195 |
| 413 | RP11-351K3 | 13 | 44939380 | 45114967 | -0.0185 | -0.1385 | 0.4226 |
| 394 | RP11-408L13 | 13 | 46981023 | 47110676 | -0.0186 | -0.1323 | 0.4241 |
| 3339 | RP11-305D15 | 13 | 47760580 | 47897921 | -0.0206 | -0.1513 | 0.4681 |
| 408 | RP11-174I10 | 13 | 47897822 | 47960646 | -0.0206 | -0.1513 | 0.4681 |
| 412 | RP11-185C18 | 13 | 48960782 | 49068912 | -0.0185 | -0.1317 | 0.4217 |
| 2563 | RP11-40A8 | 13 | 50328519 | 50471842 | -0.0188 | -0.0988 | 0.4296 |
| 3051 | RP1-126N20 | 21 | 14634832 | 14731794 | -0.0823 | -0.3210 | 0.5819 |

BRIEF DESCRIPTION OF THE DRAWINGS

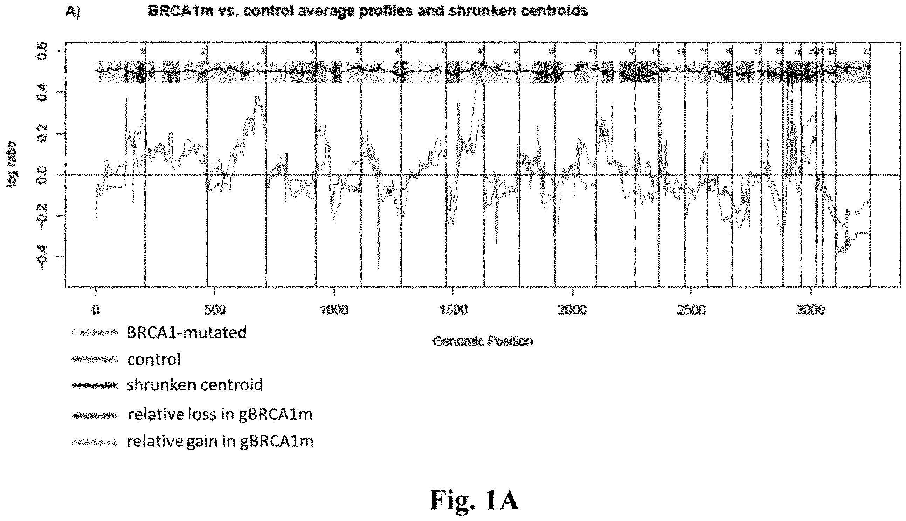

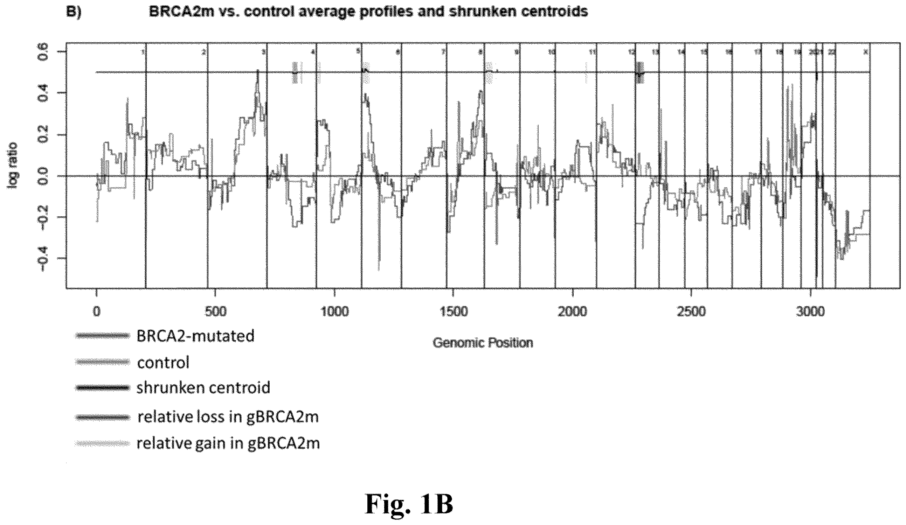

**Figure** 1: Average copy number aberration profiles of BRCA1- and BRCA2- mutated ovarian cancer. A) Average copy number aberration profile of 50 gBRCA1m ovarian cancers and 13 control ovarian cancers. On the x-axis, the cumulative genomic position, in mega bases, and the y-axis, the average 2log ratio of tumor over normal DNA. B) Average copy number aberration profile of 10 gBRCA2m ovarian cancers and 13 control ovarian cancers. On the x-axis the genomic position and on the y-axis the average 2log ratio of tumor DNA over normal DNA.

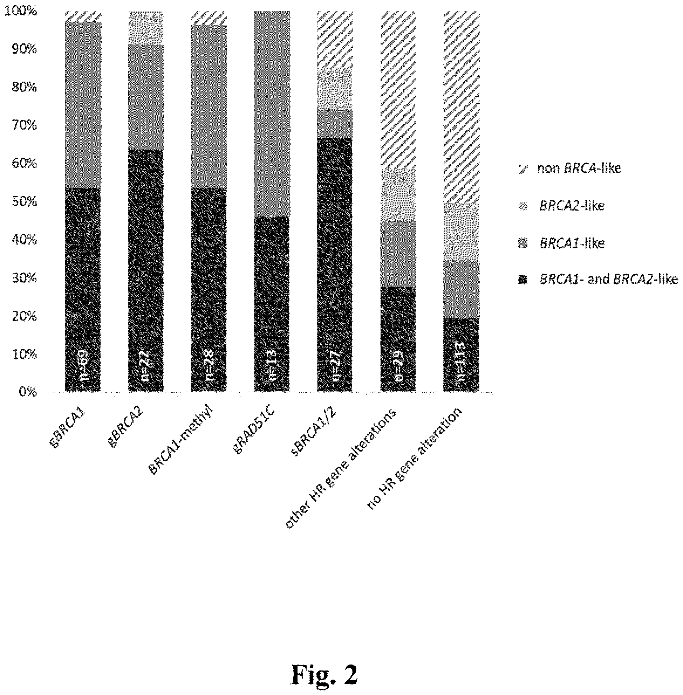

**Figure** 2: BRCA-like phenotype according to molecular genetic background. Each bar shows the distribution by percentage (y-axis) of BRCA1-and 2-like, only BRCA1-like, only BRCA2-like and non-BRCA-like profiles within the presented molecular genetic subgroup (x-axis). methyl: promoter hypermethylation, WT: wildtype.

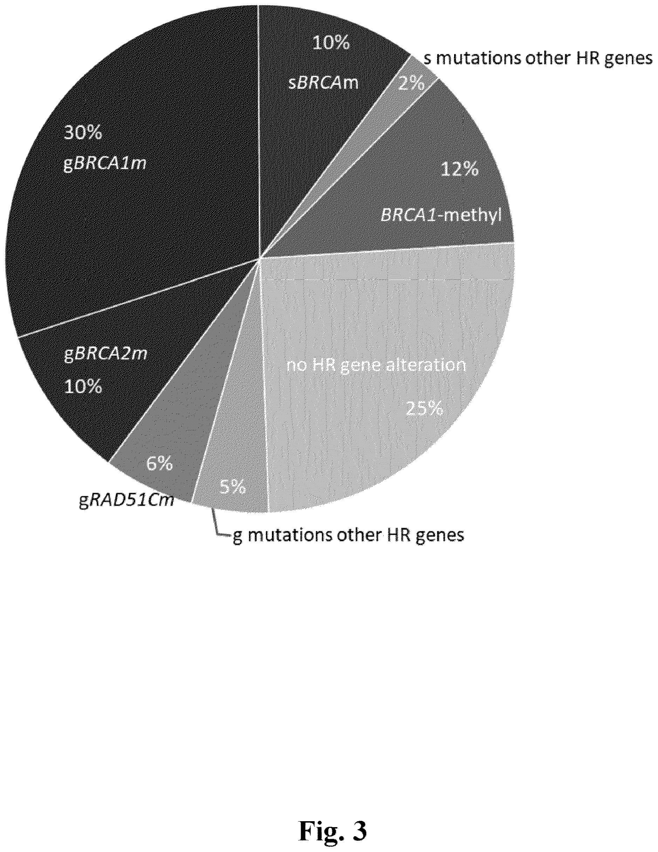

**Figure** 3: Molecular genetic background of BRCA-like cases. 223 samples are classified asBRCA1-and/ or BRCA2-like. The pie chart shows the portion of samples with a specified (epi-)genetic alteration of a gene, involved in homologous recombination in this subgroup. In 25.1% of the cases no HR-affecting variant was detected by NGS or methylation analyses. methyl: promoter hypermethylation.

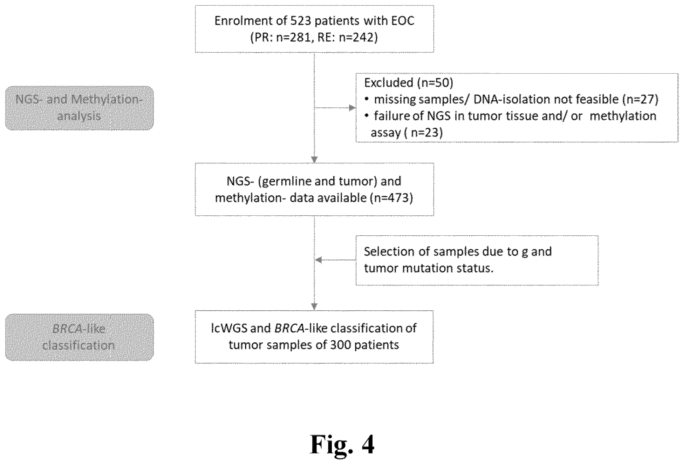

**Figure** 4: CONSORT-like flow diagram of the AGO-TR1 trial. The sample for *BRCA*-like classification has been selected with the aim to enrich for germline (g) and somatic (s) mutation status. All available samples with a deleterious mutation in *BRCA1/2* (g n=91, s n=27) and other ovarian cancer related genes (g n=34, s n=9) as well as somatic *BRCA1/2*-variants of unknown significance (VUS) (n=4) have been included, whereas g*BRCA1/2*-VUS (n=15) have been excluded. The remaining 135 samples were considered random sample of the main cohort, taking primary ovarian cancer (PR)-/platinum sensitive recurrent disease (RE)-status, age at diagnosis and sTP53-mutations into account. EOC: epithelial ovarian cancer; PR: primary disease; RE: relapsed; NGS: next generation sequencing; g: germline; lcWGS: low coverage whole genome sequencing.

DETAILED DESCRIPTION OF THE INVENTION

Tumors with HRD have been shown to be particularly sensitive to DNA crosslinking agents, such as alkylators, platinum drugs or platinating agents as well as to PARP inhibitors. The breast and ovarian cancer germline predisposition genes BRCA1 and BRCA2 play crucial roles in homologous recombination. Thus, ovarian cancers associated with a germline *BRCA1* or *BRCA2* mutation derive benefit of targeted therapy, for example with PARP inhibitors or specific classes of chemotherapy, such as platinum compounds as mentioned above. Furthermore, up to approximately 50% of tumors in ovarian cancer share characteristics with germline mutated cancers, because of other alterations in the *BRCA1/BRCA2* pathway of homologous recombination, and therefore those benefit of such therapies as well. This phenotype is called BRCAness or BRCA-like.

Breast cancer BRCA1-like and BRCA2-like copy number profile classifiers predictive for mutation status and response to therapy, targeting HRD have been developed before, but breast cancer classifiers did not sufficiently predict mutation status in ovarian cancer. Hence, new classifiers had to be developed.

Accordingly, the present invention provides predictive biomarkers to identify the germline mutated as well as said *BRCA*ness group for therapy specifically targeting *BRCA*ness in ovarian cancer. In other words, the method of the present invention is useful for identifying patients with ovarian cancer, wherein the cancer cells have a defective DNA repair system, and thus, which are responsive to the mentioned targeted anti-cancer therapy.

Furthermore, the method of the present invention is also useful for identifying a group of patients that is non-BRCA1/2 and non-BRCA1/2-like, respectively, and that does not benefit of this therapy. Until now, no reliably method has been described that also identifies the nonbenefitting population correctly.

Cancers and cell lines with BRCA mutations or mutations in the BRCA pathway exhibit genomic instability, manifesting in abnormal copy number profiles. Thus, in general, the present invention is based on the discovery that certain chromosomal copy number aberrations or variations, respectively (CNVs) in tumor cells allow tumors to be classified as either BRCA-associated-/ BRCA-like tumors or sporadic tumors. This classification of a tumor allows for the prospective prediction of responsiveness of the patient from which the tumor was removed to anti-cancer therapy.

In the literature, other assays have been described measuring HRD in ovarian cancer (15, 17, 21, 46), some of which are also partly based on derivatives of SNP/copy number profiles. The approach of the present invention differs *inter alia* from other tests because it uses genomic location-specific aberrations. This leads to additional information being retained, such as aberrations that collaborate with the underlying HRD mechanism. Additionally, the presented classifiers are tissue-specific, which is an advantage compared to other HRD assays, as a response to targeted strategies can depend on the tissue of origin.

The terms copy number aberrations, copy number variants/variations and CNVs are used interchangeable herein. Furthermore, when reference is made to BRCA1-like or BRCA2-like, for example BRCA1-like tumors, or BRCA2-like tumors, if not indicated otherwise, this includes tumors having a mutation in the BRCA1 gene or BRCA2 gene itself and tumors which share characteristics with those germline mutated cancers, because of other alterations in the *BRCA1/BRCA2* pathway of homologous recombination.

HRD is a defect in DNA repair by hampered homologous recombination repair (HRR), which is a form of DNA recombination often used to repair DNA double strand breaks. In cancers, this is often caused by loss of function mutations in BRCA1, BRCA2, RAD51C, RAD51D or PALB2, promoter hypermethylation of the BRCA1 gene promoter (leading to reduced expression of BRCA1) or a series of as yet to be defined causes. HRD is also characterized by the cellular sensitivity to for example PARP inhibitors, topoisomerase inhibitors or platinum salts.

The method of the present invention comprises the determination of a CNV of a genomic locus in a test sample from a subject comprising cancer cells, *e.g.* a DNA test sample of cancer cells, wherein the locus is selected from a pool of genomic loci comprising the loci set forth in Table 1 or in Table 2, and wherein similarity between the CNVs in the pool of genomic loci in the test sample from the subject and the CNVs in a corresponding pool of genomic loci in the DNA of a reference sample of BRCA-like mutated cancer cells and a control sample of BRCA non-mutated cancer cells, is indicative for the HR deficiency status in the subject, wherein similarity is the distance measure between centroids of the two classes, *i.e.,* the control and the reference sample, and the centroid of the test sample, wherein the centroid of a class is constructed by shrinking the class centroid by an optimization routine towards the overall centroid of both classes after standardizing by the within-class standard deviation for each CNV.

The test sample is a sample of material comprising cancer cells, in particular ovarian cancer cells. Samples include, but are not limited to material obtained from the subject of interest, *i.e.* from the ovarian cancer patient and may be directly obtained from the source, for example via tumor biopsy, or indirectly after culturing and/or further processing of the tumor cells. In particular, the test sample is a DNA sample or a sample that comprises DNA from the tumor cells of the subject of interest. Accordingly, the test sample is derived from a subject that is to be diagnosed of having HRD status or not and that is to be diagnosed of being responsive to a particular anti-cancer therapy or not. Any appropriate type of sample can be assessed to determine if the genome of the cancer cells contains an HRD signature, or lacks an HRD signature. Examples of samples containing cancer cells, in particular ovarian cancer cells that can be assessed as described herein include, without limitation, tumor biopsy samples (e.g., ovarian tumor biopsy samples), formalin-fixed, paraffin-embedded tissue samples containing cancer cells, core needle biopsies, fine needle aspirates, and samples containing cancer cells shed from a tumor. For formalin-fixed, paraffin-embedded tissue samples, the sample can be prepared by DNA extraction using a genomic DNA extraction kit optimized for FFPE tissue, for example Qiagen DNA mini kit (Qiagen, cat nr. 51306), QuickExtract™ FFPE DNA Extraction Kit (Epicentre™), and QIAamp™ DNA FFPE Tissue Kit (Qiagen™)).

In some cases, laser dissection techniques can be performed on a tissue sample to minimize the number of non-cancer cells within a cancer cell sample to be assessed. In some cases, antibody based purification methods can be used to enrich for cancer cells and/or deplete non-cancer cells. Examples of antibodies that could be used for cancer cell enrichment include, without limitation, anti-EpCAM, anti-TROP-2, anti-c-Met, anti-Folate binding protein, anti-N-Cadherin, anti-CD318, anti-antimesencymal stem cell antigen, anti-Her2, anti-MUCl, anti-EGFR, anti-cytokeratins (e.g., cytokeratin 7, cytokeratin 20, etc.), anti-Caveolin-1, anti-PSA, anti-CA125, and anti-surfactant protein antibodies. Even if dynamic ranges of the profiles are influenced, validation can be performed by the person skilled in the art as described herein.

The control sample used in the method of the present invention is a corresponding sample comprising ovarian cancer cells and the corresponding DNA, respectively, which are not associated with BRCA1-like or BRCA2-like mutations and do not have a HRD signature.

The reference sample is a corresponding sample comprising BRCA mutated ovarian cancer cells and the corresponding DNA, respectively. Based on such reference sample and a plurality of such reference samples, respectively, ovarian cancer specific DNA copy number aberration classifiers have been developed that identify a population enriched for BRCA1 and HRD-associated gene mutations (BRCA1-like), i.e. BRCA1-like classifiers, and a population enriched for BRCA2 and HR-associated gene mutations (BRCA2-like), i.e. BRCA2-like classifiers and a population enriched for BRCA1-like and BRCA2-like mutations. Those classifiers are listed in Table 1 (BRCA1-like) and Table 2 (BRCA2-like).

In particular, those classifiers have been developed by generating copy number profiles of control samples, i.e. samples derived from patients without a family history of ovarian or breast cancer, of gBRCA1m samples, i.e. samples derived from patients having ovarian cancer and having mutations in the BRCA1 gene, and of gBRCA2m samples, i.e. samples derived from patients having ovarian cancer and having mutations in the BRCA2 gene. Figure 1 presents the average profiles of gBRCA1m and gBRCA2m versus control ovarian cancers. The generation of the copy number profiles is described in Example 1 in more detail. Thus, the present invention also relates to the development of BRCA1-like and BRCA2-like classifiers by generating copy number profiles of control samples, of gBRCA1m samples and of gBRCA2m samples, respectively.

After generating the copy number profiles, the ovarian cancer specific shrunken-centroid classifiers have been trained on ovarian cancer data. These shrunken centroids select genomic regions and weights that are discriminative for the *BRCA*-like and non-*BRCA*-like class. For this, ten-fold cross-validation has been carried out and training of the shrunken centroid classifiers was performed on the class labels gBRCA1m vs. control and gBRCA2m vs. control to classify ovarian cancer as being similar to gBRCA1m (BRCA1-like) or gBRCA2m (BRCA2-like) or controls (C). Subsequently, samples were classified as *BRCA*-like if the predicted probability was > 0.5 and non-*BRCA*-like if the predicted probability was <= 0.5, as was pre-defined in the training set. This is described in more detail in Example 2. Thus, the present invention also relates to the training of the above-mentioned classifiers on ovarian cancer data. In principle any data can be used as long as they comprise data of control samples and of gBRCA1m samples and gBRCA2m samples, respectively.

The training was followed by validation of the classifiers as for example described in Examples 3 and 4. As described in Example 3 validation was performed by using the TCGA dataset. In particular, 358 of 583 samples have been classified as having a BRCA1-like profile. Within these 358, 25/26 gBRCA1, 10/11 somatic BRCA1-mutated tumors, and 67/69 BRCA1-methylated tumors were classified as BRCA1-like, resulting in an overall sensitivity of 94.7% (95% CI: 0.82-0.99) and a specificity of 40% (95% CI: 0.36-0.45). Furthermore, 350 of 583 samples were assigned to be BRCA2-like. Within these 350, 20/25 gBRCA2m 309 and 8/11 sBRCA2m samples were classified as BRCA2-like, resulting in a sensitivity of 77% (95% CI: 0.6-0.9) and a specificity of 41% (95% CI: 0.37-0.49).

Validation was also performed within AGOTR1 study samples, wherein in total 300 samples with complete genetic and epigenetic information available, were analyzed. The results of the validation are presented in Example 4. In particular, focus was first put on the detection-rate of tumors derived from deleterious germline mutations in BRCA1/2 (see Table 6, Figure 2). 67 of 69 BRCA1-mutated cases were BRCA1-like (detection rate of 97.1%), whereas 16 of 22 samples with a BRCA2 class4/5 variant in germline were BRCA2-like (detection rate of 72.7%). All remaining BRCA2-associated cases were identified by combining both classifiers, i.e. being BRCA1- or BRCA2-like. Regarding deleterious somatic variants, 15 of 17 (88.2%) samples with a BRCA1 mutation were identified by the BRCA1-like classifier. As the other two tumors displayed a BRCA2-like phenotype, both classifiers' application detected 100% of the mutated cases. The BRCA2-like classifier confirmed 6 of 10 sBRCA2m cases. The BRCA1-like classifier found no additional sample.

Of the 28 examined samples with a BRCA1 promoter hypermethylation, 27 displayed a BRCA1- like profile (96.4%), one was classified as non-BRCA-like. Thus, in one embodiment, the present invention relates to the validation of the classifiers. In principle, validation can be performed with any samples or dataset which comprise samples and data, respectively comprising a BRCA-like (BRCA1-like and BRCA2-like) profile and a control profile. Furthermore, the validation set also comprises samples and data, respectively for which the class is known, *i.e.* with pathogenic variants/mutations in for example the BRCA1 and BRCA2 gene, respectively or other HRD related genes as mentioned below. Optionally, the validation data set further comprises samples and data, respectively that are non-BRCA-like *i.e.* that do not comprise a mutation in any of the HRD pathway related genes. Alternatively or in addition, validation is also possible by determining the therapy response, *i.e.* whether a patient responds to a treatment with a treatment regimen comprising homologous recombination deficiency-targeting drugs, drugs that directly cause double strand DNA breaks, drugs that indirectly cause double strand DNA breaks; and/or drugs which have been identified in mechanistic studies to target homologous recombination deficiency.

As mentioned above, HRD is not only associated with alterations in the BRCA genes. Thus, alterations in further HR genes (ATM, BARD1, BRIP1, CHEK1, CHEK2, FAM175A, FANCM, MRE11A, NBN, PALB2, RAD50, RAD51C, RAD51D, and XRCC2) are being classified as BRCA-like and as yet unknown genes. Within the study cohort, 44 tumors without BRCA mutation or BRCA1 methylation displayed a genetic alteration in another OC-risk- or HR-gene (except sTP53 mutations), of which 32 were classified as BRCA-like (72.7%; only BRCA1-like n=11, only BRCA2-like n=4, BRCA1- and BRCA2-like n=15). The detailed results are presented in Example 5.

Thus, the present invention also relates to the development and/or training and optionally validation of the classifiers which are used in the method of the present invention, *i.e.* the method of determining HRD status in a subject having ovarian cancer and the method of predicting the response of a subject having ovarian cancer to an anti-cancer therapy, respectively.

The method of the present invention comprises the determination of a CNV of a genomic locus in a test sample from a subject comprising cancer cells, *e.g.* a DNA test sample of cancer cells, wherein the locus is selected from a pool of genomic loci comprising the loci set forth in Table 1 or in Table 2, and wherein similarity between the CNVs in the pool of genomic loci in the test sample from the subject and the CNVs in a corresponding pool of genomic loci in the DNA of a reference sample of BRCA-like mutated cancer cells and a control sample of BRCA non-mutated cancer cells, is indicative for the HRD status in the subject, i.e. whether the cancer cells have a HRD signature or not.

In particular, similarity between the CNVs of the test sample and the CNVs of the reference sample identifies the subject as HR deficient, wherein similarity between the CNVs of the test sample and the CNVs of the control sample identifies the subject as not being HR deficient. Furthermore, non-similarity between the CNVs of the test sample and the CNVs of the reference sample can also identify a subject as not being HR deficient.

Determining CNVs is usually performed at one or more genomic loci. In principle, any appropriate technique can be used to determine genotypes at loci of interest within the genome of a cell. For example, array-based comparative genomic hybridization (array-CGH) can be used. CGH refers generally to molecular-cytogenetic techniques for the analysis of copy number changes, gains or losses, in the DNA content of a given subject's DNA. CGH can be used to identify chromosomal alterations, such as unbalanced chromosomal changes, in any number of cells including, for example ovarian cancer cells. CGH can be utilized to detect one or more chromosomal amplifications and/or deletions of regions between a test sample and a reference/control sample. In particular, nucleic acids from a test sample and nucleic acids from a reference/control sample are labelled differentially, hybridized to an array comprising a plurality of probes, preferentially covering the whole genome, and wherein the ratio of the signal intensity of the test sample to that of the reference/control sample is calculated to measure the copy number changes for a particular location in the genome. This is exemplarily described in WO 2012/038837 A2 as well as in the references cited therein. Furthermore, single nucleotide polymorphism (SNP) arrays, molecular inversion probe (MIP) assays, targeted sequencing of loci of interest, and large-scale sequencing (e.g., whole exome, transcriptome, or genome sequencing) can be used to identify CNVs (as long as they cover or sufficiently approximate the loci of interest). Preferably, low coverage whole genome sequencing (LC-WGS) is used, preferably single-read sequencing, wherein the read length can vary and is for example between 30 to 200 bp, preferably between 30 and 100 bp and most preferably about 65 bp. Furthermore, the LC-WGS can be performed with a coverage of for example 0.1 - 3X, and preferably with a coverage of 0.5X. The sequencing data can then by analyzed regarding the copy numbers of the DNA in the genomic loci.

Due to the development of the classifiers set forth in Tables 1 and 2 and as explained above, it was possible to establish the method of the present invention which detects highly reliable HRD status in a subject. However, the person skilled in the art is well aware of the fact that not all genomic loci set forth in Table 1 and Table 2, respectively need to be considered in order to receive a reliable result on the HRD status of the subjects. In particular, as soon as the BRCA1-like classifiers as set forth in Table 1 and the BRCA2-like classifiers set forth in Table 2 have been developed, the person skilled in the art knows how to vary the method so that still reliable results are achieved. For example, it is possible that the CNVs in additional loci are considered, or that the CNVs in less loci are considered, or that some different loci are considered in comparison to those listed in Table 1 and Table 2, respectively. In particular, variations can be made as long as the method still reaches an appropriate sensitivity, preferably a sensitivity of 70% to 100 %, more preferably of 72 % to 97%, that allows to make a reliable statement about the HRD status of the subject to be tested.

As regards the BRCA1-like classifiers, the pool of genomic loci can vary as long as the method of the present invention still shows a sensitivity of at least 90%, *i.e.* a sensitivity between 90% and 100%, preferably of at least 94 % to 97%, *i.e.* a sensitivity between 94 to 97% and 100%. In one embodiment, the pool of genomic loci comprises at least 200, preferably 400, more preferably 600, more preferably 800, more preferably 1000, more preferably 1200, more preferably 1400, more preferably 1600, more preferably 1800, more preferably 2000, more preferably 2200, more preferably 2400, and most preferably all of those loci listed in Table 1 as long as the above-mentioned sensitivities are reached. Of course any numbers of loci in between are also encompassed and just not mentioned for simplicity.

As regards the BRCA2-like classifiers, the pool of genomic loci can vary as long as the method of the present invention still shows a sensitivity of at least 70%, i.e. a sensitivity between 70% and 100%, preferably of at least 72% to 77%, i.e. a sensitivity between 72 to 77% and 100%. In one embodiment, the pool of genomic loci comprises at least 20, preferably 40, more preferably 60, more preferably 80, more preferably 100, more preferably 120, and most preferably all of those loci listed in Table 2 as long as the above-mentioned sensitivities are reached. Of course any numbers of loci in between are also encompassed and just not mentioned for simplicity.

Thus, similarity or non-similarity between the CNVs in a pool of genomic loci in the test sample from the subject and the CNVs in a corresponding pool of genomic loci in the DNA of a reference sample of BRCA-like mutated cancer cells and a control sample of BRCA non-mutated cancer cells, can be determined in the method of the present invention by considering at least 200, preferably 400, more preferably 600, more preferably 800, more preferably 1000, more preferably 1200, more preferably 1400, more preferably 1600, more preferably 1800, more preferably 2000, more preferably 2200, more preferably 2400, and most preferably all of those loci listed in Table 1 and/or by considering at least 20, preferably 40, more preferably 60, more preferably 80, more preferably 100, more preferably 120, and most preferably all of those loci listed in Table 2, respectively.

The method of the present invention also encompasses the determination of CNV of one genomic locus in a test sample from a subject comprising cancer cells, *e.g.* a DNA test sample of cancer cells, wherein the locus is selected from a pool of genomic loci comprising the loci set forth in Table 1, wherein determination of the CNV and the CNVs, respectively, can be made in one of the genomic loci, in two, three, four, five [...], or in all 2597 of the genomic loci. [...] represents all numbers from six to 2596. The method of the present invention also encompasses the determination of a CNV of one genomic locus in a test sample from a subject comprising cancer cells, *e.g.* a DNA test sample of cancer cells, wherein the locus is selected from a pool of genomic loci comprising the loci set forth in Table 2, wherein determination of the CNV and the CNVs, respectively, can be made in one of the genomic loci, in two, three, four, five [...], or in all 138 of the genomic loci. [...] represents all numbers from six to 137.

As mentioned above, CNVs include losses and gains of genomic segments. The similarity is defined as distance measure between the centroids of the two classes, *i.e.* the control and reference sample, and the centroid of the test sample. A centroid of a class, which is in the present case the control and reference sample, respectively, is constructed by shrinking the class centroid by an optimization routine towards the overall centroid of both classes after standardizing by the within-class standard deviation for each CNVs.

A similarity score, *i.e.* a posterior probability score, is computed by providing a shrunken centroid value which is derived from the CNV of the genomic locus and wherein the comparison of the shrunken centroids of the test sample from the subject with the shrunken centroids of the reference sample and the shrunken centroids of the control sample is converted into a posterior probability score according to Tibshirani PNAS 2002 (29). This score ranges from 0-1, with 0-0.49 being non-BRCA-like and 0.5-1 being BRCA-like.

Thus, in one embodiment the method of the present invention comprises, or essentially consists of the determination of a CNV of a genomic locus in a test sample from a subject, wherein the test sample comprises cancer cells, *e.g.* a DNA test sample of cancer cells, wherein the locus is selected from a pool of genomic loci comprising the loci set forth in Table 1, the loci set forth in Table 2, or the loci set forth in Tables 1 and 2; and the provision of a shrunken centroid value which is derived from the CNV of the genomic locus, and wherein a posterior probability score of $\geq 0.5$ identifies the subject as HRD; and/or wherein a posterior probability score of $< 0.5$ identifies the subject as not being HRD.

As mentioned above, the determination of the CNV and the CNVs, respectively, can be made in one of the genomic loci, in two, three, four, five [...], or in all 2597 of the genomic loci listed in Table 1 ([...] represents all numbers from six to 2596), and/or in one of the genomic loci, in two, three, four, five [...], or in all 138 of the genomic loci listed in Table 2 ([...] represents all numbers from six to 137).

The posterior probability score is generally obtainable by comparing the shrunken centroid values derived from the CNVs of a pool of genomic loci in the test sample from the subject with the shrunken centroid values derived from the CNVs of a reference sample of BRCA-like mutated cancer cells and of a control sample of BRCA non-mutated cancer cells. In general, the loci comprised in the pool of genomic loci as regards the BRCA1-like classifiers are those listed in Table 1 and the loci comprised in the pool of genomic loci as regards the BRCA2-like classifiers are those listed in Table 2. The CNV profile of the reference and the control sample can be a profile that has been established before performing the method of the present invention or it can in principle also be established in the course of the execution of the method of the present invention. Thus, optionally, the method of the present invention further comprises the determination of the posterior probability score by comparison of the shrunken centroid values derived from the CNVs of the pool of genomic loci in the DNA of the cancer cells in the test sample from the subject with the shrunken centroid values derived from the CNVs of a reference set of samples of BRCA-like mutated cancer cells and of control samples of BRCA non-mutated cancer cells.

As mentioned above and as regards the BRCA1-like classifiers, the loci in the pool of genomic loci can vary as long as the method still shows a sensitivity of at least 90%, i.e. a sensitivity between 90% and 100%, preferably of at least 94 % to 97%, i.e. a sensitivity between 94 to 97% and 100%. In one embodiment, the pool of genomic loci comprises at least 200, preferably 400, more preferably 600, more preferably 800, more preferably 1000, more preferably 1200, more preferably 1400, more preferably 1600, more preferably 1800, more preferably 2000, more preferably 2200, more preferably 2400, and most preferably all of those loci listed in Table 1 as long as the above-mentioned sensitivities are reached. Of course any numbers of loci in between are also encompassed and just not mentioned for simplicity. Furthermore, as regards the BRCA2-like classifiers, the loci in the pool of genomic loci can vary as long as the method still shows a sensitivity of at least 70%, i.e. a sensitivity between 70% and 100%, preferably of at least 72% to 77%, i.e. a sensitivity between 72 to 77% and 100%. In one embodiment, the pool of genomic loci comprises at least 20, preferably 40, more preferably 60, more preferably 80, more preferably 100, more preferably 120, and most preferably all of those loci listed in Table 2 as long as the above-mentioned sensitivities are reached. Of course any numbers of loci in between are also encompassed and just not mentioned for simplicity.

A posterior probability score of $\geq 0.5$ identifies the subject as HRD and thus, it identifies the subject as being a likely responder to an anti-cancer therapy. A posterior probability score of $< 0.5$ identifies the subject as not being HRD and thus, it identifies the subject a not being a likely responder to an anti-cancer therapy. In other words, similarity between the CNVs of the test sample and the CNVs of the reference sample identifies the subject as HR deficient and thus, identifies the subject as being a likely responder to an anti-cancer therapy, wherein similarity between the CNVs of the test sample and the CNVs of the control sample identifies the subject as not being HR deficient and thus, it identifies the subject a not being a likely responder to an anti-cancer therapy.

The present invention is thus also directed to a method of predicting the response of a subject having cancer, in particular ovarian cancer, to an anti-cancer therapy, i.e. to a cancer treatment regimen, wherein the method comprises the steps as defined above. In particular, a posterior probability score of $\geq 0.5$ indicates an increased likelihood that the subject will respond to the anti-cancer therapy, and/or a posterior probability score of $< 0.5$ indicates an increased likelihood that the subject will not respond to the anti-cancer therapy. In other words, similarity between the CNVs of the test sample and the CNVs of the reference sample indicates an increased likelihood that the subject will respond to the anti-cancer therapy, wherein similarity between the CNVs of the test sample and the CNVs of the control sample indicates an increased likelihood that the subject will not respond to the anti-cancer therapy.

Thus, patients having cancer cells (or samples derived therefrom), and in particular ovarian cancer cells, identified as having an HRD signature status can be classified, based at least in part on such HRD signature, as being likely to respond to a particular cancer treatment regimen. For example, patients having cancer cells with an HRD signature can be classified as being likely to respond to a cancer treatment regimen that includes the use of drugs that target homologous recombination deficiency, that directly or indirectly cause double strand DNA breaks, and/or where the drug has been identified in mechanistic studies to target homologous recombination deficiency like DNA damaging agents, synthetic lethality agents (e.g., a PARP inhibitor), radiation, or a combination thereof. Examples of DNA damaging agents include, without limitation, platinum-based chemotherapy drugs (e.g., cisplatin, carboplatin, oxaliplatin, and picoplatin), anthracyclines (e.g., epirubicin and doxorubicin), topoisomerase I inhibitors (e.g. campothecin, topotecan, and irinotecan), DNA crosslinkers such as mitomycin C, and triazene compounds (e.g., dacarbazine and temozolomide). Preferably, the cancer treatment regime includes PARP inhibitors or DNA double strand break inducing agents, e.g. platinum compounds and interstrand crosslinking agents.

Synthetic lethality therapeutic approaches typically involve administering an agent that inhibits at least one critical component of a biological pathway that is especially important to a particular tumor cell's survival. For example, when a tumor cell has a deficient homologous repair pathway (e.g., as determined according to the present invention), inhibitors of poly ADP ribose polymerase (or platinum drugs, double strand break repair inhibitors, etc.) can be especially potent against such tumors because two pathways critical to survival become obstructed (one biologically, e.g., by BRCA1/2-like mutation, and the other synthetically, e.g., by administration of a pathway drug). Synthetic lethality approaches to cancer therapy are described in, e.g., O'Brien et al., Converting cancer mutations into therapeutic opportunities, EMBO MOL. MED. (2009) 1:297-299. Examples of synthetic lethality agents include, without limitation, PARP inhibitors or double strand break repair inhibitors in homologous repairdeficient tumor cells, PARP inhibitors in PTEN-deficient tumor cells, methotrexate in MSH2-deficient tumor cells, etc. Examples of PARP inhibitors include, without limitation, olaparib, iniparib, veliparib and niraparib. Examples of double strand break repair inhibitors include, without limitation, KU55933 (ATM inhibitor) and NU7441 (DNA-PKcs inhibitor). Examples of information that can be used in addition to the presence of an HRD signature to base a classification of being likely to respond to a particular cancer treatment regimen include, without limitation, previous treatment results, germline or somatic DNA mutations, gene or protein expression profiling (*e.g.* BRCA1 and/or BRCA2 status), tumor histology (*e.g.,* epithelial ovarian tumors like serous, mucinous, endometrioid, clear cell, transitional cell tumors (Brenner tumors), carcinosarcoma, mixed epithelial tumor, undifferentiated carcinoma, etc.), disease stage, tumor or cancer grade, number of previous courses of treatment, etc. Preferably, the information that can be used in addition to the presence of an HRD signature to base a classification of being likely to respond to a particular cancer treatment regimen is the tumor histology, and in particular of serous tumors.

Treatment regimens can also comprise the addition of olaparib to bevacizumab maintenance therapy after carboplatin and paclitaxel treatment. In particular, a treatment regime comprises hyperthermic intraperitoneal chemotherapy with cisplatin (100 mg per square meter) at interval debulking after standard neoadjuvant carbotaxol, *i.e.* paclitaxel and carboplatin, in stage III epithelial ovarian cancer.

Once classified as being likely to respond to a particular cancer treatment regimen as defined above, a treatment regime can be recommended to the patient, and/or the cancer patient can be treated with such a cancer treatment regimen. The invention thus relates to a method of the present invention which further comprises recommending a subject which has been identified as being HRD and/or having an increased likelihood of responding to the anti-cancer therapy, a treatment regimen comprising the above-mentioned drugs, *i.a.* homologous recombination deficiency-targeting drugs, drugs that directly cause double strand DNA breaks, drugs that indirectly cause double strand DNA breaks; and/or drugs which have been identified in mechanistic studies to target homologous recombination deficiency. On the other hand, the method may also comprise recommending a subject which has been identified as not being HRD and/or not having an increased likelihood of responding to the anti-cancer therapy, a standard treatment in the specific clinical setting of the patient, which might not include the above-mentioned drugs, *i.a.* homologous recombination deficiency-targeting drugs, drugs that directly cause double strand DNA breaks, drugs that indirectly cause double strand DNA breaks, and/or drugs which have been identified in mechanistic studies to target homologous recombination deficiency. In turn, such a subject can be classified as likely to respond to a cancer treatment regimen that includes the use of one or more cancer treatment agents not associated with HRD, such as (but not limited to) a taxane agent (e.g., paclitaxel, docetaxel, abraxane), and/or a growth factor receptor inhibitor (e.g., bevacizumab). However, in case no other treatment approach is available, the standard treatment may still comprise homologous recombination deficiency-targeting drugs, drugs that directly cause double strand DNA breaks, drugs that indirectly cause double strand DNA breaks, and/or drugs which have been identified in mechanistic studies to target homologous recombination deficiency.

Accordingly, the present invention further relates to a treatment regime comprising homologous recombination deficiency-targeting drugs, drugs that directly cause double strand DNA breaks, and/or drugs that indirectly cause double strand DNA breaks, or that otherwise have been identified as specifically targeting homologous recombination deficient tumors like a DNA damaging agent, a synthetically lethal agent (e.g., a PARP inhibitor), or a combination thereof for use in treating ovarian cancer in a subject, wherein said subject has been identified as having an HRD signature and/or identified to have an increased likelihood of responding to the anti-cancer treatment regimen as described before.

Furthermore, the present invention relates to homologous recombination deficiency-targeting drugs, drugs that directly cause double strand DNA breaks, and/or drugs that indirectly cause double strand DNA breaks, or that otherwise have been identified as specifically targeting homologous recombination deficient tumors, i.e., PARP inhibitors or DNA double strand break inducing agents, *e.g.* platinum compounds and interstrand crosslinking agents or any combination thereof for use in a method of treating a patient comprising detecting an HRD signature as described herein, wherein the method comprises administering (or recommending or prescribing) a treatment regimen comprising the above-mentioned drugs. Any appropriate method for treating the cancer at issue can be used to treat a cancer patient identified as having cancer cells having an HRD signature. For example, platinum-based chemotherapy drugs or a combination of platinum-based chemotherapy drugs can be used to treat cancer as described elsewhere (see, e.g., U.S. Patent Nos. 3,892,790, 3,904,663, 7,759,510, 7,759,488 and 7,754,684). In some cases, anthracyclines or a combination of anthracyclines can be used to treat cancer as described elsewhere (see, e.g., U.S. Patent Nos. 3,590,028, 4,138,480, 4,950,738, 6,087,340, 7,868,040, and 7,485,707). In some cases, topoisomerase I inhibitors or a combination of topoisomerase I inhibitors can be used to treat cancer as described elsewhere (see, e.g., U.S. Patent Nos. 5,633,016 and 6,403,563). In some cases, PARP inhibitors or a combination of PARP inhibitors can be used to treat cancer as described elsewhere (see, e.g., U.S. Patent Nos. 5,177,075, 7,915,280, and 7,351,701). In some cases, radiation can be used to treat cancer as described elsewhere (see, e.g., U.S. Patent No. 5,295,944). In some cases, a combination comprising different agents (e.g., a combination comprising any of platinum-based chemotherapy drugs, anthracyclines, topoisomerase I inhibitors, and/or PARP inhibitors) with or without radiation treatments can be used to treat cancer. In some cases, a combination treatment may comprise any of the above agents or treatments (e.g., PARP inhibitors or DNA double strand break inducing agents, *e.g.* platinum compounds and interstrand crosslinking agents) together with another agent or treatment, e.g., a taxane agent (e.g., doxetaxel, paclitaxel, abraxane, preferably paclitaxel), and/or a growth factor receptor inhibitor (e.g., bevacizumab).

Furthermore, patients identified as having cancer cells lacking an HRD signature can be classified, based at least in part on a sample lacking an HRD signature, as being less likely to respond to a treatment regimen that includes a homologous recombination deficiency-targeting drug, drug that directly causes double strand DNA breaks, drug that indirectly causes double strand DNA breaks, and/or drugs which have been identified in mechanistic studies to target homologous recombination deficiency, like PARP inhibitors, DNA double strand break inducing agents, *e.g.* platinum compounds and interstrand crosslinking agents or a combination thereof. In turn, such a patient can be classified as likely to respond to a cancer treatment regimen that includes the use of one or more cancer treatment agents not associated with HRD, such as a taxane agent (e.g., doxetaxel, paclitaxel, abraxane), and/or a growth factor receptor inhibitor (e.g., bevacizumab). However, in case no other treatment approach is available, the standard treatment may still comprise homologous recombination deficiency-targeting drugs, drugs that directly cause double strand DNA breaks, drugs that indirectly cause double strand DNA breaks, and/or drugs which have been identified in mechanistic studies to target homologous recombination deficiency.

Once classified as being likely to respond to a particular cancer treatment regimen (e.g., a cancer treatment regimen that includes the use of a cancer treatment agent not associated with HRD), the cancer patient can be treated with such a cancer treatment regimen. The invention thus provides a treatment regimen not comprising the use of a homologous recombination deficiency-targeting drugs, drugs that directly cause double strand DNA breaks, drugs that indirectly cause double strand DNA breaks, and/or drugs which have been identified in mechanistic studies to target homologous recombination deficiency, like PARP inhibitors, DNA double strand break inducing agents, *e.g.* platinum compounds and interstrand crosslinking agents, or a combination thereof for use in a method of treating a patient comprising detecting the absence of an HRD signature as described herein and administering (or recommending or prescribing) said treatment regimen . In some embodiments the treatment regimen comprises one or more of a taxane agent (e.g., doxetaxel, paclitaxel, abraxane, preferably paclitaxel), and/or a growth factor receptor inhibitor (e.g., bevacizumab). Any appropriate method for the cancer being treated can be used to treat a cancer patient identified as having cancer cells lacking an HRD signature. Examples of information that can be used in addition to the presence of an HRD signature to base a classification of being likely to respond to a particular cancer treatment regimen include, without limitation, previous treatment results, germline or somatic DNA mutations, gene or protein expression profiling (*e.g.* BRCA1 and/or BRCA2 status), tumor histology (*e.g.*, epithelial ovarian tumors like serous, mucinous, endometrioid, clear cell, transitional cell tumors (Brenner tumors), carcinosarcoma, mixed epithelial tumor, undifferentiated carcinoma, etc.), disease stage, tumor or cancer grade, number of previous courses of treatment, etc. Preferably, the information that can be used in addition to the presence of an HRD signature to base a classification of being likely to respond to a particular cancer treatment regimen is the tumor histology, and in particular of serous tumors.

Once treated for a particular period of time (e.g., between one to six months), the patient can be assessed to determine whether or not the treatment regimen has an effect. If a beneficial effect is detected, the patient can continue with the same or a similar cancer treatment regimen. If a minimal or no beneficial effect is detected, then adjustments to the cancer treatment regimen can be made. For example, the dose, frequency of administration, or duration of treatment can be increased. In some cases, additional anti-cancer agents can be added to the treatment regimen or a particular anti-cancer agent can be replaced with one or more different anti-cancer agents. The patient being treated can continue to be monitored as appropriate, and changes can be made to the cancer treatment regimen as appropriate.

In addition to predicting likely treatment response or selecting desirable treatment regimens, an HRD signature can be used to determine a patient's prognosis. Thus, in one aspect, a method is provided for determining a patient's prognosis based at least in part of detecting the presence or absence of an HRD signature in a sample from the patient. The method comprises, or consists essentially of, (a) determining whether a sample from the patient comprises cancer cells (or whether a sample comprises DNA derived from such cells) having an HRD signature as described herein, and (b)(1) determining, based at least in part on the presence of the HRD signature, that the patient has a relatively good prognosis, or (b)(2) determining, based at least in part on the absence of the HRD signature, that the patient has a relatively poor prognosis. Prognosis may include the patient's likelihood of survival (e.g., progression-free survival, overall survival), wherein a relatively good prognosis would include an increased likelihood of survival as compared to some reference population (e.g., average patient with this patient's cancer type/subtype, average patient not having an HRD signature, etc.). Conversely, a relatively poor prognosis in terms of survival would include a decreased likelihood of survival as compared to some reference population (e.g., average patient with this patient's cancer type/subtype, average patient having an HRD signature, etc.).

As described herein, the present invention relates to methods for assessing patients for ovarian cancer cells having an HRD signature. In some embodiments, one or more clinicians or medical professionals can determine whether a sample from the patient comprises cancer cells (or whether a sample comprises DNA derived from such cells) having an HRD signature. In some cases, one or more clinicians or medical professionals can determine if a patient contains cancer cells having an HRD signature by obtaining a cancer cell sample from the patient and assessing the DNA of cancer cells of the cancer cell sample to determine the presence or absence of an HRD signature as described herein.

In some cases, one or more clinicians or medical professionals can obtain a cancer cell sample from a patient and provide that sample to a testing laboratory having the ability to assess DNA of cancer cells of the cancer cell sample to provide an indication about the presence or absence of an HRD signature as described herein. In such cases, the one or more clinicians or medical professionals can determine if a sample from the patient comprises cancer cells (or whether a sample comprises DNA derived from such cells) having an HRD signature by receiving information about the presence or absence of an HRD signature as described herein directly or indirectly from the testing laboratory. For example, a testing laboratory, after assessing DNA of cancer cells for presence or absence of an HRD signature as described herein, can provide a clinician or medical professional with, or access to, a written, electronic, or oral report or medical record that provides an indication about the presence or absence of an HRD signature for a particular patient (or patient sample) being assessed. Such a written, electronic, or oral report or medical record can allow the one or more clinicians or medical professionals to determine if a particular patient being assessed contains cancer cells having an HRD signature.

Once a clinician or medical professional or group of clinicians or medical professionals determines that a particular patient being assessed contains cancer cells having an HRD signature, the clinician or medical professional (or group) can classify that patient as having cancer cells whose genome contains the presence of an HRD signature. In some cases, a clinician or medical professional or group of clinicians or medical professionals can diagnose a patient determined to have cancer cells whose genome contains the presence of an HRD signature as having cancer cells deficient in (or likely to be deficient in) HRD. Such a diagnosis can be based solely on a determination that a sample from the patient comprises cancer cells (or whether a sample comprises DNA derived from such cells) having an HRD signature or can be based at least in part on a determination that a sample from the patient comprises cancer cells (or whether a sample comprises DNA derived from such cells) having an HRD signature. For example, a patient determined to have cancer cells having an HRD signature can be diagnosed as likely to be deficient in HRD based on the combination of the presence of an HRD signature and deficient status in one or more tumor suppressor genes (e.g., BRCA1/2, RAD51C), a family history of cancer, or the presence of behavioral risk factors (e.g., smoking). Thus, patients can be detected that have germline mutations in HRD associated genes.

In some cases, a clinician or medical professional or group of clinicians or medical professionals can diagnose a patient determined to have cancer cells whose genome contains the presence of an HRD signature as having cancer cells which can contain genetic mutations in one or more genes in the HRD pathway. Such a diagnosis can be based solely on a determination that a particular patient being assessed contains cancer cells having a genome containing an HRD signature or can be based at least in part on a determination that a particular patient being assessed contains cancer cells having a genome containing an HRD signature. For example, a patient determined to have cancer cells whose genome contains the presence of an HRD signature can be diagnosed as having cancer cells likely to contain genetic mutations in one or more genes in the HRD pathway based on the combination of the presence of an HRD signature and a family history of cancer, or the presence of behavioral risk factors (e.g., smoking).

In some cases, a clinician or medical professional or group of clinicians or medical professionals can diagnose a patient determined to have cancer cells having an HRD signature as having cancer cells likely to respond to a particular cancer treatment regimen. Such a diagnosis can be based solely on a determination that a sample from the patient comprises cancer cells (or whether a sample comprises DNA derived from such cells) having an HRD signature or can be based at least in part on a determination that a sample from the patient comprises cancer cells (or whether a sample comprises DNA derived from such cells) having an HRD signature. For example, a patient determined to have cancer cells having an HRD signature can be diagnosed as being likely to respond to a particular cancer treatment regimen based on the combination of the presence of an HRD signature and deficient status in one or more tumor suppressor genes (e.g., BRCA1/2, RAD51), a family history of cancer, or the presence of behavioral risk factors (e.g., smoking). As described herein, a patient determined to have cancer cells having an HRD signature can be diagnosed as likely to respond to a cancer treatment regimen that includes the use of homologous recombination deficiency-targeting drugs, drugs that directly cause double strand DNA breaks, drugs that indirectly cause double strand DNA breaks; and/or drugs which have been identified in mechanistic studies to target homologous recombination deficiency, for example interstrand crosslinking agents, platinum compounds, PARP inhibitors, anthracyclines, and topoisomerase I and II inhibitors, preferably PARP inhibitors or DNA double strand break inducing agents, e.g. platinum compounds such as cisplatin, carboplatin, oxaliplatin, or picoplatin, and interstrand crosslinking agents, a combination thereof, or a combination of any of the preceding with another anti-cancer agent.

Once a clinician or medical professional or group of clinicians or medical professionals determines that a sample from the patient comprises cancer cells (or whether a sample comprises DNA derived from such cells) having a genome lacking an HRD signature, the clinician or medical professional (or group) can classify that patient as having cancer cells whose genome lacks an HRD signature. In some cases, a clinician or medical professional or group of clinicians or medical professionals can diagnose a patient determined to have cancer cells containing a genome lacking an HRD signature as having cancer cells likely to have functional HRD. In some cases, a clinician or medical professional or group of clinicians or medical professionals can diagnose a patient determined to have cancer cells containing a genome lacking an HRD signature as having cancer cells that do not likely contain genetic mutations in one or more genes in the HRD pathway. In some cases, a clinician or medical professional or group of clinicians or medical professionals can diagnose a patient determined to have cancer cells containing a genome lacking an HRD signature and that are less likely to respond to a platinum-based chemotherapy drug such as cisplatin, carboplatin, oxalaplatin, or picoplatin, an anthracycline such as epirubicin or doxorubicin, a topoisomerase I inhibitor such as campothecin, topotecan, or irinotecan, a PARP inhibitor, or radiation and/or more likely to respond to a cancer treatment regimen that includes the use of a cancer treatment agent not associated with HRD such as one or more taxane agents, growth factor or growth factor receptor inhibitors, anti-metabolite agents, etc.

The subjects/patients who are tested, diagnosed, treated, etc. are either treatment naive subjects or subjects that have already undergone treatment. For example, the subjects display primary or platinum-sensitive relapsed ovarian cancer defined as relapse after a platinum-free interval of at least 6 months.

The present invention also provides methods for performing a diagnostic analysis of a nucleic acid sample (e.g., a genomic nucleic acid sample or nucleic acids amplified therefrom) of a cancer patient to determine if a sample from the patient comprises cancer cells (or whether a sample comprises DNA derived from such cells) containing an HRD signature. In some cases, one or more laboratory technicians or laboratory professionals can detect the presence or absence of an HRD signature in the genome of cancer cells of the patient by (a) receiving a cancer cell sample obtained from the patient, receiving a genomic nucleic acid sample obtained from cancer cells obtained from the patient, or receiving a sample containing nucleic acids enriched and/or amplified from such a genomic nucleic acid sample obtained from cancer cells obtained from the patient and (b) performing an analysis (e.g., microarray-based assay or a sequencing-based assay, preferably LC-WGS) using the received material to detect the presence or absence of an HRD signature as described herein. In some cases, one or more laboratory technicians or laboratory professionals can receive a sample to be analyzed (e.g., a cancer cell sample obtained from the patient, a genomic nucleic acid sample obtained from cancer cells obtained from the patient, or a sample containing nucleic acids enriched and/or amplified from such a genomic nucleic acid sample obtained from cancer cells obtained from the patient) directly or indirectly from a clinician or medical professional.

Once a laboratory technician or laboratory professional or group of laboratory technicians or laboratory professionals detects the presence of an HRD signature as described herein, the laboratory technician or laboratory professional (or group) can associate that HRD signature or the result (or results or a summary of results) of the performed diagnostic analysis with the corresponding patient's name, medical record, symbolic/numerical identifier, or a combination thereof. Such identification can be based solely on detecting the presence of an HRD signature or can be based at least in part on detecting the presence of an HRD signature. For example, a laboratory technician or laboratory professional can identify a patient having cancer cells that were detected to have an HRD signature as having cancer cells potentially deficient in HRD (or as having an increased likelihood of responding to a particular treatment as described herein) based on a combination of the presence of an HRD signature and the results of other genetic and biochemical tests performed at the testing laboratory.

The converse of the preceding is also true. Namely, once a laboratory technician or laboratory professional or group of laboratory technicians or laboratory professionals detects the absence of an HRD signature, the laboratory technician or laboratory professional (or group) can associate the absence of an HRD signature or the result (or results or a summary of results) of the performed diagnostic analysis with the corresponding patient's name, medical record, symbolic/numerical identifier, or a combination thereof. In some cases, a laboratory technician or laboratory professional or group of laboratory technicians or laboratory professionals can identify a patient having cancer cells that were detected to lack an HRD signature as having cancer cells with potentially intact HRD (or having a decreased likelihood of responding to a particular treatment as described herein) either based solely on the absence of an HRD signature or based on a combination of the presence of an HRD signature and the results of other genetic and biochemical tests performed at the testing laboratory.

The results of any analyses according to the invention will often be communicated to physicians, genetic counselors, or a clinic and/or patients (or other interested parties such as researchers) in a transmittable form that can be communicated or transmitted to any of the above parties. Such a form can vary and can be tangible or intangible. The results can be embodied in descriptive statements, diagrams, photographs, charts, images or any other visual forms. For example, graphs or diagrams showing genotype or HRD status information can be used in explaining the results. The statements and visual forms can be recorded on a tangible medium such as papers, computer readable media such as floppy disks, compact disks, flash memory, etc., or in an intangible medium, e.g., an electronic medium in the form of email or website on internet or intranet. In addition, results can also be recorded in a sound form and transmitted through any suitable medium, e.g., analog or digital cable lines, fiber optic cables, etc., via telephone, facsimile, wireless mobile phone, internet phone and the like.

Thus, the information and data on a test result can be produced anywhere in the world and transmitted to a different location. As an illustrative example, when an assay is conducted outside Germany, the Netherlands, or the EU, the information and data on a test result may be generated, cast in a transmittable form as described above, and then imported into Germany, the Netherlands or the EU. Accordingly, the present invention also encompasses a method for producing a transmittable form of information on an HRD signature for at least one patient sample. The method comprises the steps of (1) determining an HRD signature according to methods of the present invention; and (2) embodying the result of the determining step in a transmittable form. The transmittable form is a product of such a method.

Several embodiments of the invention described herein involve a step of correlating the presence of an HRD signature according to the present invention (e.g., the CNVs in comparison to reference and control samples) to a particular clinical feature (e.g., an increased likelihood of HRD deficiency; an increased likelihood of response to a treatment regimen comprising a DNA damaging agent, an anthracycline, a topoisomerase I inhibitor, radiation, and/or a PARP inhibitor; etc.) and optionally correlating the absence of a HRD signature to one or more other clinical features. Throughout this document, wherever such an embodiment is described, another embodiment of the invention may involve, in addition to or instead of a correlating step, one or both of the following steps: (a) concluding that the patient has the clinical feature based at least in part on the presence or absence of the HRD signature; or (b) communicating that the patient has the clinical feature based at least in part on the presence or absence of the HRD signature.

As used herein, "communicating" a particular piece of information means to make such information known to another person or transfer such information to a thing (e.g., a computer). In some methods of the invention, a patient's prognosis or likelihood of response to a particular treatment is communicated. In some embodiments, the information used to arrive at such a prognosis or response prediction (e.g., HRD signature according to the present invention, etc.) is communicated. This communication may be auditory (e.g., verbal), visual (e.g., written), electronic (e.g., data transferred from one computer system to another), etc. In some embodiments, communicating a cancer classification (e.g., prognosis, likelihood of response, appropriate treatment, etc.) comprises generating a report that communicates the cancer classification. In some embodiments the report is a paper report, an auditory report, or an electronic record. In some embodiments the report is displayed and/or stored on a computing device (e.g., handheld device, desktop computer, smart device, website, etc.). In some embodiments the cancer classification is communicated to a physician (e.g., a report communicating the classification is provided to the physician). In some embodiments the cancer classification is communicated to a patient (e.g., a report communicating the classification is provided to the patient). Communicating a cancer classification can also be accomplished by transferring information (e.g., data) embodying the classification to a server computer and allowing an intermediary or end-user to access such information (e.g., by viewing the information as displayed from the server, by downloading the information in the form of one or more files transferred from the server to the intermediary or end-user's device, etc.).

A system for detecting HRD in a sample comprises, or consists essentially of, (a) a sample analyzer configured to produce a plurality of signals about genomic DNA in the test sample, and (b) a computer sub-system programmed to calculate, based on the plurality of signals, the CNVs in the pool of genomic loci. The computer sub-system can be programmed to compare the CNVs of the test sample with the CNVs in a corresponding pool of genomic loci in the DNA of a reference sample of BRCA-like mutated cancer cells and a control sample of BRCA non-mutated cancer cells, respectively, and/or to provide a shrunken centroid value which is derived from the CNVs of the genomic locus, by comparison of the shrunken centroid values derived from the CNVs of the pool of genomic loci in the DNA of the cancer cells in the test sample from the subject with the shrunken centroid values derived from the CNVs of a reference set of samples of BRCA-like mutated cancer cells and of control samples of BRCA non-mutated cancer cells, to detect (a) HRD or likelihood of HRD (e.g. , an HRD signature) in the sample, or (b) an increased likelihood that the cancer patient will respond to a cancer treatment regimen as defined above. The system can comprise an output module configured to display (a) or (b). The system can comprise an output module configured to display a recommendation for the use of the cancer treatment regimen.

A corresponding computer sub-system, *i.e.* computer program product embodied in a computer readable medium that, when executing on a computer, can be used to perform the calculations as described above.

Several documents are cited throughout the text of this specification.

A more complete understanding can be obtained by reference to the following specific Examples.

EXAMPLES

**Methods**

*OC-BRCA1/2-like classifier training*

*Training cohort*

Fifty confirmed g*BRCA1*-mutated (m), 10 confirmed *gBRCA2m* cases and 13 patients without a family history of ovarian or breast cancer (controls) were identified through the Netherlands Cancer Institute (NKI) tumor registration database and the Erasmus Medical Center (see Table 3). This study was conducted in concordance with Dutch law and national guidelines that allow for the analysis of residual tissue specimens obtained for diagnostic purposes and anonymized publication of the results (38).

**Table 3:** Pathological characteristics of samples within the training set. Histological subtyping and two-tier grading of the ovarian cancer samples in the training cohort.

|  |  | Control | *gBRCA1* | *gBRCA2* |
|---|---|---|---|---|
| Subtype | Serous | 13 | 48 | 10 |
|  | Endometrial | 0 | 1 | 0 |
|  | Other | 0 | 1 | 0 |
| Grade | Low grade | 1 | 1 | 0 |
|  | High grade | 12 | 48 | 10 |
|  | Missing | 0 | 1 | 0 |

*DNA isolation and BRCA1 methylation assay*

Histological classification and grading, according to the 2-tiered system, were performed by two expert gynaecopathologists. We isolated DNA from formalin-fixed paraffin-embedded (FFPE) tumor slides, using (micro)-dissection to obtain 60% tumor percentage with the Qiagen DNA mini kit (Qiagen, cat nr. 51306) and for *BRCA1* methylation, using an MLPA kit (MRC-Holland) as described before (30). In particular, hypermethylation of the *BRCA1* promoter was assessed using a custom methylation specific MLPA set according to the manufacturer's protocol (ME005-custom; MRC-Holland, Amsterdam, the Netherlands). Probe sequences of the MLPA set are available on request (info@mlpa.com). DNA fragments were analyzed on a 3730 DNA Analyzer (Applied Biosystems, Foster City, CA, USA). For normalization and analysis, the Coffalyzer program was used (MRC-Holland, Amsterdam, The Netherlands); peak heights below 250 were excluded from further analyses. When the *BRCA1* probes showed methylation (threshold of 0.2; MRC-Holland), we classified the result as *BRCA1* promoter methylation.

*Quantitative methylation assay*

A PCR-based methylation assay was performed centrally (Institute of Pathology, University Hospital Bonn, Germany), as previously described (37). Quantitative methylation-specific PCR (qMSP) assays were designed to allow specific amplification of the bisulfite-converted (convertion using the innuCONVERT Bisulfite Basic Kit; Analytik Jena, Jena, Germany) methylated gene promoter sequences of *BRCA1, PALB2* and *RAD51C.* Triplicate measurements were carried out for each sample, and median methylation levels were computed with values ≥5% considered positive.

*NimbleGen 135K Oligonucleotide Array Comparative Genomic Hybridization (aCGH)*

We obtained NimbleGen 135K aCGH profiles from the training set samples. The 135K array data were mapped to the BAC aCGH platform for dimension and noise reduction by averaging probes covered by the BAC clone, as described before (39,40). The data discussed here have been deposited in NCBI's Gene Expression Omnibus and are accessible through GEO Series accession number GSE111688 (https://www.ncbi.nlm.nih.gov/geo/query/acc.cgi?acc=GSE111688).

*Estimating the performance of the classifier in a 10-fold double loop cross validation loop*

We carried out nested double loop 10-fold cross validation of shrunken centroid classifiers to classify OC as being similar to g*BRCA1*m (*BRCA1*-like) or g*BRCA2*m (*BRCA2*-like) or controls (C) (29). We obtained the delta threshold by optimizing the classification error and selecting the sparsest model within one standard error of the optimal solution (41). Subsequently, we used the model at the selected threshold to predict the samples in the outer loop. The area under the curve (AUC) of the receiver/operator curve (ROC) of our predictions of the class labels is computed using samples that are left out of the training procedure. After estimating this unbiased performance, we trained the full dataset's final model using the inner loop. See Supplementary Methods for pseudocode.

*Validation in TCGA data set*

We downloaded the Infinium HumanMethylation27 BeadChip methylation, segmented genome-wide human SNP6.0 copy number, and gene expression data from the firebrowse.org archives of the ovarian cancer TCGA data (version 2015082100.0.0). *BRCA1* and *BRCA2* mutation status were obtained from cBioPortal, which stores the somatic and germline mutation status used in the original TCGA manuscripts (42). The *BRCA1* promoter methylation status was obtained by correlating the methylation and gene expression data. We used methylation probe cg10893007 because of the strongest Pearson correlation with log *BRCA1* gene expression ratio among the *BRCA1* promoter probes and close location to the MLPA probe used in the previous study (30) among those covering the *BRCA1* promoter in the TCGA dataset.

*Classifying TCGA data*

TCGA data were mapped to the NKI BAC array CGH positions, as described before (39). In short, SNP6.0 probes within the start and end positions of the BAC clone were averaged (mapped TCGA). We adjusted for differences in scaling and centering by using a method similar to quantile normalization. Briefly, we performed linear regression by fitting a generalized linear Gaussian model with identity link function to the sorted location-wise average DNA copy number values of the NimbleGen data and the sorted location-wise DNA copy number averages of the mapped TGCA dataset. Subsequently, we used the obtained alpha coefficient to correct the centering and the obtained beta coefficient to correct the scaling of the mapped TCGA data, followed by classification. We validated this method on samples that had been analyzed before (39) both on NimbleGen 135K aCGH and SNP6.0. Subsequently, samples were classified as *BRCA*-like if the predicted probability was > 0.5 and non-*BRCA*-like if the predicted probability was <= 0.5, as was pre-defined in the training set.

*Performance in TCGA data*

The sensitivity and specificity of detecting the class labels g*BRCA1/BRCA2* mutation, s*BRCA1/BRCA2* mutation, and *BRCA1* promoter hypermethylation were calculated. The Youden index, (sensitivity+specificity)-1, was used as a balanced measure to assess performance. This readout resembles the equal weighting of sensitivity and specificity in the training process.

*Independent validation within AGO-TR1 cohort study*

Within the consecutive cohort study AGO-TR1 (NCT02222883) 523 OC patients were counseled and enrolled in 20 AGO study group centers in Germany. The ethical committee approved the study protocol of the Landesaerztekammer Nordrhein (Nr. 2014340). Written informed consent was obtained before any study-related procedure. All individuals were 18 years or older and displayed a primary (PR: n=281) or platinum-sensitive relapsed (RE: n=242) OC, defined as relapse after a platinum-free interval of at least 6 months. The AGO study group documented clinical data including demographics, medical and family history as well as disease characteristics.

The Center of Hereditary Breast and Ovarian Cancer in Cologne performed the genetic analyses on blood samples of all participants and FFPE-tumor samples of 496 patients as previously described (36,37). In short, a paired multi-gene panel sequencing of germline and tumor samples analyzing 27 OC related and DNA repair genes was performed with a complementary use of CNV-analysis for the detection of large genomic rearrangements in germline samples. In particular, paired mutation sequencing of germline and tumor samples was performed at the Center of Hereditary Breast and Ovarian Cancer, Cologne, using a customer-tailored SureSelect gene panel (TruRisk® Panel, Agilent, Santa Clara, U.S.) covering the entire coding and exonflanking regions (+/- 15nt) of the below specified 27 genes. A hybridization capture-based NGS protocol, suitable for DNA derived from blood as well as FFPE-tissue, was applied (Agilent SureSelect XT; optimized for 200ng genomic DNA input). Sequencing was carried out on Mi-Seq and Hi-Seq 4000 devices (Illumina, San Diego, U.S.). For bioinformatical analyses we applied the VARBANK version 2.10 pipeline of the Cologne Center for Genomics and the SOPHiA DDM® platform (Sophia Genetics, Saint-Sulpice, Switzerland). For the germline samples CNV-detection via MLPA (multiple Ligation-dependent Probe Amplification, MRC Holland, Amsterdam, The Netherlands), CNV-tool of the SOPHIA DDM® platform, and arrayCGH (array comparative genomic hybridization) were applied, if appropriate. The analyses of all germline and 488 of 496 FFPE samples was successfully performed.

HR genes: *ATM,* NM_000051.3; *BARD1* NM_000465.3; *BRCA1* NM_007294.3; *BRCA2,* NM_000059.3; *BRIP1* NM_032043.2; *CHEK1,* NM_001330427.1; *CHEK2,* NM_007194.3; *FAM175A,* NM_139076.2; *FANCM,* NM_020937.3; *MRE11A,* NM_005591.3; *NBN,* NM_002485.4; *PALB2,* NM_024675.3; *RAD50,* NM_005732.3; *RAD51C,* NM_058216.2; *RAD51D,* NM_002878.3; *XRCC2,* NM_005431.1.

HNPCC genes: *MLH1,* NM_000249.3; *MSH2,* NM_000251.2; *MSH6,* NM_000179.2; *PMS2,* NM_000535.6
other OC associated genes: *BUB1B,* NM_001211.5; *CDH1,* NM_004360.4; *MAP3K1,* NM_005921.1; *PIK3CA,* NM_006218.3; *PTEN,* NM_000314.6; *STK11,* NM_000455.4; *TP53,* NM_000546.5

All variants were classified using a five-tier variant classification system as proposed by the International Agency for Research on Cancer Unclassified Genetic Variants Working Group, namely, deleterious=class 5, likely deleterious=class 4, variant of uncertain significance=class 3,likely benign=class 2 and benign=class 1. For somatic variants, the My Cancer Genome database (http://www.mycancergenome.org), the IARC TP53 database (https://p53.iarc.fr) and the ClinVar database (https://www.ncbi.nlm.nih.gov/clinvar/) were also considered for variant classification. Variants reported to occur in large outbred control reference groups at an allele frequency of >1% were generally considered benign. Class 4/5 variants were subsequently defined as 'deleterious variants'. Variants were considered somatic if they were not identified in a paired germline analysis of the corresponding blood sample. Also, quantitative methylation assays analyzing *BRCA1-, PALB2-,* and *RAD51C-* promoter regions were carried out as described before (37). In total, the complete data of 473 individuals were successfully generated (37) (see CONSORT-like flow diagram, Fig. 4).

*Quality control of AGO-TR1 samples*

The obtained copy number profiles were visually assessed supported by signal to noise ratios and noise variance as quality measures. The unsegmented $^2$log ratios were assumed to represent the noise, whereas the segmented profiles were assumed to represent the signal. Both values were calculated per profile in R using the base function var as follows in pseudocode:

$$Noise\_variance = var(segmented - unsegmented\ profile)$$

$$Signal\_to\_noise\_ratio = var(segmented\ profile)/noise\_variance$$

As observed earlier (39), cutoffs are experiment dependent, therefore these measures formed support for the visual assessment. In general, samples with a signal to noise ratio lower than 1 are suspicious for lower quality and with higher level of noise variance. However, determination of too low quality was done visually by identifying those samples for which visually present large aberrations were not called by the segmentation algorithm and thus resulted in an incorrect 'flat' copy number profile. Flat copy number profiles in general were not excluded as they might contain biology if not based on high noise variance. In a post-hoc analysis, flat profiles associated with non-HGS histology and non-HR-associated mutations. Samples with higher noise but also visually correctly segmented profiles were retained.

*Selection of the study sample*

We selected 300 samples with matching germline and somatic mutation status and successful performed methylation analyses for *BRCA*-like classification (see Table 4), including all available samples with deleterious germline and somatic variants (IARC class 4/5) in *BRCA1/2* (n=118), other HR-related and hereditary non-polyposis colorectal cancer (HNPCC) genes (n=43), and somatic (s) *BRCA1/2*-VUS (variants of unknown significance) (n=4). As controls, we randomly selected a similarly sized group (n=135) from the rest of the main cohort taking PR-/RE-status, age at diagnosis, and s*TP53* mutations into account. Samples with only *gBRCA1/2* VUS (n=15) were excluded.

**Table 4**: Occurrence of genetic and clinical features within the samples of the presented study cohort. A) lists all samples, whereas B) excludes all samples with an *BRCA* or other HR gene mutation, as those were enriched within the study cohort. Genetic and epigenetic features coincided within some samples and were listed in each subcategory concerned. The mean age at diagnosis was 57.6 years (range 18-83 years) in the main cohort and 59.4 years (range 21-83 years) excluding all *BRCA1/2-* or HR gene mutated cases. PR: primary ovarian cancer, RE: recurrent disease.

| | | A study cohort (n) | [%] | B study cohort (n) without BRCA/HR gene mutations | [%] |
|---|---|---|---|---|---|
| germline and somatic mutation status | gBRCA1 | 69 | 23.0 | | |
| | gBRCA2 | 23 | 7.7 | | |
| | gHR genes | 37 | 12.3 | | |
| | sBRCA1/2 | 28 | 9.3 | | |
| | sHR genes | 12 | 4.0 | | |
| | gHNPCC genes | 3 | 1.0 | 3 | 2.1 |
| | sTP53 | 251 | 83.7 | 106 | 75.2 |
| | sPTEN/PIK3CA | 17 | 5.7 | 10 | 7.1 |
| methylation status | BRCA1 methyl | 28 | 9.3 | 26 | 18.4 |
| | RAD51C methyl | 4 | 1.3 | 2 | 1.4 |
| histological subtype | high grade serous | 246 | 82.0 | 110 | 78.0 |
| | high grade endometrioid | 16 | 5.3 | 9 | 6.4 |
| | serous, grade unknown | 6 | 2.0 | 3 | 2.1 |
| | low grade serous | 7 | 2.3 | 4 | 2.8 |
| | low grade endometrioid | 4 | 1.3 | 1 | 0.7 |
| | clear cell | 2 | 0.7 | 2 | 1.4 |
| | mucinous | 4 | 1.3 | 3 | 2.1 |
| | other/missing | 15 | 5.0 | 9 | 6.4 |
| clinical features | PR | 160 | 53.3 | 83 | 58.9 |
| | RE | 140 | 46.7 | 58 | 41.1 |
| | total | 300 | | 141 | |

*DNA isolation*

Hematoxylin and eosin-stained 3 μm tissue sections were centrally investigated (Institute of Pathology, University Hospital Bonn, Germany). Tumor areas containing >80% tumor nuclei were chosen and dissected for DNA isolation. DNA isolation from FFPE tumor samples was conducted using standard procedures, as previously described (37).

*Low-coverage whole genome sequencing (lcWGS) and BRCA1/2-like classification*

LcWGS was centrally performed (NKI), as described earlier (39). Library preparation was performed with an input of 200ng double-stranded DNA derived from FFPE-tumor samples using the TruSeq®DNA LT Sample Preparation kit (Illumina, San Diego, U.S.). Ten explicit indexed samples were equimolarly pooled and sequenced in one lane on an Illumina HiSeq 2500 device (Illumina, San Diego, U.S). Single-read sequencing (read-length 65bp) was performed with an aimed coverage of 0.5X and sequences were aligned to reference genome GRCh38. Reads were counted in 20kb non-overlapping bins, corrected for CG bias and corrected for local alignment-bases estimated mappability, resulting in $^2$log count ratios. The 20kb resolution $^2$log ratios were mapped to the 1 MB resolution input for the classifier (mapped AGO-TR1). This was done by averaging the $^2$log count ratios within the 1 MB bins (surrounding the BAC clone locations of the BAC platform).

Since the classifier's training was performed on oligonucleotide array CGH data, we performed a correction of centering and scaling of the data with the next-generation sequencing (NGS) platform in this study. This correction is akin to quantile normalization and was performed by fitting a linear regression model with Gaussian distribution and identity link function using the R glm function to the sorted location-wise average of the training set and the mapped AGOTR1 dataset. Subsequently, we used the obtained alpha coefficient to correct the centering and the obtained beta coefficient to correct the scaling of the new data. We validated this method on samples that had been analyzed both on NimbleGen 135K array CGH and NGS in the cross-platform robustness dataset we previously described (39). Subsequently, samples were classified as *BRCA*-like if the predicted probability was > 0.5 and non-*BRCA*-like if the predicted probability was <= 0.5 as was pre-defined in the training set. The cross validation algorithm, pseudo code for platform correction, analysis of misclassified samples and error curves and the validation of platform correction is described in the following:

*Cross validation algorithm:*

1) Split the data in 10 folds using the pamr:::balanced.folds function from the pamr package.
2) Take 9/10 of the data (outer loop start).

    a. Train a classifier on 8/9 of this selection of the data (inner loop start).
    b. Estimate optimal shrinkage penalty from 100 shrinkage penalties on left-out 1/9 of this selection. Shrinkage penalties range from exp(-3) to exp(1.5); with step size between - 3 and 1.5 at 0.045)
    c. Calculate error between class (0 or 1) and predicted probability (range: [0-1])
    d. Save error
    e. Repeat until all 9 parts have been left out

3) Find threshold with lowest error over 9 inner loops; in case of multiple values, take the mean rounded down. Subsequently we used the one standard error rule to select the highest threshold within 1 standard error of the optimal solution, yielding a sparser model with similar prediction performance as that with the minimal error (1) (inner loop end)
4) Train model on 9/10 of the data
5) Predict with the model and threshold from 3) on 1/10 of the left-out data and save predictions
6) Repeat until all 10 parts have been left out once (outer loop end)
7) Calculate area under the receiver operator curve (AUROC) and associated confidence interval (CI) of the outer-loop predictions

*Building the final classifier models*

In the cross validation loops a sample of the data is used to obtain the unbiased performance measures. The final classifier, which uses all the data rather than only a sample, was trained on all the data using the inner loop described above.

*Pseudo code for platform correction*

1) calculate the location-wise mean of the 3277 mapped BAC clones over all the samples of the TCGA data. E.g. BAC Clone 1 has an average value of 0.03 in the dataset.
2) sort (order from lowest to highest) the location-wise mean of the 3277 mapped BAC clones over all the patients of the TCGA data
3) calculate the location-wise mean of the 3277 mapped BAC clones of the training set Nimblegen data
4) sort (order from lowest to highest) the location-wise mean of the 3277 mapped BAC clones of the training set Nimblegen data
5) fit a linear regression model using glm with gaussian distribution and identity link function between the sorted mapped TCGA means and the sorted mapped Nimblegen means using the R function: glm(sorted.mean.Nimblegen ~ sorted.mean.TCGA)
6) Scale the TCGA data (3277 mapped BAC clones in the rows with x columns equal to the number of samples with the beta coefficient of the linear model and re-center the mean using the alpha coefficient: (TCGAdata * beta coefficient) + alpha coefficient

*Validation of platform correction*

We used the cross-platform comparison dataset described before (39). In this dataset we analyzed the same sample with multiple techniques. Since we are interested in the direct comparison between NGS (NGS) and NimbleGen 135K array CGH (NG135), we intersected the dataset for overlap between these samples. For 170 samples we obtained a copy number profile with NGS and NG135. Without applying the platform correction we obtained an accuracy of 0.86 (95% CI: 0.80-0.91) and a kappa value of 0.71 (95% CI: 0.56-0.86) for the *BRCA1*-like classifier and an accuracy of 0.90 (95% CI: 0.84-0.94) and a kappa of 0.80 (95% CI: 0.65-0.94) for the *BRCA2*-like classifier. With applying the platform correction, we obtained an accuracy of NGS 0.89 (95% CI: 0.835-0.934) and a kappa of 0.79 (95% CI: 0.64-0.93) for the *BRCA1*-like classifier and an accuracy of 0.86 (95% CI: 0.80-0.91) and a kappa of 0.71 (95% CI 0.56 -0.86) for the *BRCA2*-like classifier. For SNP6 we retained an accuracy of 0.84 (95% CI 0.66-0.94) for *BRCA1*-like classification and 0.97 (95% CI 0.82-0.99) for BRCA2-like classification.

*Statistical analyses*

Fisher's exact- and $\chi^2$-square-test were applied, where appropriate, to calculate the level of significance. All tests were two-sided, and a p-value <0.05 after correction for multiple testing using the Benjamini-Hochberg approach was considered significant.

## Example 1: Generation of copy number profiles

We generated copy number profiles of 13 control, 50 g*BRCA1*m and 10 g*BRCA2*m OC, and show the pathological characteristics of these tumors in Table 3. Figure 1 presents the average profiles of g*BRCA1*m and g*BRCA2*m versus control ovarian cancers.

## Example 2: Training OC-specific classifiers

First, we classified the ovarian cancer copy number profiles with the breast cancer *BRCA1*-like and *BRCA2*-like classifiers (30,31) and obtained an AUC of 0.51 (95% CI: 0.4-0.63) for g*BRCA1*m and an AUC of 0.63 (95%CI: 0.28-0.98) for g*BRCA2*m OC in the training set. Given this low performance, we trained shrunken centroid classifiers on ovarian cancer data to investigate whether the performance could be improved. These shrunken centroids select genomic regions and weights that are discriminative for the *BRCA*-like and non-*BRCA*-like class. We used ten-fold cross-validation and trained on the class labels g*BRCA1*m vs. control and g*BRCA2*m vs. control. We observed a cross-validated AUC of 0.67 (0.55-0.78) and 0.91 (0.79-1) respectively for *BRCA1*-like and *BRCA2*-like classification (Table 5). Since the NimbleGen aCGH platform is not available anymore, we validated cross-platform compatibility of our classifier using the same methods and dataset as previously described (39), see the Methods section above.

**Table 5:** Cross validated results of training shrunken centroids classifiers to distinguish *BRCA1*- or *BRCA2*-mutated from control ovarian cancers. AUC: Area under the curve of the receiver operating characteristic. CI: confidence interval.

| | AUC (95 %CI) | Sensitivity (95% CI) | Specificity (95% CI) | | Contr. | *BRCA1* | *BRCA2* |
|---|---|---|---|---|---|---|---|
| *gBRCA1* | 0.67 | 0.76 | 0.69 | Predicted Control | 9 | 12 | 0 |
| vs.control | (0.55-0.78) | (0.62-0.87) | (0.39-0.91) | Predicted *BRCA1* | 4 | 38 | 0 |
| *gBRCA2* | 0.91 | 0.90 | 0.92 | Predicted Control | 12 | 0 | 1 |
| vs. control | (0.79-1) | (0.55-1) | (0.64-1) | Predicted *BRCA2* | 1 | 0 | 9 |

## Example 3: External validation of ovarian cancer BRCA classifiers in the TCGA data

We used the TCGA ovarian cancer data as an external validation set for our newly trained ovarian cancer classifiers. We classified 358 of 583 samples as having a *BRCA1*-like profile. Within these 358, 25/26 g*BRCA1,* 10/11 somatic *BRCA1*-mutated tumors, and 67/69 *BRCA1*-methylated tumors were classified as *BRCA1*-like, resulting in an overall sensitivity of 94.7% (95% CI: 0.82-0.99). Specificity, however, is lower, at 40% (95% CI: 0.36-0.45). 350 of 583 samples were assigned to be *BRCA2*-like. Within these 350, 20/25 g*BRCA2*m and 8/11 *sBRCA2m* samples were classified as *BRCA2*-like, resulting in a sensitivity of 77% (95% CI: 0.6-0.9) and a specificity of 41% (95% CI: 0.37-0.49).

**Example 4: Validation within the AGO-TR1 cohort**

We further analyzed both the germline/somatic mutation status of *BRCA1/2* and *BRCA1* promoter hypermethylation in the AGO TR1 study. In addition, we aimed to characterize those patients called *BRCA*-like without a mutation in *BRCA1* or *BRCA2.*

*Study sample*

In total, 300 samples with complete genetic and epigenetic information available, were analyzed. 129 germline and 43 somatic deleterious variants in *BRCA1/2* and other HR genes were present in 159 OC samples (see Table 4). The majority displayed a *BRCA1* mutation (g: n=69, s: n=17), a *BRCA2* mutation (g: n=23, s: n=11) or a *RAD51C* mutation (g: n=13). One of these samples presented a germline double mutation of g*BRCA1* and -2 and was analyzed with the g*BRCA1*m group; one somatic *BRCA2* mutation coincided with a g*BRCA2* mutation and was assigned to the g*BRCA2* group. Eight other samples with a *BRCA* mutation (g: n=6, s: n=2) displayed a class 4/5 in another HR gene as well and were analyzed with the respective *BRCA*-mutated group.

Most OCs presented with high-grade serous histology (82%), and a tumor *TP53* mutation was found in 83.7%. Promoter hypermethylation was detected in 9.3% of samples for *BRCA1* (n=28) and in 1.3% of samples (n=4) for *RAD51C.* The mean age at diagnosis was 57.6 years, and 160 primary OC and 140 platinum-sensitive recurrent cases were included. 223 tumors showed a *BRCA1*-like or *BRCA2-like* classification (74.3%). 78 OC thereof were *BRCA1*-like, 26 *BRCA2-like,* and 119 were both *BRCA1*- and BRCA2-like.

*Detection of BRCA-associated ovarian cancers with BRCA-like classification*

As there is no established gold standard to define BRCAness in general, we first focused on the detection-rate of tumors derived from deleterious germline mutations in *BRCA1/2* (see Table 6, Figure 2). 67 of 69 *BRCA1*-mutated cases had a *BRCA1*-like (detection rate of 97.1%), whereas 16 of 22 samples with a *BRCA2* class4/5 variant in germline were *BRCA2*-like (detection rate of 72.7%). All remaining BRCA2-associated cases were identified by combining both classifiers, *i.e.* being *BRCA1*- or *BRCA2*-like.

**Table 6:** Detection rate of the *BRCA1*- (A) and *BRCA2*-like classifier (B) in BRCA-associated cancers. (C) shows the complementary application of both classifiers; *BRCA1*- and/ or *BRCA2*-like. *One sample displayed both a somatic *BRCA2* mutation and a *BRCA1* methylation and was classified as *BRCA1*- and *BRCA2-like.* Therefore, it was presented in both groups but counted only once for the total sum. The sample displaying both a g*BRCA1* and 2 mutation was counted among the g*BRCA1* samples, the sample with a *BRCA2* mutation in germline and tumor was counted among the g*BRCA2* samples. WT: wild type, g: germline, s: somatic, methyl: promoter hypermethylation.

| A) | | | |
|---|---|---|---|
| | *BRCA1*-like | [%] | total |
| g*BRCA1* | 67 | 97.1 | 69 |
| s*BRCA1* | 15 | 88.2 | 17 |
| *BRCA1* methyl | 27 | 96.4 | 28 |
| *BRCA1* WT | 88 | 47.3 | 186 |
| Total | 197 | 65.7 | 300 |

| B) | | | |
|---|---|---|---|
| | *BRCA2-like* | [%] | total |
| g*BRCA2* | 16 | 72.7 | 22 |

(continued)

| B) | | | |
|---|---|---|---|
| | *BRCA2-like* | [%] | total |
| *sBRCA2* | 6 | 60.0 | 10 |
| *BRCA2* WT | 124 | 46.3 | 268 |
| Total | 146 | 48.7 | 300 |

| C) | | | |
|---|---|---|---|
| | *BRCA1/2*-like | [%] | total |
| g*BRCA1* | 67 | 97.1 | 69 |
| g*BRCA2* | 22 | 100.0 | 22 15 |
| s*BRCA1* | 17 | 100.0 | 17 |
| s*BRCA2* | 6* | 60.0 | 10* |
| *BRCA1* methyl | 27* | 96.4 | 28* |
| *BRCA* WT | 85 | 54.8 | 155 |
| Total | 223 | 74.3 | 300 |

Regarding deleterious somatic variants, 15 of 17 (88.2%) samples with a *BRCA1* mutation were identified by the *BRCA1*-like classifier. As the other two tumors displayed a *BRCA2*-like phenotype, both classifiers' application detected 100% of the mutated cases. The *BRCA2*-like classifier confirmed 6 of 10 *sBRCA2m* cases. The *BRCA1*-like classifier found no additional sample. Of the 28 examined samples with a *BRCA1* promoter hypermethylation, 27 displayed a *BRCA1*-like profile (96.4%), one was classified as non-*BRCA*-like.

**Example 5: Classification of samples with HR gene alterations beyond BRCA1/2 and mutations in HNPCC genes**

Alterations in further HR genes (*ATM, BARD1, BRIP1, CHEK1, CHEK2, FAM175A, FANCM, MRE11A, NBN, PALB2, RAD50, RAD51C, RAD51D, XRCC2*) varied in being classified as *BRCA*-like (see Table 7). Within the study cohort, 44 tumors without *BRCA* mutation or *BRCA1* methylation displayed a genetic alteration in another OC-risk- or HR-gene (except s*TP53* mutations), of which 32 were classified as *BRCA*-like (72.7%; only *BRCA1*-like n=11, only *BRCA2*-like n=4, *BRCA1*- and *BRCA2*-like n=15). A distinct statistical association was observed for the largest subgroup, the *RAD51C*-associated tumors. All germline mutationcarriers developed a *BRCA*-like OC (only *BRCA1*-like n=6, *BRCA1*- and *BRCA2*-like n=7). No clear statistical association could be observed for most other HR gene mutated samples and samples with *RAD51C* methylation (see Table 7, Figure 2) possibly due to small numbers and coinciding aberrations.

**Table 7:** *BRCA-like classification of BRCA1/2 wildtype samples with alterations in HR-and HNPCC-genes.* Two samples are described twice, each due to mutual alterations (see * and **). Both samples displayed a non-BRCA-like phenotype. m: mutation/ class4/5 variant; methyl: promotor hypermethylation; b1: *BRCA1*-like; b2: *BRCA2*-like

| Genetic alteration | g(n) | s (n) | additional alterations | *BRCA*-like (n) | (only b1; only b2; b1+2) | [%] | non-*BRCA*-like (n) | [%] |
|---|---|---|---|---|---|---|---|---|
| *RAD51C*m | 13 | 0 | X | 13 | (6;0;7) | 100.0 | 0 | 0 |
| *RAD51D*m | 3 | 1 | X | 3 | (0;0;3) | 75.0 | 1 | 25.0 |
| *FANCM*m | 2 | 3 | X | 4 | (1;0;3) | 80.0 | 1 | 20.0 |
| *PALB2*m | 3 | 2 | 1x *BRCA1* -methyl (s) | 2 | (1;0; 1) | 40.0 | 3 | 60.0 |
| *PALB2*+ *ATM*m | 1 | 0 | X | 1 | (1;0;0) | 100.0 | 0 | 0.0 |

(continued)

| Genetic alteration | g(n) | s (n) | additional alterations | BRCA-like (n) | (only b1; only b2; b1+2) | [%] | non-BRCA-like (n) | [%] |
|---|---|---|---|---|---|---|---|---|
| ATMm | 1 | 2 | X | 3 | (0;2;1) | 100.0 | 0 | 0.0 |
| BRIP1m | 1 | 1 | 1x g+sMSH6 (s) * | 1 | (0;1;0) | 50.0 | 1 | 50.0 |
| BRIP1+ CHEK2m | 1 | 0 | X | 1 | (1;0;0) | 100.0 | 0 | 0.0 |
| CHEK2m | 1 | 0 | X | 0 | (0;0;0) | 0.0 | 1 | 100.0 |
| MRE11Am | 2 | 0 | 1x RAD51C-methyl ** | 1 | (0;1;0) | 50.0 | 1 | 50.0 |
| NBNm | 1 | 0 | X | 1 | (1;0;0) | 100.0 | 0 | 0.0 |
| CHEK1m | 1 | 0 | X | 0 | (0;0;0) | 0.0 | 1 | 100.0 |
| FAM175Am | 1 | 0 | X | 0 | (0;0;0) | 0.0 | 1 | 100.0 |
| BARD1m | 0 | 1 | X | 0 | (0;0;0) | 0.0 | 1 | 100.0 |
| RAD51C-methyl | 0 | 3 | 1x gMRE11A ** | 1 | (0;0;1) | 33.3 | 2 | 66.7 |
| HNPCC-genes (2x MSH2m, 1x MSH6m) | 3 | 0 | 1x gMSH6+ sMSH6 & sBRIP1 * | 0 | (0;0;0) | 0.0 | 3 | 100.0 |

Samples with a class 4/5 variant in *RAD51D* (g: n=3, s: n=1) presented a *BRCA1*- and *2*-like profile in 3 of 4 cases and *FANCM*-associated OC (g: n=2, s: n=3) in 3 of 5 samples, one sample further presented with only a *BRCA*1-like phenotype. All four AIM-associated samples showed a *BRCA*-like profile (only *BRCA1*-like: n=1, only *BRCA2*-like: n=2, *BRCA1*- and *2*-like: n=1). Three of 6 *PALB2*-associated cancers presented a *BRCA*-like profile, including two samples with each an *ATM* mutation (see above) and a *BRCA1* methylation. Therefore, the underlying mechanism leading to a BRCA-like phenotype was unclear. Samples with each a mutation in *CHEK1* (g), *FAM175A* (g), and *BARD1* (s) as well as three tumors with a germline mutation in HNPCC genes (*MSH2* n=2, *MSH6* with additional somatic *MSH6*- and *BRIP1* mutation, n=1) were examined and showed no *BRCA*-like profile. Epigenetic alterations in *RAD51C* were rare. Four samples with a *RAD51C* promoter hypermethylation were analyzed, of which one was derived from a germline *BRCA1* mutation carrier, presenting a *BRCA1*- and BRCA2-like profile, assumingly due to the known mutation. Of the three remaining samples, one was classified as *BRCA1*- and *BRCA2*-like; the other two (one of them from a g*MRE11A* mutation-carrier) displayed a non-*BRCA*-like phenotype.

*Molecular genetic background of BRCA-like samples*

Within the cohort, 223 samples displayed a *BRCA1/2*-like phenotype. These profiles were associated with a germline mutation in *BRCA1/2* in 89 and a somatic mutation in *BRCA1/2* respectively a *BRCA1* promoter hypermethylation in 49 of these samples. In another 29 different samples, a somatic or germline class 4/5 variant in another HR gene (esp. *RAD51C*) or a *RAD51C* promoter hypermethylation can explain the presence of a *BRCA*-like phenotype. For the remaining 56 *BRCA1/2*-like samples (25.1%) no aberration affecting the HR could be detected by gene panel or methylation analyses (see Figure 3).

Regarding all OC without a genetic or epigenetic alteration in *BRCA1/2* (n=155), 54.8% (n=85) display a *BRCA1/2*-like phenotype. Excluding all HR gene mutated cases (n=113), as the study cohort was enriched for those, the *BRCA1/2*-like rate was 48.7% (n=55). Furthermore, *a BRCA*-like phenotype was correlated with high-grade serous histology, *sTP53* mutations and could be observed more often, when the patient was included with platinum-sensitive recurring disease than at primary diagnosis. A non-*BRCA*-like profile was seen in association with low-grade serous histology, in combination with *PTEN*- or *PIK3CA* mutations and with gHNPCC mutations (see Table 8).

In summary, *BRCA1*-like and *BRCA2*-like copy number classifiers have been identified and these signatures identify patients with germline and somatic mutations and promoter hypermethylation in *BRCA1/2*. In addition, we were able to investigate underlying molecular mechanisms in BRCA-like cases without a deleterious variant in *BRCA1* or *BRCA2*.

Another advantage of this study is that after initial promising results within cross-validation of the training set and in the TCGA test dataset were obtained, the AGO-TR1 study provided a large and well characterized cohort in which we could validate the prediction of mutation status. Overall, the *BRCA1*-like classifier showed a convincing performance with detecting *BRCA1*-mutated and -methylated cancers in more than 90% of the cases. When applying both classifiers in the AGO-TR1 trial, more than 95% of all *BRCA1/2*-associated cancers in the cohort were detected. Extensive germline and tumor genetic analysis provided additional support for the BRCA-like class showing alterations in *BRCA1/2* or other HR genes in 74.9% of cases. Thus, a convincing performance of the *BRCA1*-like classifier and the combination of both classifiers in detecting *BRCA* mutations/methylation has been shown. Additionally, around half of the non-*BRCA*-associated OC cases displayed a *BRCA*-like phenotype as well.

**Table 8:** Rate of *BRCA1*-, *BRCA2*-like status and either according to genetic (germline and somatic), epigenetic, and clinical features. In the first section, alterations in the HR-pathway are in focus. The sample with both a germline mutation in *BRCA1* and *BRCA2* is analyzed within the g*BRCA1* group, the sample with the germline and somatic deleterious variant in *BRCA2* with the g*BRCA2* group and the samples with an additional HR gene mutation (n=8) or a *RAD51C* methylation (n=1) in the presence of a g/s*BRCA* mutation are assigned to the respective *BRCA* group as the latter is supposed to be the superordinate feature in developing a *BRCA*-like profile. Three other samples with two mutual alterations (*BRCA1*-methyl and s*BRCA2*; *BRCA1*-methyl and s*PALB2*; *RAD51C*-methyl and g*MRE11A*) were mentioned in both categories, as the predominant mechanism was not obvious. Two of the three gHNPCC samples are included in the "no HR gene alteration" group as well, the other one within the "sHR gene" group. In the following somatic *TP53*, *PIK3CA* and *PTEN* mutations, the histological subtype and the presence of primary or recurrent disease were analyzed irrespective of alterations in the HR-pathway. As s*TP53*, s*PIK3CA* and s*PTEN* mutations could co-occur, the affected samples were mentioned in each subcategory.

p<0.05 is considered significant (Benjamini-Hochberg correction was applied). * marks p-values with a significant correlation with non-*BRCA*-like status.
G: germline, s: somatic, methyl: promoter hypermethylation, HR: homologous recombination, HNPCC: hereditary non-polyposis colorectal cancer,
HGS: high grade serous ovarian cancer (OC), HGE: high grade endometrioid OC, LGS: low grade serous OC, LGE: low grade endometrioid OC, CC: clear cell cancer, muc: mucinous OC, G: grading, PR: primary OC, RE: platinum-sensitive recurrent OC.

| | n | *BRCA1*-like | [%] | non-*BRCA1*-like | [%] | p-value | *BRCA2*-like | [%] | non-*BRCA2*-like | [%] | p-value | *BRCA1/2*-like | [%] | non-*BRCA1/2*-like | [%] | p-value |
|---|---|---|---|---|---|---|---|---|---|---|---|---|---|---|---|---|
| all | 300 | 197 | 65.7 | 103 | 34.3 | | 146 | 48.7 | 154 | 51.3 | | 223 | 74.3 | 77 | 25.7 | |
| | | | | | | | | | | | | | | | | |
| g*BRCA1* | 69 | 67 | 97.1 | 2 | 2.9 | **<0.001** | 37 | 55.2 | 32 | 46.4 | 0.867 | 67 | 97.1 | 2 | 2.9 | **<0.001** |
| g*BRCA2* | 22 | 20 | 90.9 | 2 | 9.1 | **0.023** | 16 | 72.7 | 6 | 27.3 | 0.090 | 22 | 100.0 | 0 | 0.0 | **0.009** |
| gHRgenes | 31 | 21 | 67.4 | 10 | 32.1 | 1.000 | 14 | 45.2 | 17 | 54.8 | 0.867 | 24 | 77.4 | 7 | 22.6 | 0.125 |
| g*RAD51C* | 13 | 13 | 100.0 | 0 | 0.0 | **0.015** | 7 | 53.8 | 6 | 46.2 | 0.867 | 13 | 100.0 | 0 | 0.0 | **0.050** |
| s*BRCA1* | 17 | 15 | 88.2 | 2 | 11.8 | 0.114 | 15 | 88.2 | 2 | 11.8 | **0.005** | 17 | 100.0 | 0 | 0.0 | **0.021** |
| s*BRCA2* | 10 | 5 | 50.0 | 5 | 50.0 | 0.457 | 6 | 60.0 | 4 | 40.0 | 0.867 | 6 | 60.0 | 4 | 40.0 | 0.341 |
| sHRgenes | 10 | 5 | 50.0 | 5 | 50.0 | 0.457 | 4 | 40.0 | 6 | 60.0 | 0.867 | 6 | 60.0 | 4 | 40.0 | 0.341 |
| *BRCA1*-meth | 28 | 27 | 96.4 | 1 | 3.6 | <0.001 | 15 | 53.6 | 13 | 46.4 | 0.867 | 27 | 96.4 | 1 | 3.6 | **0.010** |
| *RAD51C*-meth | 3 | 1 | 33.3 | 2 | 66.7 | 0.450 | 1 | 33.3 | 2 | 66.7 | 1.000 | 1 | 33.3 | 2 | 66.7 | 0.246 |
| no HR gene alteration | 113 | 38 | 33.6 | 75 | 66.4 | **<0.001*** | 39 | 34.5 | 76 | 65.5 | **0.001*** | 55 | 48.7 | 58 | 51.3 | **<0.001*** |
| gHNPCCgenes | 3 | 0 | 0.0 | 3 | 100.0 | 0.079* | 0 | 0.0 | 3 | 100.0 | 0.714 | 0 | 0.0 | 3 | 100.0 | **0.036*** |
| | | | | | | | | | | | | | | | | |
| s*TP53* | 251 | 185 | 73.7 | 66 | 26.3 | **<0.001** | 138 | 55.0 | 113 | 45.0 | **<0.001** | 206 | 82.1 | 45 | 17.9 | **<0.001** |

p<0.05 is considered significant (Benjamini-Hochberg correction was applied). * marks p-values with a significant correlation with non-BRCA-like status.
G: germline, s: somatic, methyl: promoter hypermethylation, HR: homologous recombination, HNPCC: hereditary non-polyposis colorectal cancer,
HGS: high grade serous ovarian cancer (OC), HGE: high grade endometrioid OC, LGS: low grade serous OC, LGE: low grade endometrioid OC, CC: clear cell cancer, muc: mucinous OC, G: grading, PR: primary OC, RE: platinum-sensitive recurrent OC.

| | n | BRCA1-like | [%] | non-BRCA1-like | [%] | p-value | BRCA2-like | [%] | non-BRCA2-like | [%] | p-value | BRCA1/2-like | [%] | non-BRCA1/2-like | [%] | p-value |
|---|---|---|---|---|---|---|---|---|---|---|---|---|---|---|---|---|
| sPIK3CA | 11 | 3 | 27.3 | 8 | 72.7 | 0.003 * | 1 | 9.1 | 10 | 90.9 | 0.053* | 4 | 36.4 | 7 | 63.6 | 0.021* |
| sPTEN | 10 | 2 | 20.0 | 8 | 80.0 | 0.079* | 4 | 40.0 | 6 | 60.0 | 0.867 | 5 | 50.0 | 5 | 50.0 | 0.217 |
| sPIK3CA/PTEN | 17 | 4 | 23.5 | 14 | 76.5 | **0.001*** | 4 | 23.5 | 14 | 76.5 | 0.090* | 7 | 41.2 | 10 | 58.8 | **0.010*** |
| | | | | | | | | | | | | | | | | |
| HGS | 246 | 172 | 69.9 | 74 | 30.1 | **0.011** | 128 | 52.0 | 118 | 48.0 | 0.076 | 191 | 77.6 | 55 | 22.4 | **0.036** |
| HGE | 16 | 6 | 37.5 | 10 | 62.5 | 0.072* | 8 | 50.0 | 8 | 50.0 | 1.000 | 9 | 56.3 | 7 | 43.7 | 0.272 |
| LGS | 7 | 1 | 14.3 | 6 | 85.7 | **0.030*** | 0 | 0.0 | 7 | 100.0 | 0.076* | 1 | 14.3 | 6 | 85.7 | **0.011*** |
| LGE | 4 | 1 | 25.0 | 3 | 75.0 | 0.192 | 3 | 75.0 | 1 | 25.0 | 0.720 | 3 | 75.0 | 1 | 25.0 | 1.000 |
| CC | 2 | 0 | 0.0 | 2 | 100.0 | 0.192 | 0 | 0.0 | 2 | 100.0 | 0.789 | 0 | 0.0 | 2 | 100.0 | 0.173 |
| muc | 4 | 3 | 75.0 | 1 | 25.0 | 1.000 | 1 | 25.0 | 3 | 75.0 | 0.789 | 3 | 75.0 | 1 | 25.0 | 1.000 |
| serous, G unknown | 6 | 3 | 50.0 | 3 | 50.0 | 0.560 | 2 | 33.3 | 4 | 66.7 | 0.789 | 4 | 67.7 | 2 | 33.3 | 1.000 |
| other/missing | 15 | 11 | 73.3 | 4 | 26.7 | 0.674 | 4 | 26.7 | 11 | 73.3 | 0.293 | 12 | 80.0 | 3 | 20.0 | 1.000 |
| | | | | | | | | | | | | | | | | |
| PR | 160 | 97 | 60.6 | 63 | 39.4 | 0.052* | 75 | 46.9 | 85 | 53.1 | 0.563 | 113 | 70.6 | 47 | 29.4 | 0.145 |
| RE | 140 | 100 | 71.4 | 40 | 28.6 | 0.052 | 71 | 50.7 | 69 | 49.3 | 0.563 | 110 | 78.6 | 30 | 21.4 | 0.145 |

**References**

1. Fitzmaurice C, Abate D, Abbasi N, Abbastabar H, Abd-Allah F, Abdel-Rahman O, et al. Global, Regional, and National Cancer Incidence, Mortality, Years of Life Lost, Years Lived With Disability, and Disability-Adjusted Life-Years for 29 Cancer Groups, 1990 to 2017: A Systematic Analysis for the Global Burden of Disease Study. JAMA Oncol 2019;5(12):1749-68.

2. National Cancer Institue. Cancer Stat Facts: Ovarian Cancer. <https://seer.cancer.gov/statfacts/html/ovary.html>. Accessed 2020 September 30th.

3. du Bois A, Reuss A, Pujade-Lauraine E, Harter P, Ray-Coquard I, Pfisterer J. Role of surgical outcome as prognostic factor in advanced epithelial ovarian cancer: a combined exploratory analysis of 3 prospectively randomized phase 3 multicenter trials: by the Arbeitsgemeinschaft Gynaekologische Onkologie Studiengruppe Ovarialkarzinom (AGO-OVAR) and the Groupe d'Investigateurs Nationaux Pour les Etudes des Cancers de l'Ovaire (GINECO). Cancer 2009;115(6):1234-44.

4. Piver MS. Treatment of ovarian cancer at the crossroads: 50 years after single-agent melphalan chemotherapy. Oncology (Williston Park) 2006;20(10):1156, 58.

5. Ledermann JA, Raja FA, Fotopoulou C, Gonzalez-Martin A, Colombo N, Sessa C, et al. Newly diagnosed and relapsed epithelial ovarian carcinoma: ESMO Clinical Practice Guidelines for diagnosis, treatment and follow-up. Ann Oncol 2013;24 Suppl 6:vi24-32.

6. Ledermann JA, Drew Y, Kristeleit RS. Homologous recombination deficiency and ovarian cancer. European journal of cancer (Oxford, England : 1990) 2016;60:49-58.

7. Moynahan ME, Chiu JW, Koller BH, Jasin M. Brca1 controls homology-directed DNA repair. Mol Cell 1999;4(4):511-8.

8. Moynahan ME, Pierce AJ, Jasin M. BRCA2 is required for homology-directed repair of chromosomal breaks. Mol Cell 2001;7(2):263-72.

9. Moore K, Colombo N, Scambia G, Kim BG, Oaknin A, Friedlander M, et al. Maintenance Olaparib in Patients with Newly Diagnosed Advanced Ovarian Cancer. N Engl J Med 2018;379(26):2495-505.

10. Robson M, Im SA, Senkus E, Xu B, Domchek SM, Masuda N, et al. Olaparib for Metastatic Breast Cancer in Patients with a Germline BRCA Mutation. N Engl J Med 2017;377(6):523-33.

11. Turner N, Tutt A, Ashworth A. Hallmarks of 'BRCAness' in sporadic cancers. Nat Rev Cancer 2004;4(10):814-9.

12. Lord CJ, Ashworth A. BRCAness revisited. Nat Rev Cancer 2016;16(2): 110-20.

13. Ledermann J, Harter P, Gourley C, Friedlander M, Vergote I, Rustin G, et al. Olaparib maintenance therapy in patients with platinum-sensitive relapsed serous ovarian cancer: a preplanned retrospective analysis of outcomes by BRCA status in a randomised phase 2 trial. Lancet Oncol 2014;15(8):852-61.

14. Mirza MR, Monk BJ, Herrstedt J, Oza AM, Mahner S, Redondo A, et al. Niraparib Maintenance Therapy in Platinum-Sensitive, Recurrent Ovarian Cancer. N Engl J Med 2016;375(22):2154-64.

15. Swisher EM, Lin KK, Oza AM, Scott CL, Giordano H, Sun J, et al. Rucaparib in relapsed, platinum-sensitive high-grade ovarian carcinoma (ARIEL2 Part 1): an international, multicentre, open-label, phase 2 trial. Lancet Oncol 2017;18(1):75-87.

16. Coleman RL, Oza AM, Lorusso D, Aghajanian C, Oaknin A, Dean A, et al. Rucaparib maintenance treatment for recurrent ovarian carcinoma after response to platinum therapy (ARIEL3): a randomised, double-blind, placebo-controlled, phase 3 trial. Lancet 2017;390(10106): 1949-61.

17. Abkevich V, Timms KM, Hennessy BT, Potter J, Carey MS, Meyer LA, et al. Patterns of genomic loss of heterozygosity predict homologous recombination repair defects in epithelial ovarian cancer. Br J Cancer 2012;107(10):1776-82.

18. Davies H, Glodzik D, Morganella S, Yates LR, Staaf J, Zou X, et al. HRDetect is a predictor of BRCA1 and BRCA2 deficiency based on mutational signatures. Nat Med 2017;23(4):517-25.

19. Birkbak NJ, Wang ZC, Kim JY, Eklund AC, Li Q, Tian R, et al. Telomeric allelic imbalance indicates defective DNA repair and sensitivity to DNA-damaging agents. Cancer Discov 2012;2(4):366-75.

20. Popova T, Manie E, Rieunier G, Caux-Moncoutier V, Tirapo C, Dubois T, et al. Ploidy and large-scale genomic instability consistently identify basal-like breast carcinomas with BRCA1/2 inactivation. Cancer Res 2012;72(21):5454-62.

21. Konstantinopoulos PA, Spentzos D, Karlan BY, Taniguchi T, Fountzilas E, Francoeur N, et al. Gene expression profile of BRCAness that correlates with responsiveness to chemotherapy and with outcome in patients with epithelial ovarian cancer. J Clin Oncol 2010;28(22):3555-61.

22. Timms KM, Abkevich V, Hughes E, Neff C, Reid J, Morris B, et al. Association of BRCA1/2 defects with genomic scores predictive of DNA damage repair deficiency among breast cancer subtypes. Breast Cancer Res 2014;16(6):475.

23. Moore KN, Secord AA, Geller MA, Miller DS, Cloven N, Fleming GF, et al. Niraparib monotherapy for late-line treatment of ovarian cancer (QUADRA): a multicentre, open-label, single-arm, phase 2 trial. Lancet Oncol 2019;20(5):636-48.

24. Coleman RL, Fleming GF, Brady MF, Swisher EM, Steffensen KD, Friedlander M, et al. Veliparib with First-Line Chemotherapy and as Maintenance Therapy in Ovarian Cancer. N Engl J Med 2019;381(25):2403-15.

25. Gonzalez-Martin A, Pothuri B, Vergote I, DePont Christensen R, Graybill W, Mirza MR, et al. Niraparib in Patients with Newly Diagnosed Advanced Ovarian Cancer. N Engl J Med 2019;381(25):2391-402.

26. Ray-Coquard I, Pautier P, Pignata S, Perol D, Gonzalez-Martin A, Berger R, et al. Olaparib plus Bevacizumab as First-Line Maintenance in Ovarian Cancer. N Engl J Med 2019;381(25):2416-28.

27. European Medicines Agency. 2020. Lynparza, Opinion. <https://www.ema.europa.eu/en/medicines/human/summaries-opinion/lynparza-0>. Accessed 2020 September 30.

28. Joosse SA, van Beers EH, Tielen IH, Horlings H, Peterse JL, Hoogerbrugge N, et al. Prediction of BRCAI-association in hereditary non-BRCA1/2 breast carcinomas with array-CGH. Breast Cancer Res Treat 2009;116(3):479-89.

29. Tibshirani R, Hastie T, Narasimhan B, Chu G. Diagnosis of multiple cancer types by shrunken centroids of gene expression. Proc Natl Acad Sci U S A 2002;99(10):6567-72.

30. Vollebergh MA, Lips EH, Nederlof PM, Wessels LF, Schmidt MK, van Beers EH, et al. An aCGH classifier derived from BRCAI-mutated breast cancer and benefit of high-dose platinum-based chemotherapy in HER2-negative breast cancer patients. Ann Oncol 2011;22(7):1561-70.

31. Vollebergh MA, Lips EH, Nederlof PM, Wessels LF, Wesseling J, Vd Vijver MJ, et al. Genomic patterns resembling BRCA1- and BRCA2-mutated breast cancers predict benefit of intensified carboplatin-based chemotherapy. Breast Cancer Res 2014;16(3):R47.

32. Schouten PC, Marme F, Aulmann S, Sinn HP, van Essen HF, Ylstra B, et al. Breast cancers with a BRCAI-like DNA copy number profile recur less often than expected after high-dose alkylating chemotherapy. Clin Cancer Res 2015;21(4):763-70.

33. Schouten PC, Gluz O, Harbeck N, Mohrmann S, Diallo-Danebrock R, Pelz E, et al. BRCAI-like profile predicts benefit of tandem high dose epirubicin-cyclophospamide-thiotepa in high risk breast cancer patients randomized in the WSG-AM01 trial. Int J Cancer 2016;139(4):882-9.

34. Vliek S, Jager A, Jonge-Lavrencic M, Lotz J-P, Goncalves A, Graeser M, et al. Abstract OT2-07-08: Substantially improving the cure rate of high-risk BRCA1-like breast cancer patients with personalized therapy (SUBITO) - an international randomized phase III trial. Cancer Research 2018;78(4 Supplement):OT2-07-08-OT2-07-08.

35. Severson TM, Wolf DM, Yau C, Peeters J, Wehkam D, Schouten PC, et al. The BRCAIness signature is associated significantly with response to PARP inhibitor treatment versus control in the I-SPY 2 randomized neoadjuvant setting. Breast Cancer Res 2017;19(1):99.

36. Harter P, Hauke J, Heitz F, Reuss A, Kommoss S, Marme F, et al. Prevalence of deleterious germline variants in risk genes including BRCA1/2 in consecutive ovarian cancer patients (AGO-TR-1). PLoS One 2017;12(10):e0186043.

37. Hauke J, Hahnen E, Schneider S, Reuss A, Richters L, Kommoss S, et al. Deleterious somatic variants in 473 consecutive individuals with ovarian cancer: results of the observational AGO-TR1 study (NCT02222883). J Med Genet 2019;56(9):574-80.

38. Federation of Dutch Medical Scientific Societies. 2011. Human Tissue and Medical Research: Code of Conduct for responsible use (2011). <https://www.federa.org/sites/default/files/images/print_version_code_of_conduct_english.pdf>. Accessed 2020 September 30.

39. Schouten PC, Grigoriadis A, Kuilman T, Mirza H, Watkins JA, Cooke SA, et al. Robust BRCAI-like classification of copy number profiles of samples repeated across different datasets and platforms. Mol Oncol 2015;9(7):1274-86.

40. Schouten PC, van Dyk E, Braaf LM, Mulder L, Lips EH, de Ronde JJ, et al. Platform comparisons for identification of breast cancers with a BRCA-like copy number profile. Breast Cancer Res Treat 2013;139(2):317-27.

41. Hastie T, Tibshirani R, Friedman J. The Elements of Statistical Learning. Springer, New York, NY; 2009.

42. Bell D, Berchuck A, Birrer M, Chien J, Cramer DW, Dao F, et al. Integrated genomic analyses of ovarian carcinoma. Nature 2011;474(7353):609-15.

43. Domanska K, Malander S, Staaf J, Karlsson A, Borg A, Jonsson G, et al. Genetic profiles distinguish different types of hereditary ovarian cancer. Oncol Rep 2010;24(4):885-95.

44. Kamieniak MM, Munoz-Repeto I, Rico D, Osorio A, Urioste M, Garcia-Donas J, et al. DNA copy number profiling reveals extensive genomic loss in hereditary BRCA1 and BRCA2 ovarian carcinomas. Br J Cancer 2013;108(8):1732-42.

45. Maxwell KN, Wubbenhorst B, Wenz BM, De Sloover D, Pluta J, Emery L, et al. BRCA locus-specific loss of heterozygosity in germline BRCA1 and BRCA2 carriers. Nature Communications 2017;8(1):319.

46. Macintyre G, Goranova TE, De Silva D, Ennis D, Piskorz AM, Eldridge M, et al. Copy number signatures and mutational processes in ovarian carcinoma. Nature genetics 2018;50(9):1262-70.

47. Prahallad A, Sun C, Huang S, Di Nicolantonio F, Salazar R, Zecchin D, et al. Unresponsiveness of colon cancer to BRAF(V600E) inhibition through feedback activation of EGFR. Nature 2012;483(7387):100-3.

48. Alexandrov LB, Kim J, Haradhvala NJ, Huang MN, Tian Ng AW, Wu Y, et al. The repertoire of mutational signatures in human cancer. Nature 2020;578(7793):94-101.

49. Watkins JA, Irshad S, Grigoriadis A, Tutt AN. Genomic scars as biomarkers of homologous recombination deficiency and drug response in breast and ovarian cancers. Breast Cancer Res 2014;16(3):211.

50. Castroviejo-Bermejo M, Cruz C, Llop-Guevara A, Gutiérrez-Enríquez S, Ducy M, Ibrahim YH, et al. A RAD51 assay feasible in routine tumor samples calls PARP inhibitor response beyond BRCA mutation. EMBO molecular medicine 2018;10(12).

**Claims**

1. An *in vitro* method of determining homologous recombination (HR) deficiency status in a subject having ovarian cancer, wherein the method comprises

   (a) determining copy number variation (CNV) of a genomic locus in a DNA test sample of cancer cells from the subject, wherein the genomic locus is selected from a pool of genomic loci comprising or consisting of the loci set forth in Table 1 and/or Table 2;
   wherein similarity between the CNVs of the pool of genomic loci in the DNA test sample and the CNVs of the pool of genomic loci of a reference sample of BRCA-like mutated cancer cells identifies the subject as HR deficient; and/or similarity between the CNVs in the pool of genomic loci in the DNA of the test sample and the CNVs in the pool of genomic loci of the control sample of BRCA non-mutated cancer cells identifies the subject as not being HR deficient, wherein similarity is the distance measure between centroids of the two classes, *i.e.*, the control and the reference sample, and the centroid of the test sample, wherein the centroid of a class is constructed by shrinking the class centroid by an optimization routine towards the overall centroid of both classes after standardizing by the within-class standard deviation for each CNV; and optionally
   (b) transmitting the result to the subject or a third party, preferably a physician or genetic counselor.

2. An *in vitro* method of determining homologous recombination (HR) deficiency status in a subject having ovarian cancer, wherein the method comprises:

   (a) determining copy number variation (CNV) of a genomic locus in a DNA test sample of cancer cells from the subject, wherein the locus is selected from a pool of genomic loci comprising or consisting of the loci set forth in Table 1 and/or the loci set forth in Table 2,
   (b) providing a shrunken centroid value which is derived from the CNV of the genomic locus; and
   wherein a posterior probability score of $\geq 0.5$ identifies the subject as HR deficient; and/or wherein a posterior probability score of < 0.5 identifies the subject as not being HR deficient, wherein the posterior probability score is obtainable by comparison of the shrunken centroid values derived from the CNVs of the pool of genomic loci in the DNA of the cancer cells in the test sample from the subject with the shrunken centroid values derived from the CNVs of a reference set of samples of BRCA-like mutated cancer cells and of control samples of BRCA non-mutated cancer cells, and optionally
   (c) transmitting the result to the subject or a third party, preferably a physician or genetic counselor.

3. The method according to claim 1 or 2, wherein a posterior probability score of $\geq 0.5$ and a similarity between the CNVs of the test sample and the CNVs of the reference sample, respectively identifies the subject as being a likely responder to an anti-cancer therapy; or wherein a posterior probability score of < 0.5 and a similarity between the CNVs of the test sample and the CNVs of the control sample, respectively identifies the subject a not being a likely responder to an anti-cancer therapy, whereby said anti-cancer therapy is selected from a PARP inhibitor, a DNA double strand break inducing agent, preferably wherein the DNA double strand break inducing agent is a platinum compound or an interstrand crosslinking agent, an anthracycline, a topoisomerase I inhibitor, or a topoisomeraase II inhibitor.

4. An in vitro method of predicting the response of a subject having ovarian cancer to an anti-cancer therapy, whereby said anti-cancer therapy is selected from a PARP inhibitor, a DNA double strand break inducing agent, preferably wherein the DNA double strand break inducing agent is a platinum compound or an interstrand crosslinking agent, an anthracycline, a topoisomerase I inhibitor, or a topoisomeraase II inhibitor, wherein the method comprises the method steps (a) of claim 1 and
the method steps (a) and (b) of claim 2, respectively, wherein

(i) a posterior probability score of $\geq$ 0.5 and a similarity between the CNVs of the test sample and the CNVs of the reference sample, respectively indicates an increased likelihood that the subject will respond to the anti-cancer therapy, and/or
(ii) a posterior probability score of < 0.5 and a similarity between the CNVs of the test sample and the CNVs of the control sample, respectively indicates an increased likelihood that the subject will not respond to the anti-cancer therapy, and optionally
(iii) transmitting the result to the subject or a third party, preferably a physician or genetic counselor.

5. The method of any one of claims 1 to 4, wherein the anti-cancer therapy comprises poly(ADP-ribose) polymerase inhibitors (PARPi) and/or platinum compounds.

6. The method of any one of claims 1 to 5, wherein the determination of the CNVs is performed by DNA sequencing.

7. The method of any one of claims 1 to 6, wherein the determination of the CNVs is performed prior to administration of the anti-cancer therapy.

8. The method of any one of claims 1 to 7 further comprising recommending a subject which

(a) has been identified as being HR deficient and/or having an increased likelihood of responding to the anti-cancer therapy, a treatment regimen comprising a PARP inhibitor, a DNA double strand break inducing agent, preferably wherein the DNA double strand break inducing agent is a platinum compound or an interstrand crosslinking agent, an anthracycline, a topoisomerase I inhibitor, or a topoisomeraase II inhibitor
(b) has been identified as not being HR deficient and not having an increased likelihood of responding to the anti-cancer therapy, a treatment regimen not comprising a PARP inhibitor, a DNA double strand break inducing agent, preferably wherein the DNA double strand break inducing agent is a platinum compound or an interstrand crosslinking agent, an anthracycline, a topoisomerase I inhibitor, or a topoisomeraase II inhibitor.

9. A PARP inhibitor of a DNA double strand break inducing agent, preferably wherein the DNA double strand break inducing agent is a platinum compound or an interstrand crosslinking agent, an anthracycline, a topoisomerase I inhibitor, or a topoisomerase II inhibitor, for use in treating ovarian cancer in a subject, wherein said subject has been identified as HR deficient in accordance with the method of any one of claims 1 to 3, and 5 to 9 and/or identified to have an increased likelihood of responding to the anti-cancer treatment regimen in accordance with the method of any one of claims 4 to 9.

**Patentansprüche**

1. *In-vitro*-Verfahren zum Bestimmen eines Status der Defizienz einer homologen Rekombination (HR) bei einem Individuum mit Eierstockkrebs, wobei das Verfahren umfasst

(a) Feststellen der Kopienzahlvariation (KZV) eines genomischen Locus in einer DNA-Testprobe von Krebszellen des Individuums, wobei der genomische Locus aus einem Pool genomischer Loci ausgewählt ist, der die in Tabelle 1 und/oder Tabelle 2 aufgeführten Loci umfasst oder aus diesen besteht; wobei die Ähnlichkeit zwischen den KZVs des Pools genomischer Loci in der DNA-Testprobe und den KZVs des Pools genomischer Loci einer Referenzprobe ähnlich BRCA-mutierter Krebszellen das Individuum als HR-defizient identifiziert; und/oder die Ähnlichkeit zwischen den KZVs in dem Pool genomischer Loci in der DNA der Testprobe und den KZVs in dem Pool genomischer Loci der Kontrollprobe nicht-BRCA-mutierter Krebszellen das Individuum als nicht HR-defizient identifiziert; wobei Ähnlichkeit das Abstandsmaß zwischen Centroiden der beiden Klassen, d. h. der Kontrolle und der Referenzprobe, und dem Centroid der Testprobe ist, wobei das Centroid einer Klasse konstruiert wird durch Schrumpfen des Klassencentroids durch eine Optimierungsroutine in Richtung des allgemeinen Centroids beider Klassen nach Standardisierung durch die klasseninterne Standardabweichung für jede KZV; und wahlweise
(b) Übermitteln des Ergebnisses an das Individuum oder einen Dritten, vorzugsweise an einen Arzt oder einen genetischen Berater.

2.  *In-vitro*-Verfahren zum Bestimmen eines Status der Defizienz einer homologen Rekombination (HR) bei einem Individuum mit Eierstockkrebs, wobei das Verfahren umfasst:

    (a) Feststellen der Kopienzahlvariation (KZV) eines genomischen Locus in einer DNA-Testprobe von Krebszellen des Individuums, wobei der Locus aus einem Pool genomischer Loci ausgewählt ist, der die in Tabelle 1 aufgeführten Loci und/oder die in Tabelle 2 aufgeführten Loci umfasst oder aus diesen besteht,
    (b) Bereitstellen eines geschrumpften Centroid-Wertes, der von der KZV des genomischen Locus abgeleitet ist; und
    wobei ein Posterior-Wahrscheinlichkeitswert von ≥ 0,5 das Individuum als HR-defizient identifiziert; und/oder wobei ein Posterior-Wahrscheinlichkeitswert von < 0,5 das Individuum als nicht HR-defizient identifiziert, wobei der Posterior-Wahrscheinlichkeitswert durch Vergleich der geschrumpften Centroid-Werte, die von den KZVs des Pools genomischer Loci in der DNA der Krebszellen in der Testprobe des Individuums abgeleitet sind, mit den geschrumpften Centroid-Werten, die von den KZVs eines Referenzsatzes von Proben ähnlicher BRCA-mutierter Krebszellen und von Kontrollproben nicht-BRCA-mutierter Krebszellen abgeleitet sind, erhältlich ist, und wahlweise
    (c) Übermitteln des Ergebnisses an das Individuum oder einen Dritten, vorzugsweise an einen Arzt oder einen genetischen Berater.

3.  Verfahren nach Anspruch 1 oder 2, wobei ein Posterior-Wahrscheinlichkeitswert von ≥ 0,5 und eine Ähnlichkeit zwischen den KZVs der Testprobe und den KZVs der Referenzprobe das Individuum als wahrscheinlichen Responder auf eine Krebstherapie identifiziert; oder wobei ein Posterior-Wahrscheinlichkeitswert von < 0,5 und eine Ähnlichkeit zwischen den KZVs der Testprobe und den KZVs der Kontrollprobe das Individuum als unwahrscheinlichen Responder auf eine Krebstherapie identifiziert, wobei die Krebstherapie aus einem PARP-Inhibitor, einem DNA-Doppelstrangbruch induzierenden Mittel, wobei es sich bei dem DNA-Doppelstrangbruch induzierenden Mittel bevorzugt um eine Platinverbindung oder ein strangvernetzendes Mittel handelt, einem Anthracyclin, einem Topoisomerase-I-Inhibitor oder einem Topoisomerase-II-Inhibitor ausgewählt ist.

4.  *In-vitro*-Verfahren zum Vorhersagen des Ansprechens eines Individuums mit Eierstockkrebs auf eine Krebstherapie, wobei die Krebstherapie aus einem PARP-Inhibitor, einem DNA-Doppelstrangbruch induzierenden Mittel, wobei es sich bei dem DNA-Doppelstrangbruch induzierenden Mittel bevorzugt um eine Platinverbindung oder ein strangvernetzendes Mittel handelt, einem Anthracyclin, einem Topoisomerase-I-Inhibitor oder einem Topoisomerase-II-Inhibitor ausgewählt ist, wobei das Verfahren die Verfahrensschritte (a) von Anspruch 1 und die Verfahrensschritte (a) und (b) von Anspruch 2, umfasst, wobei

    (i) ein Posterior-Wahrscheinlichkeitswert von ≥ 0,5 und eine Ähnlichkeit zwischen den KZVs der Testprobe und den KZVs der Referenzprobe jeweils auf eine erhöhte Wahrscheinlichkeit hindeuten, dass das Individuum auf die Krebstherapie ansprechen wird, und/oder
    (ii) ein Posterior-Wahrscheinlichkeitswert von < 0,5 und eine Ähnlichkeit zwischen den KZVs der Testprobe und den KZVs der Kontrollprobe jeweils auf eine erhöhte Wahrscheinlichkeit hinweisen, dass das Individuum nicht auf die Krebstherapie ansprechen wird, und wahlweise
    (iii) Übermitteln des Ergebnisses an das Individuum oder einen Dritten, vorzugsweise an einen Arzt oder einen genetischen Berater.

5.  Verfahren nach einem der Ansprüche 1 bis 4, wobei die Krebstherapie Poly(ADP-Ribose)-Polymerase-Inhibitoren (PARPi) und/oder Platinverbindungen umfasst.

6.  Verfahren nach einem der Ansprüche 1 bis 5, wobei die Bestimmung der KZVs durch DNA-Sequenzierung erfolgt.

7.  Verfahren nach einem der Ansprüche 1 bis 6, wobei die Bestimmung der KZVs vor der Verabreichung der Krebstherapie durchgeführt wird.

8.  Verfahren nach einem der Ansprüche 1 bis 7, ferner umfassend das Empfehlen an ein Individuum,

    (a) bei dem eine HR-Defizienz und/oder eine erhöhte Wahrscheinlichkeit des Ansprechens auf die Krebstherapie festgestellt wurden, eines Behandlungsschemas, das einen PARP-Inhibitor, ein DNA-Doppelstrangbruch induzierendes Mittel, wobei es sich bei dem DNA-Doppelstrangbruch induzierenden Mittel bevorzugt um eine Platinverbindung oder ein strangvernetzendes Mittel handelt, ein Anthracyclin, einen Topoisomerase-I-Inhibitor oder einen Topoisomerase-II-Inhibitor umfasst,

(b) bei dem keine HR-Defizienz und/oder keine erhöhte Wahrscheinlichkeit des Ansprechens auf die Krebstherapie festgestellt wurden, eines Behandlungsschemas, das keinen PARP-Inhibitor, kein DNA-Doppelstrangbruch induzierendes Mittel, wobei es sich bei dem DNA-Doppelstrangbruch induzierenden Mittel bevorzugt um eine Platinverbindung oder ein strangvernetzendes Mittel handelt, kein Anthracyclin, keinen Topoisomerase-I-Inhibitor oder keinen Topoisomerase-II-Inhibitor umfasst.

9. PARP-Inhibitor eines DNA-Doppelstrangbruch induzierenden Mittels, wobei es sich bei dem DNA-Doppelstrangbruch induzierenden Mittel bevorzugt um eine Platinverbindung oder ein strangvernetzendes Mittel handelt, ein Anthracyclin, ein Topoisomerase-I-Inhibitor oder ein Topoisomerase-II-Inhibitor zur Verwendung beim Behandeln von Eierstockkrebs bei einem Individuum, wobei bei diesem Individuum nach dem Verfahren nach einem der Ansprüche 1 bis 3 und 5 bis 9 eine HR-Defizienz festgestellt wurde und/oder eine erhöhte Wahrscheinlichkeit des Ansprechens auf das Krebs-Behandlungsschema nach dem Verfahren nach einem der Ansprüche 4 bis 9 festgestellt wurde.

## Revendications

1. Méthode *in vitro* de détermination d'un statut de déficience en recombinaison homologue (RH) chez un sujet atteint d'un cancer de l'ovaire, dans laquelle la méthode comprend

   (a) la détermination de la variation du nombre de copies (VNC) d'un locus génomique dans un échantillon de test d'ADN de cellules cancéreuses provenant du sujet, le locus génomique étant sélectionné parmi un pool de locus génomiques comprenant ou constitué des locus indiqués dans le tableau 1 et/ou le tableau 2 ;
   dans laquelle la similarité entre les VNC du pool de locus génomiques dans l'échantillon de test d'ADN et les VNC du pool de locus génomiques d'un échantillon de référence de cellules cancéreuses mutées de type BRCA identifie le sujet comme étant déficient en RH ; et/ou la similarité entre les VNC du pool de locus génomiques dans l'ADN de l'échantillon de test et les VNC du pool de locus génomiques de l'échantillon de contrôle de cellules cancéreuses non mutées BRCA identifie le sujet comme n'étant pas déficient en RH, dans laquelle la similarité est la mesure de distance entre des centroïdes de deux classes, c'est-à-dire le contrôle et l'échantillon de référence, et le centroïde de l'échantillon de test, dans laquelle le centroïde d'une classe est construit par rétrécissement du centroïde de classe par une routine d'optimisation vers le centroïde global des deux classes après normalisation par l'écart type intra-classe pour chaque VNC ; et éventuellement
   (b) la transmission du résultat au sujet ou à un tiers, de préférence un médecin ou un généticien.

2. Méthode *in vitro* de détermination d'un statut de déficience en recombinaison homologue (RH) chez un sujet atteint d'un cancer de l'ovaire, dans laquelle le procédé comprend :

   (a) la détermination de la variation du nombre de copies (VNC) d'un locus génomique dans un échantillon de test d'ADN de cellules cancéreuses provenant du sujet, le locus étant sélectionné parmi un pool de locus génomiques comprenant ou constitué des locus indiqués dans le tableau 1 et/ou des locus indiqués dans le tableau 2,
   (b) la fourniture d'une valeur de centroïde rétréci qui est dérivée de la VNC du locus génomique ; et
   dans laquelle un score de probabilité a posteriori $\geq 0{,}5$ identifie le sujet comme déficient en RH ; et/ou dans laquelle un score de probabilité a posteriori $< 0{,}5$ identifie le sujet comme n'étant pas déficient en RH, dans laquelle le score de probabilité a posteriori peut être obtenu par comparaison des valeurs de centroïdes rétrécis dérivées des VNC du pool de locus génomiques dans l'ADN des cellules cancéreuses dans l'échantillon de test provenant du sujet avec les valeurs de centroïdes rétrécis dérivées des VNC d'un ensemble de référence d'échantillons de cellules cancéreuses mutées de type BRCA et d'échantillons de contrôle de cellules cancéreuses non mutées BRCA, et éventuellement
   (c) la transmission du résultat au sujet ou à un tiers, de préférence un médecin ou un généticien.

3. Méthode selon la revendication 1 ou 2, dans laquelle un score de probabilité a posteriori $\geq 0{,}5$ et une similarité entre les VNC de l'échantillon de test et les VNC de l'échantillon de référence, identifient respectivement le sujet comme étant un répondeur probable à une thérapie anticancéreuse ; ou dans laquelle un score de probabilité a posteriori $< 0{,}5$ et une similarité entre les VNC de l'échantillon de test et les VNC de l'échantillon de contrôle, identifient respectivement le sujet comme n'étant pas un répondeur probable à une thérapie anticancéreuse, moyennant quoi ladite thérapie anticancéreuse est sélectionnée parmi un inhibiteur de PARP, un agent induisant une cassure double brin de l'ADN, de préférence dans laquelle l'agent induisant une cassure double brin de l'ADN est un composé de platine ou un agent de réticulation inter-brins, une anthracycline, un inhibiteur de la topoisomérase I ou un inhibiteur de la topoisomérase II.

4. Méthode *in vitro* de prédiction de la réponse d'un sujet atteint d'un cancer de l'ovaire à une thérapie anticancéreuse, moyennant quoi ladite thérapie anticancéreuse est sélectionnée parmi un inhibiteur de PARP, un agent induisant une cassure double brin de l'ADN, de préférence dans laquelle l'agent induisant une cassure double brin de l'ADN est un composé de platine ou un agent de réticulation inter-brins, une anthracycline, un inhibiteur de la topoisomérase I ou un inhibiteur de la topoisomérase II,

dans laquelle la méthode comprend respectivement les étapes (a) de la méthode de la revendication 1 et les étapes (a) et (b) de la méthode de la revendication 2, dans laquelle

(i) un score de probabilité a posteriori ≥ 0,5 et une similarité entre les VNC de l'échantillon de test et les VNC de l'échantillon de référence, indiquent respectivement une probabilité accrue que le sujet réponde à la thérapie anticancéreuse, et/ou

(ii) un score de probabilité a posteriori < 0,5 et une similarité entre les VNC de l'échantillon de test et les VNC de l'échantillon de contrôle, indiquent respectivement une probabilité accrue que le sujet ne réponde pas à la thérapie anticancéreuse, et éventuellement

(iii) la transmission du résultat au sujet ou à un tiers, de préférence un médecin ou un généticien.

5. Méthode selon l'une quelconque des revendications 1 à 4, dans laquelle la thérapie anticancéreuse comprend des inhibiteurs de la poly(ADP-ribose) polymérase (PARPi) et/ou des composés de platine.

6. Méthode selon l'une quelconque des revendications 1 à 5, dans laquelle la détermination des VNC est effectuée par séquençage d'ADN.

7. Méthode selon l'une quelconque des revendications 1 à 6, dans laquelle la détermination des VNC est effectuée avant l'administration de la thérapie anticancéreuse.

8. Méthode selon l'une quelconque des revendications 1 à 7 comprenant en outre la recommandation à un sujet qui

(a) a été identifié comme étant déficient en RH et/ou ayant une probabilité accrue de répondre à la thérapie anticancéreuse, d'un schéma de traitement comprenant un inhibiteur de PARP, un agent induisant une cassure double brin de l'ADN, de préférence dans laquelle l'agent induisant une cassure double brin de l'ADN est un composé de platine ou un agent de réticulation inter-brins, une anthracycline, un inhibiteur de la topoisomérase I ou un inhibiteur de la topoisomérase II ; ou

(b) a été identifié comme n'étant pas déficient en RH et n'ayant pas une probabilité accrue de répondre à la thérapie anticancéreuse, d'un schéma de traitement ne comprenant pas un inhibiteur de PARP, un agent induisant une cassure double brin de l'ADN, de préférence dans laquelle l'agent induisant une cassure double brin de l'ADN est un composé de platine ou un agent de réticulation inter-brins, une anthracycline, un inhibiteur de la topoisomérase I ou un inhibiteur de la topoisomérase II.

9. Inhibiteur de PARP d'un agent induisant une cassure double brin de l'ADN, de préférence dans lequel l'agent induisant une cassure double brin de l'ADN est un composé de platine ou un agent de réticulation inter-brins, une anthracycline, un inhibiteur de la topoisomérase I ou un inhibiteur de la topoisomérase II, pour une utilisation dans le traitement d'un cancer de l'ovaire chez un sujet, dans lequel ledit sujet a été identifié comme déficient en RH selon la méthode selon l'une quelconque des revendications 1 à 3 et 5 à 9 et/ou identifié pour avoir une probabilité accrue de répondre au schéma de traitement anticancéreux selon le procédé selon l'une quelconque des revendications 4 à 9.

BRCA1m vs. control average profiles and shrunken centroids

A)

log ratio

Genomic Position

BRCA1-mutated
control
shrunken centroid
relative loss in gBRCA1m
relative gain in gBRCA1m

Fig. 1A

**B)** BRCA2m vs. control average profiles and shrunken centroids

BRCA2-mutated

control

shrunken centroid

relative loss in gBRCA2m

relative gain in gBRCA2m

**Fig. 1B**

**Fig. 2**

Fig. 3

Enrolment of 523 patients with EOC
(PR: n=281, RE: n=242)

NGS- and Methylation-
analysis

Excluded (n=50)
• missing samples/ DNA-isolation not feasible (n=27)
• failure of NGS in tumor tissue and/ or  methylation
  assay ( n=23)

NGS- (germline and tumor) and
methylation- data available (n=473)

Selection of samples due to g and
tumor mutation status.

BRCA-like
classification

lcWGS and BRCA-like classification of
tumor samples of 300 patients

# Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012038837 A2
- US 3892790 A
- US 3904663 A
- US 7759510 B
- US 7759488 B
- US 7754684 B
- US 3590028 A
- US 4138480 A
- US 4950738 A
- US 6087340 A
- US 7868040 B
- US 7485707 B
- US 5633016 A
- US 6403563 B
- US 5177075 A
- US 7915280 B
- US 7351701 B
- US 5295944 A

### Non-patent literature cited in the description

- **ZHANG et al.**, *Gynecologic Oncology,* 2016, vol. 141, 57-64
- **DAVIES et al.** *Nature Medicine,* 2017, vol. 23, 517-525
- **O'BRIEN et al.**, Converting cancer mutations into therapeutic opportunities. *EMBO MOL. MED.,* 2009, vol. 1, 297-299
- **FITZMAURICE C ; ABATE D ; ABBASI N ; AB-BASTABAR H ; ABD-ALLAH F ; ABDEL-RAHMAN O et al.** Global, Regional, and National Cancer Incidence, Mortality, Years of Life Lost, Years Lived With Disability, and Disability-Adjusted Life-Years for 29 Cancer Groups, 1990 to 2017: A Systematic Analysis for the Global Burden of Disease Study. *JAMA Oncol,* 2019, vol. 5 (12), 1749-68
- National Cancer Institue. *Cancer Stat Facts: Ovarian Cancer,* 30 September 2020, <https://seer.cancer.gov/statfacts/html/ovary.html>
- **DU BOIS A ; REUSS A ; PUJADE-LAURAINE E ; HARTER P ; RAY-COQUARD I ; PFISTERER J.** Role of surgical outcome as prognostic factor in advanced epithelial ovarian cancer: a combined exploratory analysis of 3 prospectively randomized phase 3 multicenter trials: by the Arbeitsgemeinschaft Gynaekologische Onkologie Studiengruppe Ovarialkarzinom (AGO-OVAR) and the Groupe d'Investigateurs Nationaux Pour les Etudes des Cancers de l'Ovaire (GINECO). *Cancer,* 2009, vol. 115 (6), 1234-44
- **PIVER MS.** Treatment of ovarian cancer at the crossroads: 50 years after single-agent melphalan chemotherapy. *Oncology (Williston Park),* 2006, vol. 20 (10), 1156, , 58
- **LEDERMANN JA ; RAJA FA ; FOTOPOULOU C ; GONZALEZ-MARTIN A ; COLOMBO N ; SESSA C et al.** Newly diagnosed and relapsed epithelial ovarian carcinoma: ESMO Clinical Practice Guidelines for diagnosis, treatment and follow-up. *Ann Oncol,* 2013, vol. 24, 24-32
- **LEDERMANN JA ; DREW Y ; KRISTELEIT RS.** Homologous recombination deficiency and ovarian cancer. *European journal of cancer,* 1990, vol. 60, 49-58
- **MOYNAHAN ME ; CHIU JW ; KOLLER BH ; JASIN M.** Brca1 controls homology-directed DNA repair. *Mol Cell,* 1999, vol. 4 (4), 511-8
- **MOYNAHAN ME ; PIERCE AJ ; JASIN M.** BRCA2 is required for homology-directed repair of chromosomal breaks. *Mol Cell,* 2001, vol. 7 (2), 263-72
- **MOORE K ; COLOMBO N ; SCAMBIA G ; KIM BG ; OAKNIN A ; FRIEDLANDER M et al.** Maintenance Olaparib in Patients with Newly Diagnosed Advanced Ovarian Cancer. *N Engl J Med,* 2018, vol. 379 (26), 2495-505
- **ROBSON M ; IM SA ; SENKUS E ; XU B ; DOMCHEK SM ; MASUDA N et al.** Olaparib for Metastatic Breast Cancer in Patients with a Germline BRCA Mutation. *N Engl J Med,* 2017, vol. 377 (6), 523-33
- **TURNER N ; TUTT A ; ASHWORTH A.** Hallmarks of 'BRCAness' in sporadic cancers. *Nat Rev Cancer,* 2004, vol. 4 (10), 814-9
- **LORD CJ ; ASHWORTH A.** BRCAness revisited. *Nat Rev Cancer,* 2016, vol. 16 (2), 110-20

- **LEDERMANN J ; HARTER P ; GOURLEY C ; FRIEDLANDER M ; VERGOTE I ; RUSTIN G et al.** Olaparib maintenance therapy in patients with platinum-sensitive relapsed serous ovarian cancer: a preplanned retrospective analysis of outcomes by BRCA status in a randomised phase 2 trial. *Lancet Oncol,* 2014, vol. 15 (8), 852-61
- **MIRZA MR ; MONK BJ ; HERRSTEDT J ; OZA AM ; MAHNER S ; REDONDO A et al.** Niraparib Maintenance Therapy in Platinum-Sensitive, Recurrent Ovarian Cancer. *N Engl J Med,* 2016, vol. 375 (22), 2154-64
- **SWISHER EM ; LIN KK ; OZA AM ; SCOTT CL ; GIORDANO H ; SUN J et al.** Rucaparib in relapsed, platinum-sensitive high-grade ovarian carcinoma (ARIEL2 Part 1): an international, multicentre, open-label, phase 2 trial. *Lancet Oncol,* 2017, vol. 18 (1), 75-87
- **COLEMAN RL ; OZA AM ; LORUSSO D ; AGHAJA-NIAN C ; OAKNIN A ; DEAN A et al.** Rucaparib maintenance treatment for recurrent ovarian carcinoma after response to platinum therapy (ARIEL3): a randomised, double-blind, placebo-controlled, phase 3 trial. *Lancet,* 2017, vol. 390 (10106), 1949-61
- **ABKEVICH V ; TIMMS KM ; HENNESSY BT ; POTTER J ; CAREY MS ; MEYER LA et al.** Patterns of genomic loss of heterozygosity predict homologous recombination repair defects in epithelial ovarian cancer. *Br J Cancer,* 2012, vol. 107 (10), 1776-82
- **DAVIES H ; GLODZIK D ; MORGANELLA S ; YATES LR ; STAAF J ; ZOU X et al.** HRDetect is a predictor of BRCA1 and BRCA2 deficiency based on mutational signatures. *Nat Med,* 2017, vol. 23 (4), 517-25
- **BIRKBAK NJ ; WANG ZC ; KIM JY ; EKLUND AC ; LI Q ; TIAN R et al.** Telomeric allelic imbalance indicates defective DNA repair and sensitivity to DNA-damaging agents. *Cancer Discov,* 2012, vol. 2 (4), 366-75
- **POPOVA T ; MANIE E ; RIEUNIER G ; CAUX-MON-COUTIER V ; TIRAPO C ; DUBOIS T et al.** Ploidy and large-scale genomic instability consistently identify basal-like breast carcinomas with BRCA1/2 inactivation. *Cancer Res,* 2012, vol. 72 (21), 5454-62
- **KONSTANTINOPOULOS PA ; SPENTZOS D ; KARLAN BY ; TANIGUCHI T ; FOUNTZILAS E ; FRANCOEUR N et al.** Gene expression profile of BRCAness that correlates with responsiveness to chemotherapy and with outcome in patients with epithelial ovarian cancer. *J Clin Oncol,* 2010, vol. 28 (22), 3555-61
- **TIMMS KM ; ABKEVICH V ; HUGHES E ; NEFF C ; REID J ; MORRIS B et al.** Association of BRCA1/2 defects with genomic scores predictive of DNA damage repair deficiency among breast cancer subtypes. *Breast Cancer Res,* 2014, vol. 16 (6), 475
- **MOORE KN ; SECORD AA ; GELLER MA ; MILLER DS ; CLOVEN N ; FLEMING GF et al.** Niraparib monotherapy for late-line treatment of ovarian cancer (QUADRA): a multicentre, open-label, single-arm, phase 2 trial. *Lancet Oncol,* 2019, vol. 20 (5), 636-48
- **COLEMAN RL ; FLEMING GF ; BRADY MF ; SWISHER EM ; STEFFENSEN KD ; FRIEDLANDER M et al.** Veliparib with First-Line Chemotherapy and as Maintenance Therapy in Ovarian Cancer. *N Engl J Med,* 2019, vol. 381 (25), 2403-15
- **GONZALEZ-MARTIN A ; POTHURI B ; VERGOTE I ; DEPONT CHRISTENSEN R ; GRAYBILL W ; MIRZA MR et al.** Niraparib in Patients with Newly Diagnosed Advanced Ovarian Cancer. *N Engl J Med,* 2019, vol. 381 (25), 2391-402
- **RAY-COQUARD I ; PAUTIER P ; PIGNATA S ; PEROL D ; GONZALEZ-MARTIN A ; BERGER R et al.** Olaparib plus Bevacizumab as First-Line Maintenance in Ovarian Cancer. *N Engl J Med,* 2019, vol. 381 (25), 2416-28
- *European Medicines Agency,* 30 September 2020, <https://www.ema.europa.eu/en/medicines/human/summaries-opinion/lynparza-0>.
- **JOOSSE SA ; VAN BEERS EH ; TIELEN IH ; HORLINGS H ; PETERSE JL ; HOOGERBRUGGE N et al.** Prediction of BRCAI-association in hereditary non-BRCA1/2 breast carcinomas with array-CGH. *Breast Cancer Res Treat,* 2009, vol. 116 (3), 479-89
- **TIBSHIRANI R ; HASTIE T ; NARASIMHAN B ; CHU G.** Diagnosis of multiple cancer types by shrunken centroids of gene expression. *Proc Natl Acad Sci U S A,* 2002, vol. 99 (10), 6567-72
- **VOLLEBERGH MA ; LIPS EH ; NEDERLOF PM ; WESSELS LF ; SCHMIDT MK ; VAN BEERS EH et al.** An aCGH classifier derived from BRCAI-mutated breast cancer and benefit of high-dose platinum-based chemotherapy in HER2-negative breast cancer patients. *Ann Oncol,* 2011, vol. 22 (7), 1561-70
- **VOLLEBERGH MA ; LIPS EH ; NEDERLOF PM ; WESSELS LF ; WESSELING J ; VD VIJVER MJ et al.** Genomic patterns resembling BRCA1- and BRCA2-mutated breast cancers predict benefit of intensified carboplatin-based chemotherapy. *Breast Cancer Res,* 2014, vol. 16 (3), R47
- **SCHOUTEN PC ; MARME F ; AULMANN S ; SINN HP ; VAN ESSEN HF ; YLSTRA B et al.** Breast cancers with a BRCAI-like DNA copy number profile recur less often than expected after high-dose alkylating chemotherapy. *Clin Cancer Res,* 2015, vol. 21 (4), 763-70

- **SCHOUTEN PC ; GLUZ O ; HARBECK N ; MOHR-MANN S ; DIALLO-DANEBROCK R ; PELZ E et al.** BRCAl-like profile predicts benefit of tandem high dose epirubicin-cyclophospamide-thiotepa in high risk breast cancer patients randomized in the WSG-AM01 trial. *Int J Cancer,* 2016, vol. 139 (4), 882-9
- **VLIEK S ; JAGER A ; JONGE-LAVRENCIC M ; LOTZ J-P ; GONCALVES A ; GRAESER M et al.** Abstract OT2-07-08: Substantially improving the cure rate of high-risk BRCA1-like breast cancer patients with personalized therapy (SUBITO) - an international randomized phase III trial. *Cancer Research,* 2018, 78
- **SEVERSON TM ; WOLF DM ; YAU C ; PEETERS J ; WEHKAM D ; SCHOUTEN PC et al.** The BRCAlness signature is associated significantly with response to PARP inhibitor treatment versus control in the I-SPY 2 randomized neoadjuvant setting. *Breast Cancer Res,* 2017, vol. 19 (1), 99
- **HARTER P ; HAUKE J ; HEITZ F ; REUSS A ; KOMMOSS S ; MARME F et al.** Prevalence of deleterious germline variants in risk genes including BRCA1/2 in consecutive ovarian cancer patients (AGO-TR-1). *PLoS One,* 2017, vol. 12 (10), e0186043
- **HAUKE J ; HAHNEN E ; SCHNEIDER S ; REUSS A ; RICHTERS L ; KOMMOSS S et al.** Deleterious somatic variants in 473 consecutive individuals with ovarian cancer: results of the observational AGO-TR1 study (NCT02222883). *J Med Genet,* 2019, vol. 56 (9), 574-80
- Federation of Dutch Medical Scientific Societies. 2011. *Human Tissue and Medical Research: Code of Conduct for responsible use,* 2011, <https://www.federa.org/sites/default/files/images/print_version_code_of_conduct_en glish.pdf>.
- **SCHOUTEN PC ; GRIGORIADIS A ; KUILMAN T ; MIRZA H ; WATKINS JA ; COOKE SA et al.** Robust BRCAl-like classification of copy number profiles of samples repeated across different datasets and platforms. *Mol Oncol,* 2015, vol. 9 (7), 1274-86
- **SCHOUTEN PC ; VAN DYK E ; BRAAF LM ; MULDER L ; LIPS EH ; DE RONDE JJ et al.** Platform comparisons for identification of breast cancers with a BRCA-like copy number profile. *Breast Cancer Res Treat,* 2013, vol. 139 (2), 317-27

- **HASTIE T ; TIBSHIRANI R ; FRIEDMAN J.** The Elements of Statistical Learning. Springer, 2009
- **BELL D ; BERCHUCK A ; BIRRER M ; CHIEN J ; CRAMER DW ; DAO F et al.** Integrated genomic analyses of ovarian carcinoma. *Nature,* 2011, vol. 474 (7353), 609-15
- **DOMANSKA K ; MALANDER S ; STAAF J ; KARLSSON A ; BORG A ; JONSSON G et al.** Genetic profiles distinguish different types of hereditary ovarian cancer. *Oncol Rep,* 2010, vol. 24 (4), 885-95
- **KAMIENIAK MM ; MUNOZ-REPETO I ; RICO D ; OSORIO A ; URIOSTE M ; GARCIA-DONAS J et al.** DNA copy number profiling reveals extensive genomic loss in hereditary BRCA1 and BRCA2 ovarian carcinomas. *Br J Cancer,* 2013, vol. 108 (8), 1732-42
- **MAXWELL KN ; WUBBENHORST B ; WENZ BM ; DE SLOOVER D ; PLUTA J ; EMERY L et al.** BRCA locus-specific loss of heterozygosity in germline BRCA1 and BRCA2 carriers. *Nature Communications,* 2017, vol. 8 (1), 319
- **MACINTYRE G ; GORANOVA TE ; DE SILVA D ; ENNIS D ; PISKORZ AM ; ELDRIDGE M et al.** Copy number signatures and mutational processes in ovarian carcinoma. *Nature genetics,* 2018, vol. 50 (9), 1262-70
- **PRAHALLAD A ; SUN C ; HUANG S ; DI NICOLANTONIO F ; SALAZAR R ; ZECCHIN D et al.** Unresponsiveness of colon cancer to BRAF(V600E) inhibition through feedback activation of EGFR. *Nature,* 2012, vol. 483 (7387), 100-3
- **ALEXANDROV LB ; KIM J ; HARADHVALA NJ ; HUANG MN ; TIAN NG AW ; WU Y et al.** The repertoire of mutational signatures in human cancer. *Nature,* 2020, vol. 578 (7793), 94-101
- **WATKINS JA ; IRSHAD S ; GRIGORIADIS A ; TUTT AN.** Genomic scars as biomarkers of homologous recombination deficiency and drug response in breast and ovarian cancers. *Breast Cancer Res,* 2014, vol. 16 (3), 211
- **CASTROVIEJO-BERMEJO M ; CRUZ C ; LLOP-GUEVARA A ; GUTIÉRREZ-ENRÍQUEZ S ; DUCY M ; IBRAHIM YH et al.** A RAD51 assay feasible in routine tumor samples calls PARP inhibitor response beyond BRCA mutation. *EMBO molecular medicine,* 2018, vol. 10 (12